# EUROPEAN PATENT SPECIFICATION

(11) **EP 4 346 630 B1**
(45) Date of publication and mention of the grant of the patent: **08.04.2026**
(21) Application number: 22738778.4
(22) Date of filing: 31.05.2022
(51) Int. Cl.: A61B 17/04, A61F 2/24

(54) **LINE TRIMMING TOOL FOR HEART WALL REMODELING DEVICES**
LINIENSCHNEIDEWERKZEUG FÜR VORRICHTUNGEN ZUR HERZWANDREMODELLIERUNG
OUTIL DE COUPE DE LIGNE POUR DISPOSITIFS DE REMODELAGE DE LA PAROI CARDIAQUE

(30) Priority: 04.06.2021 US 202163197296 P
(43) Date of publication of application: 10.04.2024
(73) Proprietor: Edwards Lifesciences Corporation, Irvine, CA 92614 (US)
(72) Inventor: ELSHEIKH, Alzuebeir, Abdelbagi, Irvine, CA 92614 (US); KWON, Yoon, Hee, Mission Viejo, CA 92691 (US); TRAN, Bryant, Quoc, Irvine, CA 92614 (US); CATANIA, Pablo, Hernan, Irvine, CA 92614 (US); WITTEL, James, G., Irvine, CA 92617 (US)
(74) Representative: Eisenführ Speiser
(86) International application number: PCT/US2022/031569
(87) International publication number: WO 2022/256309

(56) References cited:
- US-A1- 2006 161 183
- US-A1- 2019 240 023
- US-B2- 7 785 348

## Description

### RELATED APPLICATIONS

The present application claims the benefit of US Provisional Patent Application No. 63/197,296, filed June 4, 2021.

### BACKGROUND

Ischemic heart failure and systolic heart failure are conditions whereby the left ventricle becomes enlarged and dilated. With ischemic heart failure, a cardiac infarction occurs and the left ventricle remodels over a period of time, such as over days or months. With systolic heart failure, the left ventricle undergoes dilation for some other reason. For example, initial causes of heart systolic heart failure include chronic hypertension, mitral valve incompetency, and other dilated cardiomyopathies. A dilated heart, and particularly a dilated left ventricle, can significantly increase the tension and/or stress in the heart wall both during diastolic filling and systolic contraction, which contributes to ongoing dilatation of the left ventricular chamber.

Mitral valve incompetency or mitral valve regurgitation often accompanies ischemic and systolic heart failure. As the dilation of the ventricle proceeds, valve function may worsen. For example, as the dilation of the left ventricle progresses, the papillary muscles (to which the leaflets are connected via the chordae tendinea) may move radially outward and downward relative to the mitral valve, and relative to their normal positions. During this movement of the papillary muscles, however, the various chordae lengths remain substantially constant. This compromises the full closure ability of the leaflets by exerting tension prematurely on the leaflets. In addition, the enlargement of the left ventricle can cause the size of the mitral valve annulus to increase, while the area of the leaflets of the valve remains constant. This may lead to an area of less coaptation of the valve leaflets. Moreover, in normal hearts, the size of the mitral valve contracts during systole, aiding in valve coaptation. Right ventricular enlargement reduces annular contraction and distorts annular size, often exacerbating mitral valve regurgitation. The combination of the changes to the mitral valve annulus and the movement of the papillary muscles can result in a regurgitant mitral valve. This increase in regurgitation can, in turn, increase ventricular wall stress thereby advancing the dilation process, which can even further worsen mitral valve dysfunction.

US 7,785,348 B2 discloses a suture lock and cut assembly that forms a hollow body that is slidably connected upon a stem through which one or more sutures is threaded. Depending on the slidable position of the body upon the stem, a locking arm is first engaged to clamp the suture permanently within the stem, and a cutting arm is engaged next to cut any surplus suture, which is then removed from the patient.

US 2019/0240023 A1 discloses a system for introducing an element into tissue proximate (i.e., either at or close to) the mitral valve annulus of the heart of a patient. The element comprises a guide wire. The system includes a first catheter device having a first distal end portion capable of being introduced through the vascular system of the patient and into the coronary sinus proximate the mitral valve annulus. The first catheter device includes first, second and third spaced apart radiopaque markers at the first distal end portion. The system further includes a second catheter device having a second distal end portion capable of being introduced through the vascular system of the patient and into the left ventricle of the heart proximate the mitral valve annulus. The second catheter device includes a fourth radiopaque marker at the second distal end portion and a lumen for delivering an element from the second distal end portion.

US 2006/0161183 A1 discloses an instrument for remote placement of knots in a loop of suture having first and second ends extending through tissue and trimming of suture away from said knots. The instrument comprises a shaft having a distal end, a distal tip at said distal end having means for facilitating the formation of one or more knots in the suture extending from the tissue, in which said distal tip of said shaft when moved forward pushes said formed knots for placement to the tissue, two openings at said distal end of said shaft through which is receivable two ends of the suture extending from said placed knots and means for cutting positionable to cut said suture extending through said openings.

### SUMMARY

The presently claimed invention provides a trimming tool for trimming a line within a patient according to claim 1. Further developments of the herein claimed invention are described in the dependent claims. This summary is meant to provide some examples and is not intended to be limiting of the scope of the invention in any way. For example, any feature included in an example of this summary is not required by the claims, unless the claims explicitly recite the features. Also, the features, components, steps, concepts, etc. described in examples in this summary and elsewhere in this disclosure can be combined in a variety of ways. Various features and steps as described elsewhere in this disclosure may be included in the examples summarized here.

An implantable device or implant (e.g., implantable device, etc.) is configured to be positioned within a native heart valve to remodel one or more walls of a heart to allow the heart to work more effectively.

In one exemplary embodiment, a trimming tool for trimming a line within a patient includes an actuator, an outer shaft, a movable inner shaft, a compressible member, and a cutter or blade. The actuator is manipulatable by a user outside the patient. The moveable inner shaft is coupled to the actuator. The compressible member can be compressed from an expanded length to a compressed length by the actuator. The cutter has a length between the expanded length and the compressed length of the compressible member. The outer sleeve is coupled to the inner shaft, such that the inner shaft is movable with respect to the outer shaft. A backstop is disposed along an inner wall of the outer sleeve. The outer sleeve includes a cutting channel. The trimming tool is configured to trim a line received in the cutting channel of the outer sleeve.

In one exemplary embodiment, a handle for manipulating a trimming tool includes an outer frame and a two-stage trigger. A cavity extends partially into the outer frame. The cavity houses a first spring and the two-stage trigger. The trigger is coupled with the outer housing and moveable withing the cavity. The two-stage trigger includes an outer housing, an inner member, and a spring coupled with the outer housing and the inner member.

In one exemplary embodiment, an assembly includes a trimming tool and a two-stage trimming tool control handle. The trimming tool includes an actuator, an outer shaft, a movable inner shaft, a compressible member, and a cutter or blade. The actuator is manipulatable via the two-stage trimming control handle outside the patient. The moveable inner shaft is coupled to the actuator. The compressible member can be compressed from an expanded length to a compressed length by the two-stage trigger. The cutter has a length between the expanded length and the compressed length of the compressible member. The outer sleeve is coupled to the inner shaft, such that the inner shaft is movable with respect to the outer shaft. A backstop is disposed along an inner wall of the outer sleeve. The outer sleeve includes a cutting channel. The trimming tool is configured to trim a line received in the cutting channel of the outer sleeve. The handle includes an outer frame and a two-stage trigger. A cavity extends partially into the outer frame. The cavity houses a first spring and the two-stage trigger. The trigger is coupled with the outer housing and moveable withing the cavity. The two-stage trigger includes an outer housing, an inner member, and a spring coupled with the outer housing and the inner member.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures can be drawn to scale for some embodiments, the figures are not necessarily drawn to scale for all embodiments. Embodiments and other features and advantages of the present disclosure will be described and explained with additional specificity and detail through the use of the accompanying drawings in which:
Figure 1 illustrates a cutaway view of the human heart in a diastolic phase;
Figure 2 illustrates a cutaway view of the human heart in a systolic phase;
Figure 3 illustrates a cutaway view of the human heart in a diastolic phase, in which the chordae tendineae are shown attaching the leaflets of the mitral and tricuspid valves to ventricle walls;
Figure 4 illustrates a healthy mitral valve with the leaflets closed as viewed from an atrial side of the mitral valve;
Figure 5 illustrates a dysfunctional mitral valve with a visible gap between the leaflets as viewed from an atrial side of the mitral valve;
Figure 6 illustrates a cutaway view of the human heart showing the papillary muscles;
Figure 7 illustrates a cutaway view of the human heart showing the multilayer heart wall;
Figure 8 is an enlarged cutaway view of the human heart wall;
Figure 9 is an enlarged cutaway view of the human heart wall of Figure 8 showing a needle about to pierce the heart wall;
Figure 10 is an enlarged cutaway view of the human heart wall of Figure 8 showing a needle inserted into the myocardium of the heart wall;
Figure 11 is an enlarged cutaway view of the human heart wall of Figure 8 showing a needle inserted into the parietal tissue of the pericardium of the heart wall;
Figure 12 is an enlarged cutaway view of the human heart wall of Figure 8 showing a needle inserted into the pericardial cavity of the heart wall;
Figure 13 is an enlarged cutaway view of the human heart wall of Figure 8 showing a needle injecting a dye into the pericardial cavity of the heart wall;
Figure 14 is an enlarged cutaway view of the human heart wall of Figure 8 showing a needle inserted into the pericardial cavity of the heart wall and a catheter adjacent the endocardium of the heart wall;
Figure 15 is an enlarged cutaway view of the human heart wall of Figure 8 showing a needle and a catheter inserted into the pericardial cavity of the heart wall;
Figure 16 is an enlarged cutaway view of the human heart wall of Figure 8 showing an anchor deployed into the pericardial cavity of the heart wall;
Figure 16A is a view similar to the view of Figure 16 where the anchor is deployed through an introducer or needle;
Figures 16B and 16C are views similar to Figure 16 where the anchor is deployed over an introducer or needle;
Figure 17 is an enlarged cutaway view of the human heart wall of Figure 8 showing the anchor of Figure 16 seated in the pericardial cavity of the heart wall;
Figure 18 is an enlarged cutaway view of the human heart wall of Figure 8 showing a screw catheter adjacent the endocardium of the heart wall;
Figure 19 is an enlarged cutaway view of the human heart wall of Figure 8 showing a screw catheter anchored into the myocardium of the heart wall;
Figure 20 is an enlarged cutaway view of the human heart wall of Figure 19 showing a needle delivered through the screw catheter;
Figure 21 is an enlarged cutaway view of the human heart wall of Figure 19 showing a needle inserted into the myocardium of the heart wall;
Figure 22 is an enlarged cutaway view of the human heart wall of Figure 19 showing a needle inserted into the parietal tissue of the heart wall;
Figure 23 is an enlarged cutaway view of the human heart wall of Figure 19 showing a needle inserted into the pericardial cavity of the heart wall;
Figure 24 is an enlarged cutaway view of the human heart wall of Figure 19 showing a needle injecting a dye into the pericardial cavity of the heart wall;
Figure 25 is an enlarged cutaway view of the human heart wall of Figure 19 showing a needle inserted into the pericardial cavity of the heart wall and a secondary catheter adjacent the endocardium of the heart wall;
Figure 26 is an enlarged cutaway view of the human heart wall of Figure 19 a needle and a secondary catheter inserted into the pericardial cavity of the heart wall;
Figure 26A is an enlarged cutaway view of the human heart wall showing a needle and a secondary catheter inserted into the pericardial cavity of the heart wall;
Figure 27 is an enlarged cutaway view of the human heart wall of Figure 19 showing an anchor deployed into the pericardial cavity of the heart wall;
Figure 27A is an enlarged cutaway view of the human heart wall showing an anchor deployed into the pericardial cavity of the heart wall;
Figure 28 is an enlarged cutaway view of the human heart wall of Figure 19 showing the anchor of Figure 26 seated in the pericardial cavity of the heart wall;
Figure 28A is an enlarged cutaway view of the human heart wall showing an anchor seated in the pericardial cavity of the heart wall
Figure 29 is an enlarged cutaway view of a human heart wall showing a needle about to pierce the heart wall;
Figure 30 is an enlarged cutaway view of the human heart wall of Figure 29 showing the needle extending through the heart wall;
Figure 31 is an enlarged cutaway view of the human heart wall of Figure 29 showing the needle extending through the heart wall and a catheter adjacent the endocardium of the heart wall;
Figure 32 is an enlarged cutaway view of the human heart wall of Figure 29 showing the needle and a catheter extending through the heart wall;
Figure 33 is an enlarged cutaway view of the human heart wall of Figure 32 showing an anchor deployed through the catheter;
Figure 34 is an enlarged cutaway view of the human heart wall of Figure 29 showing the anchor seated against the parietal tissue of the heart wall;
Figure 35 is an enlarged cutaway view of the human heart wall showing a screw catheter anchored into the myocardium of the heart wall;
Figure 36 is an enlarged cutaway view of the human heart wall of Figure 35 showing a needle delivered through the screw catheter;
Figure 37 is an enlarged cutaway view of the human heart wall of Figure 35 showing a needle inserted through the heart wall;
Figure 38 is an enlarged cutaway view of the human heart wall of Figure 35 showing a needle inserted through the heart wall and a secondary catheter adjacent the endocardium of the heart wall;
Figure 39 is an enlarged cutaway view of the human heart wall of Figure 35 showing a needle and a secondary catheter inserted through the heart wall;
Figure 40 is an enlarged cutaway view of the human heart wall of Figure 35 showing an anchor deployed through the secondary catheter;
Figure 41 is an enlarged cutaway view of the human heart wall of Figure 35 showing the anchor of Figure 40 seated against the parietal tissue of the heart wall;
Figure 42 illustrates a cutaway view of the human heart showing a needle inserted through a papillary muscle of the heart and through the heart wall;
Figure 43 illustrates a cutaway view of the human heart of Figure 42 showing the needle inserted through the papillary muscle and a delivery catheter positioned adjacent the papillary muscle;
Figure 44 is an enlarged cutaway view of the human heart wall showing a needle and a catheter inserted through a papillary muscle of the heart and into the pericardial cavity of the heart wall;
Figure 45 is an enlarged cutaway view of the human heart wall of Figure 44 showing an anchor deployed into the pericardial cavity of the heart wall;
Figure 46 is an enlarged cutaway view of the human heart wall of Figure 44 showing an anchor seated in the pericardial cavity of the heart wall;
Figure 47 illustrates a cutaway view of the human heart showing an anchor seated against the exterior of the heart wall with a line extending through a papillary muscle;
Figure 48 illustrates a cutaway view of the human heart showing a first anchor seated against the exterior of the heart wall with a line extending through a first papillary muscle and a second anchor seated against the exterior of the heart wall with a second line extending through a second papillary muscle;
Figure 49A illustrates the cutaway view of the human heart of Figure 48 showing the first line and a second line being pulled through a connector to pull the papillary muscles toward one another;
Figure 49B shows the first and second lines secured in the connector and trimmed;
Figure 50 illustrates the cutaway view of the human heart of Figure 49B showing a third anchor seated against the intraventricular septum and a third line coupled to the first and second lines;
Figure 51 illustrates a cutaway view of the human heart showing a first anchor seated against the exterior of the heart wall with a first line extending through a papillary muscle and a second anchor seated against the intraventricular septum and a second line connected to the first line;
Figure 52 illustrates a cutaway view of the human heart showing a first anchor seated against the exterior of the heart wall with a first line extending through the heart wall and a second anchor seated against the intraventricular septum and a second line connected to the first line;
Figures 53 and 54 are exploded views of a line clamp according to an exemplary embodiment;
Figures 53A and 54A are exploded views of a line clamp according to an exemplary embodiment;
Figures 55 and 56 are cross-section views of a line clamp according to an exemplary embodiment;
Figures 55A and 56A are cross-section views of a line clamp according to an exemplary embodiment;
Figures 57A-57E are illustrations of anchors, lines, and a line clamp located between tissue walls according to an exemplary embodiment;
Figures 57F-57J are illustrations of anchors, lines, and a line clamp located between tissue walls according to an exemplary embodiment;
Figure 58 is a perspective view of the operating end of a line clamp installation tool according to an exemplary embodiment;
Figure 58A is a perspective view of the operating end of a line clamp installation tool according to an exemplary embodiment;
Figure 59 is a cross-section view of the operating end of a line clamp installation tool according to an exemplary embodiment;
Figure 59A is a cross-section view of the operating end of a line clamp installation tool according to an exemplary embodiment;
Figure 60 is a perspective view of a line clamp according to an exemplary embodiment;
Figure 60A is a perspective view of a line clamp according to an exemplary embodiment;
Figure 61 is a perspective view of an engagement device according to an exemplary embodiment;
Figure 61A is a perspective view of an engagement device according to an exemplary embodiment;
Figure 62 is a cross-section view of an engagement device secured to a component of a line clamp according to an exemplary embodiment;
Figure 62A illustrates a cross-section view of an engagement device secured to a component of a line clamp according to an exemplary embodiment;
Figure 63 is an exploded view of Figure 62;
Figure 63A is an exploded view of the components illustrated by Figure 62A;
Figures 63B-63G are schematic illustrations of an engagement device being used to place an insert in a body of a locking device according to an exemplary embodiment;
Figure 64 illustrates a socket positioned adjacent to the operating end of a line clamp installation tool according to an exemplary embodiment;
Figure 64A illustrates a socket positioned adjacent to the operating end of a line clamp installation tool according to an exemplary embodiment;
Figure 65 is a perspective cross-section view of a line clamp installation tool according to an exemplary embodiment;
Figure 66 is a perspective cross-section view of the operating handle portion of a line clamp installation tool according to an exemplary embodiment;
Figure 67 is a cross-section view of a portion of the operating handle portion of a line clamp installation tool according to an exemplary embodiment;
Figure 68 is a cross-section illustration of the operating end and operating handle portion of a line clamp installation tool according to an exemplary embodiment;
Figure 69 is an enlarged perspective cross-section view of the operating handle portion of a line clamp installation tool according to an exemplary embodiment;
Figures 70A-70G are illustrations of the line clamp being installed by a line clamp installation tool according to an exemplary embodiment;
Figures 71A-71E are illustrations of a line trimming tool in use according to an exemplary embodiment;
Figures 72A-72B illustrate an alternative exemplary embodiment of a line trimming tool according to an exemplary embodiment;
Figures 73A-73B are views of the components of the line trimming tool of Figures 72A-72B;
Figures 74A-74C are illustrations of a line clamp according to an exemplary embodiment;
Figures 75A-75B are illustrations of a line clamp according to an exemplary embodiment;
Figures 76A-76B are illustrations of a line clamp according to an exemplary embodiment;
Figures 77A-77B are illustrations of a line clamp according to an exemplary embodiment;
Figures 78A-78B are illustrations of a line clamp according to an exemplary embodiment;
Figures 79A-79B are illustrations of a line clamp according to an exemplary embodiment;
Figures 80A-80B are illustrations of a line clamp according to an exemplary embodiment;
Figures 81A-81B are illustrations of a line clamp according to an exemplary embodiment;
Figure 82 is an illustration of a line clamp according to an exemplary embodiment;
Figure 83 is an illustration of a line clamp according to an exemplary embodiment;
Figure 84 is an illustration of a line clamp according to an exemplary embodiment;
Figures 85A-85B are illustrations of a line clamp according to an exemplary embodiment;
Figure 86 is an illustration of a line clamp according to an exemplary embodiment;
Figures 87A-87B are illustrations of a line clamp according to an exemplary embodiment;
Figures 88A-88B are illustrations of a line clamp according to an exemplary embodiment;
Figures 89A-89B are illustrations of a line clamp according to an exemplary embodiment;
Figures 90A-90B are illustrations of a line clamp according to an exemplary embodiment;
Figures 91A-91B are illustrations of a line clamp according to an exemplary embodiment;
Figure 92 is an illustration of a line clamp according to an exemplary embodiment;
Figures 93A-93B are illustrations of a line clamp according to an exemplary embodiment.
Figure 94 illustrates an exemplary embodiment of an anchor for a papillary muscle approximation system, the anchor illustrated in an extended configuration;
Figure 95 illustrates the anchor of Figure 94 in a non-deployed, extended configuration;
Figure 95A illustrates another exemplary embodiment of an anchor in a substantially non-deployed configuration;
Figure 95B is a plan view of a piece of material that has been cut in a pattern for use in a connector of the anchor illustrated by Figure 95A;
Figure 95C is a side view of the material illustrated by Figure 95B;
Figure 95D is a plan view of a connector made by folding the material illustrated by Figure 95B;
Figure 95E is a side view of the connector illustrated by Figure 95D;
Figure 95F is a plan view of a portion of the connector illustrated by Figure 95D attached to a portion of an anchor;
Figure 95G is a side view of the connector and anchor illustrated by Figure 95F;
Figure 95H is a plan view of another exemplary embodiment of a piece of material that has been cut in a pattern for use in a connector of the anchor illustrated by Figure 95A;
Figure 95I is illustrates an exemplary embodiment of an anchor that is similar to the embodiment illustrated by Figure 95A where high strength fibers are oriented to increase pull strength;
Figure 96 illustrates the anchor of Figure 94 in a deployed configuration;
Figure 96A illustrates the anchor of Figure 95A in a deployed configuration;
Figures 96B and 96C illustrates exemplary embodiments of anchors that are similar to the anchor illustrated by Figure 96A where high strength fibers are oriented to increase strength of the anchor;
Figure 96D illustrates an exemplary embodiment of a hybrid cloth;
Figure 96E illustrates an exemplary embodiment of a hybrid cloth;
Figure 97 illustrates the anchor of Figure 94 in a non-deployed, extended configuration along with a hemostatic plug;
Figure 98 illustrates the anchor of Figure 94 in a deployed configuration along with a hemostatic plug;
Figure 99 illustrates a delivery sheath and a steerable catheter for delivering the anchor of Figure 94;
Figure 100 illustrates an anchoring delivery catheter extending from the delivery sheath and the steerable catheter of Figure 99;
Figure 101 illustrates a needle extending from the anchoring delivery catheter of Figure 100;
Figure 102 illustrates the anchor extending along the needle of Figure 101;
Figure 103 illustrates the anchor and hemostatic plug along with the needle and a pusher of the delivery system of Figure 98;
Figure 104 illustrates the anchor and hemostatic plug of Figure 103;
Figure 105 illustrates the anchor and hemostatic plug of Figure 103 with lines attached;
Figure 106 illustrates the anchor with lines attached without a hemostatic plug;
Figure 107 illustrates the delivery system deploying the anchor and hemostatic plug without lines;
Figure 108 illustrates the delivery system, anchor, and hemostatic plug of Figure 107 with lines;
Figure 109 illustrates the delivery system, anchor, and hemostatic plug of Figure 108 with the anchor in a deployed state;
Figure 110 illustrates an enlarged view of the anchor and hemostatic plug in a deployed state;
Figure 110A illustrates an enlarged view of the anchor of Figure 95A and a hemostatic plug in a deployed state;
Figure 111 illustrates the anchor and hemostatic plug in a deployed state with the needle and delivery catheter of the delivery system withdrawn;
Figure 112 is an enlarged cutaway view of the human heart wall showing the delivery sheath and steerable catheter positioned adjacent the heart wall;
Figure 112A illustrates a delivery catheter, a piston, and an anchoring device for delivering an anchor;
Figure 112B is a cross sectional view of the delivery catheter, piston, and anchoring device of Figure 112A;
Figure 112C is a cross sectional view of the delivery catheter of Figure 112B;
Figure 112D is a cross sectional view of the delivery catheter of Figure 112C taken along the plane indicted by lines 133-133 in Figure 112C;
Figure 112E is a cross sectional view of the piston of Figure 112B;
Figure 112F is a cross sectional view of the piston of Figure 112E taken along the plane indicted by lines 135-135 in Figure 112E;
Figure 112G is a cross sectional view of the delivery catheter and piston of Figure 112B;
Figure 113 is an enlarged cutaway view of the human heart wall showing the delivery catheter anchored to the myocardium of the heart wall;
Figure 113A is a cross sectional view of the piston of Figure 112B, illustrating a clutch mechanism;
Figure 113B is a cross sectional view of the piston of Figure 113A taken along the plane indicated by lines 138-138 in Figure 113A;
Figures 113C-113E are views similar to Figure 113B, illustrating increasing torque applied between the piston and anchor;
Figure 113F and 113G are cross sectional views of an alternate clutch arrangement between the piston and the clutch;
Figure 113H is an enlarged cutaway view of a human heart wall showing the delivery catheter, piston, and anchoring device positioned adjacent the heart wall;
Figure 113I is an enlarged cutaway view of the human heart wall showing the delivery catheter, piston, and anchoring device positioned adjacent the heart wall with the piston compressed;
Figure 113J is an enlarged cutaway view of the human heart wall showing the anchoring device partially anchored to the myocardium of the heart wall;
Figure 113K is a cross sectional view showing the position of the clutch of the piston of Figure 113J;
Figure 113L is an enlarged cutaway view of the human heart wall showing the anchoring device anchored to the myocardium of the heart wall;
Figures 113M and 113N are cross sectional views showing the movement of the clutch of the piston when the anchor reaches the position illustrated by Figure 113L;
Figure 114 is an enlarged cutaway view of the human heart wall showing the needle inserted into the pericardial cavity of the heart wall;
Figure 114A is an enlarged cutaway view of the human heart wall showing a needle inserted into the pericardial cavity of the heart wall;
Figure 115 is an enlarged cutaway view of the human heart wall showing a needle injecting a dye into the pericardial cavity of the heart wall;
Figure 115A is an enlarged cutaway view of the human heart wall showing the needle injecting a dye into the pericardial cavity of the heart wall;
Figure 115B is an enlarged cutaway view of the human heart wall showing a needle injecting a wire into the pericardial cavity of the heart wall;
Figure 116 is an enlarged cutaway view of the human heart wall showing the anchor being deployed along the needle;
Figure 116A is an enlarged cutaway view of the human heart wall showing the anchor being deployed along the needle;
Figure 117 is an enlarged cutaway view of the human heart wall showing the anchor extending inside the pericardial cavity;
Figure 117A is an enlarged cutaway view of the human heart wall showing the anchor extending inside the pericardial cavity;
Figure 118 is an enlarged cutaway view of the human heart wall showing the anchor deployed inside the pericardial cavity;
Figure 118A is an enlarged cutaway view of the human heart wall showing the anchor deployed inside the pericardial cavity;
Figure 119 is an enlarged cutaway view of the human heart wall showing the anchor being seated in the pericardial cavity;
Figure 119A is an enlarged cutaway view of the human heart wall showing the anchor seated in the pericardial cavity;
Figure 119B is an enlarged cutaway view of the human heart wall showing the anchor seated in the pericardial cavity;
Figure 119C is an enlarged cutaway view of the human heart wall showing the anchoring device being detached from the myocardium of the heart wall;
Figure 119D is a cross sectional view of the piston of Figure 158, illustrating the position of the clutch as the anchor is removed from the heart wall;
Figure 120 is an enlarged cutaway view of the human heart wall showing the anchor seated in the pericardial cavity and the delivery system removed;
Figure 120A is an enlarged cutaway view of the human heart wall showing the anchor seated in the pericardial cavity and the delivery system removed;
Figure 121 is an enlarged cutaway view of the human heart wall showing the delivery sheath and steerable catheter positioned adjacent a papillary muscle of the heart;
Figure 121A illustrates a cutaway view of the human heart in a diastolic phase, in which a steerable catheter and delivery catheter are positioned adjacent a papillary muscle of the heart;
Figure 122 is an enlarged cutaway view of the human heart wall showing the delivery catheter anchored to the papillary muscle of the heart;
Figure 122A illustrates a cutaway view of the human heart in a diastolic phase, in which a steerable catheter and delivery catheter are positioned adjacent a papillary muscle of the heart
Figure 123 is an enlarged cutaway view of the human heart wall showing the needle inserted through the papillary muscle and into the pericardial cavity of the heart wall;
Figure 124 is an enlarged cutaway view of the human heart wall showing a needle injecting a dye into the pericardial cavity of the heart wall;
Figure 125 is an enlarged cutaway view of the human heart wall showing the anchor being deployed along the needle;
Figure 126 is an enlarged cutaway view of the human heart wall showing the anchor extending inside the pericardial cavity;
Figure 127 is an enlarged cutaway view of the human heart wall showing the anchor deployed inside the pericardial cavity;
Figure 128 is an enlarged cutaway view of the human heart wall showing the anchor being seated in the pericardial cavity;
Figure 129 is an enlarged cutaway view of the human heart wall showing the anchor seated in the pericardial cavity and the delivery system removed.
Figures 130A-130D illustrate an exemplary embodiment of a helical anchor being implanted in tissue;
Figures 131A-131E illustrate an exemplary embodiment of a helical anchor being implanted in tissue;
Figures 132A and 132B illustrate and exemplary embodiment of a helical anchor and a delivery system for the helical anchor;
Figures 133A-133F illustrate the helical anchor of Figures 132A and 132B being implanted in tissue;
Figure 134 illustrates an exemplary embodiment of a helical anchor implanted in a ventricular heart wall;
Figure 135 illustrates an exemplary embodiment of two helical anchors implanted in a ventricular heart wall;
Figure 136 illustrates an exemplary embodiment of a helical anchor implanted in a ventricular heart wall;
Figure 137 illustrates an exemplary embodiment of two helical anchors implanted in a ventricular heart wall;
Figure 138 illustrates an exemplary embodiment of a helical anchor implanted in a ventricle of a heart;
Figure 139 illustrates an exemplary embodiment of a helical anchor implanted in a ventricle of a heart;
Figure 140 illustrates an exemplary embodiment of a helical anchor implanted in a ventricular heart wall;
Figure 141 illustrates an exemplary embodiment of a helical anchor implanted in a ventricular heart wall;
Figure 142 illustrates an exemplary embodiment of a helical anchor and a delivery rail;
Figure 143 is a side view of an exemplary embodiment of a helical anchor;
Figure 144 is a leading end view of the helical anchor illustrated by Figure 143;
Figures 145A-145C illustrate delivery of a helical anchor to an endocardial layer of a heart wall;
Figure 146 is an enlarged cutaway view of a human heart wall showing a portion of a helical anchoring device applying a compressive force to a myocardium layer of a heart wall;
Figures 147A and 147B illustrate an exemplary embodiment of a guard for use during implantation of a helical anchor;
Figure 148 illustrates an exemplary embodiment of a guard for use during implantation of a helical anchor;
Figures 149, 150, and 151A-151D illustrate use of the guard of Figure 148;
Figures 152A illustrates an exemplary embodiment of a lubricant being applied to a cloth covering on a wire of a helical anchor;
Figures 152B illustrates an exemplary embodiment of a lubricated cloth covering on a wire of a helical anchor;
Figure 152C illustrates an exemplary embodiment of a cloth for covering a wire of a helical anchor;
Figures 153A-153K illustrate use of an exemplary embodiment of a tissue remodeling system;
Figures 154A-154M illustrate use of an exemplary embodiment of a tissue remodeling system;
Figures 155A-155K illustrate use of an exemplary embodiment of a tissue remodeling system;
Figures 156A-156L illustrate use of an exemplary embodiment of a tissue remodeling system;
Figures 157A-157P illustrate use of an exemplary embodiment of a tissue remodeling system;
Figures 158A-158G illustrate use of an exemplary embodiment of a tissue remodeling system;
Figures 159-162 illustrate use of an exemplary embodiment of a tissue remodeling system to approximate papillary muscles;
Figure 163 illustrates an exemplary embodiment of a tissue remodeling system with anchors implanted on two papillary muscles and a heart wall;
Figure 164 illustrates an exemplary embodiment of a tissue remodeling system with anchors implanted on one papillary muscle and a heart wall;
Figure 165 illustrates an exemplary embodiment of a tissue remodeling system with anchors implanted on spaced apart portions of a heart wall;
Figure 166 illustrates an exemplary embodiment of a depth of a helical anchor in an endocardial layer of a heart wall;
Figure 167 illustrates alternate embodiments of anchors implanted in an endocardial layer of a heart wall;
Figure 168A-168G illustrate deployment of an exemplary embodiment of a tissue remodeling system;
Figure 169A illustrates an exemplary embodiment of an alternate force application arrangement for the tissue remodeling system of Figures 168A-168H;
Figure 169B illustrates an exemplary embodiment of an alternate force application arrangement for the tissue remodeling system of Figures 168A-168H;
Figure 170A-170G illustrate use of an exemplary embodiment of a tissue remodeling system to remodel a shape of a heart ventricle;
Figure 171 illustrates a cutaway view of the human heart with an anchor delivery device inside the heart;
Figure 172A is a perspective view of an exemplary embodiment of a coiled anchor;
Figure 172B is a top view of the coiled anchor illustrated by Figure 172A;
Figure 172C-172E are different side elevational views of the coiled anchor illustrated by Figure 172A;
Figure 172F is a bottom view of the coiled anchor illustrated by Figure 172A;
Figure 173A is a perspective view of an exemplary embodiment of a coiled anchor;
Figure 173B is a top view of the coiled anchor illustrated by Figure 173A;
Figure 173C-173E are different side elevational views of the coiled anchor illustrated by Figure 173A;
Figure 173F is a bottom view of the coiled anchor illustrated by Figure 173A;
Figure 174A is a perspective view of an exemplary embodiment of a coiled anchor;
Figure 174B is a top view of the coiled anchor illustrated by Figure 173A;
Figure 174C-174E are different side elevational views of the coiled anchor illustrated by Figure 174A;
Figure 174F is a bottom view of the coiled anchor illustrated by Figure 174A;
Figures 175A-175H illustrate an exemplary embodiment of a helical anchor being implanted in tissue;
Figures 176A-176H are schematic illustrations showing a coiled anchor penetrating the endocardial tissue and embedding in myocardial tissue;
Figures 177A-177B illustrate deployment of a coiled anchor and an attached tether;
Figures 178A-178D illustrate use of an exemplary embodiment of a tissue remodeling system;
Figures 179A-179B illustrate deployment of a coiled anchor and an attached tether;
Figures 180A-180D illustrate use of an exemplary embodiment of a tissue remodeling system;
Figures 181A-181C illustrate deployment of a coiled anchor and an attached tether;
Figures 182A-182H illustrate deployment of a coiled anchor and an attached tether into a ventricular wall;
Figures 183A-183G illustrate deployment of a coiled anchor and an attached tether into a ventricular wall;
Figure 184A illustrates a cutaway view of the human heart showing a first anchor attached to the intraventricular septum and a second anchor attached to a ventricular wall with lines connecting the first and second anchors;
Figure 184B illustrates a cutaway view of the human heart showing a first anchor attached to the intraventricular septum, a second anchor attached to a ventricular wall, and a third anchor attached to a ventricular wall with lines connecting the first, second, and third anchors;
Figure 184C illustrates a cutaway view of the human heart showing a first anchor attached to the intraventricular septum, a second anchor attached to a ventricular wall, a third anchor attached to a ventricular wall, and a fourth anchor attached to a ventricular wall with lines connecting the first, second, third and fourth anchors;
Figure 184D illustrates a cutaway view of the human heart showing a first anchor attached to a papillary muscle and a second anchor attached to a papillary muscle with lines connecting the first and second anchors;
Figure 184E illustrates a cutaway view of the human heart showing a first anchor attached to an intraventricular septum, a second anchor attached to a papillary muscle, and a third anchor attached to a papillary muscle with lines connecting the first, second, and third anchors;
Figure 185A is a perspective view of an exemplary embodiment of a coiled anchor;
Figure 185B is a side elevational views of the coiled anchor illustrated by Figure 185A;
Figures 186A-186C illustrate an exemplary embodiment of a coiled anchor with an anti-rotation mechanism;
Figure 187 illustrates an exemplary embodiment of a coiled anchor having a cloth disposed over at least a portion of an outer ring portion;
Figure 188 illustrates the coiled anchor illustrated by Figure 187 implanted in tissue;
Figure 189 illustrates a line trimming tool according to an exemplary embodiment;
Figures 190-194 illustrate cross sectional views of a line trimming tool and a line according to an exemplary embodiment;
Figure 195 illustrates a cross-section view of a handle of a line trimming tool according to an exemplary embodiment;
Figures 196-197 illustrate a trigger of a handle according to an exemplary embodiment;
Figures 198-199 illustrate a handle of a line trimming tool according to an exemplary embodiment; and
Figures 200-202 illustrate a handle and a line trimming tool according to an exemplary embodiment.

### DETAILED DESCRIPTION

In this disclosure the following non-SI unit is used, which may be converted to the respective SI or metric unit according to the following conversion factor: 1 inch = 0.0254 meter. The following description refers to the accompanying drawings, which illustrate specific embodiments of the present disclosure. Other embodiments having different structures and operation do not depart from the scope of the present disclosure.

Exemplary embodiments of the present disclosure are directed to devices and methods for remodeling the shape of one or more walls of a human heart. It should be noted that various embodiments of devices and systems for delivery are disclosed herein, and any combination of these options can be made unless specifically excluded. In other words, individual components of the disclosed devices and systems can be combined unless mutually exclusive or otherwise physically impossible.

As described herein, when one or more components are described as being connected, joined, affixed, coupled, attached, or otherwise interconnected, such interconnection can be direct as between the components or may be indirect such as through the use of one or more intermediary components. Also, as described herein, reference to a "member," "component," or "portion" shall not be limited to a single structural member, component, or element but can include an assembly of components, members, or elements. Also, as described herein, the terms "substantially" and "about" are defined as at least close to (and includes) a given value or state (preferably within 10% of, more preferably within 1% of, and most preferably within 0.1% of).

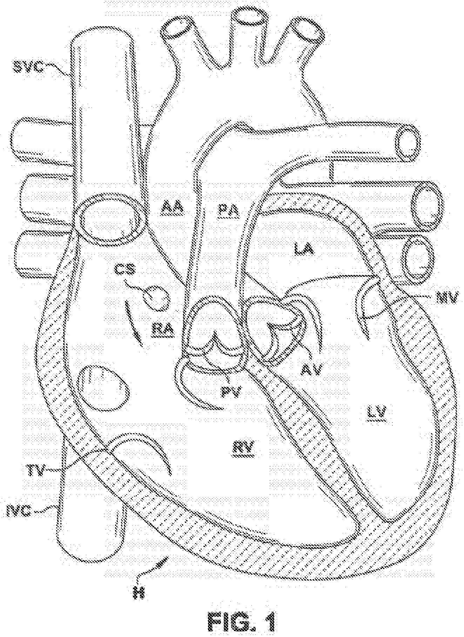

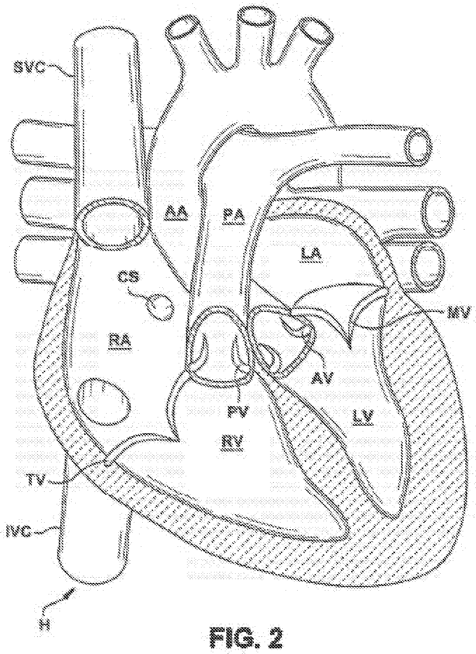

Figures 1 and 2 are cutaway views of the human heart H in diastolic and systolic phases, respectively. The right ventricle RV and left ventricle LV are separated from the right atrium RA and left atrium LA, respectively, by the tricuspid valve TV and mitral valve MV; i.e., the atrioventricular valves. Additionally, the aortic valve AV separates the left ventricle LV from the ascending aorta AA, and the pulmonary valve PV separates the right ventricle from the pulmonary artery PA. Each of these valves has flexible leaflets extending inward across the respective orifices that come together or "coapt" in the flow stream to form the one-way, fluid-occluding surfaces. The remodeling devices, systems, and methods of the present application are described primarily with respect to the left ventricle LV. Therefore, anatomical structures of the left side of the heart will be explained in greater detail. It should be understood that the devices, systems, and methods described herein may also be used in remodeling the right ventricle.

The left atrium LA receives oxygenated blood from the lungs. During the diastolic phase, or diastole, seen in Figure 1, the blood that was previously collected in the left atrium LA (during the systolic phase) moves through the mitral valve MV and into the left ventricle LV by expansion of the left ventricle LV. In the systolic phase, or systole, seen in Figure 2, the left ventricle LV contracts to force the blood through the aortic valve AV and ascending aorta AA into the body. During systole, the leaflets of the mitral valve MV close to prevent the blood from regurgitating from the left ventricle LV and back into the left atrium LA, and blood is collected in the left atrium from the pulmonary vein.

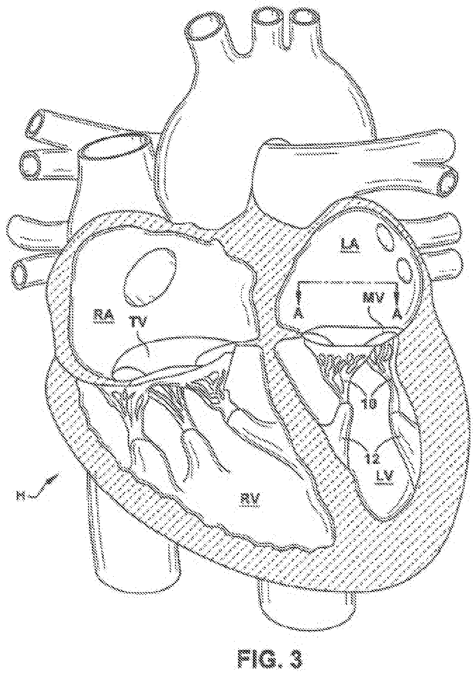

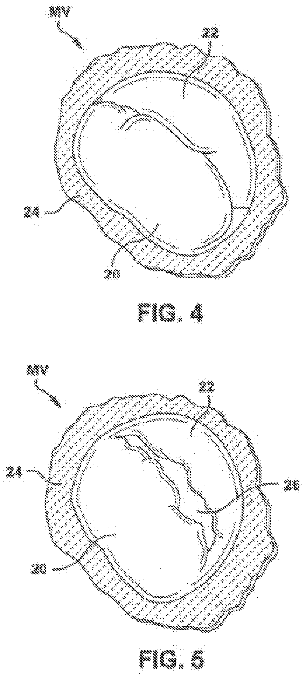

Referring now to Figures 1-5, the mitral valve MV includes two leaflets, the anterior leaflet 20 and the posterior leaflet 22. The mitral valve MV also includes an annulus 24, which is a variably dense fibrous ring of tissues that encircles the leaflets 20, 22. Referring to Figure 3, the mitral valve MV is anchored to the wall of the left ventricle LV by chordae tendineae 10. The chordae tendineae 10 are cord-like tendons that connect the papillary muscles 12 (i.e., the muscles located at the base of the chordae tendineae and within the walls of the left ventricle) to the leaflets 20, 22 of the mitral valve MV. The papillary muscles 12 serve to limit the movements of the mitral valve MV and prevent the mitral valve from being inverted or prolapsed. The mitral valve MV opens and closes in response to pressure changes in the left atrium LA and the left ventricle LV. The papillary muscles do not open or close the mitral valve MV. Rather, the papillary muscles brace the mitral valve MV against the high pressure needed to circulate blood throughout the body. Together the papillary muscles and the chordae tendineae are known as the subvalvular apparatus, which functions to keep the mitral valve MV from prolapsing into the left atrium LA when the mitral valve closes.

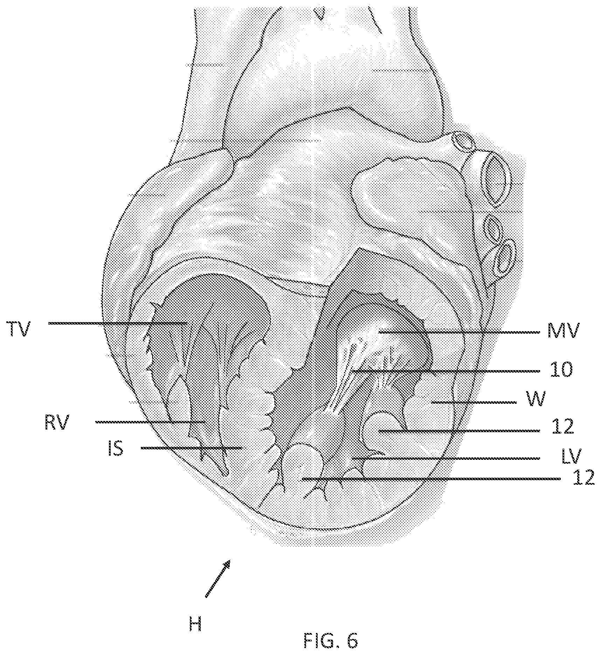

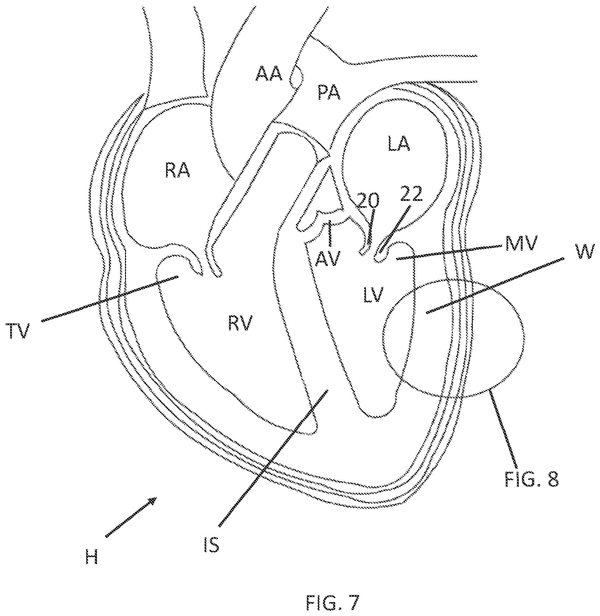

Figure 6 is a cutaway view of the human heart with the section through the papillary muscles of the left ventricle. The right ventricle RV is separated from the left ventricle LV by the interventricular septum IS. The mitral valve leaflets 20, 22 shown Figure 7) extending inward across the respective orifices that come together or "coapt" in the flow stream to form the one-way, fluid-occluding surfaces. The devices and methods for remodeling the shape of the heart walls W are described primarily with respect to the left ventricle LV. The devices and methods can be used to approximate the papillary muscles in some embodiments, which are also described primarily with respect to the left ventricle LV. In addition to reducing the size of the ventricle to increase the ventricular function, bringing the papillary muscles closer together can cause the valve leaflets to coapt and prevent mitral valve regurgitation. It should be understood that the devices described herein may also be used in remodeling the right ventricle RV and approximate the papillary muscles of the tricuspid valve TV.

In one exemplary embodiment, the devices described by the present application are used to remodel the shape of a ventricle to improve heart function. Heart function can be improved by reducing the size of the ventricle, approximating the papillary muscles, and/or correcting the function of the mitral valve MV. In one exemplary embodiment, the devices are configured to reshape the wall of a human heart H in a way that causes the mitral valve MV to prevent blood from regurgitating from the left ventricle LV and back into the left atrium LA.

When a healthy mitral valve MV is in a closed position, the leaflets 20, 22 coapt, which prevents blood from leaking from the left ventricle LV to the left atrium LA. Regurgitation can occur when one or both of the leaflets 20, 22 of the mitral valve MV prolapse into the left atrium LA during systole or the leaflets fail to coapt or close together against one another. This prolapse and/or a failure to coapt causes a gap between the leaflets 20, 22, which allows blood to flow back into the left atrium LA from the left ventricle LV during systole.

The devices and procedures disclosed herein refer to remodeling the left ventricle, with the possible consequence of better coaption of the leaflets of the mitral valve. However, it should be understood that the devices and concepts provided herein can be used to remodel the right ventricle, with the possible consequence of better coaption of the tricuspid valve TV leaflets.

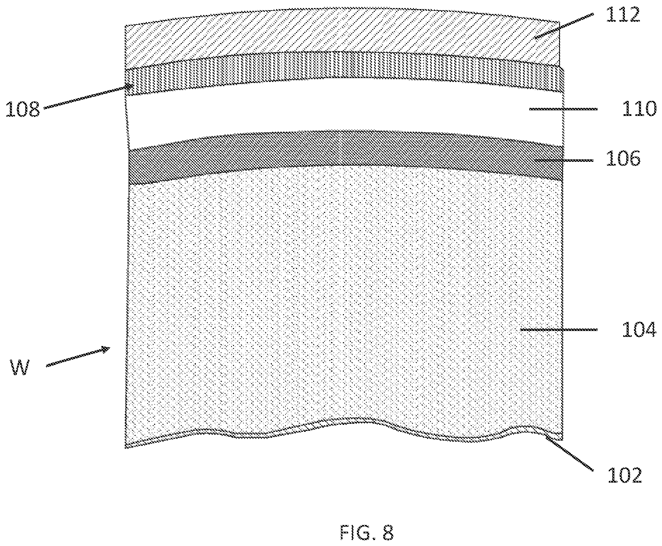

Referring now to Figure 8, an enlarged cutaway view of the human heart wall W is illustrated. The heart wall W has multiple layers, which include the endocardium 102, the myocardium 104, and the epicardium 106. The endocardium 102 is the most inner layer of the heart H. It forms the inner layer of all four heart chambers and is directly connected to all the inner cardiac appendages, such as the bicuspid valve BV, the tricuspid valve TV, the pulmonary valve (not shown), the aortic valve AV, and the chordae tendineae CT by way of the papillary muscles 12.

The myocardium 104 sits between the inner endocardium 102 and the outer epicardium 106. The myocardium 104 is the basic muscle that makes up the heart H and it functions by providing a scaffolding for the heart chambers. The myocardium 104 contracts and relaxes the cardiac walls so that blood can pass between the chambers.

The epicardium 106 is a visceral layer of serous pericardium. The epicardium is the innermost of the two layers of the pericardium. The epicardium covers the external surfaces of the heart. It is directly fused with the myocardium internally. It is comprised mainly of connective tissue and protectively encompasses the heart.

The pericardium 108 is the double-walled sac that contains the heart and roots of the great vessels that leave from or enter the heart. A space is formed between epicardium 106 and the serous layer of the pericardium 108, which is known as the pericardial cavity 110, which contains pericardial fluid. A layer of parietal pericardium 112 is disposed around the heart. The outer parietal layer 112 and the inner serous pericardium layer are on the outside of the pericardial cavity 110.

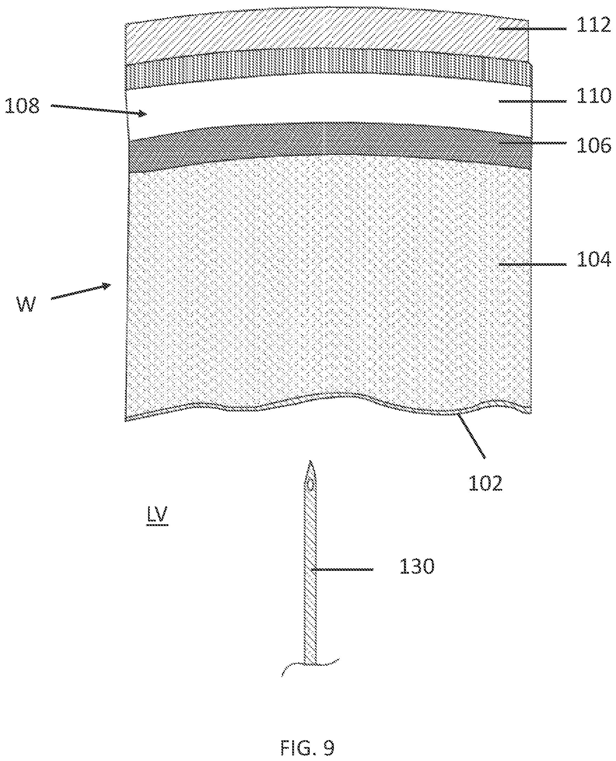

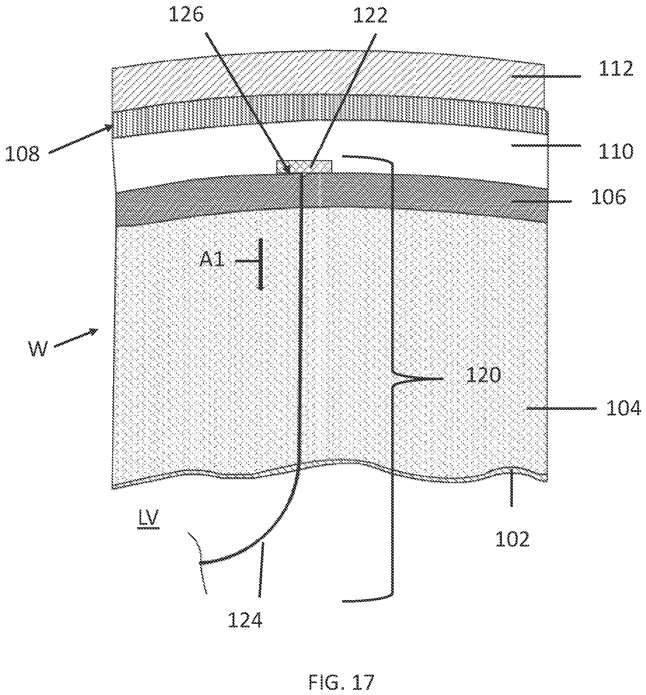

Referring to Figures 9-17, an exemplary embodiment of a device 120 for remodeling the shape of a heart wall W, and an exemplary embodiment of a system and method for delivering and deploying the device 120 into the pericardial cavity 110 is illustrated. Referring to Figure 17, the device 120 includes an anchor 122 and a line 124 engaging or connected to the anchor 122 and extending therefrom. The line 124 can take a wide variety of different forms. Examples of lines 124 include, but are not limited to, sutures, wires, cables, chords, bendable rods, any combination thereof, etc. The line 124 can be any element or combination of elements that is configured to extend from the anchor 122, through the heart wall W, and into the internal chamber.

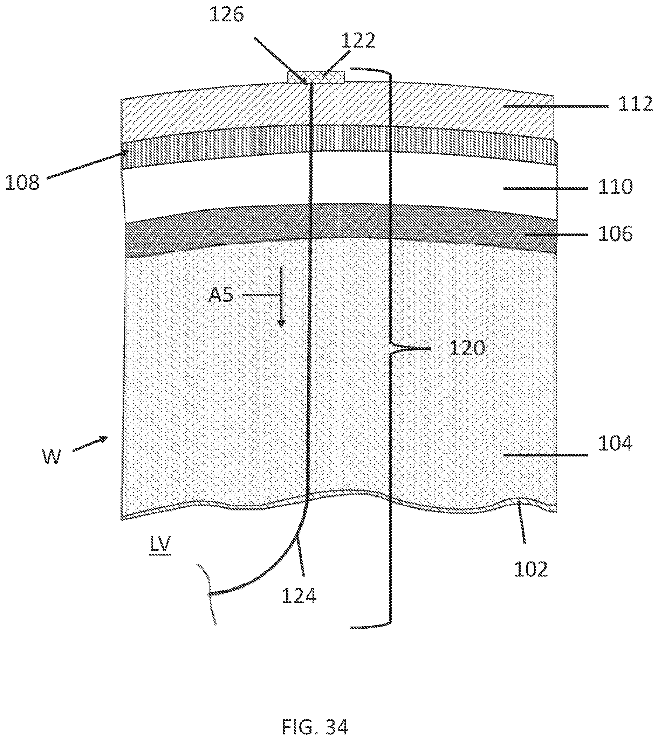

The anchor 122 is configured to be positioned against a surface 126 facing outward relative to an internal chamber of the heart H, such as for example, the left ventricle LV or right ventricle of the heart H. In the illustrated embodiment of Figure 17, the outward facing surface 126 is a portion of the epicardium 106 and the anchor 122 is disposed in the pericardial cavity 110. In the embodiment illustrated by Figure 34, the outward facing surface 126 is the pericardium 108.

The anchor 122 can be configured in a variety of ways. Any configuration that can be positioned to engage an outward facing surface 126 of the heart wall W to support pulling a portion of the heart wall W inward (i.e., toward the internal chamber) can be used. For example, the anchor 122 can be a pledget, a sufficiently sized knot formed in the line 124, a stop, or some other line anchoring device. The anchor 122 can be collapsible/expandable or reconfigurable, such that the anchor can be delivered through a catheter or sheath in a delivered state (e.g., collapsed or elongated) that fits within a lumen of the catheter, and can be reshaped or expanded to a deployed state once it has been delivered to the appropriate location. In one exemplary embodiment, the anchor 122 includes a shape-memory alloy-such as Nitinol-to provide shape-setting capability.

Referring to Figure 9, in one exemplary embodiment, deployment of the device 120 includes delivering a piercing device 130 into an internal chamber (e.g. left ventricle LV) of the heart H and adjacent the heart wall W. The piercing device 130 can be any suitable device for piercing or creating a passage into the human heart wall W, such as for example, a needle, wire, or other similar device. In the illustrated embodiment, the piercing device 130 is a needle or hollow wire having an inner passage (not shown) and an opening 131 proximate the distal end 133 of the piercing device the fluidly connects the inner passage (not shown) to the exterior of the piercing device 130. However, in other exemplary embodiments, the piercing device is not hollow. In the illustrated embodiment, the piercing device 130 is pointed or has a sharp tip. However, in other exemplary embodiments, the piercing device 130 has a blunt tip.

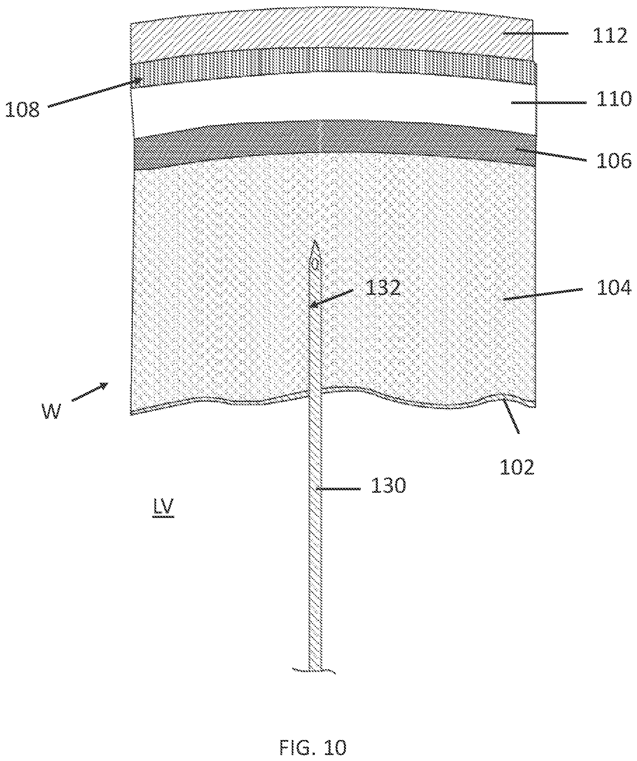

In Figure 10, the piercing device 130 is extended into the heart wall W through the endocardium 102 and into the myocardium 104 to create a passage 132 through the heart wall W. The piercing device 130, however, is not yet sufficiently inserted to deploy the anchor 122 into the pericardial cavity 110. Thus, the piercing device 130 in Figure 10 is shown in an under-inserted position.

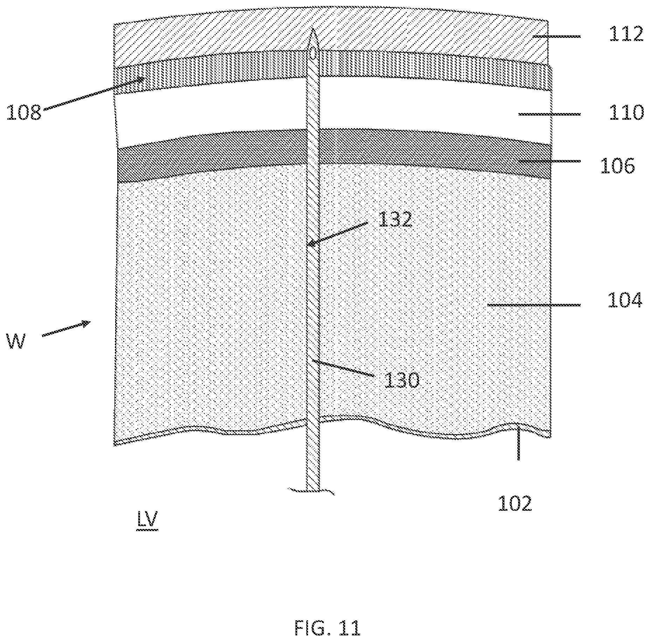

In Figure 11, the piercing device 130 is extended through the endocardium 102, the myocardium 104, the epicardium 106, and the pericardium 108, and into the external parietal pericardium tissue 112 to extend the passage 132 in the heart wall W. The piercing device 130, however, has extended past the pericardial cavity 110 where the anchor 122 is to be positioned in this exemplary embodiment. Thus, the piercing device 130 in Figure 11 is shown in an over-inserted position for this exemplary embodiment (however, this can be the correct position for other embodiments).

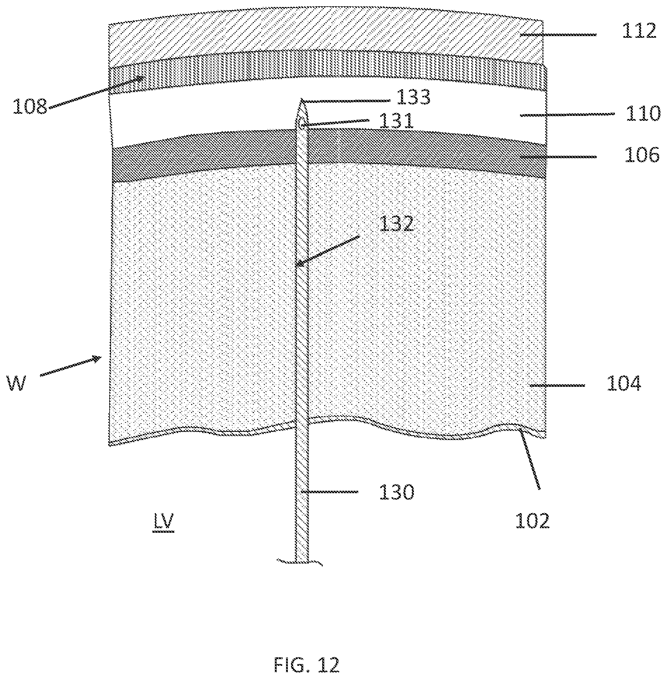

In Figure 12, the piercing device 130 is extended through the endocardium 102, the myocardium 104, the epicardium 106, and into the pericardial cavity 110, such that the opening 131 and the distal end 133 are within the within the pericardial cavity 110. Thus, the piercing device 130 in Figure 12 is properly positioned for deploying the anchor 122 into the pericardial cavity 110.

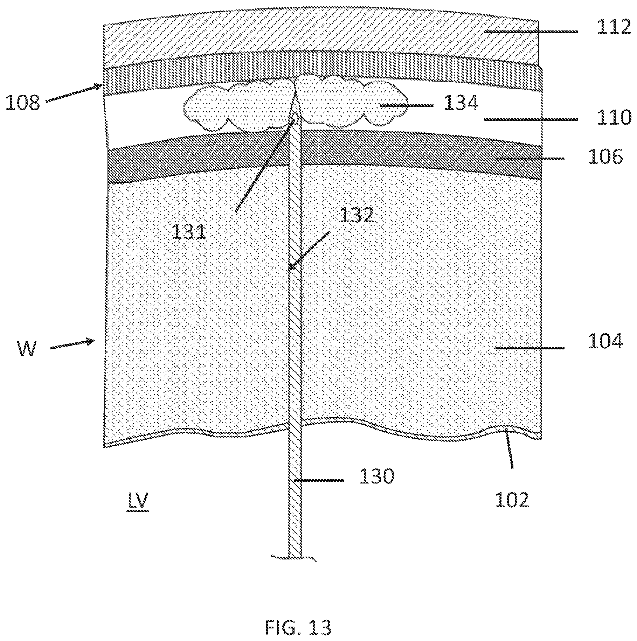

In one exemplary embodiment, proper positioning of the piercing device 130 is optionally verified. The proper positioning of the piercing device 130 can be verified in a wide variety of different ways. For example, the positioning of the piercing device can be visually determined by providing the piercing device with a marker, such as a radio-opaque marker, by discharging a material into cavity, such as the pericardial cavity, by sensing a pressure required to discharge fluid from the piercing device, by sensing a force required to advance the piercing device, by positioning a small guide wire in the pericardial cavity, and/or with electrical signals, such as by electrical signals provided by and/or sensed by the piercing device, etc. In one exemplary embodiment, to verify that the piercing device 130 is properly positioned for deploying the anchor 122 into the pericardial cavity 110, a dye 134, or other detectable fluid, is delivered through the piercing device 130 and injected into the pericardial cavity 110, as shown in Figure 13. The dye 134 can be detected by any suitable technique such as X-ray or other imaging techniques, to verify that the piercing device 130 is properly positioned.

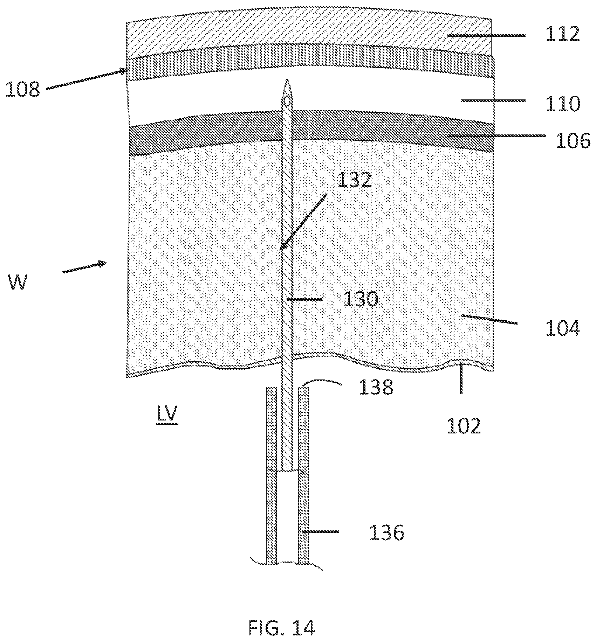

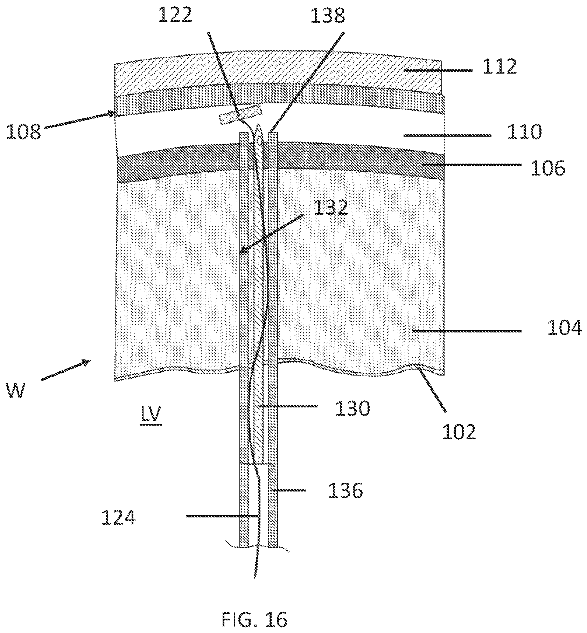

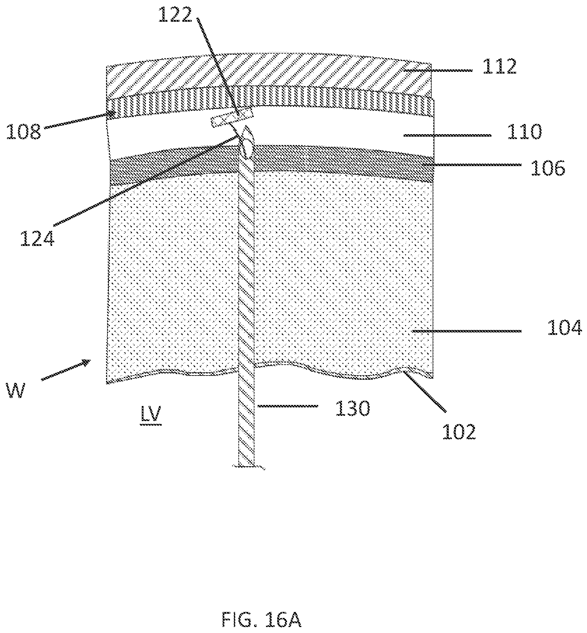

In Figure 14, the piercing device 130 is extended into the pericardial cavity 110 and a delivery catheter 136 is positioned within the heart chamber (e.g. the left ventricle LV) such that a distal end 138 of the delivery catheter 136 is adjacent the endocardium 102. In the illustrated embodiment, the delivery catheter 136 is concentric with the piercing device 130. In other embodiments, however, the delivery catheter 136 may not be concentric with the piercing device 130. In yet other embodiments, the delivery catheter 136 can be omitted. For example, the device 120 can be delivered directly through or over the piercing device 130, rather than through a separate catheter (See FIGS 16A-16C).

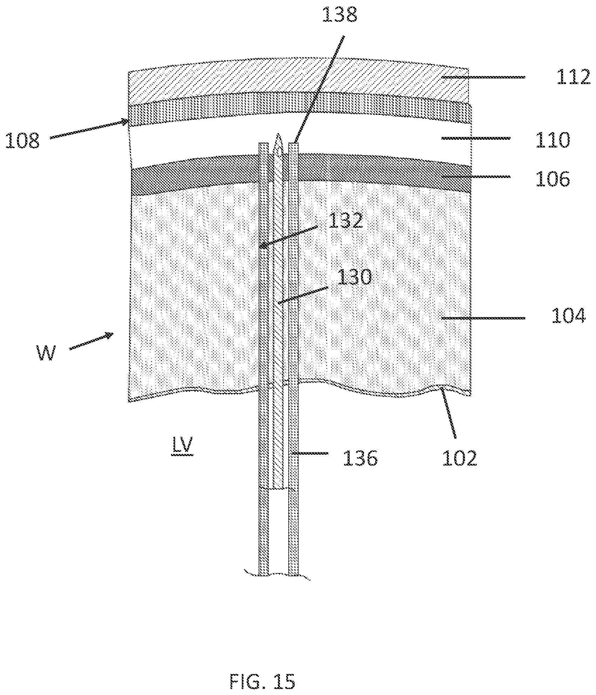

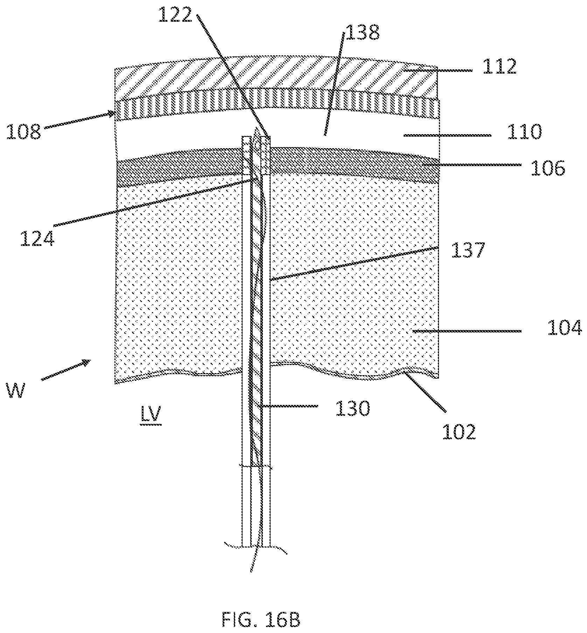

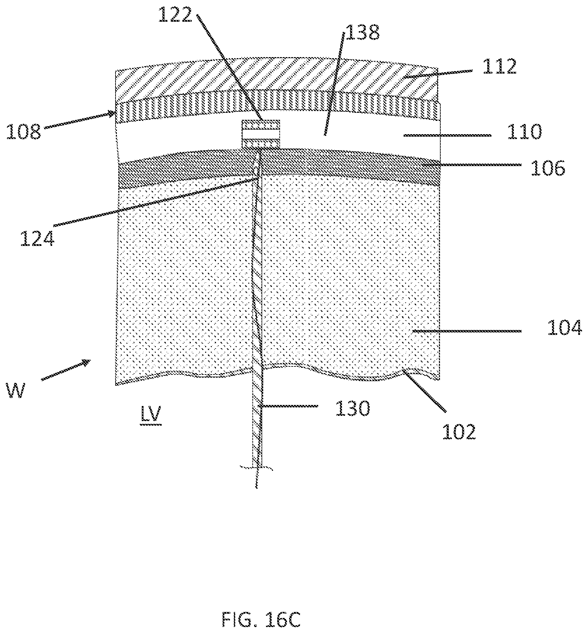

In Figure 15, the delivery catheter 136 is extended through the passage 132 such that the distal end 138 of the delivery catheter 136 is positioned within the pericardial cavity 110. Referring to Figure 16, with the distal end 138 of the delivery catheter 136 is positioned within the pericardial cavity 110, the device 120 (See Figure 17) for remodeling the shape of a heart wall W can be delivered through the delivery catheter 136. In particular, the anchor 122 (See Figures 16 and 17) can be extended or pushed out of the distal end of the 138 of the delivery catheter 136 and into the pericardial cavity 110 while the attached line 124 (See Figures 16 and 17) extends through the delivery catheter 136 in the passage 132.

As is mentioned above, the anchor 122 can take a wide variety of different forms and can be delivered in a wide variety of different ways. In the examples illustrated by Figures 16A-16C, the delivery catheter 136 is omitted. In Figure 16A, the anchor 122 is delivered through the piercing device 130 and the catheter 136 can be omitted. The anchor 122 can be delivered through the piercing device 130 in any of the embodiments disclosed herein. In Figure 16B, the anchor 122 is delivered over the piercing device 130. In this example, a pusher 137 pushes the anchor 122 along the outside surface of the piercing device 130 to deploy the anchor 122 as illustrated by Figure 16C. The anchor 122 can be delivered over the piercing device 130 in any of the embodiments disclosed herein.

As shown by comparing Figures 16 and 17, the delivery catheter 136 and the piercing device 130 can be removed by withdrawing them from the passage 132 and from the heart chamber. The device 120 is left deployed in the heart wall W with the anchor 122 within the pericardial cavity 110 and the line 124 extending through the epicardium 106, the myocardium 104, and the endocardium 102 and into the heart chamber (e.g., the left ventricle LV or the right ventricle RV).

To seat the anchor 122 and to remodel the heart wall W, the line 124 can be placed in tension by pulling the line 124 in an inward direction toward the heart chamber as shown by the arrow A1 in Figure 17. As will be described in more detail below, two or more devices can be deployed, coupled, and pull against one another to pull the heart wall inward and remodel the shape of the heart wall(s). The anchor 122 will engage and press in on the outward facing surface 126, which in the illustrated embodiment is the epicardium 106. The anchor 122 (See Figure 17) in its deployed state is too large to fit through the passage 132 (See Figure 14) formed by the piercing device 130 (See Figure 14). Thus, further tensioning of the line 124 can pull the heart wall W inward toward the heart chamber (e.g., the left ventricle LV).

In one exemplary embodiment, the device 120 is configured to prevent blood leakage through the passage 132 (see Figure 14) into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 closes immediately or substantially immediately upon withdrawal of the piercing device 130 and/or the delivery catheter 136.

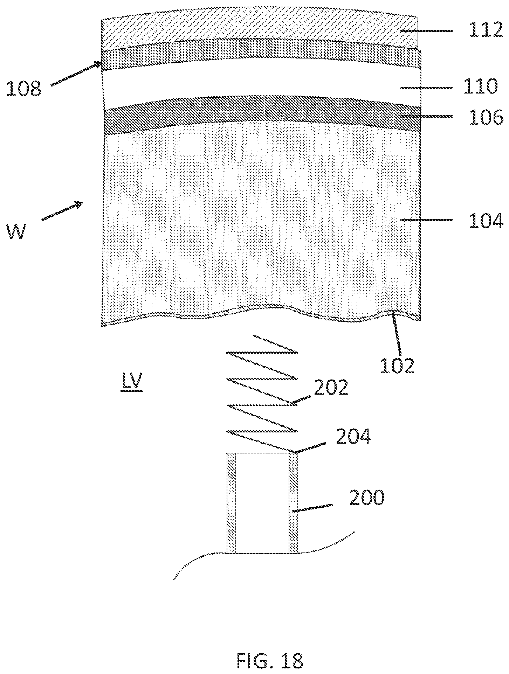

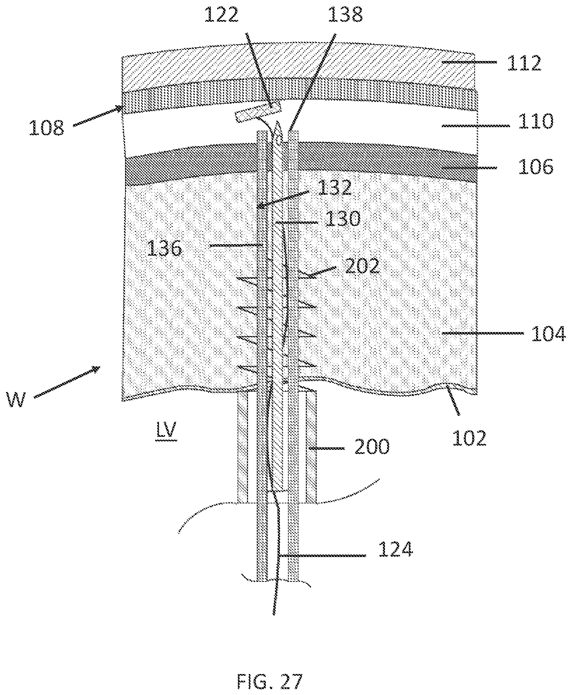

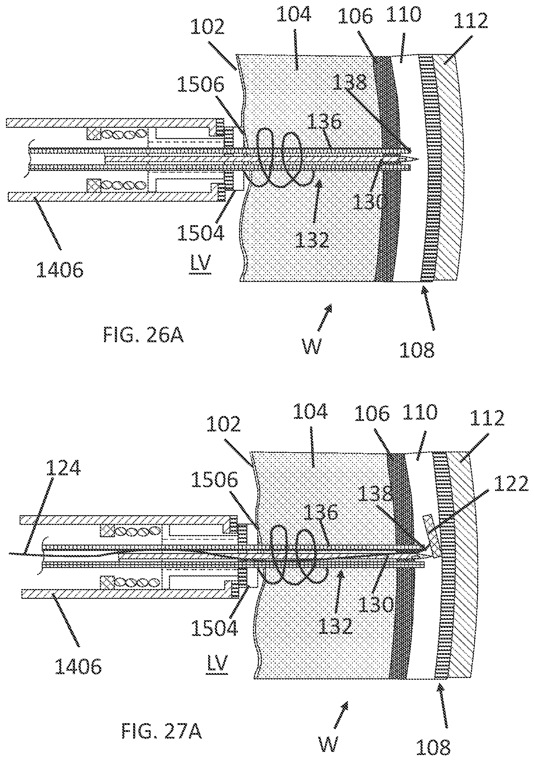

Referring to Figures 18-28, another exemplary embodiment of deployment of the device 120 (See Figure 27) into the pericardial cavity 110 for remodeling the shape of a heart wall W and system and method for delivering the device 120 is illustrated. Referring to Figure 18, deployment of the device 120 includes delivering an anchoring catheter 200 into an internal chamber (e.g. left ventricle LV) of the heart H and adjacent the heart wall W. The anchoring catheter 200 can be any suitable catheter or catheter-like device that is capable of attaching to, or anchoring to, the heart wall W.

In the exemplary embodiment, the anchoring catheter 200 includes an anchoring device 202 attached to a distal end 204 of the anchoring catheter 200. Examples of suitable anchoring devices include, but are not limited to expandable barbs, a suction tip, such as a suction cone, and/or a corkscrew shaped tip as illustrated. The anchoring device 202 can be any device capable of temporarily attaching the anchoring catheter 200 to the heart wall W. In the illustrated embodiment, the anchoring device 202 is a wire formed into a helical shape.

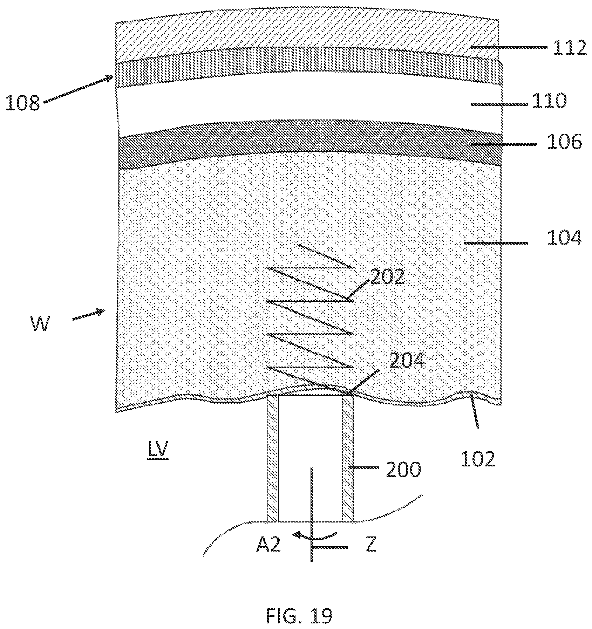

Referring to Figure 19, the anchoring catheter 200 is attached to the heart wall W. In the illustrated embodiment, the anchoring catheter 200 can be attached to the heart wall W by rotating the anchoring catheter 200 about illustrated axis Z in the direction of arrow A2, as shown in Figure 19. As a result, the helical anchoring device 202 can screw into the heart wall W through the endocardium 102 and into the myocardium 104 to secure the anchoring catheter 200 to the heart wall W. The anchoring device 202 thereby fixes the position of the catheter 200 relative to the heart wall W. Since the heart H is beating during the procedures described herein, the anchoring of the catheter 200 relative to the heart wall greatly simplifies and increases the accuracy of the procedure of piercing the wall W with the piercing device 130, positioning the distal end 133 of the piecing device in the pericardial space 110, positioning the delivery catheter 136 in the pericardial space 110, and/or deploying the anchor 122 into the pericardial space 110 (See Figure 27).

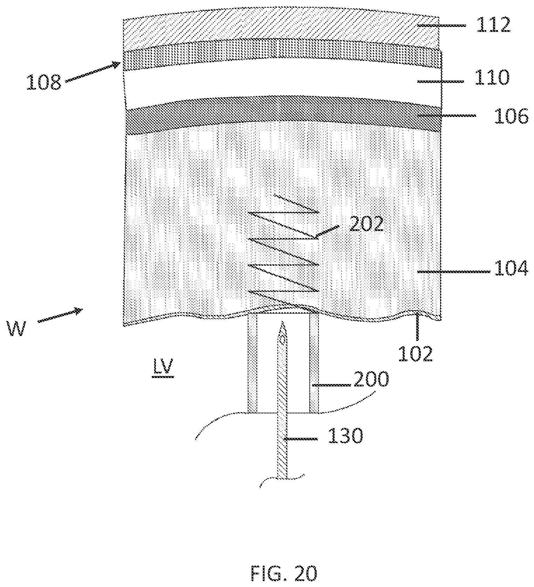

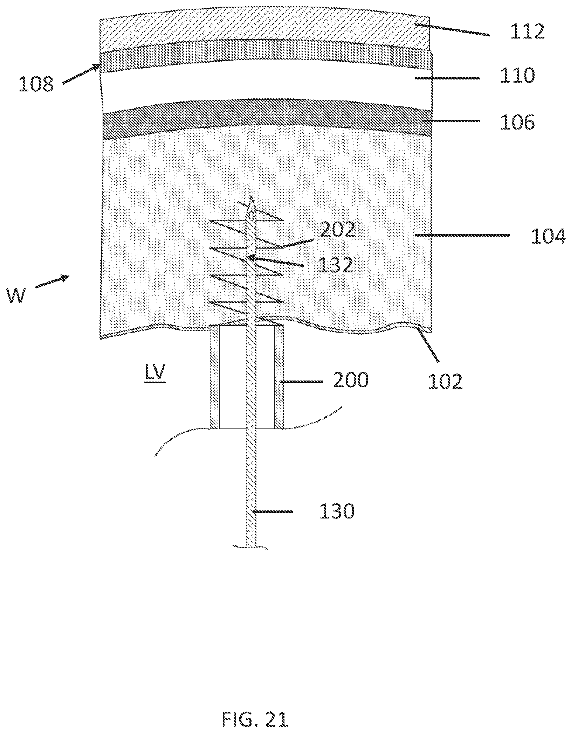

Referring to Figure 20, the piercing device 130 is delivered through the anchoring catheter 200 into an internal chamber (e.g. left ventricle LV) of the heart H such that the distal end of the piercing device is adjacent the heart wall W. In Figure 21, the piercing device 130 is extended into the heart wall W through the endocardium 102 and into the myocardium 104 to create the passage 132 into the heart wall W. The piercing device 130, however, is not yet sufficiently inserted to deploy the anchor 122 (See Figure 27) into the pericardial cavity 110. Thus, the piercing device 130 in Figure 21 is shown in an under-inserted position.

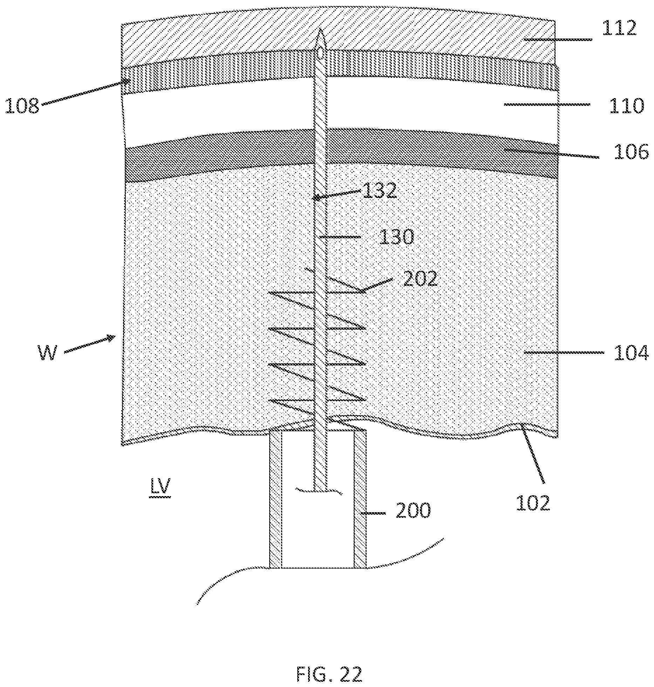

In Figure 22, the piercing device 130 is extended through the endocardium 102, the myocardium 104, the epicardium 106, and the pericardium 108, and into the external parietal pericardium tissue 112. The piercing device 130, however, has extended past the pericardial cavity 110 where the anchor 122 (See Figure 27) is to be positioned in this exemplary embodiment. Thus, the piercing device 130 in Figure 22 is shown in an over-inserted position in this exemplary embodiment.

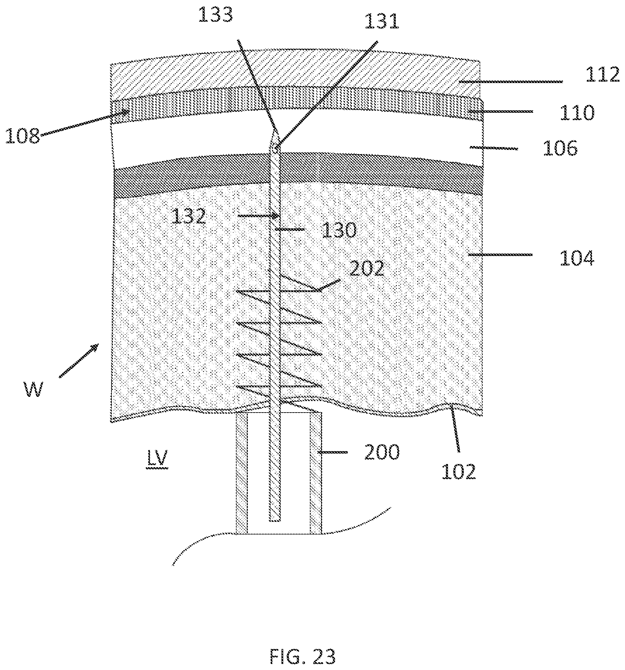

In Figure 23, the piercing device 130 is extended through the endocardium 102, the myocardium 104, the epicardium 106, and into the pericardial cavity 110, such that the opening 131 and the distal end 133 are within the pericardial cavity 110. Thus, the piercing device 130 in Figure 23 is properly positioned for deploying the anchor 122 (See Figure 27) into the pericardial cavity 110.

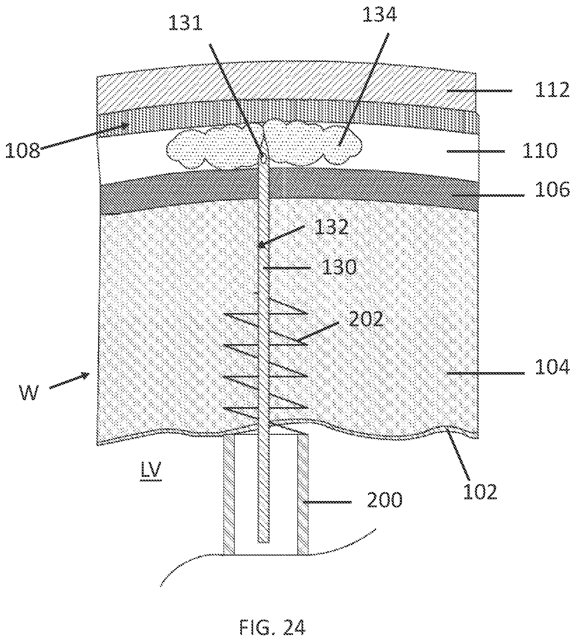

In one exemplary embodiment, proper positioning of the piercing device 130 is optionally verified. The proper positioning of the piercing device 130 can be verified in a wide variety of different ways. For example, the positioning of the piercing device can be visually determined by providing the piercing device with a marker, such as a radio-opaque marker, by discharging a material into cavity, such as the pericardial cavity, by sensing a pressure required to discharge fluid from the piercing device, by sensing a force required to advance the piercing device, by providing and/or sensing an electrical signal with the piercing device (e.g. the piercing device can be used to sense an ECG signal generated by the heart). In one exemplary embodiment, to verify that the piercing device 130 is properly positioned for deploying the anchor 122 into the pericardial cavity 110, a dye 134, or other detectable fluid, is delivered through the piercing device 130 and injected into the pericardial cavity 110, as shown in Figure 24. The dye 134 can be detected by any suitable technique such as X-ray or other imaging techniques, to verify that the piercing device 130 is properly positioned.

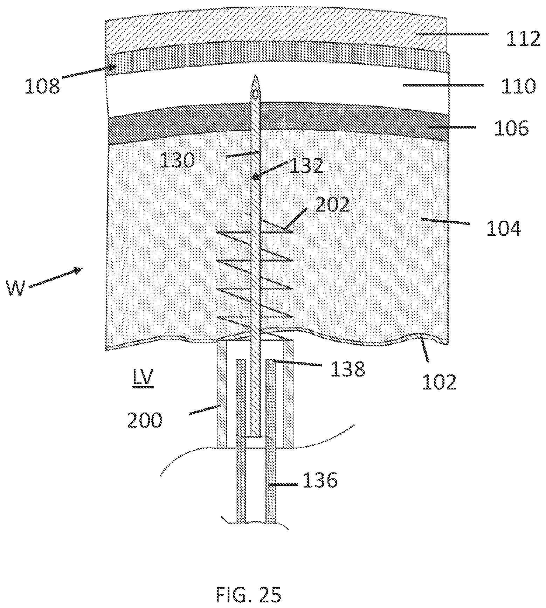

In Figure 25, the piercing device 130 is extended into the pericardial cavity 110 and the delivery catheter 136 is extended through the anchoring catheter 200 such that the distal end 138 of the delivery catheter 136 is adjacent the endocardium 102.

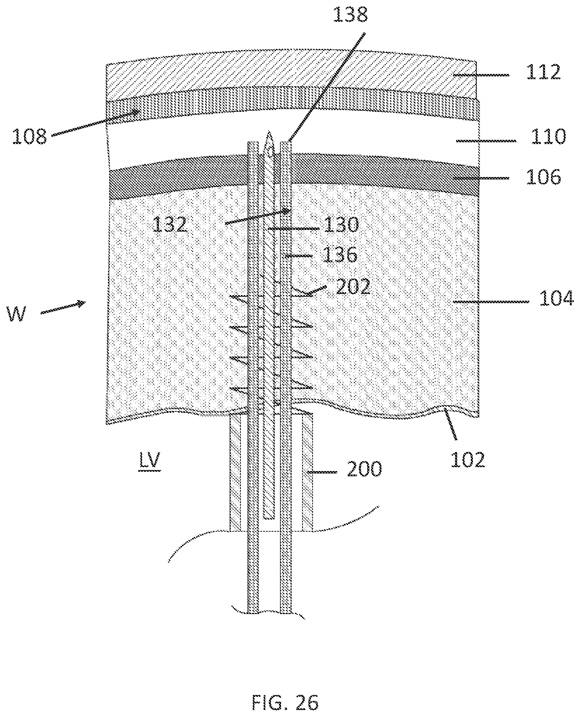

In Figure 26, the delivery catheter 136 is extended through the passage 132 such that the distal end 138 of the delivery catheter 136 is positioned within the pericardial cavity 110. As shown in Figure 27, with the distal end 138 of the delivery catheter 136 is positioned within the pericardial cavity 110, the device 120 for remodeling the shape of a heart wall W can be delivered through the delivery catheter 136. In particular, the anchor 122 can be extended out of the distal end of the 138 of the delivery catheter 136 and into the pericardial cavity 110 while the attached line 124 extends through the delivery catheter 136 in the passage 132.

As shown by comparing Figures 26-28, the delivery catheter 136 and the piercing device 130 can be removed by withdrawing them from the passage 132 and from the heart chamber. The device 120 is left deployed in the heart wall W with the anchor 122 within the pericardial cavity 110 and the line 124 extending through the epicardium 106, the myocardium 104, and the endocardium 102 and into the heart chamber (e.g., the left ventricle LV).

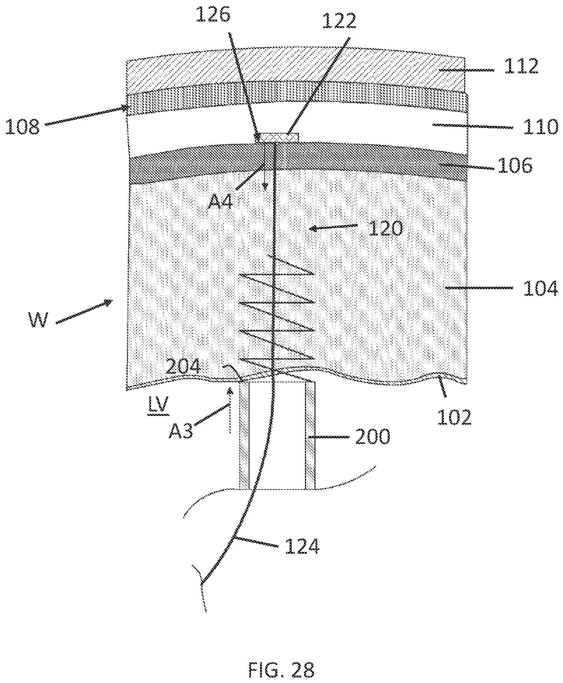

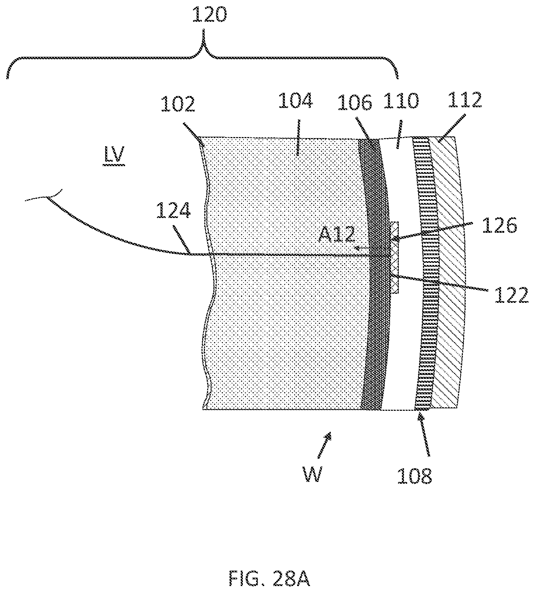

To seat the anchor 122 against the heart wall W, the anchoring catheter 200 can optionally remain attached to the heart wall W and the distal end 204 can be pushed against the heart wall W, as shown by arrow A3. At the same time, the line 124 can be placed in tension by pulling the line 124 in an inward direction toward the heart chamber as shown by the arrow A4 in Figure 28.

To remodel the heart wall W, the anchoring catheter 200 is removed (if it had not previously been removed) The line 124 can be placed in tension by pulling the line 124 in an inward direction toward the heart chamber as shown by the arrow A4 in Figure 28. As will be described in more detail below, two or more devices can be deployed, coupled, and pull toward one another to pull the heart wall inward and remodel the shape of the heart wall(s). The anchor 122 will engage and press in on the outward facing surface 126, which in the illustrated embodiment is the epicardium 106. The anchor 122 in its deployed state is too large to fit through the passage 132 formed by the piercing device 130. Thus, once the anchoring catheter 200 is removed, further tensioning of the line 124 can pull the heart wall W inward toward the heart chamber (e.g., the left ventricle LV).

In one exemplary embodiment, the device 120 is configured to prevent blood leakage through the passage 132 into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 closes immediately or substantially immediately upon withdrawal of the piercing device 130 and/or the delivery catheter 136.

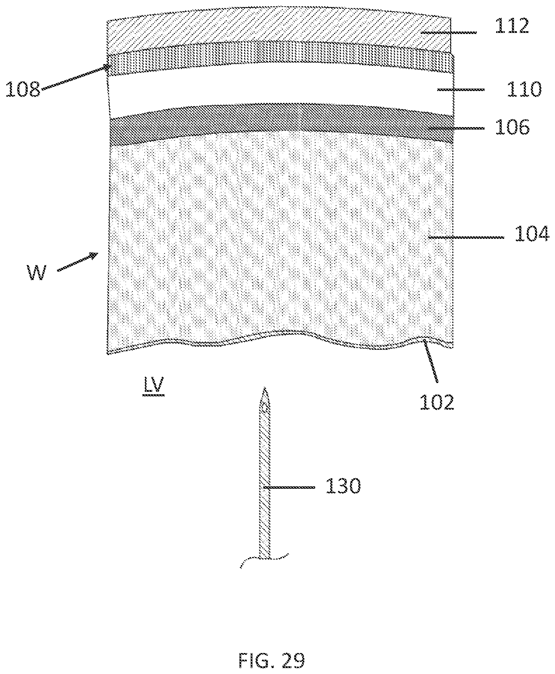

Referring to Figures 29-34, an exemplary embodiment of a device 120 (Figure 34) for remodeling the shape of a heart wall W, and an exemplary embodiment of a system and method for delivering and deploying the device 120 against an exterior surface of the heart H is illustrated. Referring to Figure 29, in one exemplary embodiment, deployment of the device 120 includes delivering the piercing device 130 into an internal chamber (e.g. left ventricle LV) of the heart H and adjacent the heart wall W.

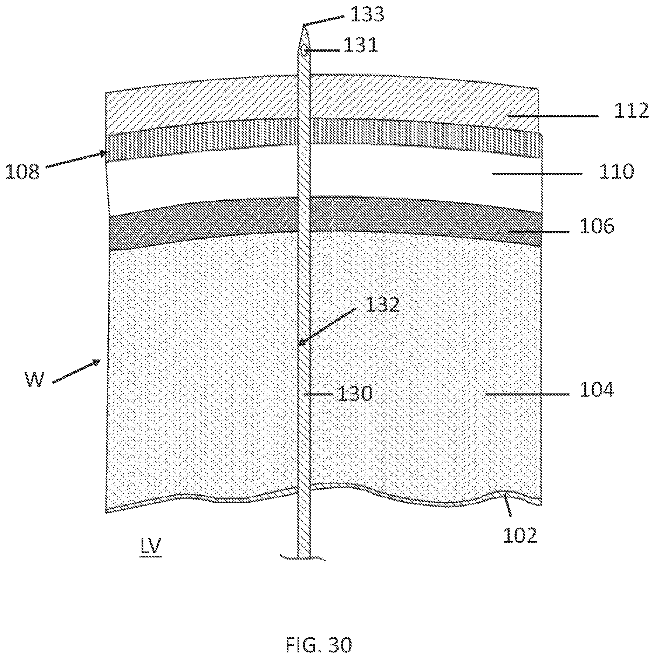

In Figure 30, the piercing device 130 is extended into the heart wall W through the endocardium 102, the myocardium 104, the epicardium 106, the pericardium 108, and the external parietal pericardium tissue 112 to create a passage 132 through the heart wall W, such that the opening 131 and the distal end 133 of the piercing device 130 are external to the heart H. Thus, the piercing device 130 in Figure 30 is properly positioned for deploying the anchor 122 in this exemplary embodiment.

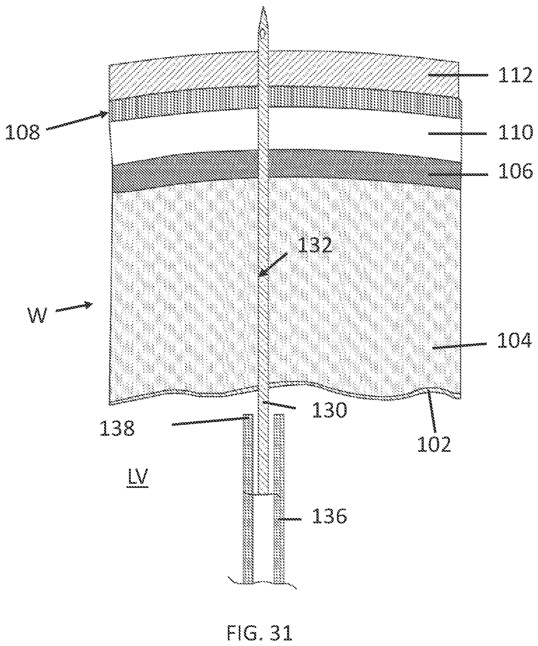

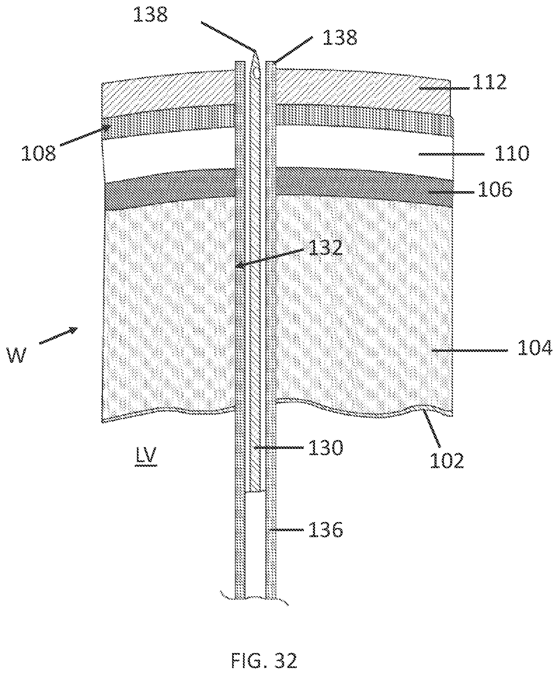

In Figure 31, the piercing device 130 is extended though the heart wall W and the delivery catheter 136 is positioned within the heart chamber (e.g. the left ventricle LV) such that the distal end 138 of the delivery catheter 136 is adjacent the endocardium 102. In Figure 32, the delivery catheter 136 is extended through the passage 132 such that the distal end 138 of the delivery catheter 136 is external the heart H, such as adjacent the distal end 133 of the piercing device 130. In the illustrated embodiment, the delivery catheter 136 is concentric with the piercing device 130. In other embodiments, however, the delivery catheter 136 may not be concentric with the piercing device 130. In yet other embodiments, the delivery catheter 136 can be omitted. For example, the device 120 can be delivered directly through the piercing device 130, rather than through a separate catheter.

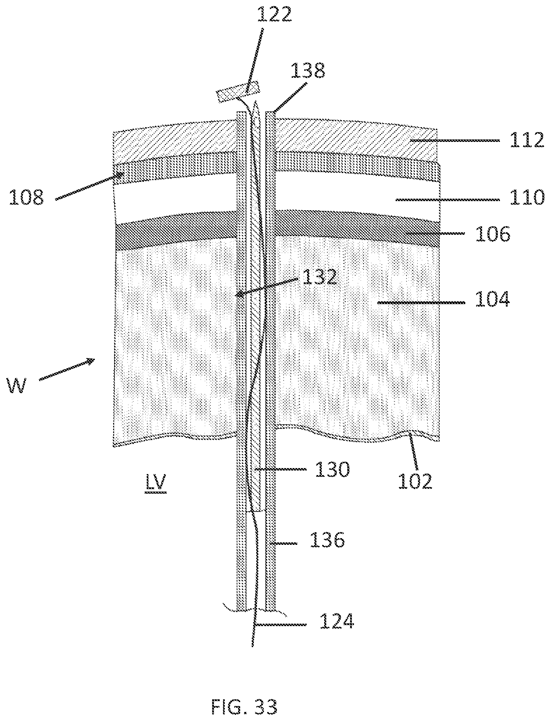

In Figure 33, the device 120 (Figure 34) for remodeling the shape of a heart wall W can be delivered through the delivery catheter 136. In particular, the anchor 122 can be extended out of the distal end of the 138 of the delivery catheter 136 external to the heart wall H while the attached line 124 extends through the delivery catheter 136 in the passage 132.

As shown in Figure 34, the delivery catheter 136 and the piercing device 130 can be removed by withdrawing them from the passage 132 and from the heart chamber. The device 120 is left deployed through heart wall W with the anchor 122 external to the heart wall W and the line 124 extending through the parietal pericardium tissue 112, the pericardium 108, the pericardial space 110, the epicardium 106, the myocardium 104, and the endocardium 102 and into the heart chamber (e.g., the left ventricle LV).

To seat the anchor 122 and to remodel the heart wall W, the line 124 can be placed in tension by pulling the line 124 in an inward direction toward the heart chamber as shown by the arrow A5 in Figure 34. As will be described in more detail below, two or more devices can be deployed, coupled, and pulled toward one another to pull the heart wall inward and remodel the shape of the heart wall(s). The anchor 122 will engage the outward facing surface 126, which in the illustrated embodiment is the exterior parietal pericardium tissue layer 112. In one exemplary embodiment, the anchor 122 presses a localized area of the pericardium 108 against the epicardium and thus pushes the myocardium 104 inward to remodel the heart wall W. The anchor 122 in its deployed state is too large to fit through the passage 132 formed by the piercing device 130. Thus, further tensioning of the line 124 can pull the heart wall W inward toward the heart chamber (e.g., the left ventricle LV).

In one exemplary embodiment, the device 120 is configured to prevent blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 and/or the hole in the pericardium closes immediately or substantially immediately upon withdrawal of the piercing device 130 and/or the delivery catheter 136.

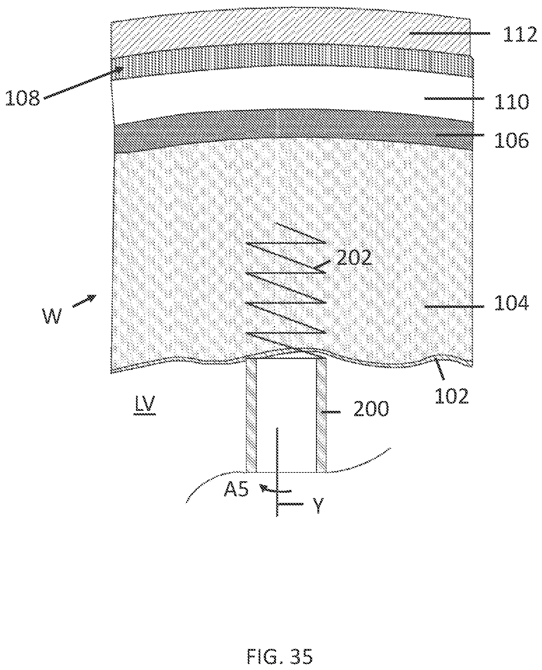

Referring to Figures 35-41, another exemplary embodiment of deployment of the device 120 for remodeling the shape of a heart wall W and system and method for delivering the device 120 against an exterior surface of the heart H is illustrated. Referring to Figure 35 deployment of the device 120 includes delivering the anchoring catheter 200 into an internal chamber (e.g. left ventricle LV) of the heart H and attaching the anchoring catheter to the heart wall W.

In the exemplary embodiment, the anchoring catheter 200 includes an anchoring device 202 attached to a distal end 204 of the anchoring catheter 200. Examples of suitable anchoring devices include, but are not limited to expandable barbs, a suction tip, such as a suction cone, and/or a corkscrew shaped tip as illustrated. The anchoring device 202 can be any device capable of temporarily attaching the anchoring catheter 200 to the heart wall W. In the illustrated embodiment, the anchoring device 202 is a wire formed into a helical shape.

In the illustrated embodiment, the anchoring catheter 200 can be attached to the heart wall W by rotating the anchoring catheter 200 about axis Y in the direction of arrow A5, as shown in Figure 35. As a result, the helical anchoring device 202 may screw into the heart wall W through the endocardium 102 and into the myocardium 104 to secure the anchoring catheter 200 to the heart wall W.

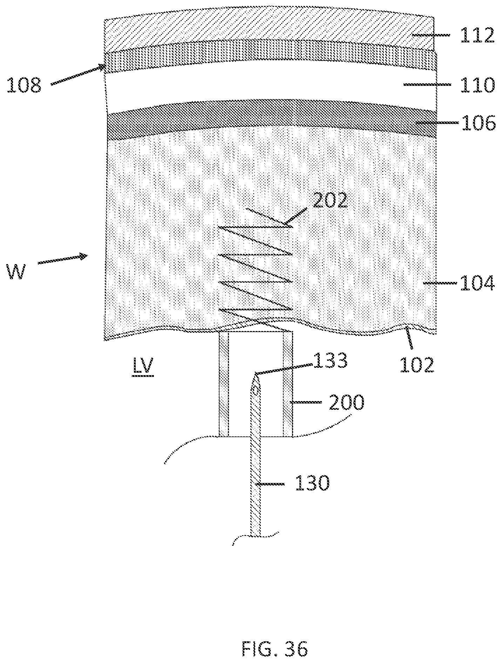

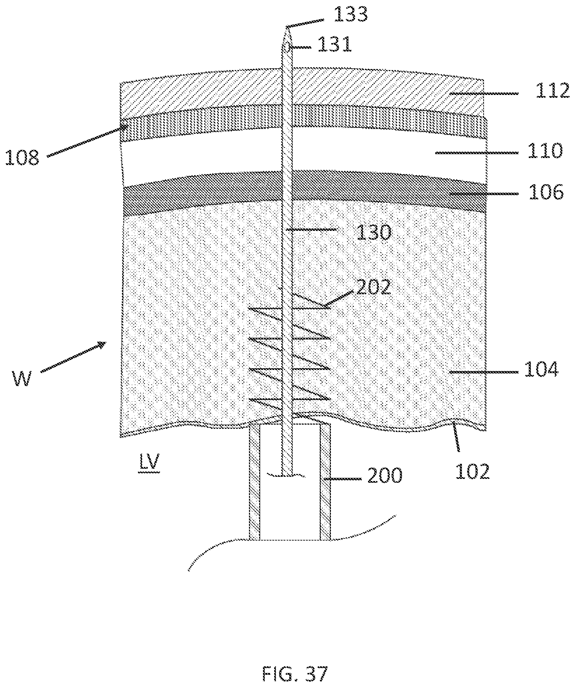

Referring to Figure 36, the piercing device 130 is delivered into an internal chamber (e.g. left ventricle LV) of the heart H via the anchoring catheter 200. The distal end 133 of the piercing device is adjacent the heart wall W. In Figure 37, the piercing device 130 is extended into the heart wall W through the endocardium 102, the myocardium 104, the epicardium 106, and the pericardium 108 to create the passage 132 through the heart wall W. The opening 131 and the distal end 133 of the piercing device 130 are external to the heart H. The piercing device 130 in Figure 37 is properly positioned for deploying the anchor 122 in this exemplary embodiment.

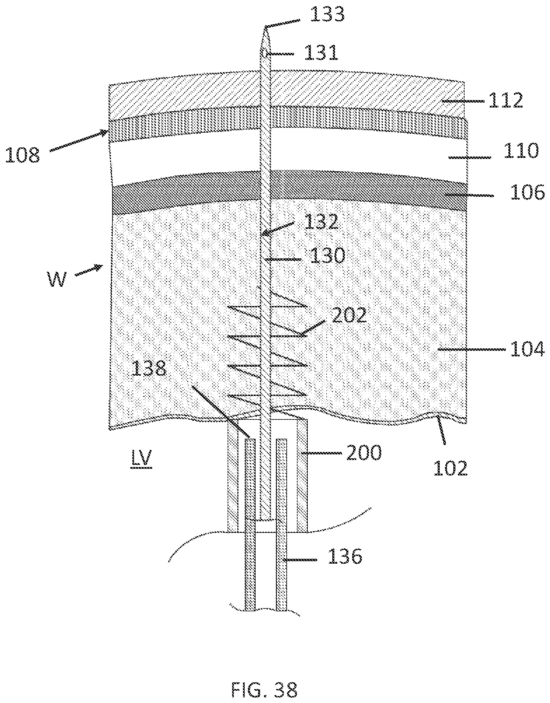

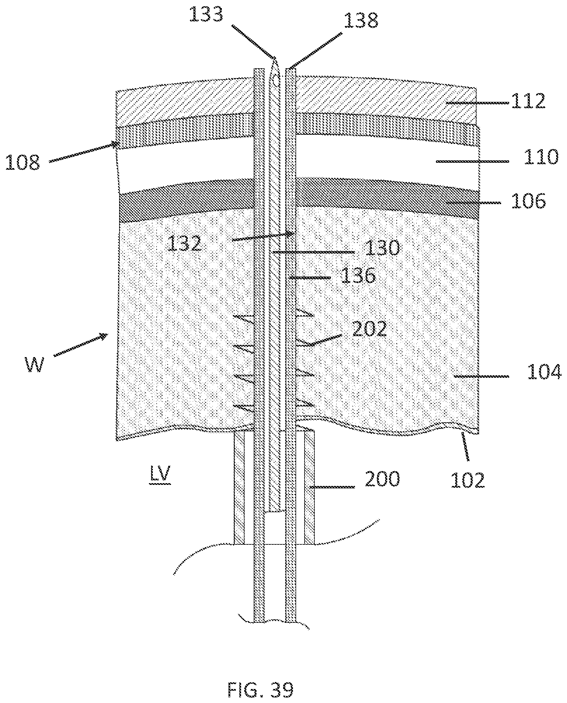

In Figure 38, the piercing device 130 is extended though the heart wall W and the delivery catheter 136 is positioned within the temporary anchoring catheter 200 such that the distal end 138 of the delivery catheter 136 is adjacent the endocardium 102. In Figure 39, the delivery catheter 136 is extended through the passage 132 such that the distal end 138 of the delivery catheter 136 is external the heart H, such as adjacent the distal end 133 of the piercing device 130.

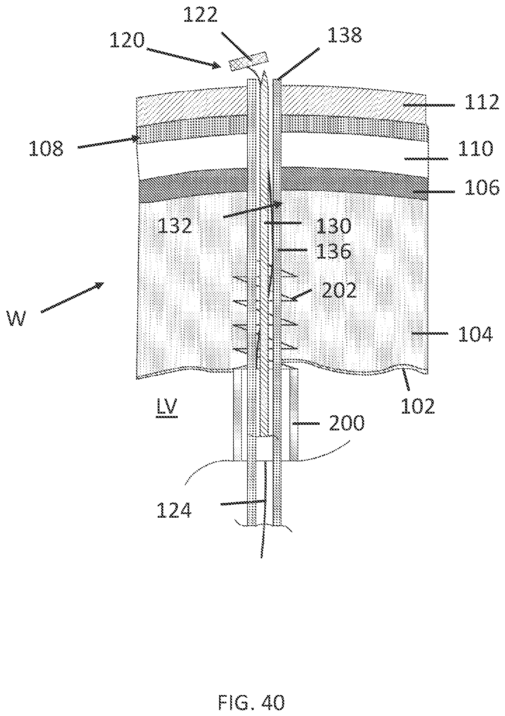

In Figure 40, the device 120 for remodeling the shape of a heart wall W can be delivered through the delivery catheter 136. In particular, the anchor 122 can be extended out of the distal end of the 138 of the delivery catheter 136 external to the heart wall H while the attached line 124 extends through the delivery catheter 136 in the passage 132.

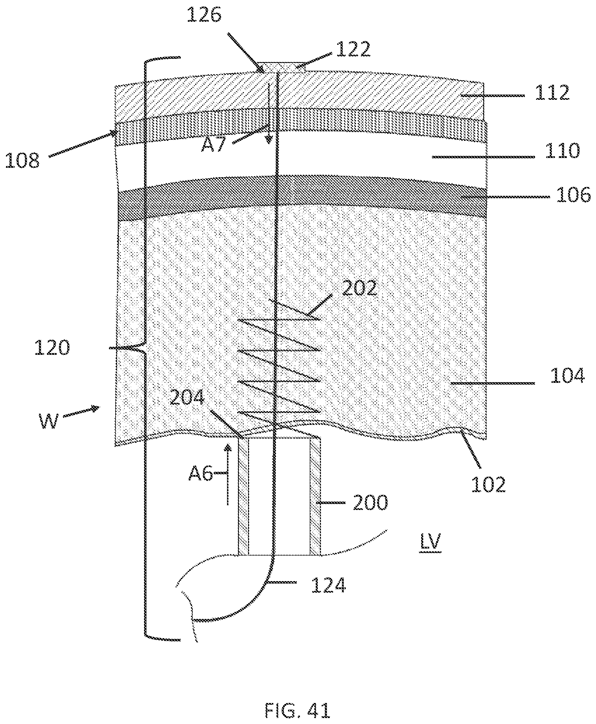

As shown in Figure 41, the delivery catheter 136 and the piercing device 130 can be removed by withdrawing them from the passage 132 and from the heart chamber. The device 120 is left deployed through heart wall W with the anchor 122 external to the heart wall W and the line 124 extending through the epicardium 106, the myocardium 104, and the endocardium 102 and into the heart chamber (e.g., the left ventricle LV).

To seat the anchor 122 firmly against the heart wall W, the anchoring catheter 200 can optionally remain attached to the heart wall W and the distal end 204 can be maintained against the heart wall W, as shown by arrow A6. At the same time, the line 124 can be placed in tension by pulling the line 124 in an inward direction toward the heart chamber as shown by the arrow A7 in Figure 41. This seating step is optional and can be omitted.

Once the anchor 122 is firmly seated, the anchoring catheter 200 is removed and the line 124 is placed in tension to remodel the heart wall. As will be described in more detail below, two or more devices can be deployed, coupled, and pulled toward one another to pull the heart wall inward and remodel the shape of the heart wall(s). The anchor 122 will engage the outward facing surface 126, which in the illustrated embodiment is the exterior parietal tissue layer 112. In one exemplary embodiment, the anchor 122 presses a localized area of the pericardium 108 against the epicardium and thus pushes the myocardium 104 inward to remodel the heart wall W. The anchor 122 in its deployed state is too large to fit through the passage 132 formed by the piercing device 130. Thus, once the anchoring catheter 200 is removed, further tensioning of the line 124 can pull the heart wall W inward toward the heart chamber (e.g., the left ventricle LV).

In one exemplary embodiment, the device 120 is configured to prevent blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 and/or the hole in the pericardium closes immediately or substantially immediately upon withdrawal of the piercing device 130 and/or the delivery catheter 136.

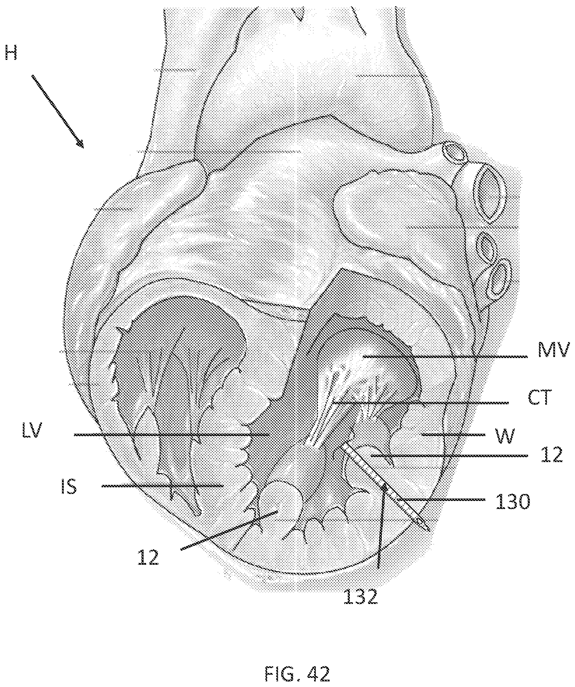

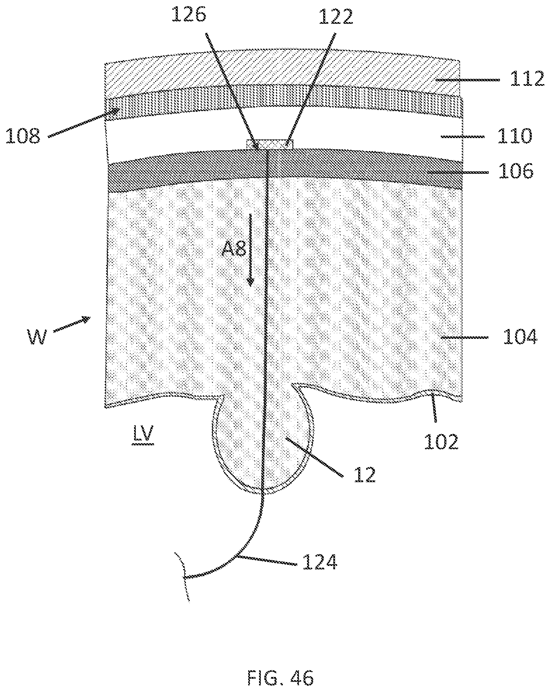

Referring to Figures 42-47, another exemplary embodiment of deployment of the device 120 for remodeling the shape of a heart wall W and system and method for delivering the device 120. The example illustrated by Figures 42-47 can deploy the anchor 122 within the pericardial cavity 110 as illustrated by Figure 46 or outside the pericardium (see, for example Figure 34 - location can also be selected to pass the line 124 through the papillary muscle). Referring to Figure 42, in one exemplary embodiment, deployment of the device 120 includes delivering the piercing device 130 into an internal chamber (e.g. left ventricle LV or right ventricle RV) of the heart H. The piercing device 130 is then extended through one of the papillary muscles 12 and through the heart wall W to create a passage 132.

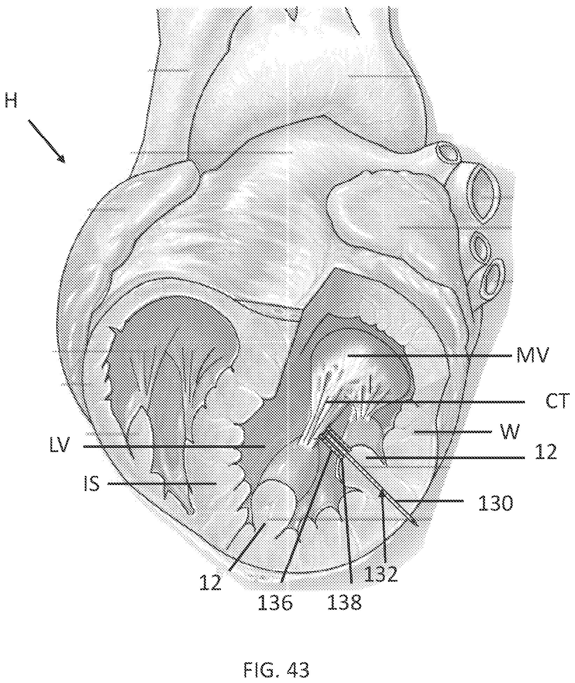

In Figure 43, the delivery catheter 136 is disposed around the piercing device 130 and positioned within the heart chamber (e.g. the left ventricle LV). The distal end 138 of the delivery catheter 136 is positioned adjacent the papillary muscle 12. In Figures 42 and 43, the pericardium 108 is not illustrated. As is noted above, in this embodiment the anchor 122 can be deployed into the pericardial space 110 or onto the outside of the pericardium.

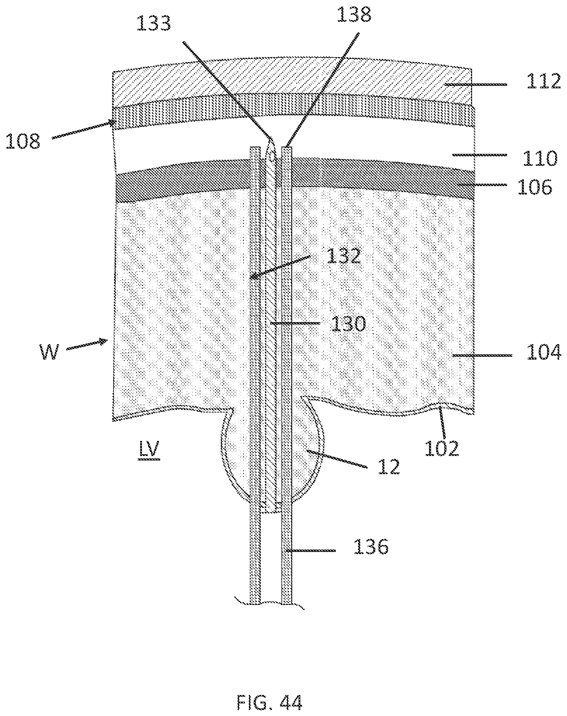

In Figure 44, the piercing device 130 is illustrated in the proper position for deploying the anchor 122 in the pericardial cavity 110. The delivery catheter 136 is extended through the passage 132 such that the distal end 138 of the delivery catheter 136 is within the pericardial cavity 110 adjacent the distal end 133 of the piercing device 130.

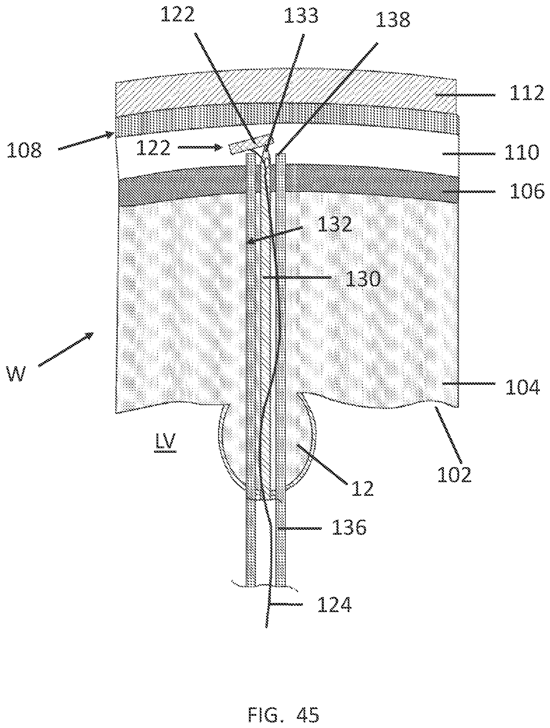

In Figure 45, the device 120 for remodeling the shape of a heart wall W can be delivered through the delivery catheter 136. In particular, the anchor 122 can be extended out of the distal end of the 138 of the delivery catheter 136 into the pericardial cavity 110 while the attached line 124 extends through the delivery catheter 136 in the passage 132 through the heart wall and the papillary muscle 12.

As shown in Figure 46, the delivery catheter 136 and the piercing device 130 can be removed by withdrawing them from the passage 132 and from the heart chamber. The device 120 is left deployed through heart wall W with the anchor 122 within the pericardial cavity 110 and the line 124 extending through the epicardium 106, the myocardium 104, and the endocardium 102 and into the heart chamber (e.g., the left ventricle LV).

To seat the anchor 122 and to remodel the heart wall W, the line 124 can be placed in tension by pulling the line 124 in an inward direction toward the heart chamber as shown by the arrow A8 in Figure 46. As will be described in more detail below, two or more devices can be deployed, coupled, and pulled toward one another to pull the heart wall inward and remodel the shape of the heart wall(s). The anchor 122 will engage and press in on the outward facing surface 126, which in the illustrated embodiment is the epicardium 106. The anchor 122 in its deployed state is too large to fit through the passage 132 formed by the piercing device 130. Thus, further tensioning of the line 124 can pull the heart wall W inward toward the heart chamber (e.g., the left ventricle LV).

In one exemplary embodiment, the device 120 is configured to prevent blood leakage through the passage 132 into the pericardial space 110. The blood leakage can be blocked in a wide variety of different ways. For example, the anchor 122 can locally pull the pericardium into contact with the epicardium to block the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 and/or the hole in the pericardium closes immediately or substantially immediately upon withdrawal of the piercing device 130 and/or the delivery catheter 136.

In one exemplary embodiment, the device 120 is configured to prevent blood leakage through the passage 132 into the pericardial space 110. The blood leakage can be blocked in a wide variety of different ways. For example, the anchor 122 can cover the hole through the epicardium, a seal separate from the anchor 122 can be placed over the hole in the epicardium, a seal can be placed over the hole in the endocardium, a portion of the anchor 122 can plug the passage 132, the line 124 can be configured to plug the passage, and/or a component disposed over the line can plug the passage. In one exemplary embodiment, the piercing device 130 and/or the delivery catheter 136 are configured such that the passage 132 closes immediately or substantially immediately upon withdrawal of the piercing device 130 and/or the delivery catheter 136.

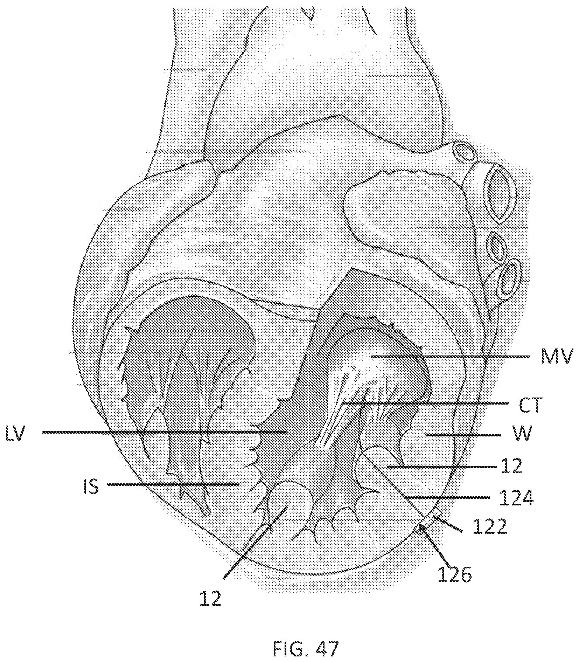

In Figure 47, the device 120 is illustrated in an installed position with the anchor 122 engaging the outward facing surface 126 and the line extending through the heart wall W and one of the papillary muscles 12 and into the left ventricle LV and through the mitral valve MV. The device illustrated by Figure 47 can be installed in any manner described herein. In the example illustrated by Figures 42-47, the device 120 is installed without and anchoring catheter 200. However, in other embodiments, the device 120 can be installed using and anchoring catheter 200 in any of the manners described herein.

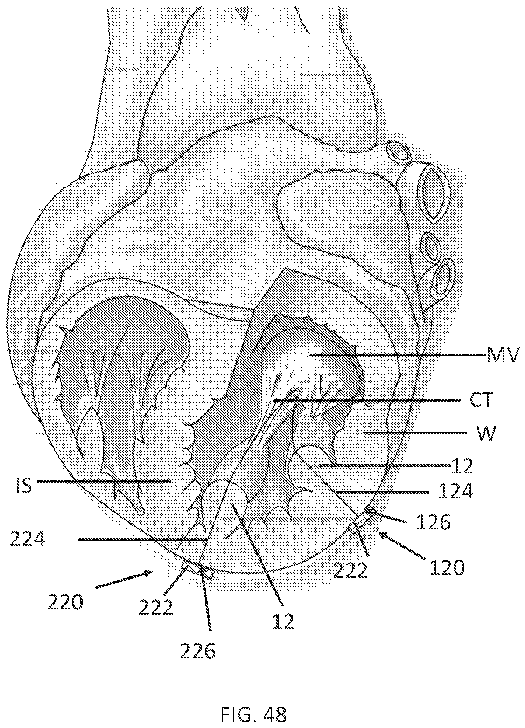

In Figure 48, along with the installed first device 120, a second device 220 is illustrated in an installed position. The second device 220 includes a second anchor 222 engaging a second outward facing surface 126 and a second line extending through the heart wall W and through another of the papillary muscles 12 and into the left ventricle LV and through the mitral valve MV. The second device 120 can be installed in any of the manners described herein. In one exemplary embodiment, the second device 220 is installed in the same manner as the first device 120.

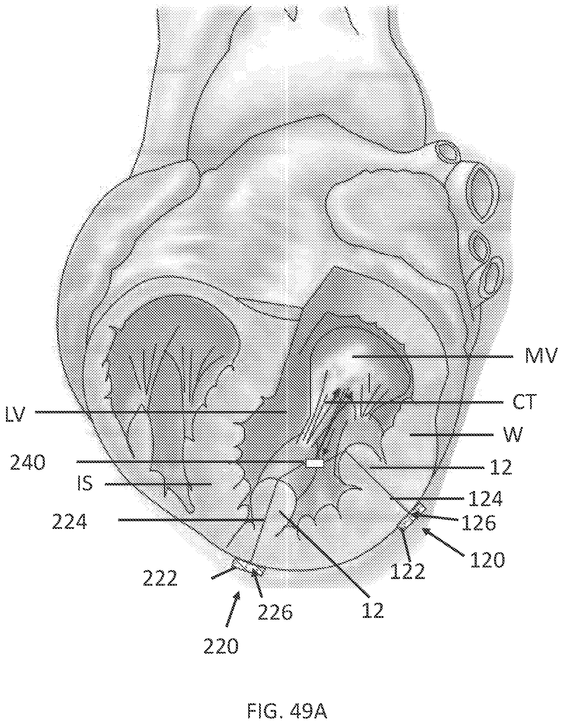

Referring to Figure 49A, in one exemplary embodiment the first and second lines are routed through a connector 240. In the example illustrated by Figure 49A, the papillary muscles 12 are pulled together or approximated by pushing or holding the position of the connector 240 as indicated by arrow 241 and pulling on the lines 124, 224 as indicated by arrows 243, 245 respectively. The distance the connector 240 is pushed as indicated by arrow 241 and the distances the lines 124, 224 are pulled controls how far the papillary muscles 12 are pulled toward one another, which in turn determines the remodeling of the heart walls.

Still referring to Figure 49A, in one exemplary embodiment, the papillary muscles 12 can be pulled toward one another in a manner that improves coaption between the leaflets of the mitral valve MV. That is, the chordae tendinea are attached to the mitral valve MV leaflets and the papillary muscles. Approximating the papillary muscles toward one another causes the chordae tendinea CT to pull the mitral valve leaflets toward one another and enhance coaption of the mitral valve leaflets. The enhanced or corrected leaflet coaption can reduce or eliminate mitral valve regurgitation.

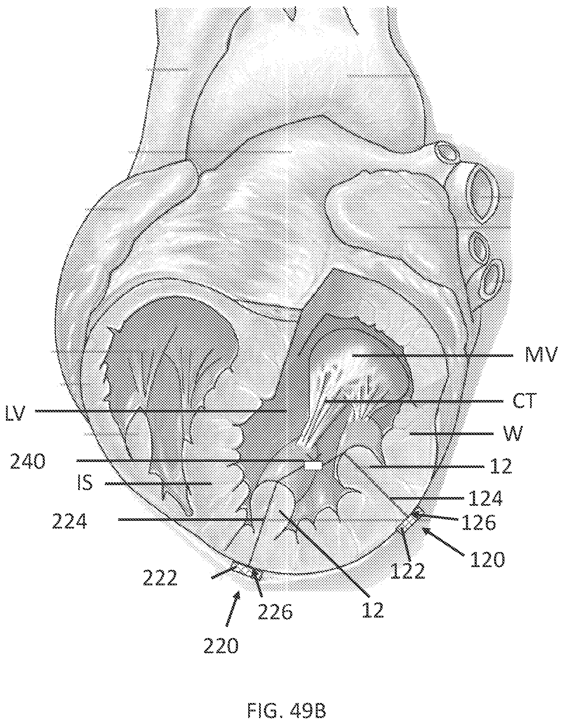

Referring to Figure 49B, after the papillary muscles 12 and the heart walls W are pulled to the desired remodeled position by the lines 124, 224, the connector 240 secures the positions of the lines in the connector. Once secured, the lines can be trimmed as shown. As such, both the device 120 and the second device 220 are shown in installed positions with the anchors 122, 222 engaging the outward facing surfaces 126, 226 and the lines 124, 224 extending through the heart wall W, through papillary muscles 12 and into the left ventricle LV. The lines 124, 224 remain in tension to pull inward to pull the heart wall W inward to remodel the shape of the heart wall W.

The lines 124, 224 can be connected together in a variety of ways. For example, the lines can be tied together or held together by a line locking device 240. The line locking device 240 can be any suitable device that can hold the lines 124, 224 together in tension such that the heart wall W is held in the remodeled position. A variety of different line locking devices 240 are shown and described below.

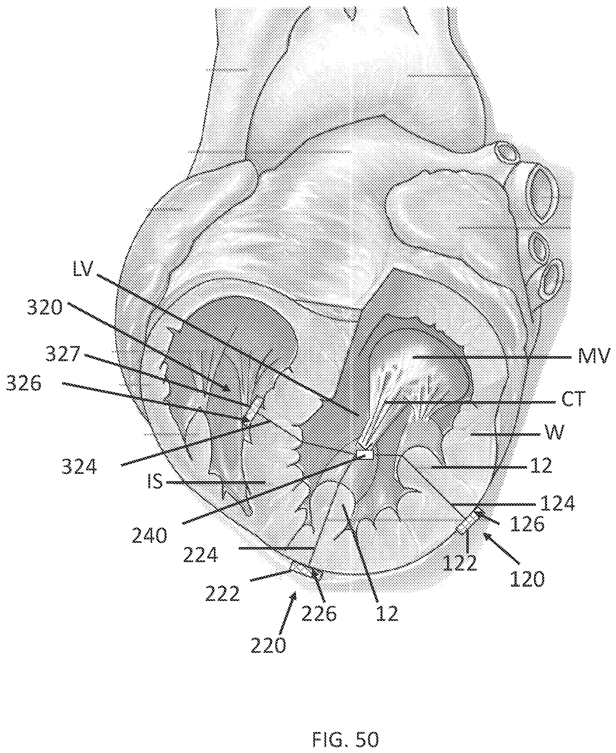

Any number of devices 120 and any number of line locking devices 240 can be used to tailor the heart wall remodeling to each individual patient. In one exemplary embodiment, two or more lines 124 are locked together in each of the locking devices. Figure 50 illustrates one example where three lines are connected together by one locking device. In some embodiments, more than one locking device is used, with at least two lines being connected together by each locking device. In the example illustrated by Figure 50, along with the installed first device 120 and a second device 220, a third device 320 is illustrated in an installed position with a third anchor 322 engaging a third outward facing surface 326 and a third line 324 extending through a heart wall and into the left ventricle LV. In the illustrated embodiment of Figure 48, the heart wall associated with the third device 320 is the interventricular septum IS. Thus, the interventricular septum IS defines the third outward facing surface 326 and the third line 324 extends through the interventricular septum IS.

As with the devices of Figure 49B, the lines 124, 224, 324 can be pulled inward to pull the heart wall W inward to remodel the shape of the heart wall W. To hold the heart wall W in the remodeled position, the lines 124, 224, 324, while in tension, can be connected within the left ventricle LV, such as for example, by the line locking device 240 or other suitable means for connecting the lines 124, 224, 324.

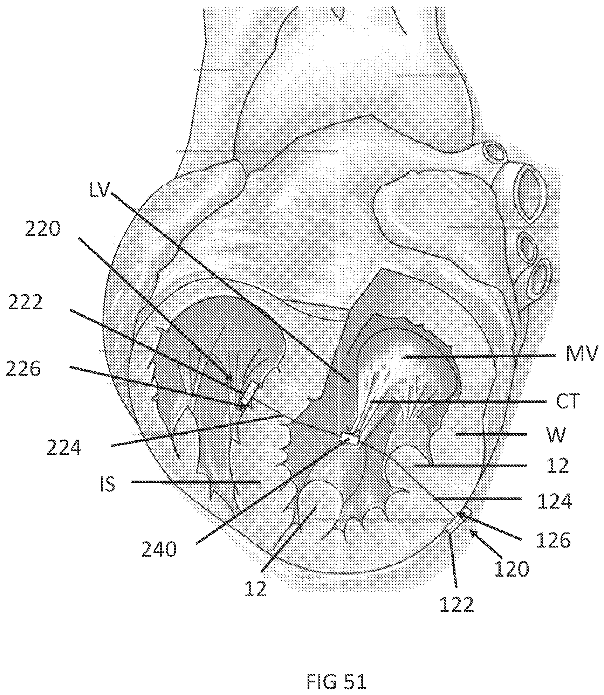

In Figure 51, both the device 120 and the second device 220 are shown in installed positions with the anchors 122, 222 engaging the outward facing surfaces 126, 226. The line 124 extends through the heart wall W, through a papillary muscle 12 and into the left ventricle LV. The second device 220 is installed such that the second anchor 222 engages the outward facing surface 226 on the interventricular septum IS and the second line 224 extends through the interventricular septum IS and into the left ventricle LV.

As with the devices of Figure 49B, the lines 124, 224, can be pulled inward to pull the heart wall W inward and to pull the intraventricular septum IS inward to remodel the shape of the heart wall W and the intraventricular septum IS. To hold the heart wall W and the intraventricular septum IS in the remodeled positions, the lines 124, 224, while in tension, can be connected within the left ventricle LV, such as for example, by the line locking device 240 or other suitable means for connecting the lines 124, 224.

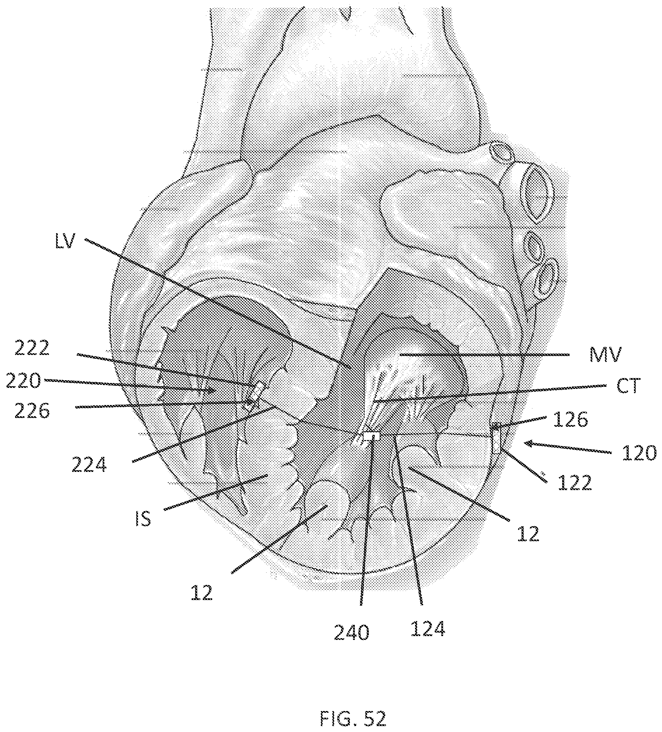

In Figure 52, both the device 120 and the second device 220 are shown in installed positions with the anchors 122, 222 engaging the outward facing surfaces 126, 226. The line 124 extends through the heart wall W and into the left ventricle LV, but not through a papillary muscle 12. The second device 220 is installed such that the second anchor 222 engages the outward facing surface 226 on the interventricular septum IS and the second line 224 extends through the interventricular septum IS and into the left ventricle LV.

As with the devices of Figure 49B, the lines 124, 224, can be pulled inward to pull the heart wall W inward to remodel the shape of the heart wall W and the intraventricular septum IS. To hold the heart wall W and the intraventricular septum IS in the remodeled position, the lines 124, 224, while in tension, can be connected within the left ventricle LV, such as for example, by the line locking device 240 or other suitable means for connecting the lines 124, 224.

The devices 120 can be used in a wide variety of different ways to remodel the heart and/or approximate the papillary muscles. A single locking device 240 and two or more devices 120 can be deployed or more than one line locking device 240 with two or more devices 120 per locking device 240 can be deployed to remodel the heart of a single patient. For example, any of the configurations illustrated by Figures 50, 51, and 52 can be used in combination on the heart of a single patient. For example, one pair of devices 120, 220 can be used to approximate the papillary muscles 12, while one or more additional pairs (or three, or four, etc.) of devices can be used to remodel the shape of the right ventricle, in the same patient. Or, in one exemplary embodiment, one pair of devices 120, 220 pulls one papillary muscle and a portion of the heart wall or intraventricular septum IS relatively toward one another and another on pair of devices 120, 220 pulls another papillary muscle and a portion of the heart wall or intraventricular septum IS relatively toward one another. In another embodiment, in one exemplary embodiment, the lines are not deployed through the papillary muscles and one pair of devices 120, 220 pulls two portions of the heart wall or intraventricular septum IS relatively toward one another and another pair of devices 120, 220 pulls two other portions of the heart wall or intraventricular septum IS relatively toward one another.

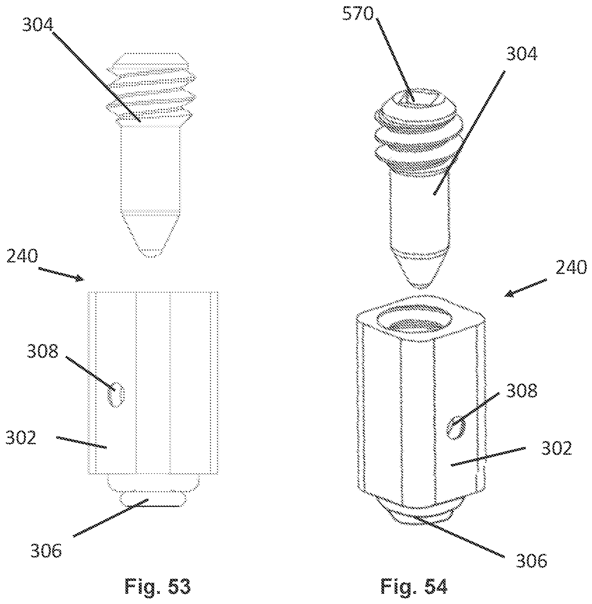

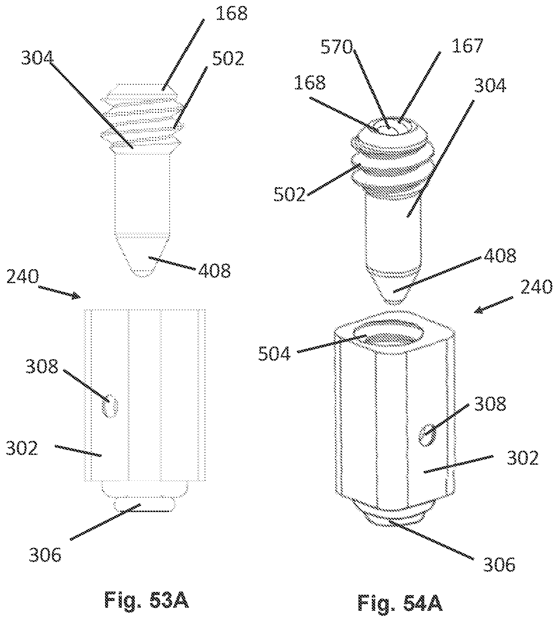

The line locking device 240 can take a wide variety of different forms. An exemplary embodiment of a line locking device 240 is illustrated in Figures 53 and 54. Figure 53 illustrates an exploded side view of the line locking device 240 which is comprised of a body 302 and a threaded insert 304. The body 302 has an input opening 306 and two outlets 308 (the second outlet is not visible in each of the figures). Figure 54 illustrates an orthogonal view of Figure 53.

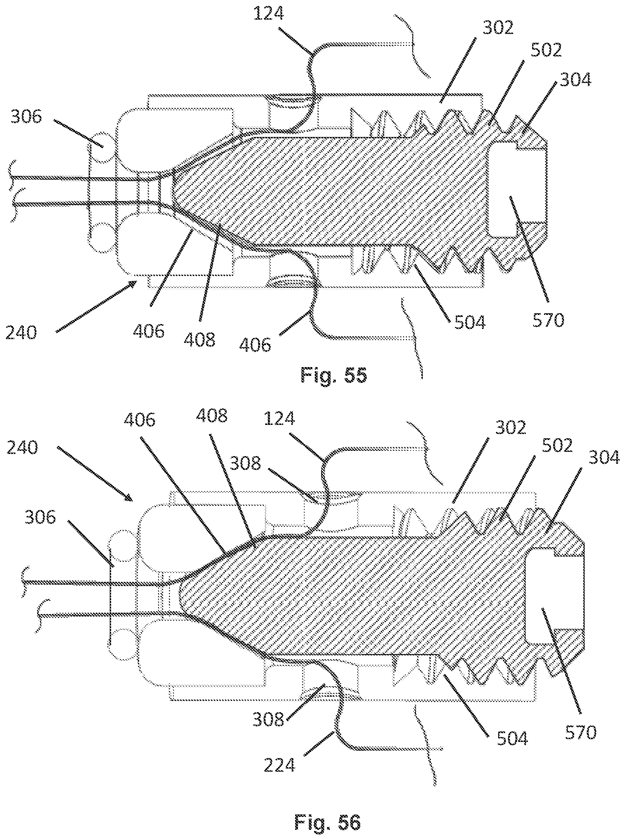

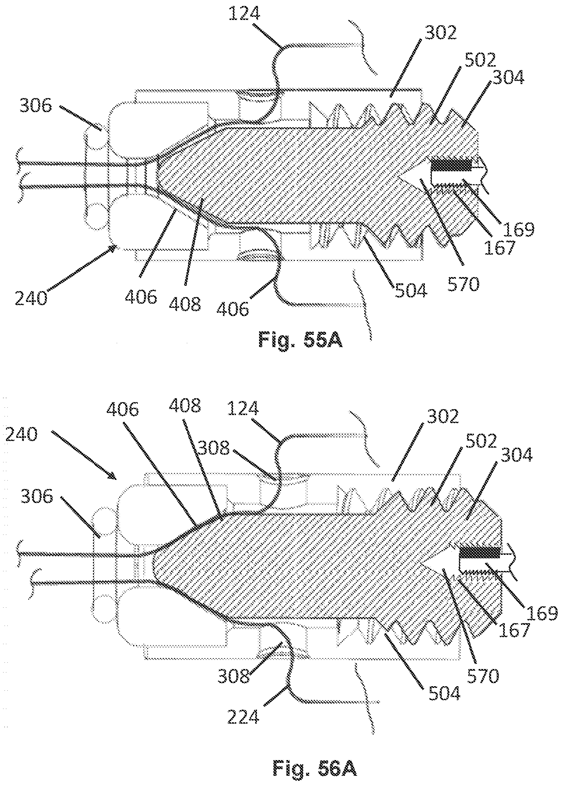

Figure 55 illustrates cross section view of the line locking device 240 of Figure 53 and 54. As illustrated, the threaded insert 304 is partially inserted into the body 302. A first line 124 and a second line 224 are shown entering the body 302 via the input opening 306 where they pass between a conical-shaped clamping surface 406 of the body 302 and a conical surface 408 of the insert 304. After passing between the two clamping surfaces, the first line 124 exits the line locking device 240 through one outlet 308 and the second line 224 extends through the other outlet 308. The lines 124, 224 can take a wide variety of different forms. For example, the lines can be sutures, wires, cables, chords, bendable rods, any combination thereof, etc.

Figure 56 illustrates the threaded insert 304 inserted into the body 302 such that the first and second lines (124 and 224) are captured or clamped between the conical-shaped clamping surface 406 of the body 302 and the conical surface 408 of the insert 304. This insertion is performed by causing the threaded insert 304 to be rotated such that the threaded insert is drawn into the body 302 by the action of threads 502 formed on the insert and mating threads 504 formed in a body chamber.

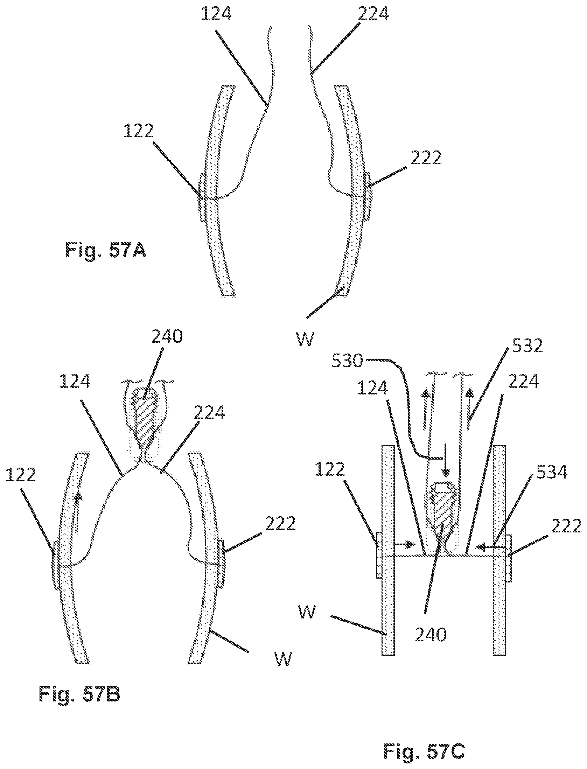

Figures 57A-57E illustrate the clamp in use. As shown, a first anchor 122 and second anchor 222 are positioned on an outside surface 126 of a heart wall W. The first anchor 122 is attached to the first line 124 and the second anchor 222 is attached to the second line 224. The first and second anchors 122, 222 and lines 124, 224 can be deployed in the manner described above.

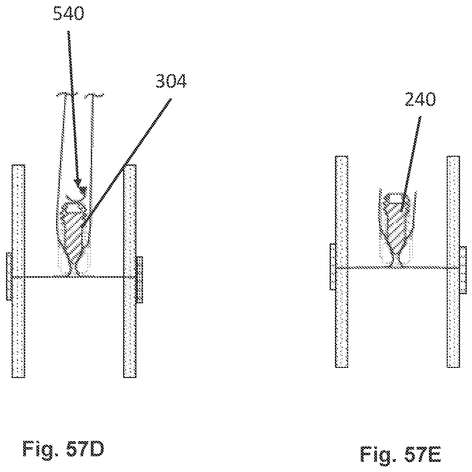

As illustrated in Figure 57B, the first and second lines 124 and 224 are positioned in a line locking device 240, such as the device illustrated by Figure 55. In Figure 57C, the line locking device 240 is pushed along the lines 124, 224 as indicated by arrow 530 and is positioned and held proximately to the first and second anchors 122 and 222. The first and second lines 124 and 224 are pulled as indicated by arrows 532 through the clamp, causing the first and second anchors to be drawn inward as shown as indicated by arrows 534. This has the effect of drawing the heart walls W together. Once the heart walls W are in the position desired, the threaded insert 304 is rotated as shown in Figure 57D at 540. This serves to secure the first and second lines 124 and 224 as illustrated in Figure 56. This securing maintains the tension on the walls W and thereby keeps the heart walls in the remodeled shape. When the first and second lines 124 and 224 are secured they can be cut as illustrated in Figure 57E.

Still referring to Figures 57A-57E, in one exemplary embodiment the line locking device 240 is configured to delivered through a catheter (i.e. transcatheter) to a ventricle LV, RV. Tension is applied to the lines 124, 224 while the line locking device is inside the ventricle by pulling on the lines from outside the patient's body (i.e. from the patient's groin, collar bone area, or chest). The line locking device 240 is locked while the line locking device is inside the ventricle by rotating (or otherwise actuating) a line locking tool or driver 550 from outside the patient's body (i.e. also from the patient's groin, collar bone area, or chest). As such, in one exemplary embodiment the operation of rotating the threaded insert 304 includes a tool or driver 550 that can position the line locking device 240 and rotate the threaded insert 304 inside a heart.

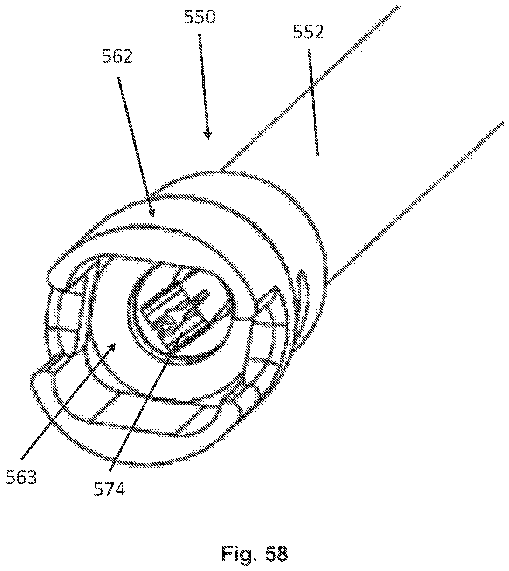

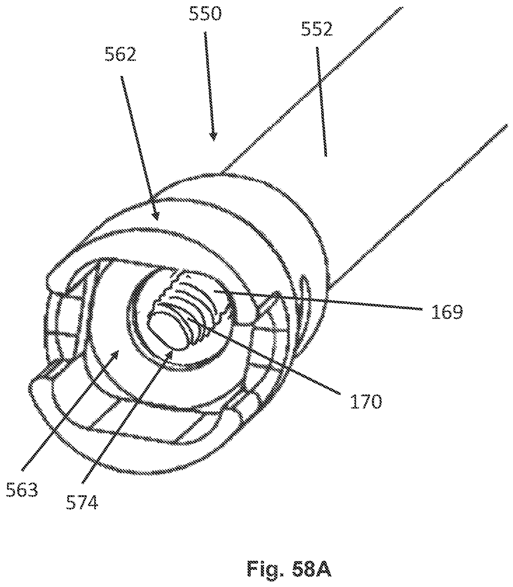

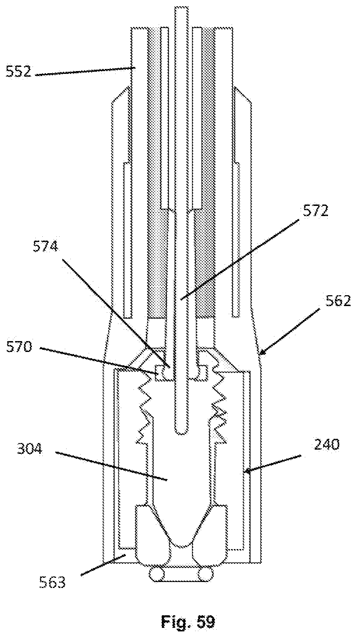

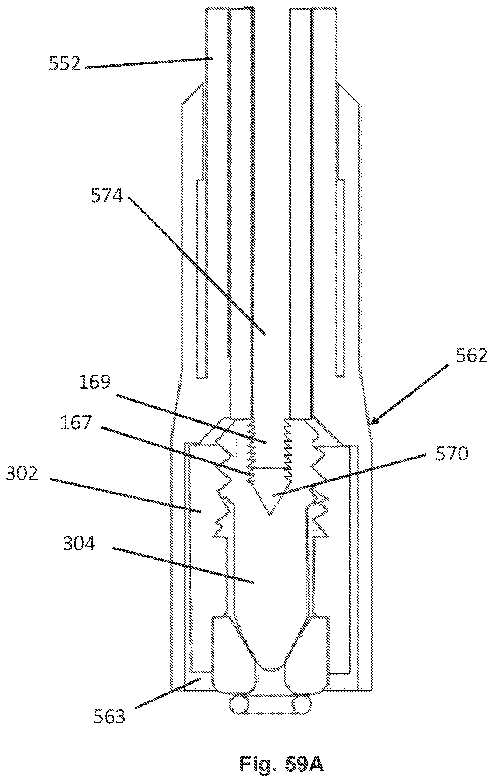

The tool or driver 550 can take a wide variety of different forms. In the example illustrated by Figures 58 and 59, the tool 550 includes a catheter 552, a socket 562, and a drive member 574. The socket 562 and drive member 574 are configured to retain the line locking device 240, while the catheter 552 moves the line locking device 240 along the lines 124, 224 through the patient's vasculature (typically through one or more guide catheters) to the patient's ventricle. The socket 562 includes a recess 563 for receiving the body 302 and is shaped to releasably engage and prevent rotation of the body 302 when the threaded insert 304 is rotated. Referring again to Figure 55, a recess 570 for engagement with the drive member 574 is located in the threaded insert 304, opposite the conical surface 408 of the insert 304. The drive member 574 is positionable in the recess 570 and can latch to and disengage from the threaded insert 304.

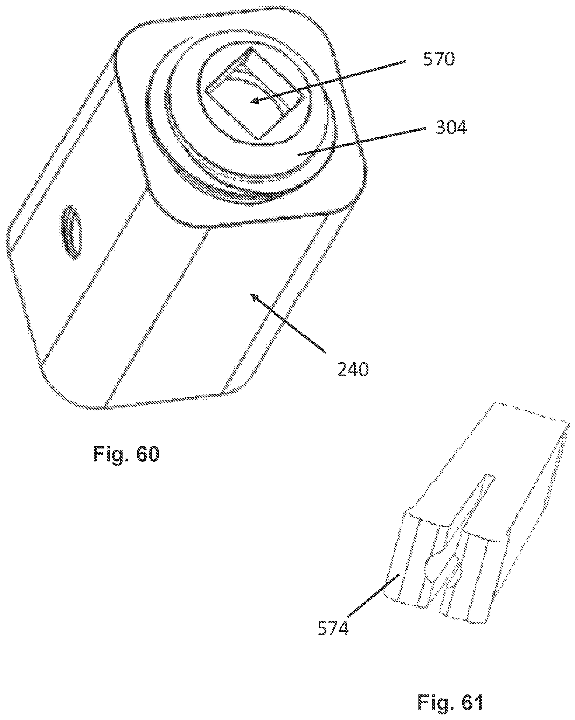

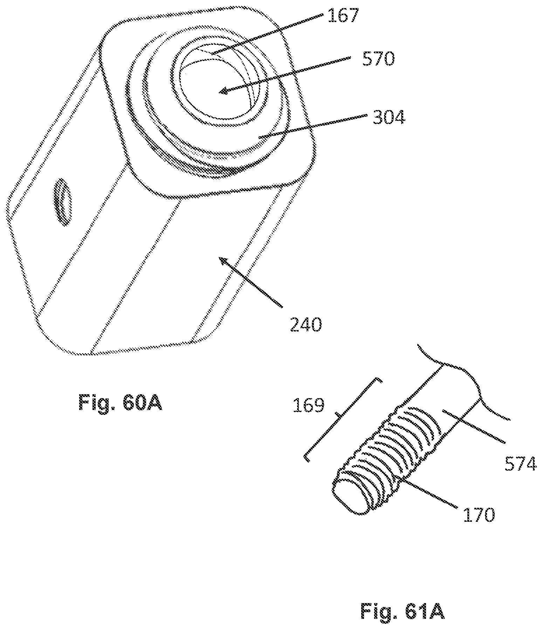

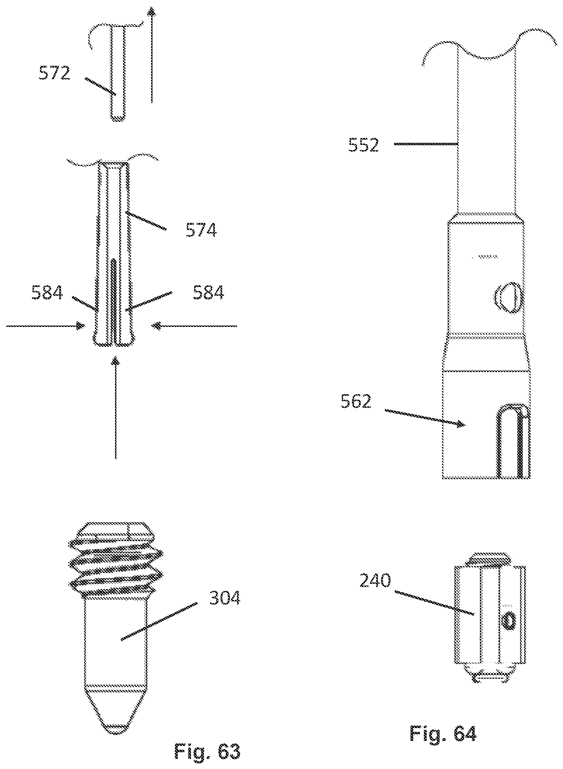

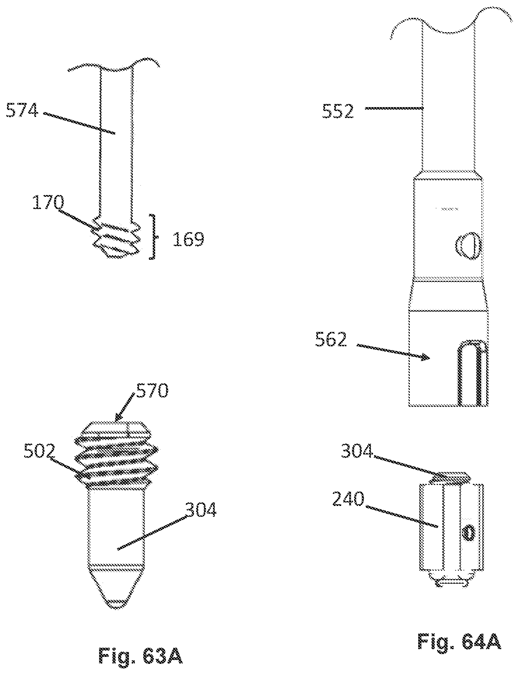

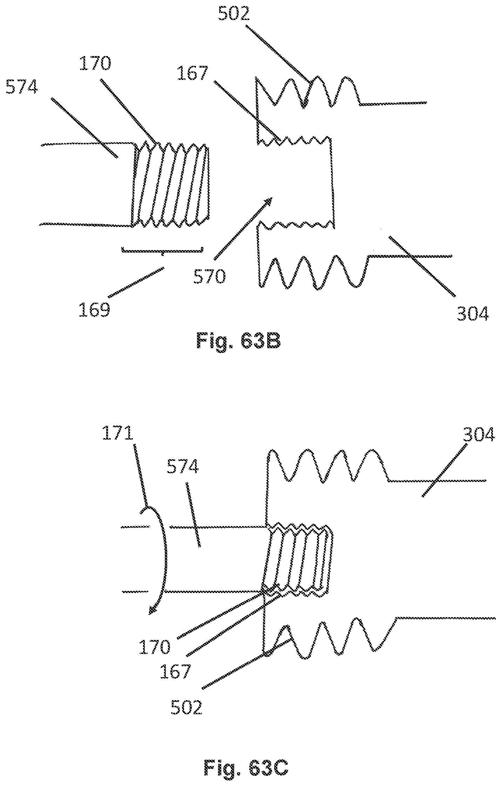

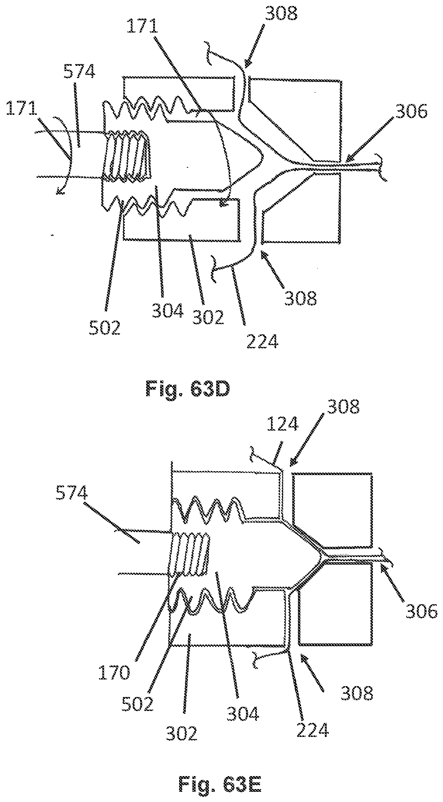

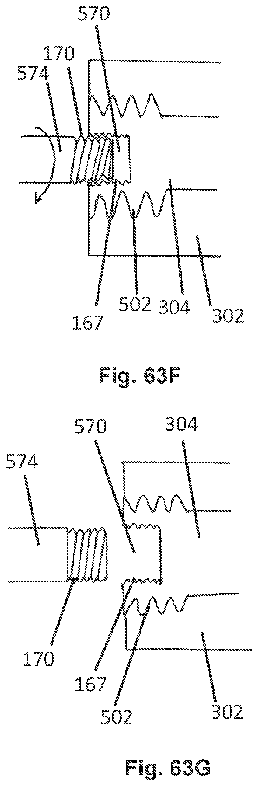

Figure 59 illustrates an operating end of the tool 550 with a line locking device 240 inserted into the recess 563 of the socket 562. In one exemplary embodiment, the drive member 574 is moveable between an engaged or expanded condition (Figure 59) and a disengaged or collapsed condition (indicated by arrows in Figure 63). The drive member 574 can be configured to engage and disengage in a wide variety of different forms. In the example illustrated by Figure 59, the driver 574 is positioned in the insert 304 recess 570 before the driver 574 is moved from the disengaged condition to the engaged condition. In the disengaged condition, a retaining rod 572 is not disposed in the end of the drive member. Once in the recess 570, the retaining rod 572 is moved to the position illustrated by Figure 59 and causes the driver 574 to expand to the engaged condition in the recess 570 of the insert 304, and thereby couple the driver 574 to the insert 304 (until the rod 572 is removed). Figure 60 shows a perspective view of the line locking device 240 which shows the recess 570 located in the insert 304. Figure 61 shows another view of the driver 574, without the retaining rod 572.

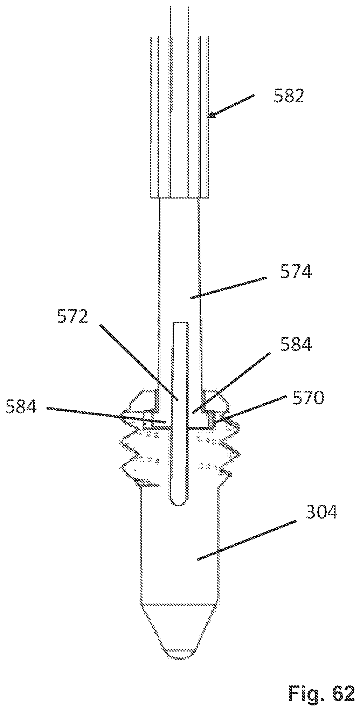

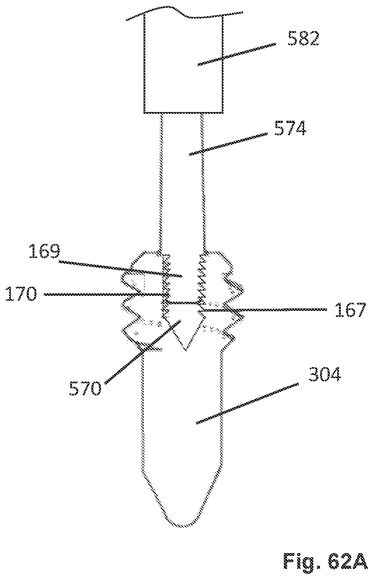

Figure 62 illustrates a view of the insert 304 which is positioned on the drive member 574 with the retaining rod 572 positioned to secure the insert 304 to the engagement device 574. A shaft 582 is attached to the driver 574, which allows for rotation of the driver 574 relative to the catheter 552 and socket 562. The illustrated driver 574 comprises fingers 584 that are forced outward by the retaining rod 572. This causes the fingers 584 to expand into the recess 570 of the insert 304 and secure the insert 304 to the engagement device 574. For clarity, this view omits the remaining components of the tool 550.

As illustrated in Figure 63, when the retaining rod 572 is withdrawn from the insert 304, the fingers 584 retract to the disengaged state. When the fingers retract to the disengaged state, the end of the driver 574 becomes smaller than the recess 570 in the insert 304, thereby releasing the insert 304. For example, the fingers 584 can be made from steel, a shape memory alloy or other resilient material spring toward the disengaged state when the retaining rod 572 is removed. Figure 64 illustrates the socket 562 in proximity to a line locking device 240 that has been released. Also illustrated is a portion of a catheter 552 attached to the socket 562.

Figures 53A, 54A, 55A, 56A, 57F-57J, 58A, 59A, 60A, 61A, 62A, 63A-63G, and 64A illustrate another exemplary embodiment of a locking device 240 having a body 302 and a threaded insert 304 for positioning within the body. In this embodiment, as in other exemplary embodiments described herein, the threaded insert can have exterior threads 502 on a proximal exterior portion of the threaded insert 304. The insert 304 can be substantially cylindrical and can have a tapered or conical distal end 408. The insert can be rotated into the body 302 by applying torque to rotate and screw the insert into the body. The exterior insert threads 502 screw into the mating threads 504 of the body 302, and the threads are aligned or mate in a first direction. This first direction can be that which requires the insert to be rotated clockwise to screw the insert into the body, i.e., the threads are right-handed. In another embodiment, the threads can be left-handed.

Referring to Figure 62A, in an exemplary embodiment, the insert can have an opening 570 with interior threads 167. A driver 574 (see Figure 59A) having external threads 170 can be rotated into the interior threads 167 of the opening 570 to secure the driver to the insert. This attachment of the driver to the insert can be done before the insert is rotated to attach the insert to the body. The threads of the driver that mate with the interior threads of the body can be aligned or mate in a second direction. In exemplary embodiments where the first direction is clockwise (right-handed), the threads of the second direction can require the driver to be rotated counterclockwise to attach the driver to the insert, i.e., left-handed threads (or vice versa). When the driver 574 is secured to the insert 304, the insert can be rotated into the body by rotation of the driver, described in detail below.

To deploy the implant, the driver 574 is secured to the insert 304. The driver is secured to the insert by rotating it in the second direction. Once the driver is secured to the insert, the driver is rotated in the first direction to rotate the insert into the body. This rotation in the first direction twists the insert into the body 302 of the device 240 so that the insert and body are secured together by being screwed together. Once the threads are secured between the insert and the body, the driver can continue to be rotated in the first direction. Then, the distal end of the driver will unscrew from the insert so that the driver can be removed. In particular, the rotation of the driver in the first direction when secured to the insert twists the insert into the body. Once the insert is fully twisted into the body, the driver which is still turning in the first direction, causes the torsional load to increase until it reaches a torsional preload value. Once the torsional preload value is reached, the insert will stop rotating with the driver. Continued application of the rotational force on the driver will cause the torque to increase past the threshold value, which will cause the distal end of the driver to begin to unscrew from the internal threads of the insert. The torque then drops when the driver begins to rotate with respect to the insert. Continued rotation of the driver in this direction causes the driver to detach from the insert, so that the delivery tools (driver, catheter) can be removed.

The line locking device 240 and the threaded insert 304 for positioning within the body 302 can take a wide variety of different forms. An exemplary embodiment of a line locking device 240 is illustrated in Figures 53A and 54A. Figure 53A illustrates an exploded side view of the line locking device 240 which is comprised of a body 302 and a threaded insert 304. The body 302 has an input opening 306 and two outlets 308 (the second outlet is not visible in each of the figures). Figure 54A illustrates an orthogonal view of Figure 53A. The threaded insert 304 can have a threaded opening 570 at the top (proximal) end 168 of the insert 304. The threaded insert can have exterior threads 502 in a first orientation, for example, right-handed threads, which align with the threads 504 in the body. The interior threads of this same insert can be left-handed threads. (The opposite can also be true; the exterior threads 502 can be left-handed with right-handed interior threads in the opening 570.) The driver 574 can be a threaded rod with threads that fit into the opening 570 having left-handed threads.

Figures 55A and 56A illustrate a cross section view of the line locking device 240 of Figures 53A and 54A. As illustrated in Figure 55A, the threaded insert 304 is partially inserted into the body 302. The threaded distal end 169 of driver 574 is secured into the threaded opening 570 of the insert 304 (the threaded distal end 169 is shown smaller than the threaded opening 570 to illustrate bit features. However, in most exemplary embodiments these two features will mate and/or be substantially the same size). A first line 124 and a second line 224 are shown entering the body 302 via the input opening 306 where they pass between a conical-shaped clamping surface 406 of the body 302 and a conical surface 408 of the insert 304. After passing between the two clamping surfaces, the first line 124 exits the line locking device 240 through one outlet 308 and the second line 224 extends through the other outlet 308. The lines 124, 224 can take a wide variety of different forms. For example, the lines can be sutures, wires, cables, chords, bendable rods, or any combination thereof.

Figure 56A illustrates the threaded insert 304 inserted into the body 302 such that the first and second lines (124 and 224) are captured and/or clamped between the conical-shaped clamping surface 406 of the body 302 and the conical surface 408 of the insert 304. This insertion is performed by causing the threaded insert 304 to be rotated such that the threaded insert is drawn into the body 302 by the action of threads 502 formed on the insert and mating threads 504 formed in a body chamber. In Figure 56A, the driver 574 with a threaded distal end 169 remains inserted into the opening 570 of the insert 304.

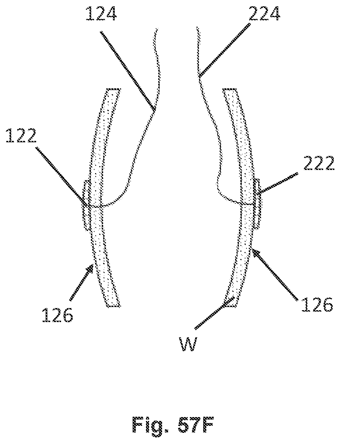

Figures 57F-57J illustrate the clamp in use. As shown, a first anchor 122 and second anchor 222 are positioned on an outside surface 126 of a heart wall W. The first anchor 122 is attached to the first line 124 and the second anchor 222 is attached to the second line 224. The first and second anchors 122, 222 and lines 124, 224 can be deployed in the manner described above.

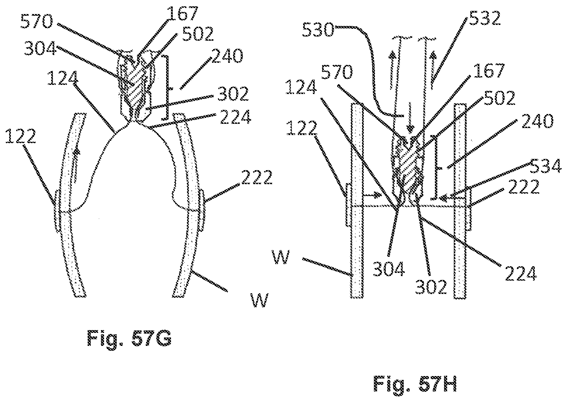

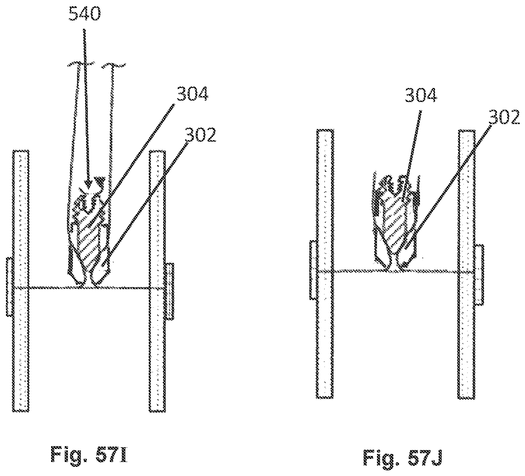

As illustrated in Figure 57G, the first and second lines 124 and 224 are positioned in a line locking device 240, such as the device illustrated by Figure 55A. In Figure 57H, the line locking device 240 is pushed along the lines 124, 224 as indicated by arrow 530 and is positioned and held proximately to the first and second anchors 122 and 222. The first and second lines 124 and 224 are pulled as indicated by arrows 532 through the clamp, causing the first and second anchors to be drawn inward as shown as indicated by arrows 534. This has the effect of drawing the heart walls W inward. Once the heart walls W are in the position desired, the threaded insert 304 is rotated as shown in Figure 57I at 540. This serves to secure the first and second lines 124 and 224 as illustrated in Figure 56A. This securing maintains the tension on the walls W and thereby keeps the heart walls in the remodeled shape. When the first and second lines 124 and 224 are secured they can be cut as illustrated in Figure 57J.

Still referring to Figures 57F-57J, in one exemplary embodiment the line locking device 240 is configured to delivered through a catheter (i.e. transcatheter) to a ventricle LV, RV. Tension is applied to the lines 124, 224 while the line locking device is inside the ventricle by pulling on the lines from outside the patient's body (i.e. from the patient's groin, collar bone area, or chest). The line locking device 240 is locked while the line locking device is inside the ventricle by rotating (or otherwise actuating) a line locking tool or driver 550 from outside the patient's body (i.e. also from the patient's groin, collar bone area, or chest). As such, in one exemplary embodiment the operation of rotating the threaded insert 304 includes a tool or driver 550 that can position the line locking device 240 and rotate the threaded insert 304 inside a heart.

The tool or driver 550 can take a wide variety of different forms. In the example illustrated by Figures 58A and 59A, the tool 550 includes a catheter 552, a socket 562, and a drive member 574. The socket 562 and drive member 574 are configured to retain the line locking device 240, while the catheter 552 moves the line locking device 240 along the lines 124, 224 through the patient's vasculature (typically through one or more guide catheters) to the patient's ventricle. The socket 562 includes a recess 563 for receiving the body 302 and is shaped to releasably engage and prevent rotation of the body 302 when the threaded insert 304 is rotated. Referring again to Figure 55A, a recess 570 for engagement with the drive member 574 is located in the threaded insert 304, opposite the conical surface 408 of the insert 304. The drive member 574 with a threaded distal end 169 can mate to and disengage from the threaded insert 304 by mating threads 170 of the driver with the interior threads 167 of the recess 570 of the insert 304.

Figure 59A illustrates an operating end of the tool 550 with a line locking device 240 inserted into the recess 563 of the socket 562. In one exemplary embodiment, the drive member 574 is moveable between an engaged condition (FIG. 59A) and a disengaged condition. The drive member 574 can be configured to engage and disengage in a wide variety of different forms. In the example illustrated by FIG. 59A, the driver 574 is threaded within the threaded opening 570 of the insert 304. Figure 60A shows a perspective view of the line locking device 240 which shows the threaded insert 304 located in the body 302 of the line locking device 240. Figure 61A shows a view of the threaded distal end 169 of the driver 574.

Figure 62A illustrates a view of the insert 304 which is threaded onto the drive member 574 A shaft 582 is attached to the driver 574, which allows for rotation of the driver 574 relative to the catheter 552 and socket 562. The illustrated driver 574 comprises a threaded distal end 169. The threads 170 of the distal end 169 mate with the threads 502 in the recess 540 of the insert 304 to secure the driver to the insert, so that continued rotation of the driver 574 can rotate the insert 304 into the body 302. For clarity, this view omits the remaining components of the tool 550.

As illustrated in Figure 63A, after the insert 304 bottoms out in the body the continued rotation of the driver 574 in the same direction of rotation that was used to secure the insert 304 into the body 302, causes the driver to unscrew from the interior threads of the cavity 570 of insert 304. Figure 64A illustrates the socket 562 in proximity to a line locking device 240 that has been released. Also illustrated is a portion of a catheter 552 attached to the socket 562.

Referring now to Figures 63B and 63C, schematics of insert 304 and the driver 574 engaging with each other are illustrated. In Figure 63B, the insert 304 has an opening 570 with interior threads 167. The driver 574 has a distal end 169 with threads 170 that are oriented to mate with the interior threads 167 of the insert. The threads 170 at the distal end of the driver can be left-handed threads. In the same exemplary embodiment, the interior threads 167 of the insert 304 are left-handed threads, and the exterior threads 502 of the insert 304 that mate with the body 302 are right-handed threads. In another exemplary embodiment, all the threads are the opposite hand of those just described. In Figure 63C, the driver has been rotated in a first direction, as indicated by arrow 171 such that the threads are torqued to a preload threshold value, and the driver is engaged with the insert.

Referring now to Figures 63D and 63E, schematics of the insert being deployed into the body 302 of the device 240 are illustrated. In Figure 63D, the insert 304 is deployed into the body 302 by rotation of the insert 304 with the driver 574 to rotate the external threads 502. Torque is applied to the driver 574 at the catheter handle (not pictured), and the torsion translates through the system to the insert, as indicated by arrows 171. The torque increases throughout the system as the insert is further rotated into the body and the insert engages the body 302 and/or the lines 124. In Figure 63D, the first line 124 and second line 224 are threaded through the body 302 and can be pulled to remodel the heart walls. In Figure 63E, the insert 304 is fully deployed into the body 302, and the first line 124 and second line 224 are each secured between the conical end 408 of the insert 304 and the body 302. Continued rotation from this position causes the insert 304 to reach or exceed the torsional preload value necessary to secure the lines 124. The insert is about to stop rotating upon application of any more torque from the driver. Further, in this position, the driver is about to start rotating relative to the insert once its torsional preload within the insert is surpassed.

Referring now to Figures 63F and 63G, schematics of the driver 574 disengaging from the insert 304 are illustrated. In Figure 63F, torque is applied to the driver in the same direction that it was applied in to secure the insert 304 to the body 302. The torque can be applied in the same direction as before because the external insert threads 502 and interior insert threads 167 are threaded in opposite directions. The driver begins to unscrew and therefore disengage from the insert in Figure 63F. In Figure 63G, the driver is fully unthreaded and separated from the insert and can be removed from the implanted device 240 by being pulled in a proximal direction through the catheter.

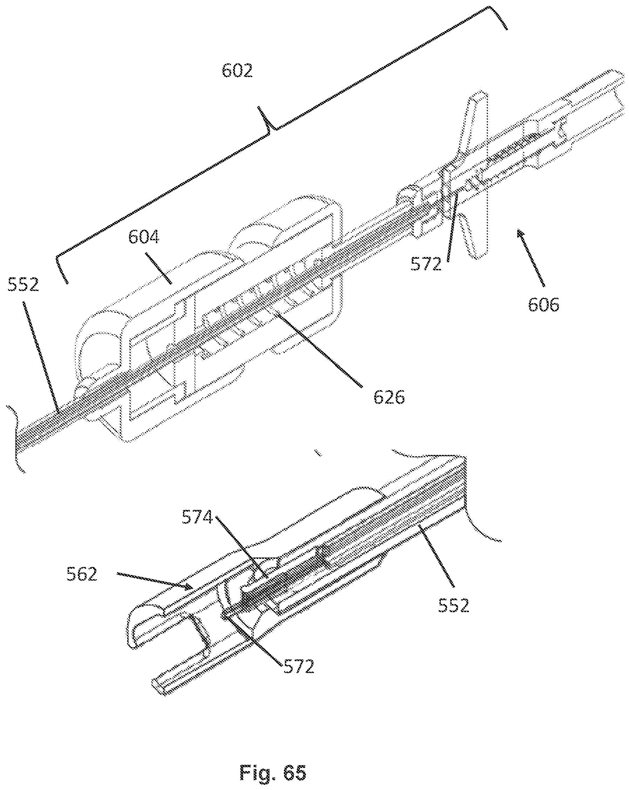

The tool 550 can be operated in a wide variety of different ways to lock the lines 124, 224 with the line lock device 240. Figure 65 illustrates one exemplary embodiment of a handle 602 for operating the tool 550 described above. The handle 602 can operate the tool 550 from outside the patient's body, while the socket 562 and driver 574 are in the patient's ventricle. The socket 562 is shown slightly enlarged relative to the handle 602 for clarity. Additionally, for clarity, a portion of the catheter 552 between the handle 602 and the socket 562 is also not shown. One of ordinary skill in the art will understand that the shaft or catheter 552 can be of sufficient length to allow the socket 562 to be positioned inside a patient's heart while allowing the operating handle 602 to be outside the patient's body, with the catheter 552 extending through the patient's vasculature.

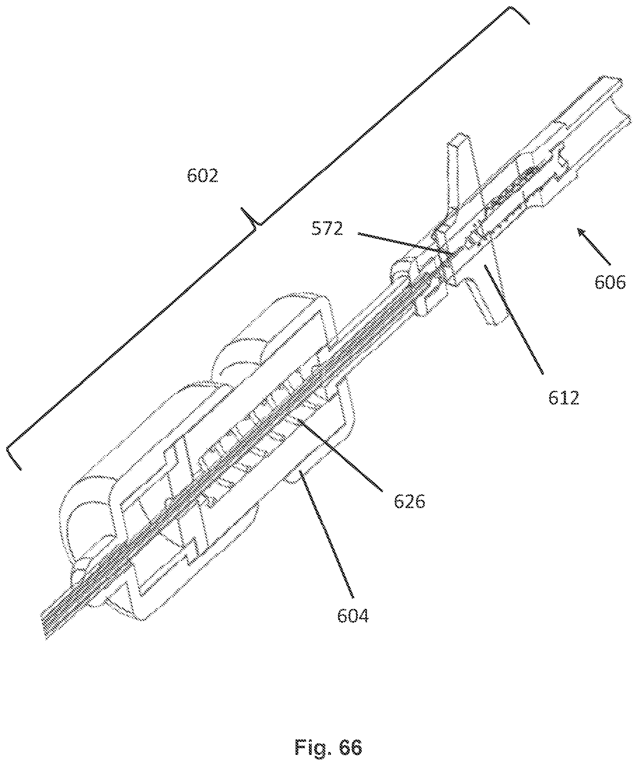

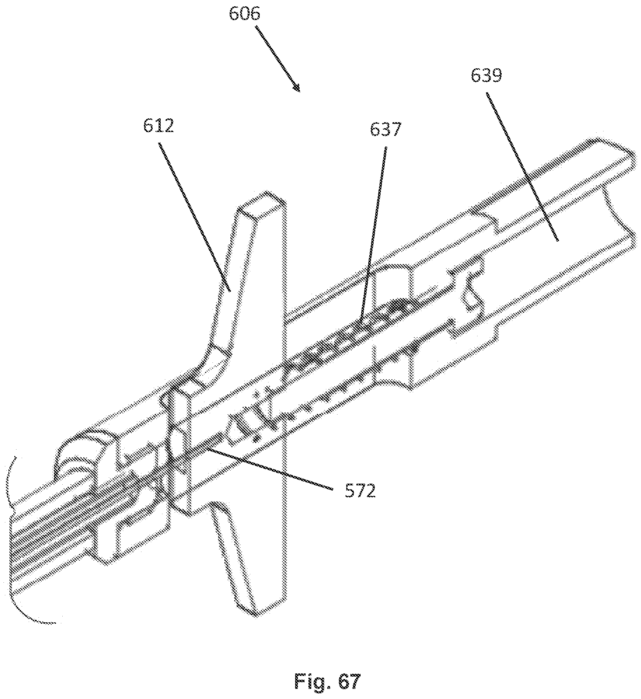

As illustrated, the operating handle 602 comprises a grip 604 and an engagement handle 606. Figure 66 shows a detailed view of the operating handle 602 including the grip 604, the engagement handle 606 and a release lever or control 612. Figure 67 provides an enlarged view of the engagement handle 606. The release lever or control 612 can take a wide variety of different forms. For example, the control 612 can be a lever, a button, a trigger, etc. In the illustrated embodiment, the release control 612 has a wing shape for ease of operation and is connected to an end of the retaining rod 572.

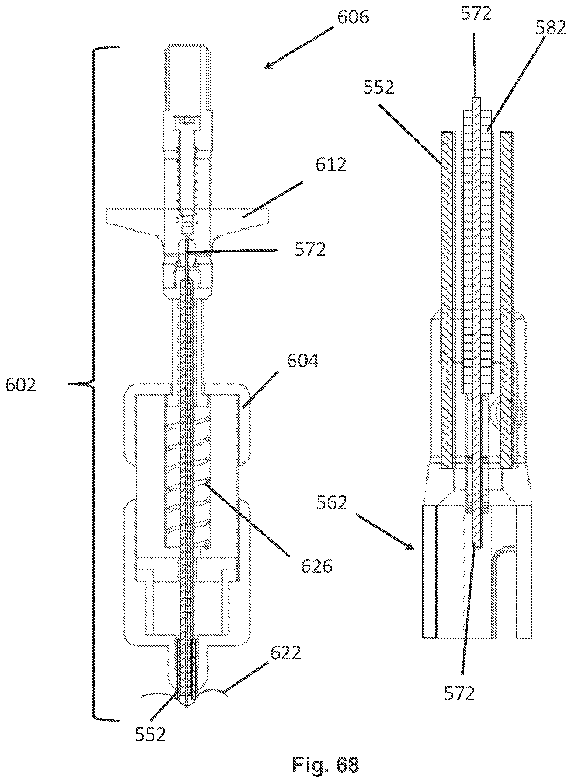

Figure 68 illustrates a cut-away view of the socket 562 and the operating handle 602. As shown the retaining rod 572 passes from the release lever 612 through the catheter 552 to the socket 562. The shaft can pass into the body of a patient around location 622 where it can be threaded through a vein or artery to position the line locking device 240. As is illustrated, an inner driver shaft 582 passes through the catheter 552 from the engagement handle 606 to an area near the socket 562. As will be illustrated, this shaft 582 functions to move the line locking device 240 further into the socket 562 and to rotate the drive member 574. This takes place when a user pushes the engagement handle 606 relative to the grip 604 and rotates the grip. A spring 626 positioned within the grip 604 resists this pushing motion and rotational movement and biases the engagement handle 606 and the connected drive member 574 toward a retracted position.

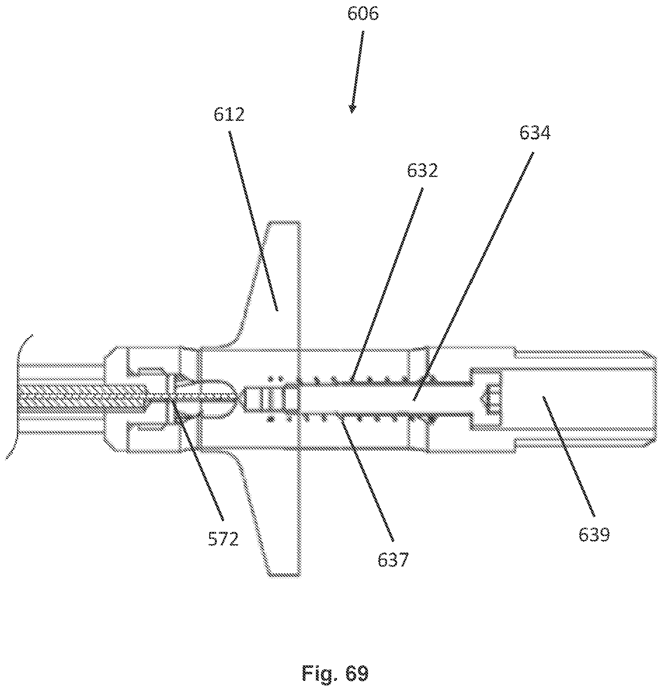

As illustrated in Figure 69, a second spring 632 positions the release control 612 such that the retaining rod extends from the engagement device 574 (not shown). This position secures the line locking device 240 to the engagement device 574 located at the end of the inner shaft 582 opposite the engagement handle 606. In an exemplary embodiment, a positioning shaft 634 serves to keep the release lever 612 aligned as the release lever moves between the engaged and disengaged positions. The positioning shaft 634 is connected to the release control 612 at an end 635 and slides within bores 637, 639.

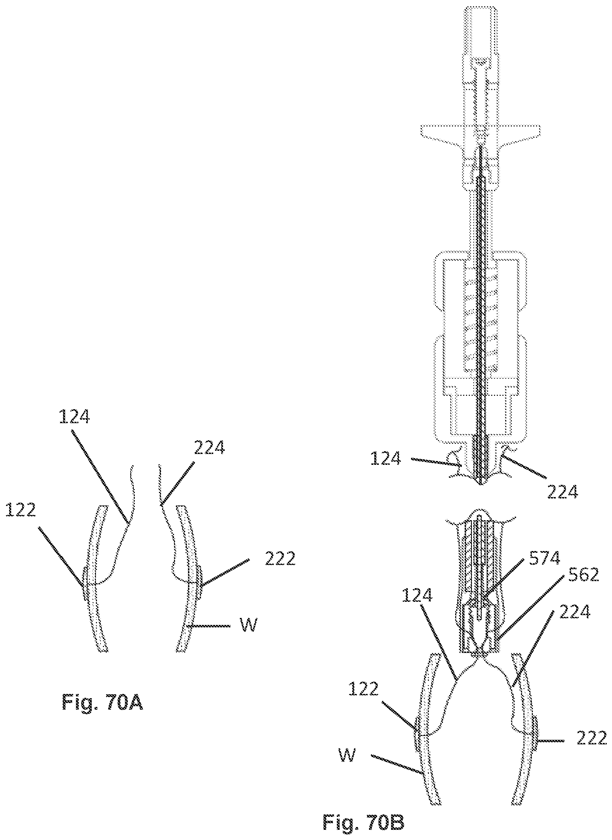

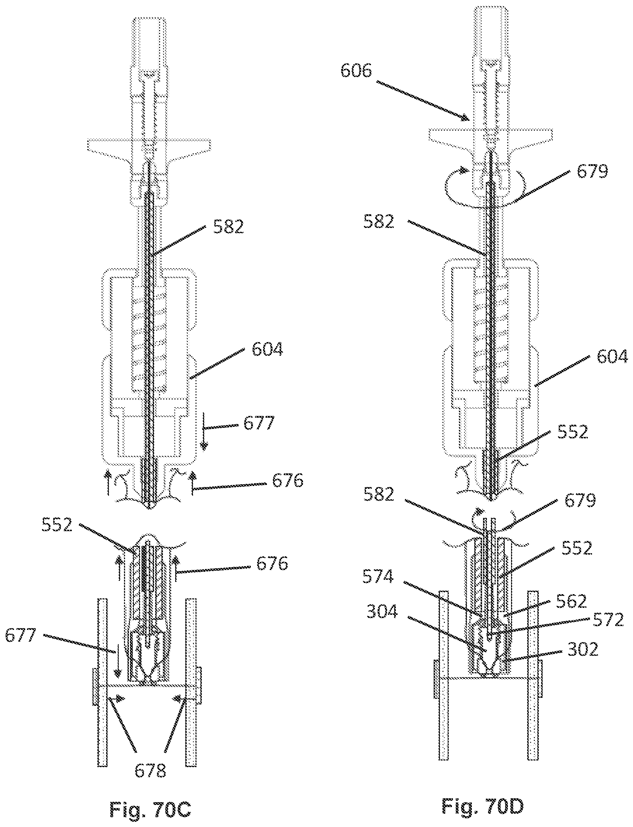

Referring to Figure 70A, in an exemplary embodiment, during use, the first anchor 122 and second anchor 222 are positioned on an outer surface of the heart wall W with a first line 124 and a second line 224 attached and threaded thru the heart wall W. The lines (124 and 224) are threaded through the line locking device 240 as illustrated in Figure 55. This threading through the locking device 240 is done external to the patient's body. The line locking device 240 is secured inside the socket 562 to the driver 574 as shown in Figure 70B. The socket 562 is inserted into a patient and threaded through the vein or artery through a guide catheter as the lines (124 and 224) are held in place outside the patient. The line locking device 240 is positioned adjacent to the first and second anchor (122 and 222) using the socket 562. Referring to Figure 70C, when this positioning is done, the first and second lines (124 and 224) are pulled as indicated by arrows 676 while the socket is maintained in position or advanced as indicated by arrows 677. This pulling 676 of the lines 124, 224 and maintaining or advancing 677 of the line locking device 240 causes the first anchor 122 and second anchor 222 to be drawn inward 678 as first illustrated in Figure 70C,

After the first anchor 122 and second anchor 222 are drawn inward 678, the line locking device 240 is secured as illustrated and described in Figures 56 and 57D. This is performed by rotating as indicated by arrow 679 the engagement handle 606 relative to the grip 604. This rotation causes the shaft 582 to rotate 579 relative to the catheter 552, which in turn causes the driver 574 to rotate relative to the socket 562. The rotation of the driver 574 relative to the socket 562 causes the threaded insert 304 to rotate and axially advance in the body 302. As such, the engagement threaded insert 304 and the body 302 secure the first and second lines (124 and 224) as illustrated in Figure 56.

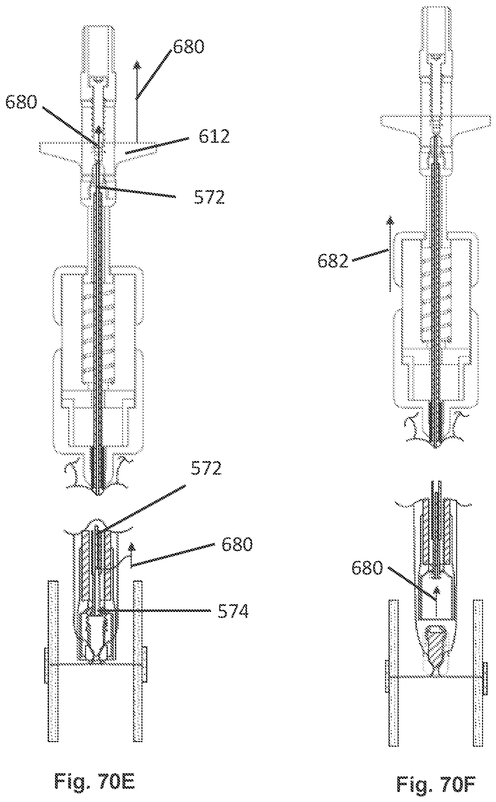

Referring to Figure 70E, after the first and second lines (124 and 224) are secured by the line locking device 240, the engagement device 574 is released from the threaded insert 304 portion of the line locking device 240 by retracting the retaining rod 572 by pulling the release control 612 rearward as illustrated by arrow 680. This rearward pulling pulls 680 the driver shaft or rod 572 out of the end of the driver 574. This allows the driver 574 to spring back to its retracted state, which is smaller than the recess 570 (See Figure 60), releasing the driver 574 from the insert 304.

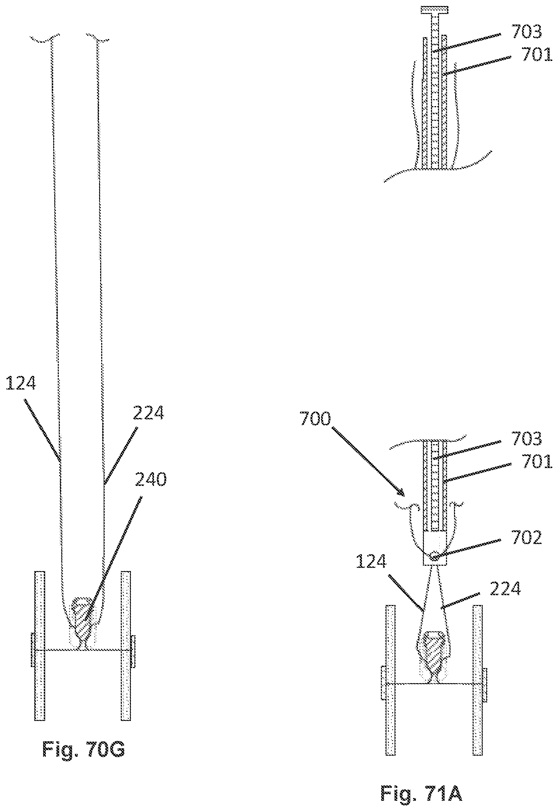

Referring to Figure 70F, after the driver 574 is released from the line locking device 240, the tool 550 is withdrawn from the patient as indicated by arrows 682. The line locking device 240 and first and second lines (124 and 224) remain in the patient as illustrated by Figure 70G.

The first and second lines (124 and 224) can be trimmed at the line locking device 240 using a trimming tool 700. The trimming tool can take a wide variety of different forms. An exemplary embodiment of such a trimming tool 700 is illustrated in Figure 71A. The trimming tool 700 includes an outside component or sleeve 701 and an inside component or plunger 703 that are slidably or telescopically coupled together. As illustrated, the first and second lines 124 and 224 are threaded through an opening 702 in the trimming tool 700. For example, the lines 124, 224 can be threaded through the opening 702 outside the patients' body.

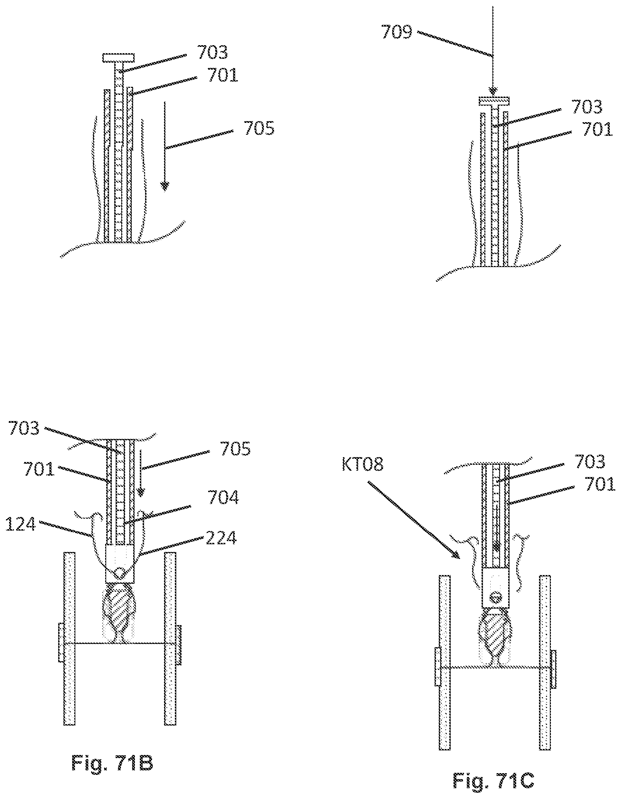

The trimming tool 700 is advanced 705 through a guide catheter to position a cutting end 704 of the tool at the line locking device 240 as illustrated in Figure 71B. In an exemplary embodiment, the lines 124, 224 are held in place outside the patient's body as the cutting end 704 is advanced into place.

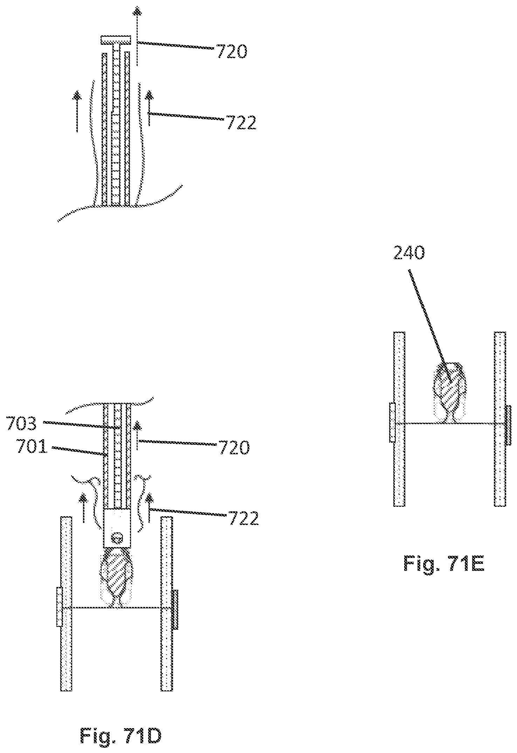

Referring to Figure 71C, in an exemplary embodiment, a plunger 703 is depressed as indicated by arrow 709, causing the first and second lines (124 and 224) to be trimmed. Referring to Figure 71D, after the lines 124, 224 are cut, the trimming tool 700 and first and second lines 124 and 224 are withdrawn from the patient as indicated by arrows 720, 722 respectively. The result is the line locking device 240 remaining in place as shown in Figure 71E.

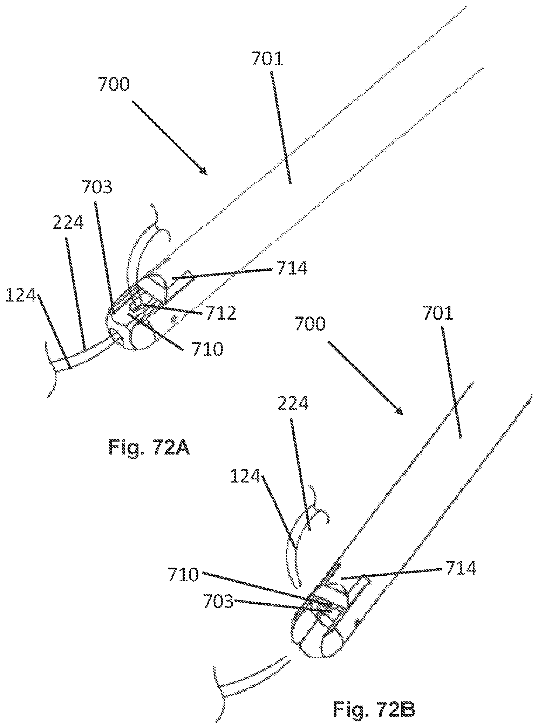

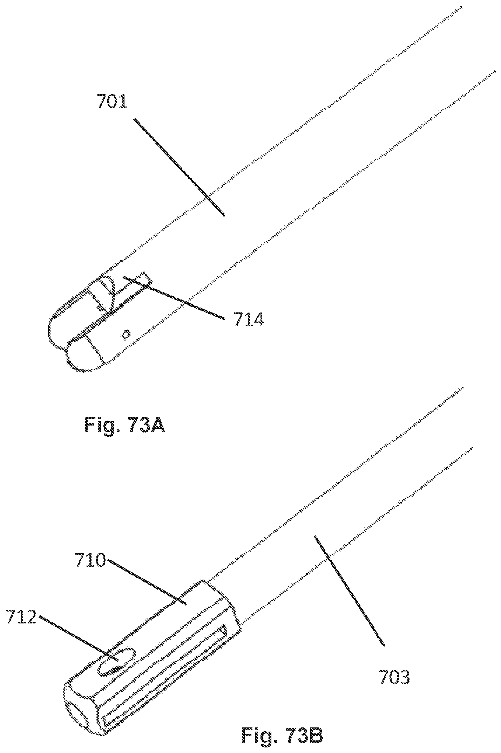

A more specific example of a trimming tool 700 is illustrated in Figures 72A and 72B. The trimming tool includes an outer sleeve 701 and an inner shaft 703. The outer shaft 701 is shown separately in Figure 73A and the inner shaft 703 of the trimming tool 700 is illustrated in Figure 73B. The inner shaft 703 includes a head 710 with a passage 712 that the lines 124, 224 can be routed through. For example, the lines 124, 224 can be routed through the passage 712 outside the patient's body. The inner shaft 703 is slidably disposed in the outer sleeve 701, such that the head 710 pulls the lines 124, 224 against a blade 714 of the outer sleeve to cut the lines 124, 224.

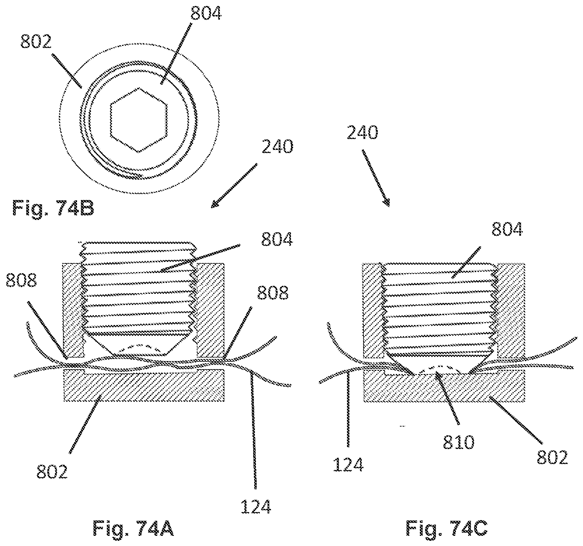

The line securing device 240 can take a wide variety of different forms. Figures 74A-93B illustrate a variety of non-limiting examples of line securing devices 240 that can be used in the heart wall remodeling techniques described herein. An exemplary embodiment of a line locking device 240 is illustrated in Figures 74A and 74B. As illustrated, the line locking device 240 is comprised of a housing 802 and a threaded insert 804. Figure 74B shows a view of the line locking device 240 looking at the outward end of the threaded insert 804. As shown in Figure 74A, when the threaded insert 804 is partially inserted into the housing 802, one or more lines 124 can be threaded through a pair of openings 808. With the lines 124 threaded as shown in Figure 74A, the threaded insert 804 can be turned such that the treaded insert is drawn into the housing 802, causing the lines to be trapped between the threaded insert 804 and the housing 802 as shown in Figure 74C. As illustrated in Figure 74C, the threaded insert 804 can be formed with a concave surface 810 or a flat surface located at the inward end of the threaded insert 804. In certain embodiment, this concave surface may result in a more secure clamping action because the configuration avoids the small area of contact that might occur if the insert 804 were provided with a convex shaped pointed or rounded end.

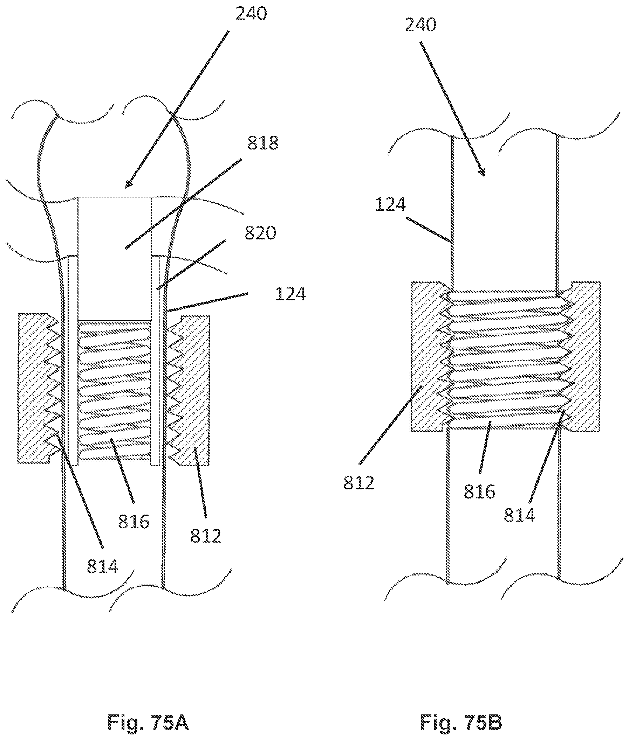

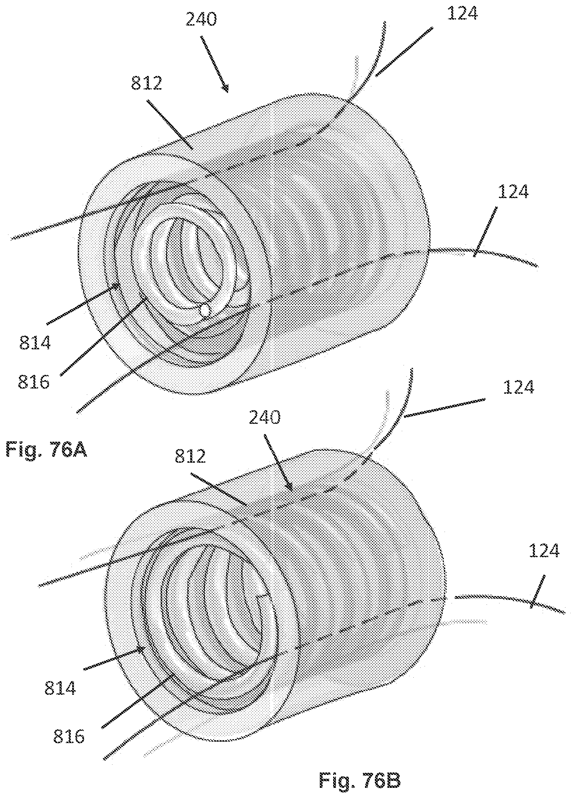

Figures 75A, 75B, 76A and 76B illustrate another exemplary embodiment of a line locking device 240. In this exemplary embodiment, an outer housing 812 has a threaded or serrated opening 814 passing longitudinally through the outer housing 812. Lines 124 are caused to pass through the threaded opening 814 and a spring 816 is disposed within the threaded opening 814. The spring 816 can be formed from a shape memory metal such as, without limitation, Nickel titanium (Nitinol) or another resilient material, such as steel, etc. The resilient characteristic of the spring 816 cause it to expand to engage the threads in the threaded opening 814, trapping the lines between the threads of the threaded opening 814 and the spring 816.

Figures 75A and 75B illustrate one exemplary embodiment of a system for deploying the line locking device illustrated by Figures 76A and 76B. In the illustrated example, the spring 816 is compressed inside a catheter 820. The catheter 820 is positioned inside the outer housing 812. The lines 124 are threaded between the catheter 820 and the body 812. The spring 816 is released from the catheter 820 by retracting the catheter 820 while a pusher 818, such as a rod or a catheter, holds the axial position of the spring 816. Once the spring 816 is released, the spring 816 expands outward to the position illustrated by Figure 75B. In the position illustrated by Figure 75B, the lines are captured between the spring 816 and the threaded or serrated opening 814 of the housing 812.

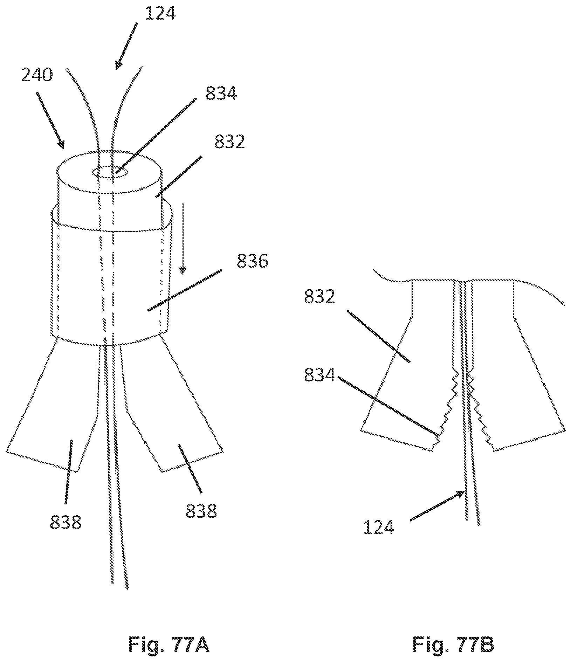

Figures 77A and 77B illustrate another exemplary embodiment of a line locking device 240. In this embodiment, lines are passed through a clamp member 832 that has an opening 834 passing longitudinally through the clamp member 832. When it is desired to clamp the lines 124, a sleeve 836 is moved along the clamp member 832 to cause jaws 838 formed in the clamp member 832 to close on and secure the lines 124. As illustrated in Figure 77B, teeth or serrations 840 can be formed in the clamp member 832 to more securely engage the lines 124. The sleeve 836 can be maintained in the closed position on the clamp member 832 in a wide variety of different ways. For example, the sleeve 836 can have an interference fit with the clamp member 832, the sleeve 836 and the clamp member 832 can threadedly engage one another, etc.

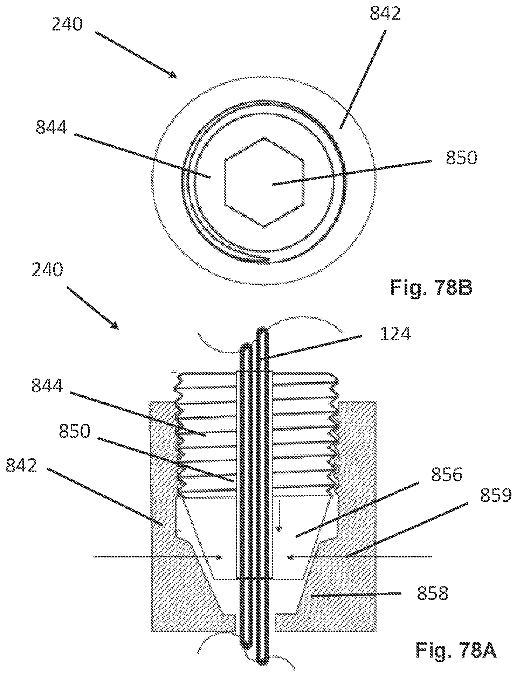

An exemplary embodiment of a line locking device 240 is illustrated in Figures 78A and 78B. As shown in Figure 78A, the line locking device 240 is comprised of a housing 842 and a threaded insert 844. The threaded insert 844 is formed with a passage 850 that extends longitudinally through the insert. In use, lines 124 are passed through the opening 850 as illustrated. The threaded insert 844 is rotated such that the threads formed in the insert engage with threads formed in the housing 842. As the threaded insert 844 is drawn into the housing 842, a tapered end 856 of the threaded insert 844 engages a taper 858 formed in the housing. This engagement causes the tapered end of the insert to deform as indicated by arrows 859 and close a portion of the passage onto the lines 124, thus clamping the lines 124 in position relative to the line locking device 240. Figure 78B shows a top view of the line locking device 240 that illustrates the end of the threaded insert 844 opposite the tapered end 856. As illustrated, an exemplary embodiment has a hex shaped opening 850 in the threaded insert 844 that facilitates the use of a tool (not illustrated) to rotate the threaded insert 844 relative to the housing 842, thus clamping the lines 124 as described herein.

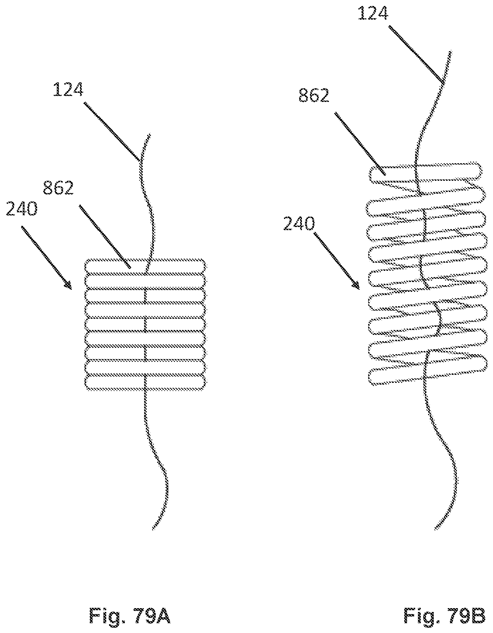

Another exemplary embodiment of a line locking device 240 is illustrated in Figures 79A and 79B. In this embodiment, lines 124 are woven through a spring 862 as illustrated in Figure 79B. The spring 862 can be formed from a resilient material, such as steel, a shape memory metal such as, without limitation, Nickel titanium (Nitinol), etc. As shown, the spring 862 will return to its tightly coiled state as illustrated in Figure 79A. This serves to trap the lines 124 between the coils of the spring 862, locking the lines 124 in place.

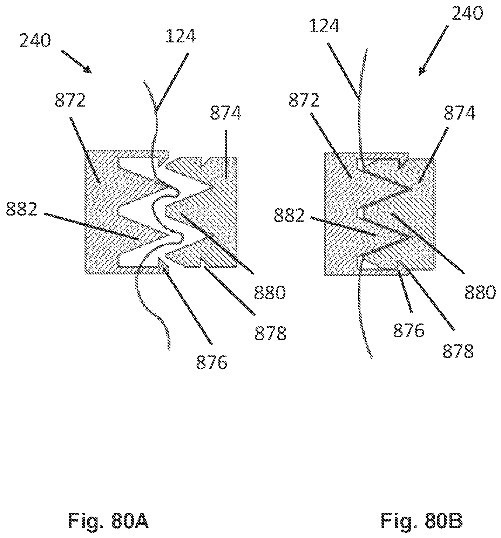

Another exemplary embodiment of a line locking device 240 is shown in Figures 80A and 80B. The line locking device 240 is comprised of two parts, a receiver 872 and a locking insert 874. As shown, lines 124 are placed between the receiver 872 and locking insert 874 and the two are brought together as shown in Figure 80B. The receiver 872 and the locking insert can be coupled together in a wide variety of different ways to connect the lines and lock them in place. In the example illustrated by Figures 80A and 80B, the receiver has locking hooks 876 that engage locking grooves 878 in the locking insert 874. When the locking hooks 876 are engaged in the locking grooves 878, the teeth 880 of the locking insert 874, interweave with the teeth 882 of the receiver 872. These two sets of teeth 880 and 882 capture the line 124, locking it in place with respect to the line locking device 240.

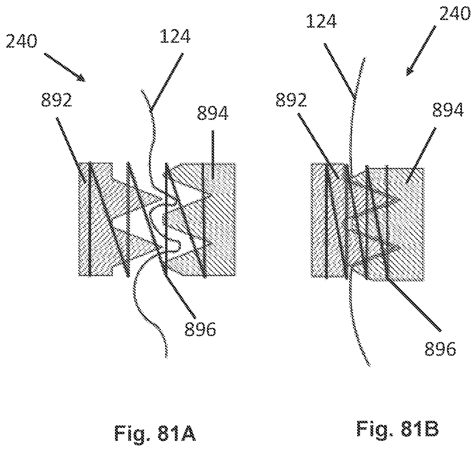

Another exemplary embodiment of a line locking device 240 is shown in Figures 81A and 81B. As shown, the device has a receiver 892 and a locking insert 894. Lines 124 are placed between the receiver 892 and locking insert 894 and the two are brought together as shown in Figure 81B. A spring 896 draws the receiver 892 and locking insert 894 together and holds them in this state, locking the line 124 as shown in Figure 81B.

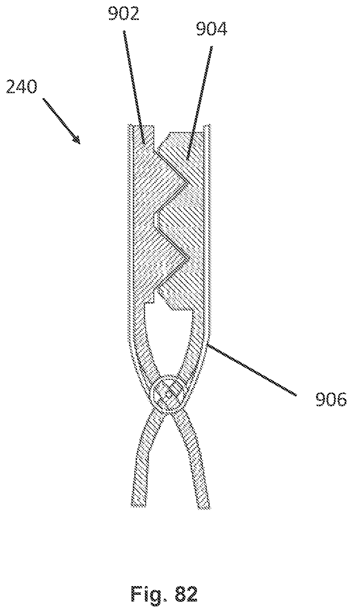

As shown in Figure 82, an exemplary embodiment of a line locking device 240 comprises a pair of toothed jaws 902 and 904 that are held together by a spring 906. Closing force exerted by the spring 906 causes the toothed jaws 902 and 904 to lock a line (not shown) in place relative to the clamp 240.

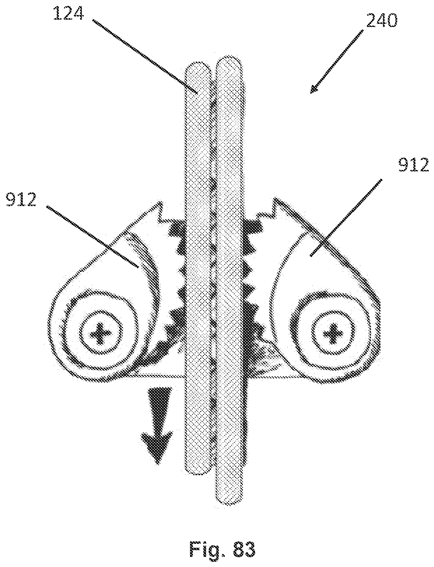

In the exemplary embodiment of the line locking device 240 illustrated in Figure 83, a pair of toothed cams 912 are positioned such that movement of a pair of lines 124 results in the rotation of the cams 912. Depending on the direction the lines are pulled and the corresponding direction the cams 912 rotate, the locking device 240 either allows the lines to move through the locking device or prevents the lines from moving through the locking device. For example, when the lines 124 are pulled relative to the locking device 240 in the direction indicated by the arrow, the cams 912 clamp the lines 124 such that the lines 124 are locked in place relative to the line locking device 240. The tension achieved by a pair of anchors 122 in the embodiments described above can cause the force indicated by the arrow, and thereby cause the locking device 240 to lock the position of the lines 124. In some exemplary embodiments, the cams can optionally be spring-loaded such that the cams 912 are biased against the lines 124.

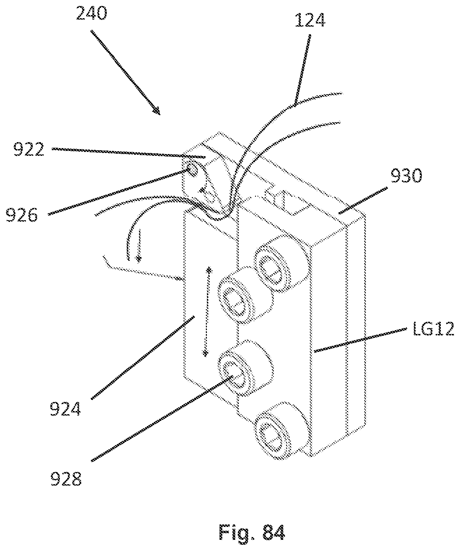

In another exemplary embodiment, a line locking device 240 is formed from a movable plate and cleat that are used to secure two or more lines. As illustrated in Figure 84, a line 124 is threaded between a cleat 922 and an adjustable plate 924. The cleat 922 rotates about a pin 926 to secure the lines 124 between the cleat 922 and the plate 924. The plate 924 can be moved closer or farther from the cleat 922 depending upon the thickness and number of lines 124. In the illustrated exemplary embodiment, the plate 924 can be secured in position using one or more fasteners 928 which secure the plate 924 between a backing plate 930 and a clamp plate 932.

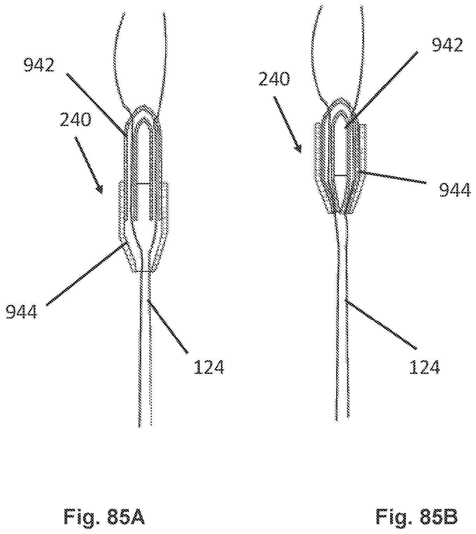

In another exemplary embodiment, a line locking device 240, illustrated in Figures 85A and 85B, secures a line 124 that is passed through an inner component 942 of the line locking device 240. The inner component 942 is inserted into an outer component 944 which serves to compress the inner component 942 as illustrated in Figure 85B, such that the lines 124 are secured by the compression of the inner component 942.

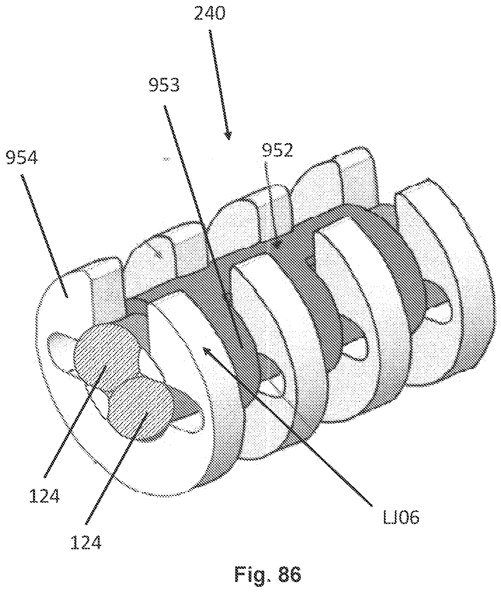

An exemplary embodiment of a line locking device 240 is shown in Figure 86. Such a line locking device could be made from a resilient material, such as steel or a shape memory metal such as, without limitation, Nickel titanium (Nitinol), etc. As shown, a line 124 is passed between an inner lock component 952 and a memory metal outer lock component 954. The inner lock housing 952 has fingers 953 that fit within slots between fingers 956 of the outer lock housing 954 The two parts 952, 954 are brought together to snap onto the lines 124. The fingers 953, 956 mesh together to hole the lines 124 in place in the line locking device 240.

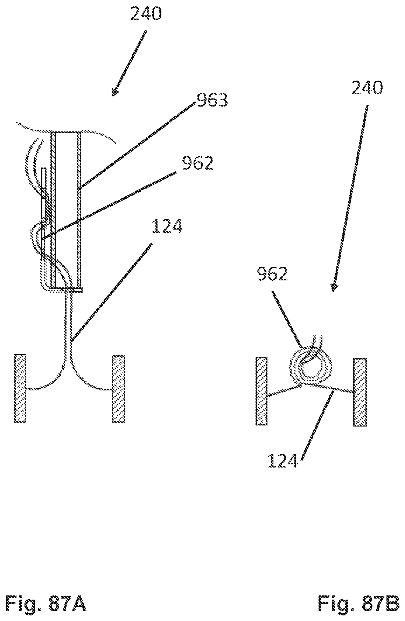

In another exemplary embodiment of a line locking device 240 is illustrated in Figures 87A and 87B that comprises a material strap 962. In Figure 87A, the strap 962 is held in an elongated condition by a holder 963. A line 124 is weaved in and out of an elongated strap 962 and easily slides relative to the strap, when the strap is in the elongated position. The strap can be made of a shape memory material such as, without limitation, Nickel titanium (Nitinol) and shape set to the coiled shape (or other line constraining shape) illustrated by Figure 87B. When the locking strap 962 is released from the holder, it returns to its set position as a spiral shape. The spiral shape causes the line to be wrapped up with the strap locked in place as illustrated in Figure 87B.

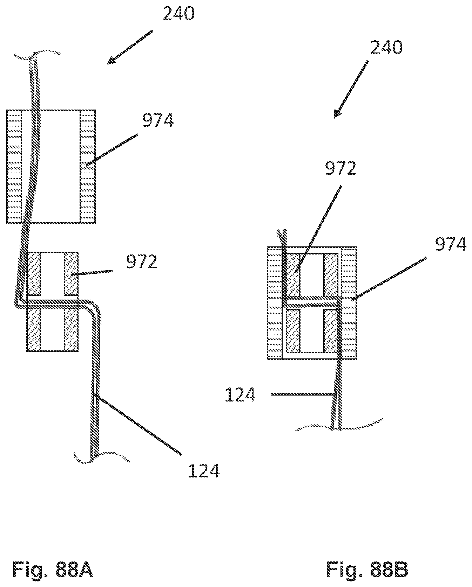

**In** another exemplary embodiment of a line locking device 240, as illustrated in Figures 88A and 88B, a line 124 is passed through an inner clamp barrel 972. The inner clamp barrel 972 is placed inside an outer clamp barrel 974 with an inner diameter slightly larger than the outer diameter of the inner clamp barrel 972. The result is that the line 124 is wedged between the inner barrel 972 and outer barrel 974, locking the line 124 in place relative to the line locking device 240.

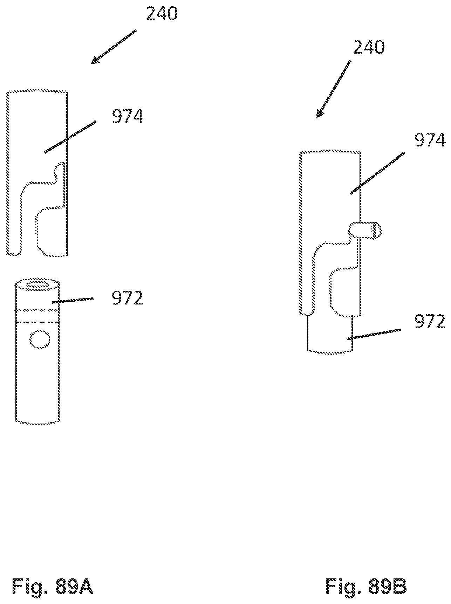

Figures 89A and 89B illustrate an exemplary embodiment of a line locking device 240 that is similar to the device of Figures 88A and 88B. In the example illustrated by Figures 88A and 88B, the inner clamp barrel 972 includes a pin 976 and the outer clamp barrel 974 includes a slot 978. To lock the lines 124, the pin 976 is placed in the slot 978, inner clamp barrel 972 is advanced into the outer clamp barrel 974, and the inner clamp barrel 972 is rotated in the outer barrel 974 to move the pin circumferentially along the slot. As a result, the inner clamp barrel 972 has to be rotated relative to the outer clamp barrel 974 before the inner clamp barrel can be withdrawn from the outer clamp barrel. As such, the pin 976 and slot 978 lock the device 240 to prevent unintentional release of the lines 124.

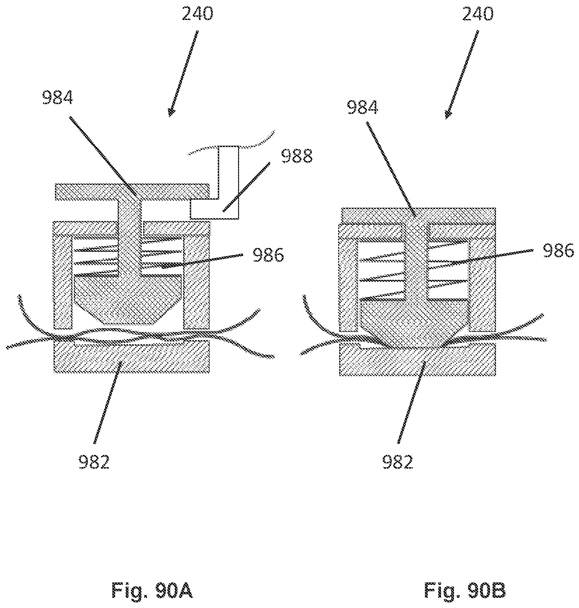

Figures 90A and 90B shown another exemplary embodiment of a line locking device 240. As shown, a line 124 is passed through an opening in an outer housing 982. A plunger 984 and spring 986 are deposed inside the outer housing 982. As shown in Figure 90A, during installation a spacer 988 is positioned such that the plunger 984 is held in a retracted state. The spacer 988 may be a part of an installation tool (not shown) that guides the line locking device 240 into position. The retracted state allows the line 124 to move freely relative to the line locking device 240. When the spacer 988 is removed, the plunger 984 is pressed against the outer housing 982 by the spring 986, trapping the lines 124 in place.

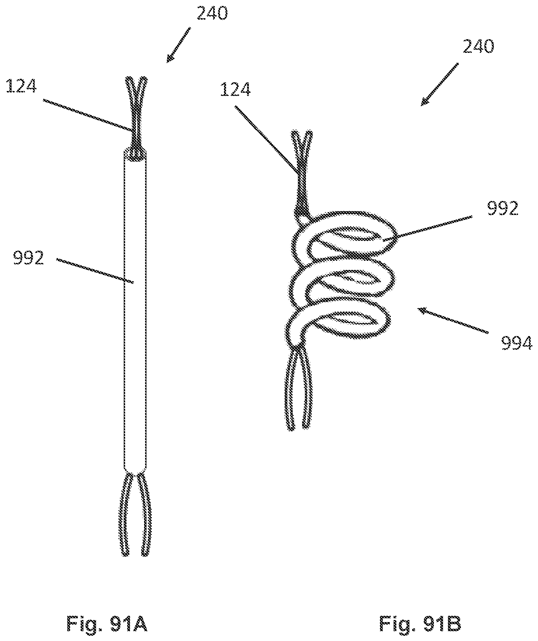

The properties of a shape memory metal such as, without limitation, Nickel titanium (Nitinol) can be used in an exemplary embodiment of a line locking device. One such embodiment of a line locking device 240 is illustrated in Figures 91A and 91B. As shown, lines 124 are threaded through a tube 992 formed from a shape memory metal. When it is desired to clamp the lines 124 in place, the memory metal of the tube 992 is allowed to return a shape set coiled state 994 as illustrated in Figure 91B. This forces the lines 124 into a circuitous path, holding them in place.

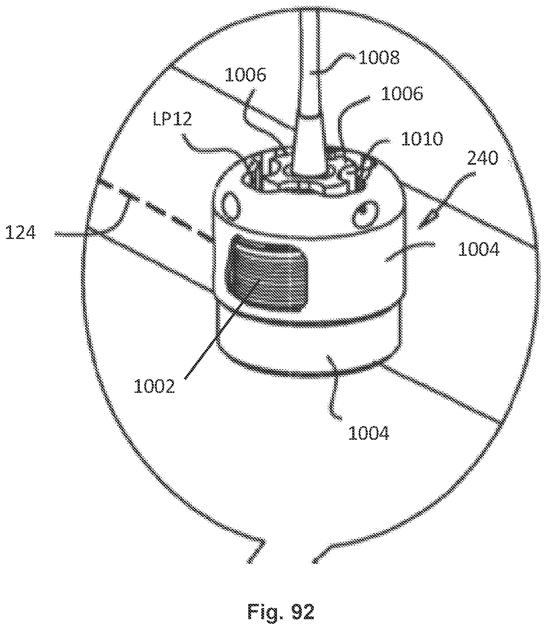

In another exemplary embodiment, a line locking device 240 uses a spool to retract a line, locking it in place. Such a line locking device 240 is illustrated in Figure 92. As is illustrated, a spool 1002 is used to draw a line 124 into an outer housing 1004. The line locking device 240 comprises an engagement hub 1006 that is affixed to the spool 1002. The engagement hub 1006 can be turned by an installation tool 1008. The engagement hub 1006 is formed with teeth 1010 that engage pawls 1012 that allow the engagement hub 1006 to turn only in one direction such that the spool 1002 tightens the line 124 and holds it in the tightened state.

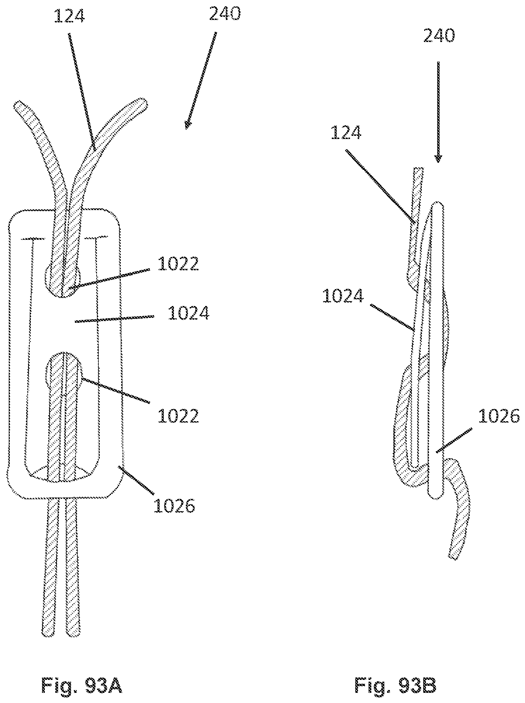

An exemplary embodiment of a one-piece line locking device 240 is illustrated in Figures 93A and 93B. As shown, lines 124 are threaded through openings 1022 formed in the line locking device 240. A tab portion 1024 is formed in the line locking device 240. The tab portion 1024 captures the lines 124 between the tab portion 1024 and a base portion 1026. This capture, together with the circuitous path that the line 124 takes through the openings 1022, serves to lock the line 124 in place relative to the clamp 240. In certain embodiments, such an exemplary embodiment of a line locking device 240 can be formed from a resilient material, such as a shape memory metal such as, without limitation, Nickel titanium (Nitinol), etc.

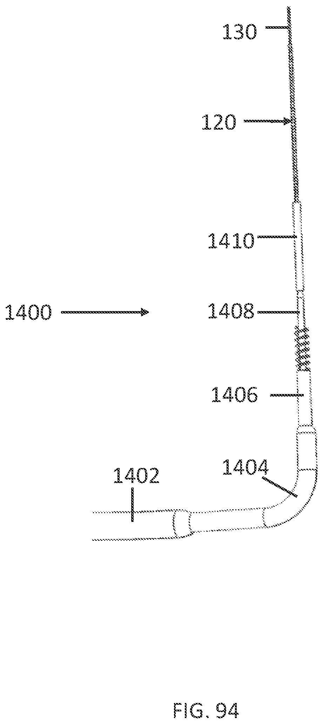

Referring to Figures 94-111, an exemplary embodiment of a device 120 for remodeling the shape of a heart wall and a system 1400 and method for delivering the device 120 against an exterior surface of the heart H is illustrated. The system 1400 and the device 120 can be configured in a variety of ways.

In the illustrated embodiment, the system 1400 includes a guide sheath 1402, a steerable catheter 1404, a delivery catheter 1406, a pusher 1408, a hemostatic plug 1410, a piercing device 130, and the device 120.

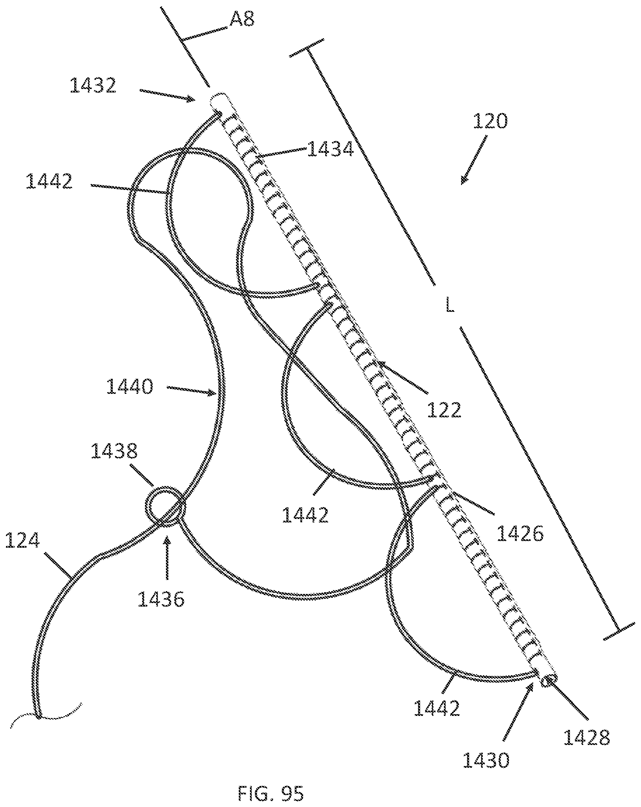

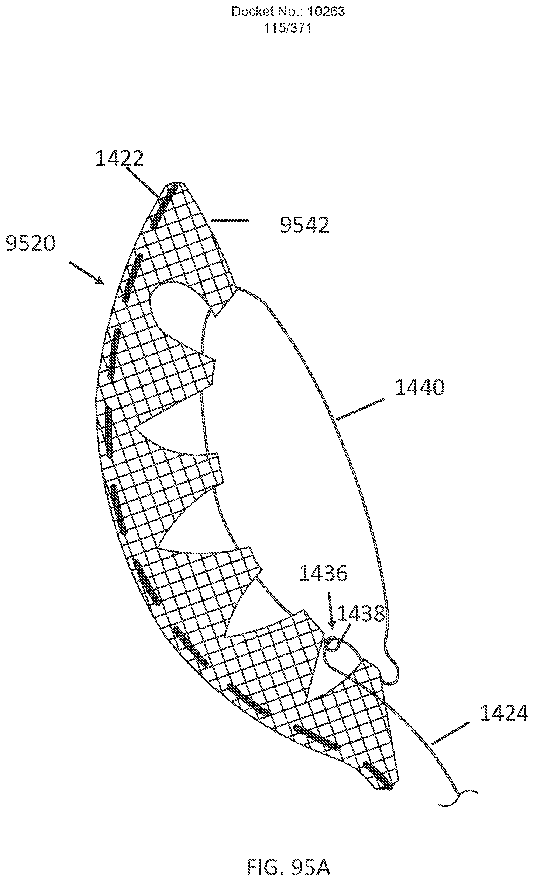

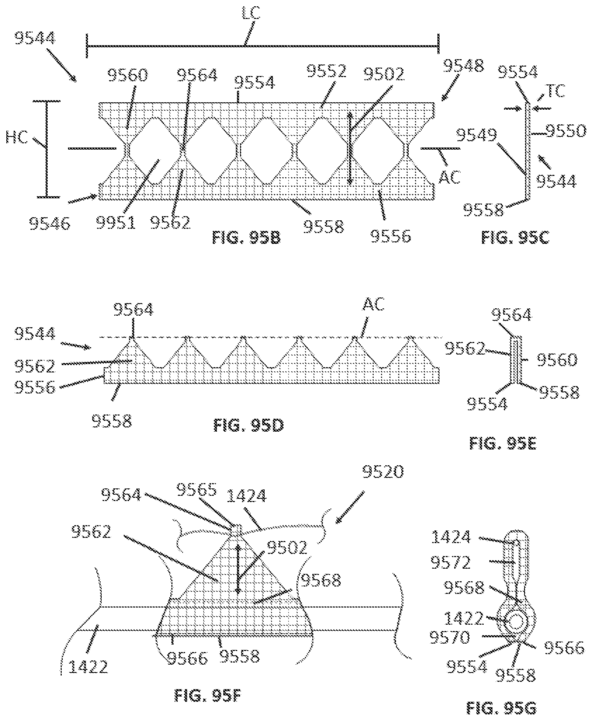

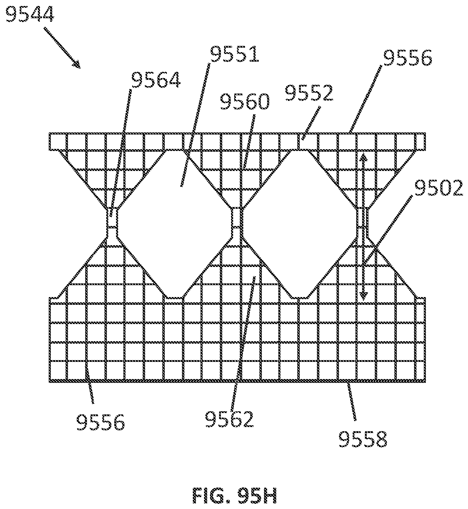

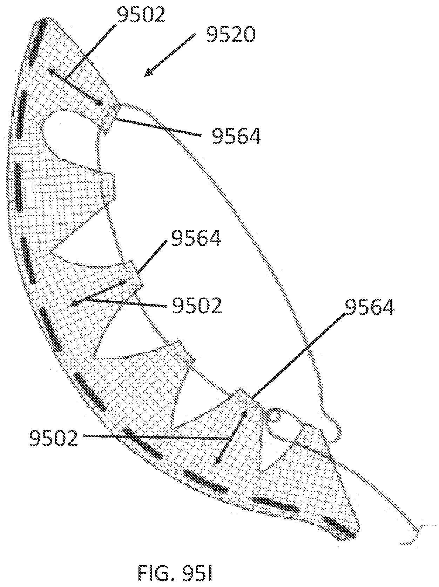

Referring to Figure 95, an exemplary embodiment of the device 120 for remodeling the shape of a heart wall is illustrated. The device 120 includes an anchor 122 and a line 124. The anchor 122 can be configured in a variety of ways. Any configuration that can be positioned to engage an outward facing surface of a heart wall W to support pulling a portion of the heart wall W inward (i.e., toward an internal chamber of the heart) can be used. In the illustrated embodiment, the anchor 122 is reconfigurable, such that the anchor can be delivered through a catheter or sheath in a delivered state (e.g., elongated as shown in Figure 95) that fits within a lumen of delivery catheter 1406, and can be reshaped to a deployed state once it has been delivered to the appropriate location.

In the illustrated embodiment, the anchor 122 has a generally cylindrical elongated body 1426 forming a tube having a central passage 1428. In other embodiments, however, the body 1426 can be shaped other than cylindrical. For example, the elongated body 1426 may have an oval, rectangular, or other shaped cross section. The body 1426 has a length L and includes a first end portion 1430 and a second end portion 1432 opposite the first end portion.

In the illustrated embodiment, the body 1426 includes one or more features to facilitate bending of the body 1426. The features can be configured in a variety of ways. In the illustrated embodiment, the features include a series of traverse cuts 1434 along the body 1426. In one embodiment, the series of cuts 1434 are a plurality of cuts where each of the cuts is generally perpendicular to a longitudinal axis A8 of the body 1426. In the illustrated embodiment, the cuts in the series of cuts 1434 are evenly spaced along the body 1426 and extend from the first end portion 1430 to the second end portion 1432. For example, the series of cuts 1434 may extend over at least 80% of the length L of the body M. In other embodiments, the series of cuts 1434 may not be evenly spaced and may extend less than 80% of the length L of the body 1426.

Further, each of the cuts of the series of cuts 1434 extends partially into the body 1426. In one embodiment, each of the cuts extend between 25%-75% through the body.

The anchor 122 can be made from any suitable material that can be reshaped from an elongated state to a curved, deployed state. In one exemplary embodiment, the anchor 122 includes a shape-memory alloy-such as Nitinol-to provide shape-setting capability.

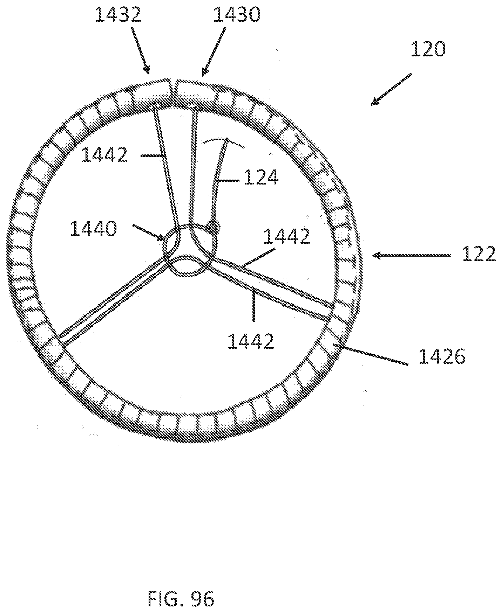

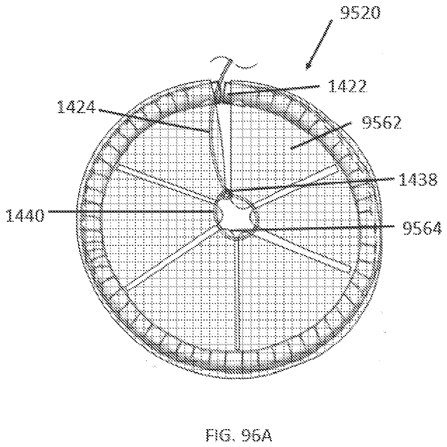

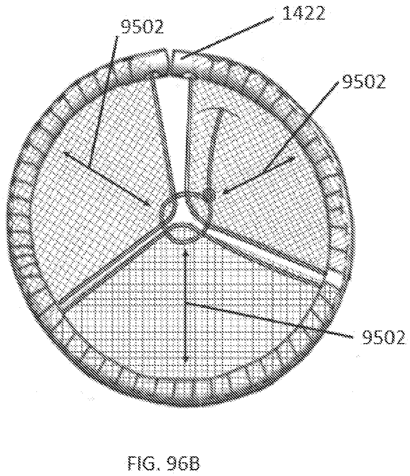

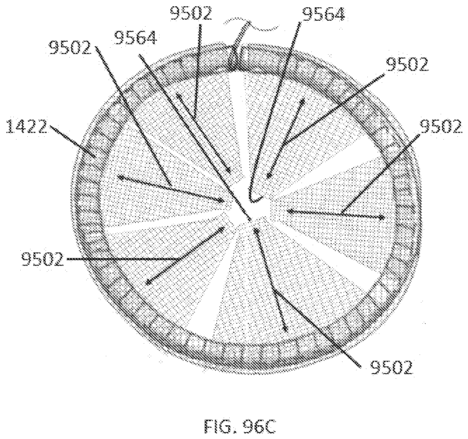

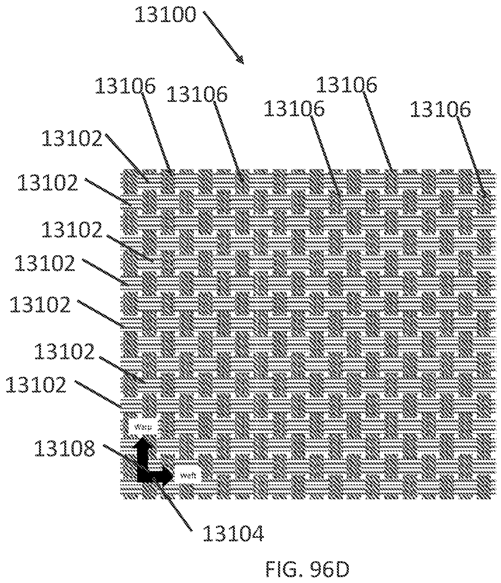

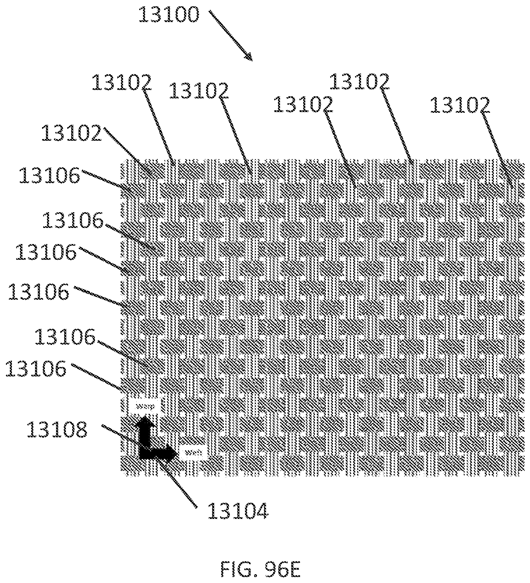

The line 124 is connected to the anchor 122 such that pulling the line 124 may pull the anchor 122. In some embodiments, pulling the line 124 may also reshape the anchor from an elongated state (Figure 95) to a curved, deployed state (Figure 96). In the illustrated embodiment, the line 124 has a terminal end 1436 formed in a closed shape 1438, such as a circle 1438. The line 124 is arranged such that the line 124 passes through the closed-shaped terminal end 1436 to form a loop 1440 in the line 124 that can be withdrawn by pulling the line 124.

The line 124 can be connected to the anchor 122 in a variety of ways. In the illustrated embodiment, the anchor includes a plurality of loops 1442 and the line 124 passes through each of the loops 1442 to connect the line 124 to the anchor 122. The number, size, location, and configuration of the loops 1442 may vary in different embodiments. In the illustrated embodiment, the anchor includes three equal sized and evenly spaced loops 1442 along the length L of the anchor 122.

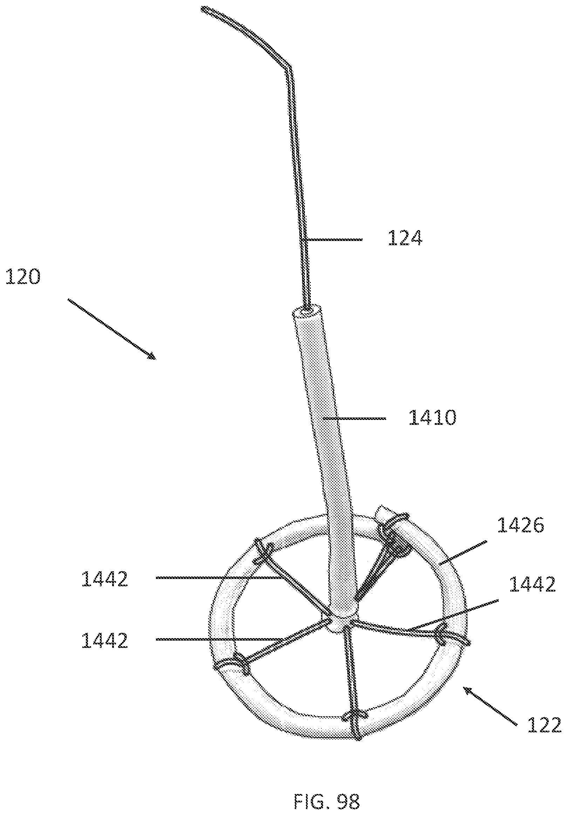

In the illustrated embodiment, each of the loops 1442 is formed by a line fixedly attached, at its ends, to the body 1426 of the anchor 122. The line forming the loops 1442 can be fixedly attached in any suitable manner. In some embodiments, the body 1426 may include openings that accommodate insertion of the ends of the line forming the loops 1442 into the interior of body 1426 to attach the ends to the body 1426 122 (see, for example, Figure 96). In other embodiments, the lines that form the loops 1442 can be tied to the exterior of the body 1426 of the anchor 122 (see, for example, Figure 98) or otherwise attached to the exterior, such as for example, by an adhesive or other fastening device.

In other embodiments, however, the loops 1442 can be formed from a material other than a line. The loop 1440 in the line 124 passes through each of the loops 1442 to connect the line 124 to the anchor 122.

Referring to Figures 95A-95G, another exemplary embodiment of a device 9520 for remodeling the shape of a heart wall is illustrated. The device 9520 is similar to the illustrated device 120 of Figure. 95 in that the device 9520 includes an anchor 1422 and a line 1424. The device 9520, however, instead of utilizing a plurality of loops 1442, formed by lines, through which the line 124 passes through, the device 9520 utilizes a cloth, fabric, or similar pliable material that is configured to facilitate reshaping the anchor in a deployed state that can be used to pull a portion of a heart wall inward to remodel the shape of the heart wall.

The anchor 1422 may be configured in a variety of ways. Any configuration that can be positioned to engage an outward facing surface of a heart wall W to support pulling a portion of the heart wall W inward (i.e., toward an internal chamber of the heart) may be used. In the illustrated embodiment, the anchor 1422 is the same or substantially similar to the anchor 122 of Figure 95. Thus, the description of the anchor 122 applies equally to the anchor 1422

The line 1424 is connected to the anchor 1422 such that pulling the line 1424 pulls the anchor 1422. In some embodiments, pulling the line 1424 can also reshape the anchor from a substantially elongated state (Figure 95A) to a round, deployed state (Figure 96A). In the illustrated embodiment, the line 1424 has a terminal end 1436 formed in a closed shape 1438, such as a circle. The line 1424 is arranged such that the line 1424 passes through the closed-shaped terminal end 1436 to form a loop 1440 in the line 1424 that can be withdrawn by pulling the line 1424.

The line 1424 can be connected to the anchor 1422 in a variety of ways. In the illustrated embodiment, a connector 9542, made from a cloth, fabric, or similar pliable material, is used to connect the line 1424 to the anchor 1422. The connector 9542 may be configured in a variety of ways, such as for example, different shapes and different materials. In one exemplary embodiment, the connector 9542 is made from a low-profile cloth. This cloth can take a wide variety of different forms. It can be woven, knitted, braided or non-woven. When woven, knitted or braided the cloth can optionally utilize high-strength yarns. The cloth can comprise any of the fabrics, yarns, and yarn components disclosed by US Patent No. 8,833,402, issued on September 16, 2014 to Rasmussen et al.. In one exemplary embodiment, a permanent implant grade high strength yarn can be made from ultra-high molecular weight polyethylene (UHMwPE), polyethylene terephthalate (PET) and/or other materials and blends of materials. In some exemplary embodiments, the materials of the yarns are oriented to increase the strength of the yarn. In some exemplary embodiments, the connector 9542 is made from a thin material that when used with the disclosed anchor 1422 has a minimum pull strength of 60N or more in combination with the pulling line 1424. In one exemplary embodiment, the cloths have a low-profile yarns and high orientation in molecular chains of the polymer to achieve high strength and low connector profile. The yarn used in the functional direction for realization of high pull-strength can possess a tenacity of at least 40 grams/denier. In one exemplary embodiment, high-strength Dyneema^{®} yarn or other UHMwPE yarn with PET yarn can yield approximately 3 times the pull-out strength of the line 1424 through the connector compared to an otherwise identical connector made only from high density PET cloth.

In one exemplary embodiment, the connector cloth 9542 is a high density PET cloth and/or a hybrid cloth (described below) that has between 150 and 270 ends per inch, such as between 170 and 250 ends per inch, such as between 190 and 230 ends per inch, such as 210 ends per inch or about 210 ends per inch and between 110 and 230 picks per inch, such as between 130 and 210 picks per inch, such as between 150 and 190 picks per inch, such as about 170 picks per inch, such as 170 picks per inch. A wide variety of different yarns can be used for warp and weft. For example, a 40d/24f PET yarn can be used for warp and weft. In one exemplary embodiment, a cloth thickness of a cloth made from a high strength yarn, such as a UHMwPE yarn, such as a Dyneema^{®} yarn with a conventional yarn, such as PET yarn is also lower compared to an otherwise identical cloth made only from conventional yarns, such as PET. The use of cloth as the connector 9542 provides an even force distribution against the tissue

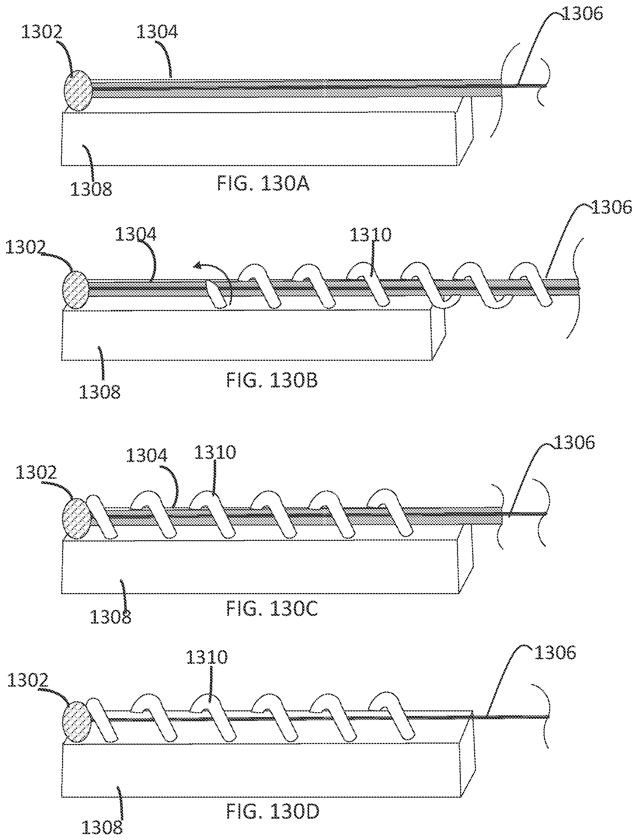

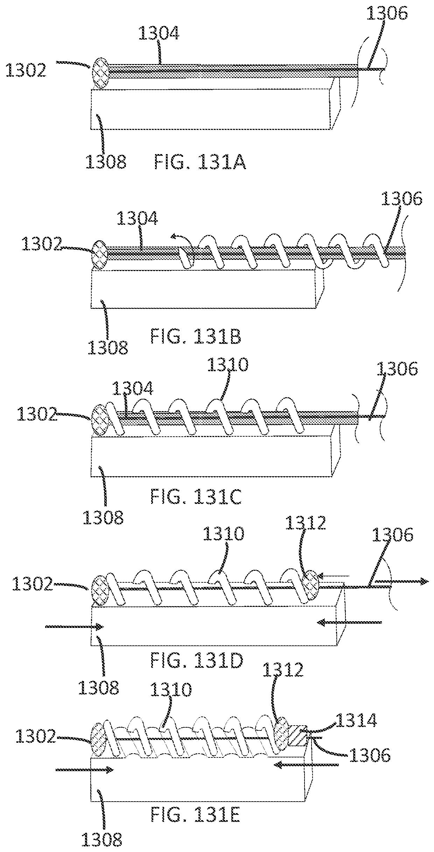

Figures 130 and 131 illustrate two examples of woven cloth 13100 that can be used to make the connector 9542. In the example illustrated by Figure 96D, the woven cloth 13110 includes high strength yarn 13102 in the weft direction 13104 and a conventional yarn 13106 in the warp direction 13108. In the example illustrated by Figure 96E, the woven cloth 13110 includes a conventional yarn 13106 in the weft direction 13104 and a high strength yarn 13102 in the warp direction. The high strength yarn 13102 can take a wide variety of different forms. For example, the high strength yarn 13102 can be an UHMwPE yearn, such as a Dyneema^{®} yarn. The conventional yarn 13106 can take a variety of different forms. For example, the conventional yarn 13106 can be a conventional plastic, such as PET. The conventional yarn 13106 and the high strength yarn 13102 are woven to form a hybrid fabric. In one exemplary embodiment, the high strength yarn size can be 25dtex/10filament and the conventional yarn size can be 44dtex/24filament, with the fabric being constructed using at least 180 picks per inch and 160 ends per inch. For example, a UHMWPE yarn size can be 25dtex/10filament and a PET yarn can be 44dtex/24filament, with the fabric being constructed using at least 180 picks per inch and 160 ends per inch. The high strength yarn and the conventional yarn can be multifilament or monofilament. For example, a UHMWPE yarn and a PET yarn can be multifilament or monofilament. UHMPWPE yarn can be of size 11dtex up to 55dtex while PET yarn can be 11dtex up to 44dtex. UHMPWPE yarn can be of size 11dtex up to 55dtex while PET yarn can be 11dtex up to 44dtex.

The connector 9542 can take a variety of different shapes. In some exemplary embodiments, the connector 9542 is configured such that pulling the line 1424 also reshapes the anchor from an elongated state (Figure 95A) to a round, deployed state (Figure 96A). In some other exemplary embodiments, the anchor 1422 is shape-set to the deployed state and the connector 9542 is not configured such that pulling the line reshapes the anchor. In the exemplary embodiment illustrated by Figures 95B-95C, the connector 9542 includes a patterned cloth or other material 9544 that is laser-cut, or otherwise formed. In the illustrated embodiment, the patterned material 9544 has a first end 9546, a second end 9548, a first face 9549, and a second face 9550 opposite the first face 9549.

The patterned material 9544 includes a series of cut-outs 9551 extending across the length LC of the patterned material 9544 along a central longitudinal axis AC. The cut-outs 9551 may be configured in a variety of ways, such as the shape, size, and a number of cut-outs. In the illustrated embodiment, the cut-outs 9551 are generally diamond-shaped, with the cut-outs 9551 adjacent the first end 9546 and adjacent the second end 9548 being half-diamonds. In other embodiments, however, the cutouts 9551 may take other shapes, such as for example, circular, oval, triangular hexagonal, octagonal and/or rectangular, etc. The cut-outs 9551, in the illustrated embodiment, are positioned and shaped such that the patterned material 9544 is symmetric relative to the longitudinal axis AC. Further, in the illustrated embodiment, the patterned material 9544 includes five, full cut-outs, and two half, open cut-outs adjacent the first end 9546 and adjacent the second end 9548. In other embodiments, however, the patterned material 9544 may include more or less than five, full cut-outs.

In one exemplary embodiment, the cut-out dimensions at the folded section (i.e. the section connecting the diamonds in Figure 95B) are selected to achieve a required pull-strength. Based upon the desired minimum pull strength, the number of cut-outs, the width of the section connecting the diamonds and cloth parameters such as tenacity, size, and yarn density of high strength yarn in the cloth is optimized. In one exemplary embodiment, a hybrid cloth of high strength yarn, such as UHMWPE yarn with conventional yarn, such as PET yarn utilizes melt-sealing of edges from the filaments of plastic conventional filaments, such as PET filaments when the connector 9542 component is cut into different shapes to avoid fraying of the yarns and/or premature failure of components i.e. low pull strength. In one exemplary embodiment, the low melting temperature and melt flow characteristics of high strength yarns, such as UHMWPE yarns do not evenly melt at the edges when cutting the fabric into the shape of the connector 9542 using high temperature techniques, such laser cutting. The inclusion of the conventional yarns, such as PET yarn results in an edge having an even thickness, even though the edge is cut using a high temperature technique, such as laser cutting and the fabric includes the high strength yarns, such as UHMWPE yarns.

The cut-outs 9551 result in the patterned material 9544 having a first band 9552 forming an upper edge 9554 and a lower band 9556 forming a lower edge 9558. The cut-outs 9551 are defined by a series of first inward tapering sections 9560 and a series of opposing second inward tapering sections 9562 aligned with the series of first inward tapering sections 9560. Each of the first and second inward tapering sections 9560, 9562 are generally triangular, such as for example, forming an isosceles or equilateral triangle. However, the sections 9560 can have a wide variety of different shapes. Each of the first inward tapering sections 9560 is connected to a corresponding second inward tapering section 9562 by a bridge 9564.

Referring to Figures 95D-95E, to form the connector 9542, the patterned material 9544 is folded over upon itself, lengthwise along the central longitudinal axis AC, as best shown in Figure 95E. Thus, the bridges 9564 are folded in half such that the upper edge 9554 is positioned adjacent the lower edge 9558 and the first inward tapering sections 9560 are positioned adjacent the second inward tapering sections 9562. In this folded configuration, the material 9544 resembles a saw-tooth shape.

The connector can be connected to the anchor in a wide variety of different ways. Referring to Figures 95F-95G, to connect the connector to the anchor and to connect the line 1424 to the anchor 1422, the first band 9552 is connected to the second band 9556 at two locations. In the illustrated example, first, the first band 9552 is connected to the second band 9556 adjacent the upper edge 9554 and the lower edge 9558 as shown by line 9566. Second, the first band 9552 is connected to the second band 9556 adjacent the first inward tapering sections 9560 and the second inward tapering sections 9562 as shown by line 9568. The first band 9552 may be connected to the second band 9556 in any suitable manner, such as stitching, stapling, and/or adhesive or other suitable attachment. As a result of the first band 9552 being connected to the second band 9556 as disclosed, the material 9544 forms a first passage (or passages) 9570 through which the anchor 1422 extends and a second passage 9572 through which the line 1424 extends.

Figure 95H illustrates another patterned material 9544 where the first band 9552 is narrower than the second band 9556. To form the connector 9542, from the patterned material 9544 illustrated by Figure 95H, the material is folded over upon itself, lengthwise along centers of the bridges 9564. The upper edge 9554 is positioned significantly inward of the lower edge 9558 and the first inward tapering sections 9560 are positioned adjacent the second inward tapering sections 9562. The connector can be connected to the anchor in a wide variety of different ways.

In the Figure 95H embodiment, the second band 9556 is folded over the anchor. The folded second band 9556 can be connected to itself (since it folds back onto itself) and/or to the first band 9552. The second band 9556 can be connected to the first band 9552 and/or itself in any suitable manner, such as stitching, stapling, and/or adhesive or other suitable attachment. As a result of the second band 9556 being folded, the material 9544 forms a first passage (or passages) through which the anchor extends and a second passage through which the line extends.

Referring to Figures 95B, 95F, 95H, 95I, 96B, and 96C, in some exemplary embodiments the cloth 13100 can be oriented and cut into a pattern 9544 (Figure 95B) that maximizes the pull strength of the connector 9542. In one exemplary embodiment, the cloth 13100 is oriented and cut such that the high strength fibers 13102 (See Figures 96D and 96E) extend in the direction indicated by arrow 9502 (See Figures 95I, 96B, and 96C). In the examples illustrated by Figures 95I, 96B, and 96C, the direction 9502 corresponds to a line that is perpendicular to an edge 9565 (or the axis AC) of the connection portions 9564. This direction can also be characterized as extending in the direction of a shortest line that extend from the anchor 1422 to the edge 9565 of the connection portions. In another exemplary embodiment, the cloth 13100 is oriented and cut such that the high strength fibers 13102 extend in a direction that is perpendicular to the direction indicated by arrow 9502 (See Figures 95I, 96B, and 96C). In the examples illustrated by Figures 95I, 96B, and 96C, the direction that is perpendicular to the direction 9502 corresponds to a line that is parallel to an edge 9565 of the connection portions 9564 and/or to the edge 9558.

Referring to Figure 96, the anchor 122 is illustrated in a deployed state. The deployed state of the anchor 122 can be a variety of shapes. Any shape that can be used to pull a portion of a heart wall inward to remodel the shape of the heart wall can be used, such as for example, a curved shape. In the illustrated embodiment, the anchor 122 is ring-shaped in the deployed state such that the first end portion 1430 is adjacent the second end portion 1432 and the body 1426 is curved in a circle. In some embodiments, the first end portion 1430 and the second end portion 1432 may abut. In other embodiments, the first end portion 1430 and the second end portion 1432 may not abut. For example, the first end portion 1430 and the second end portion 1432 can be spaced apart from one another or may overlap,

The anchor 122 can be reshaped from the elongated state to the deployed state in a variety of ways. For example, the anchor 122 may include a shape-memory alloy and be shape set to the shape of the deployed state. Alternatively, or in conjunction with the anchor being shape set, the line 124 can be used to reshape the anchor 122. In particular, since the line loop 1440 passes through each of the loops 1442, by pulling on the line 124, the line loop 1440 is withdrawn which pulls the first end portion 1430 and second end portion 1432 together while bending the body 1426 into an annulus.

Referring to Figure 96A, the anchor 1422 is illustrated in a deployed state. The deployed state of the anchor 1422 can be a variety of shapes. Any shape that can be used to pull a portion of a heart wall inward to remodel the shape of the heart wall can be used, such as for example, a curved shape. In the illustrated embodiment, the anchor 1422 is ring-shaped in the deployed state.

The anchor 1422 can be reshaped from the elongated state to the deployed state in a variety of ways. For example, the anchor 1422 can include a shape-memory alloy and be shape set to the shape of the deployed state. Alternatively, or in conjunction with the anchor being shape set, the line 1424 can be used to reshape the anchor 1422. In particular, since the line loop 1440 passes through the second passage 9572, by pulling on the line 1424, the line loop 1440 decreases in size. The decreasing size of the line loop 1440 pulls the bridges 9564 toward one another, which pulls the anchor 1422 into an annular shape. The bridges 9564 on the connector body converge toward a center point of the annulus. Since the overlapping tapering sections 9560, 9562 are generally triangular, when the connector body into pulled into an annulus, the overlapping tapering sections 9560, 9562 are pulled together to substantially form a solid disc, which can be pulled against tissue, such as heart wall tissue.

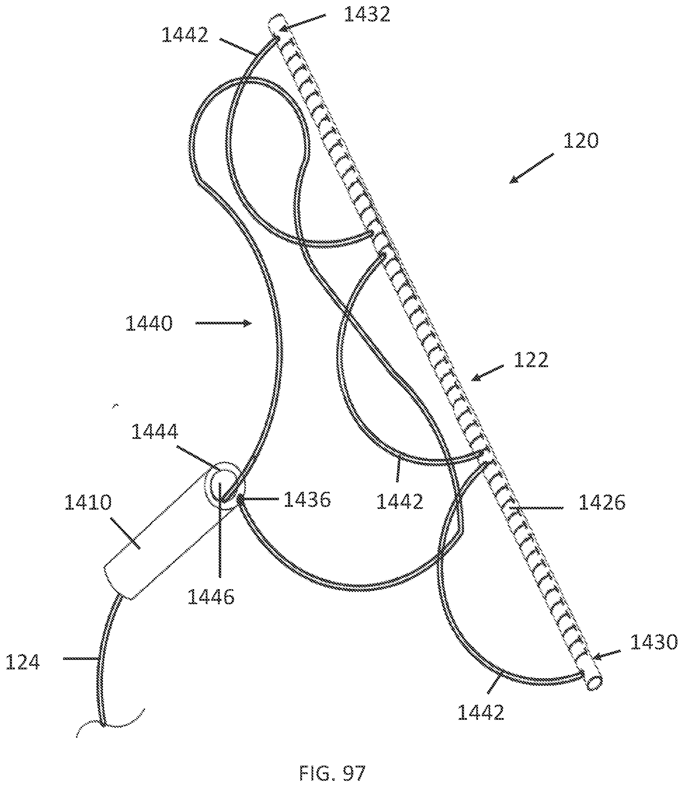

In Figure 97, the device 120 includes the hemostatic plug 1410. The hemostatic plug 1410 can be configured in a variety of ways. Any device that can stop bleeding from occurring from a passage formed by the piercing device 130 in a heart wall can be used. In the illustrated embodiment, the hemostatic plug 1410 is formed as a cylindrical tube having a distal end 1444 and a central passage 1446 through which the line 124 extends. In other embodiments, however, the hemostatic plug 1410 can be configured other than cylindrical.

In the illustrated embodiment, the terminal end 1436 of the line 124 is attached to the distal end 1444 of the hemostatic plug 1410. The line 124 forms the loop 1440 and is connected to the anchor 122 by passing through each of the loops 1442.

Referring to Figure 98, the anchor 122 is illustrated in a deployed state. In the illustrated embodiment, the anchor 122 is ring-shaped in the deployed state such that the first end portion 1430 is adjacent the second end portion 1432, the body 1426 is curved in a circle, and the distal end 1444 of the hemostatic plug 1410 is generally adjacent the center of the circle.

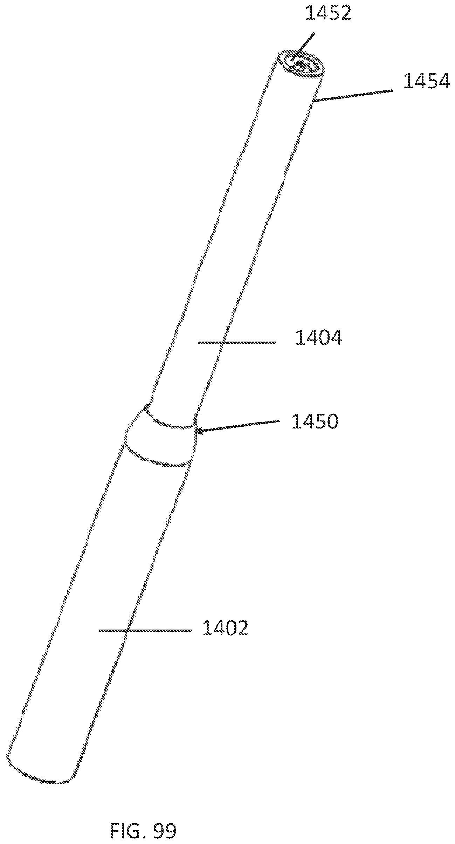

In Figure 99, an exemplary embodiment of a portion of the guide sheath 1402 and a portion of the steerable catheter 1404 is illustrated. Both the guide sheath 1402 and the steerable catheter 1404 can be configured in a variety of ways. Any suitable known guide sheath 1402 and steerable catheter 1404 can be used. In the illustrated embodiment, the guide sheath 1402 and the steerable catheter 1404 are concentric where the guide sheath 1402 includes an inner lumen (not shown) and the steerable catheter extends through the inner lumen and out of a distal end 1450 of the guide sheath 1402. Likewise, the steerable catheter 1404 includes an inner lumen 1452 that is open at a distal end 1454 of the steerable catheter 1404.

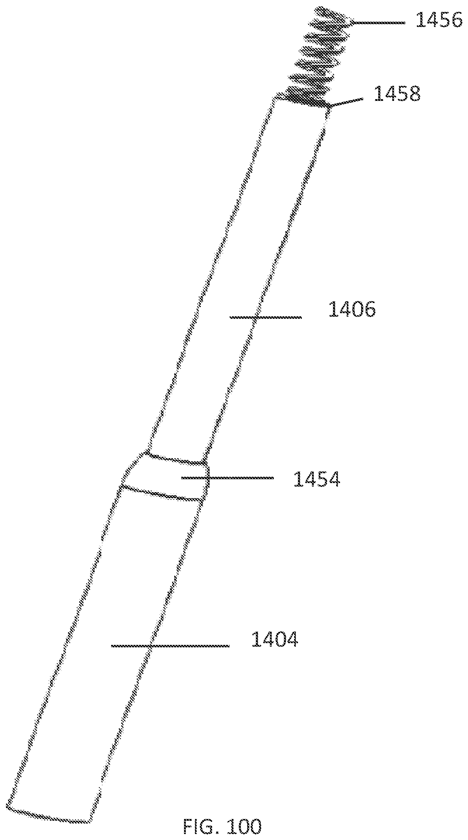

In Figure 100, a portion of the steerable catheter 1404 and a portion of the delivery catheter 1406 are illustrated. The delivery catheter 1406 includes an anchoring device 1456 attached to a distal end 1458 of the delivery catheter 1406. The anchoring device 1456 can be any suitable device capable of attaching to the heart wall W. In the illustrated embodiment, the anchoring device 1456 is a wire formed in a helical shape configured to be screwed into the heart wall W to secure the delivery catheter 1406 to the heart wall W. The delivery catheter 1406 includes an inner lumen (not shown) open at the distal end 1458.

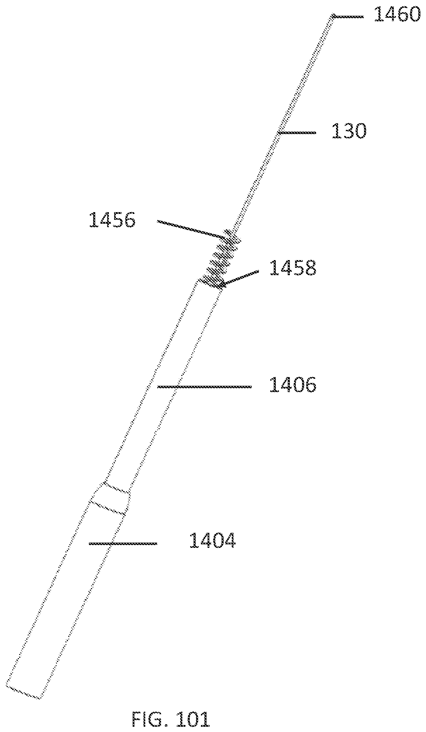

In Figure 101, a portion of the steerable catheter 1404, a portion of the delivery catheter 1406, and the piercing device 130 are illustrated. The piercing device 130 can be any suitable device for piercing or creating a passage into a human heart wall, such as for example, a needle, wire, or other similar device. In the illustrated embodiment, the piercing device 130 is a needle or hollow wire having an inner passage (not shown) and an opening (not shown) proximate a distal end 1460 of the piercing device 130 the fluidly connects the inner passage (not shown) to the exterior of the piercing device 130.

In the illustrated embodiment, the piercing device 130 is delivered through the inner lumen (not shown) of the delivery catheter 1406 and extends out of the distal end 1458 of the delivery catheter 1406.

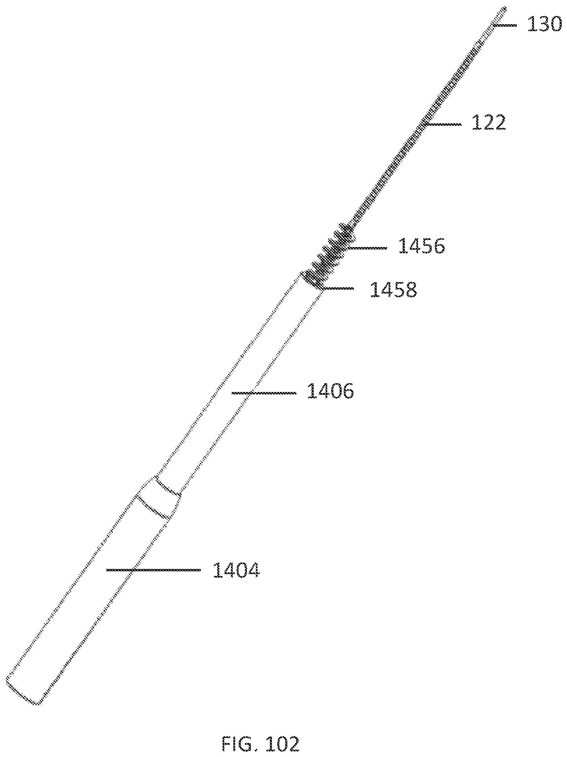

In Figure 102, a portion of the steerable catheter 1404, a portion of the delivery catheter 1406, the piercing device 130, and the anchor 122 are illustrated. The anchor 122 is shown in the elongated state and it extends from the distal end 1458 of the delivery catheter 1406 over top of the piercing device 130 such that the piercing device 130 is received in the central passage 1428 of the anchor 122 and the anchor 122 and the piercing device 130 are concentric.

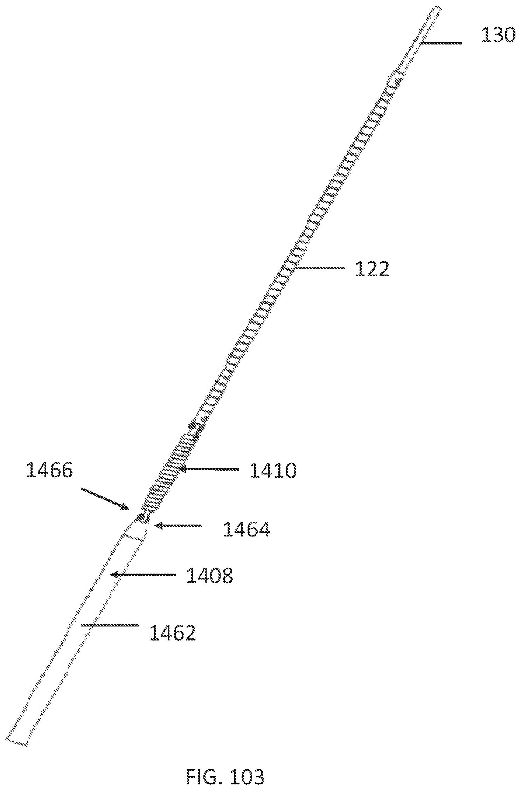

In Figure 103, exemplary embodiments of the piercing device 130, the anchor 122, the hemostatic plug 1410, and the pusher 1408 are illustrated. The pusher 1408 can be configured in a variety of ways. Any configuration capable of pushing the hemostatic plug 1410 and anchor 122 over the piercing device 130 and beyond the distal end 1460 of the piercing device 130 can be used. In the illustrated embodiment, the pusher 1408 has an elongated, cylindrical body 1462 having a distal end 1464 configured to abut a proximal end 1466 of the hemostatic plug 1410. The body 1462 includes an inner passage (not shown) extending through the pusher body 1462. The piercing device 130 can be received in the passage (not shown) such that the pusher 1408 is slidable over the piercing device 130 and concentric with the piercing device 130.

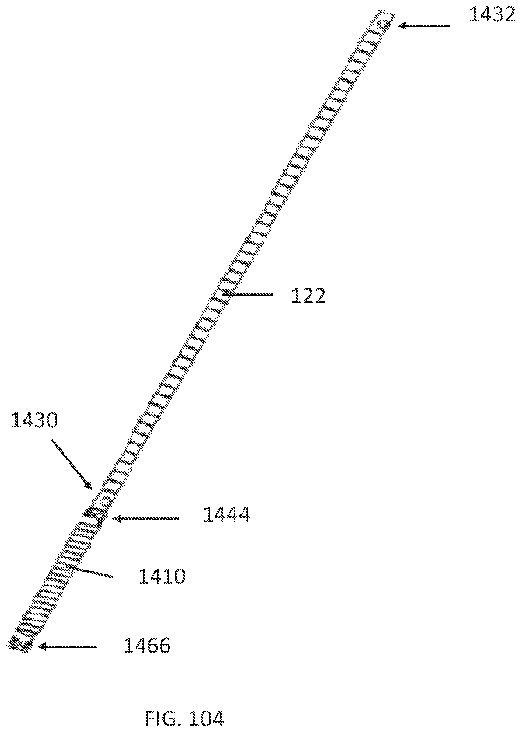

**In** Figure 104, the anchor 122 and the hemostatic plug 1410 are illustrated. The anchor 122 is shown in the elongated state. and the hemostatic plug 1410 is illustrated as a cylindrical tube. Both the anchor 122 and the hemostatic plug 1410 are configured to be concentric with and slidable over the piercing device 130. When being delivered over the piercing device 130, the anchor 122 and the hemostatic plug 1410 are longitudinally aligned, with the distal end 1444 of the hemostatic plug 1410 adjacent the first end 1430 of the anchor 122, as shown in Figure 104.

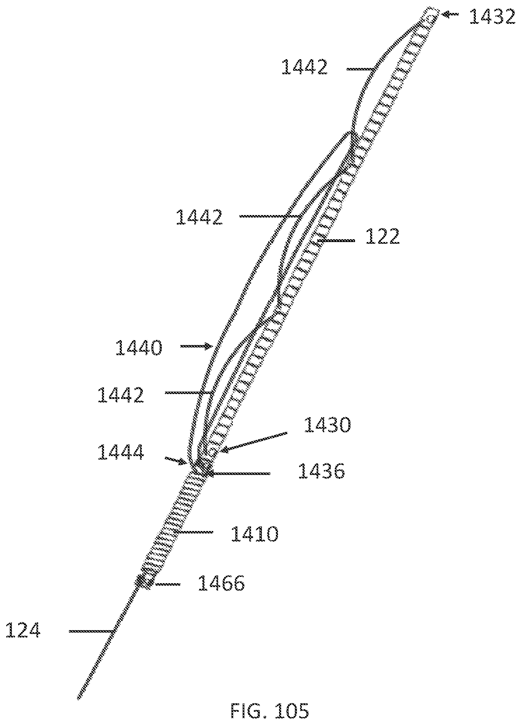

**In** Figure 105, the anchor 122 and the hemostatic plug 1410 are illustrated along with the line 124 and the loops 1442 on the anchor 122. The terminal end 1436 of the line 124 is attached to the distal end 1444 of the hemostatic plug 1410. The loop 1440 of the line 124 passes through each of the loops 1442 to connect the line 124 to the anchor 122. The line 124 then extends through the central passage 1446 (Figure 97) of the hemostatic plug from the distal end 1444 to the proximal end 1466.

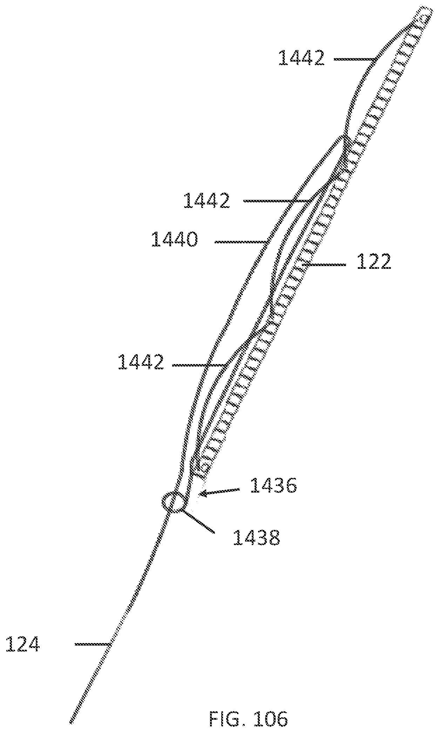

**In** Figure 106, the anchor 122 without a hemostatic plug 1410 is illustrated along with the line 124 and the loops 1442 on the anchor 122. The terminal end 1436 of the line 124 is formed in a closed, circular shape 1438 and the line 124 is arranged such that the line 124 passes through the closed-shaped terminal end 1436. The loop 1440 of the line 124 passes through each of the loops 1442 to connect the line 124 to the anchor 122.

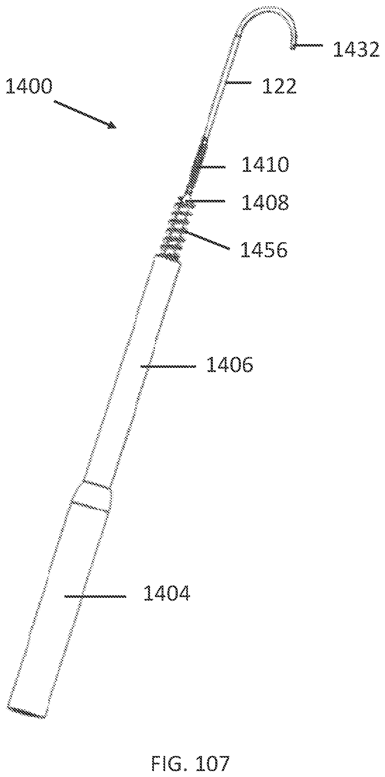

**In** Figure 107, the system 1400 is illustrated showing the anchor 122 partially deployed beyond the distal end 1460 of the piercing device 130 (Figure 101). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the proximal end 1466 of the hemostatic plug 1410 to push both the hemostatic plug 1410 and the anchor 122 over the piercing device 130. As the distal end 1432 of the anchor 122 moves beyond the distal end 1460 of the piercing device 130 (Figure 101), the anchor 122 may begin to reshape into its curved deployed state. For example, the anchor 122 may include a shape memory alloy that is shape set to the curved deployed state. Thus, the portion of the anchor 122 that is no longer received on the piercing device 130 may revert to the curved deployed state that that shape memory alloy was set to.

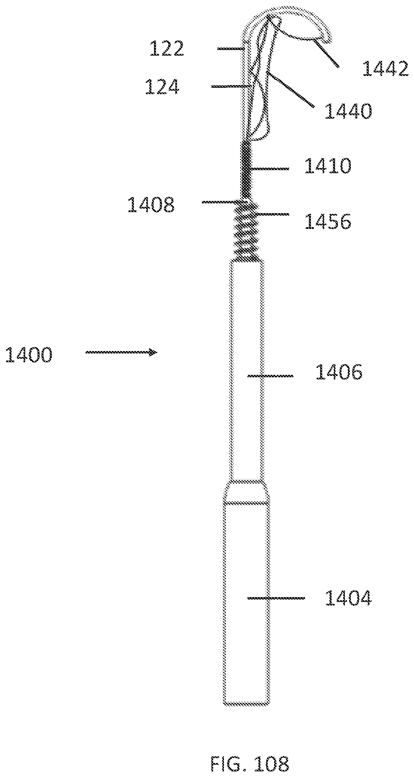

In Figure 108, the system 1400 is illustrated showing the anchor 122 partially deployed beyond the distal end 1460 of the piercing device 130 (Figure 101) along with the line 124 connected to the anchor 122. In particular, the line 124 connects to the hemostatic plug 1410, extends through the loops 1442, and then loops around and extend though the central passage 1446 (Figure 97) of the hemostatic plug 1410.

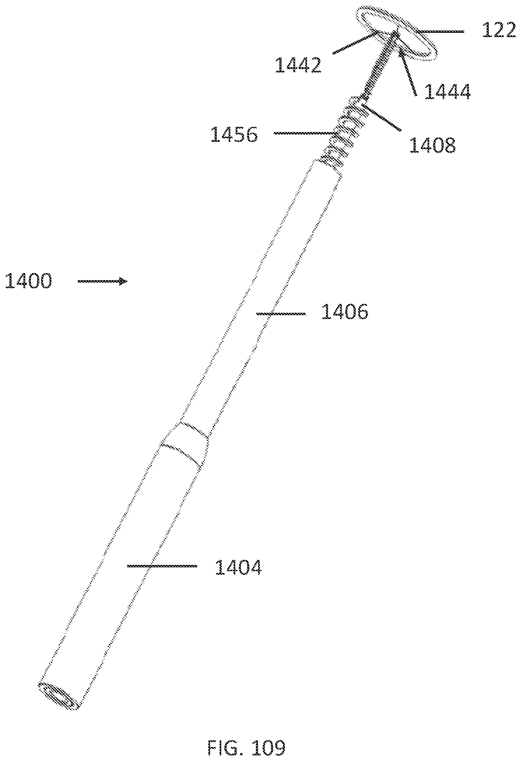

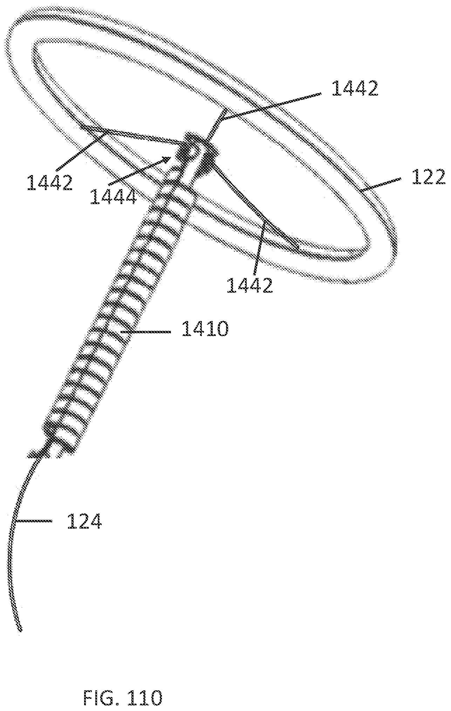

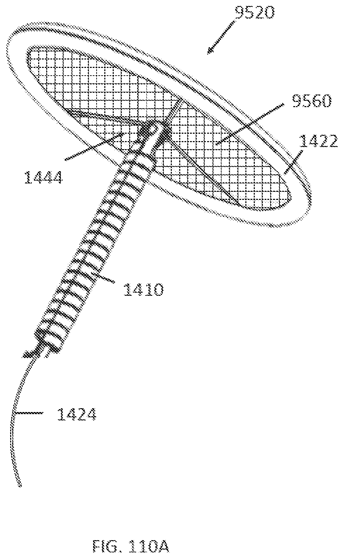

In Figures 109-110, the system 1400 is shown with the anchor 122 in the deployed state. In particular, the line 124 (Figure 110) is withdrawn by pulling the line 124 in a direction away from the anchor and into the delivery catheter 1406. Since the loop 1440 (Figure 97) of the line 124 passes through the loops 1442 on the anchor 122, pulling the line 124 closes the loop 1440 and pulls the loops 1442 together to help facilitate, along with any shape setting properties of the anchor, reshaping the anchor 122 from the elongated state to the curved deployed state shown in Figures 109-110. At the same time, the distal end 1444 of the hemostatic plug 1410 is positioned adjacent the center of the curved anchor 122.

Similarly, in Figure 110A, the system is shown with the anchor 1422 in the deployed state. In particular, the line 1424 is withdrawn by pulling the line 1424 in a direction away from the anchor 1422 and into the delivery catheter 1406. Since the loop 1440 (Figure 96A) of the line 1424 passes through the second passage 9572 on the anchor 1422, pulling the line 1424 closes the loop 1440 and pulls the overlapping tapering sections 9560, 9562 together to help facilitate, along with any shape setting properties of the anchor, reshaping the anchor 1422 from the elongated or undeployed state to the curved deployed state. At the same time, the distal end 1444 of the hemostatic plug 1410 is positioned adjacent the center of the curved anchor 1422.

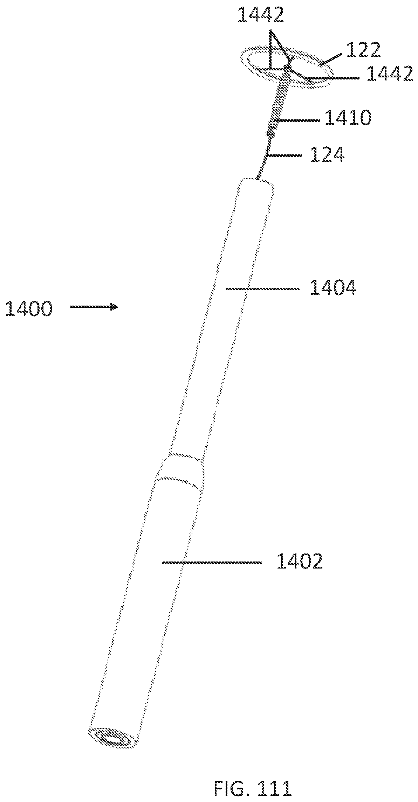

In Figure 111, the system 1400 is shown with the anchor 122 in the deployed state and the delivery catheter 1406 retracted back into the steerable catheter 1404. The line 124 (see Figure 110) is withdrawn by pulling the line 124 in a direction away from the anchor and into the delivery catheter 1406. Since the loop 1440 (Figure 97) of the line 124 passes through the loops 1442 on the anchor 122, pulling the line 124 closes the loop 1440 and pulls the loops 1442 together to help facilitate, along with any shape setting properties of the anchor, reshaping the anchor 122 from the elongated state to the curved deployed state shown in Figures 109-110. At the same time, the distal end 1444 of the hemostatic plug 1410 is pulled up to the center of the curved anchor 122 as the loop 1440 is shortened by pulling the line 124.

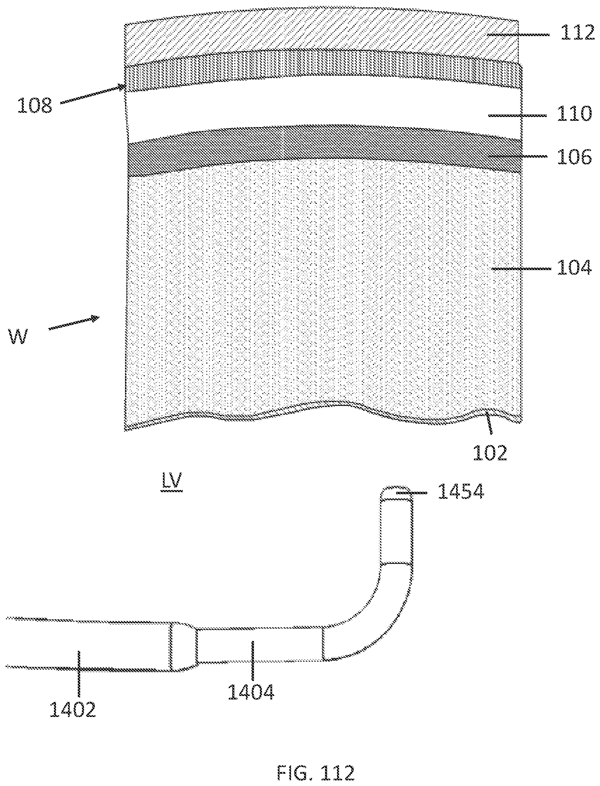

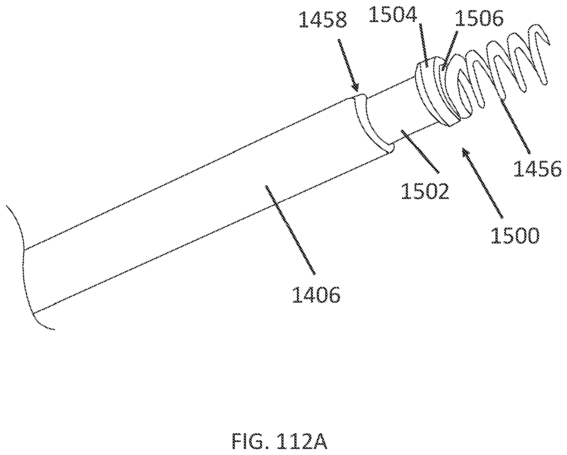

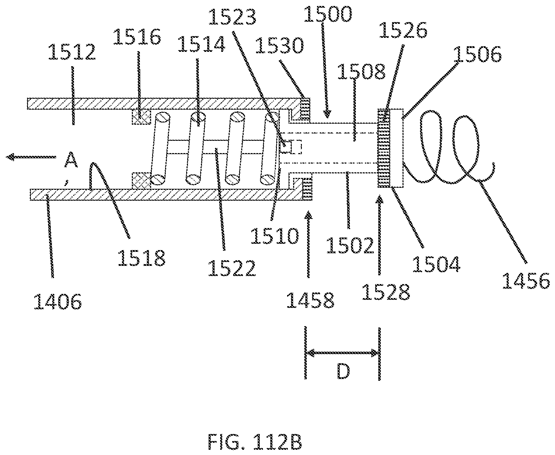

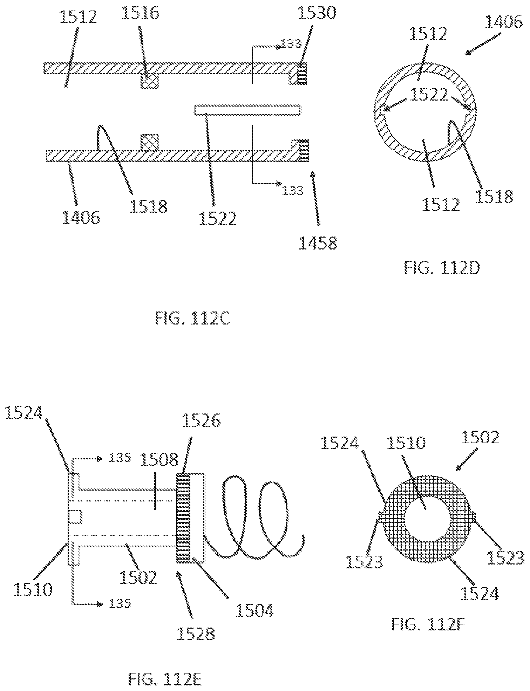

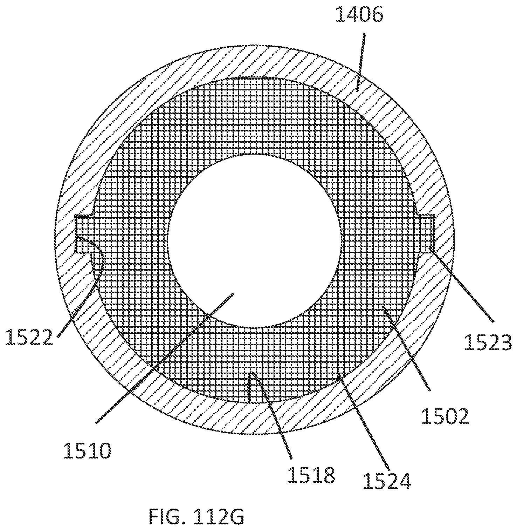

Referring to Figures 112-120, the deployment of the device 120 into the pericardial cavity 110 for remodeling the shape of a heart wall W and system and method for delivering the device 120 is illustrated. Referring to Figure 112, deployment of the device 120 includes delivering the guide sheath 1402 and the steerable catheter 1404 into an internal chamber (e.g. left ventricle LV) of the heart H. The steerable catheter 1404 is arranged such that the distal end 1454 of the steerable catheter 1404 is adjacent the heart wall W.

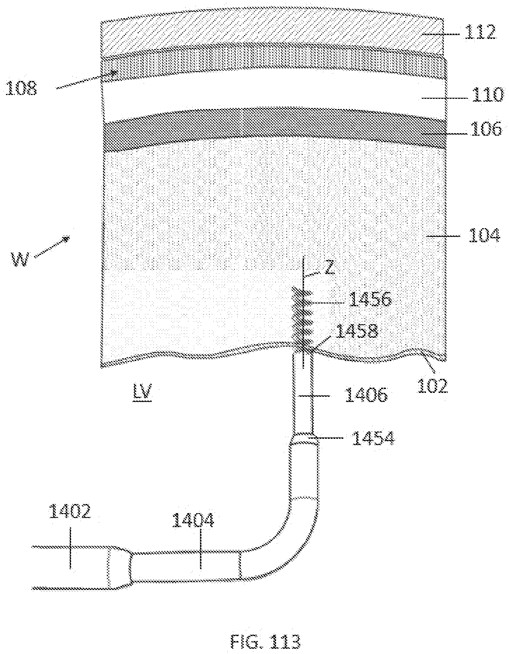

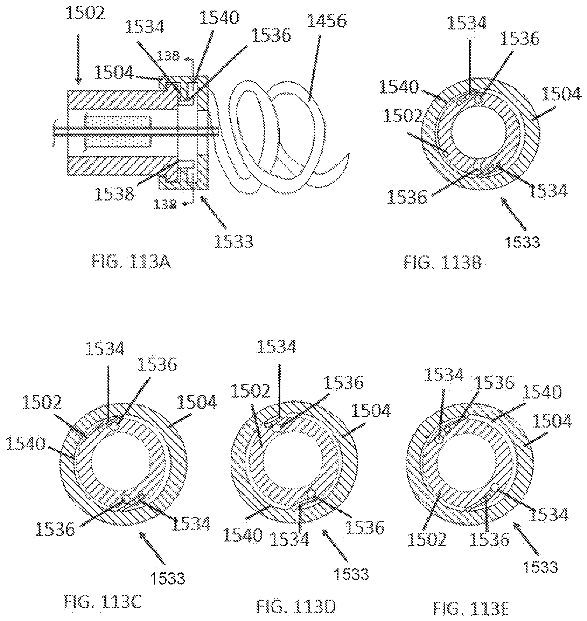

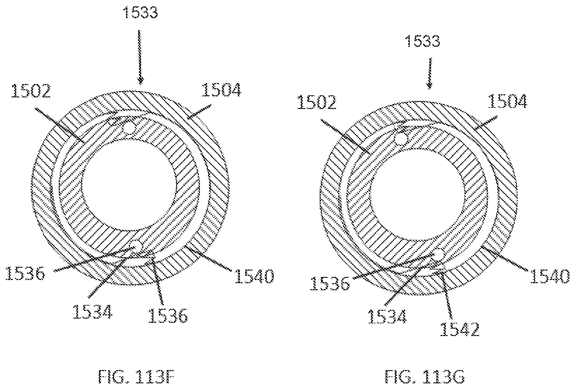

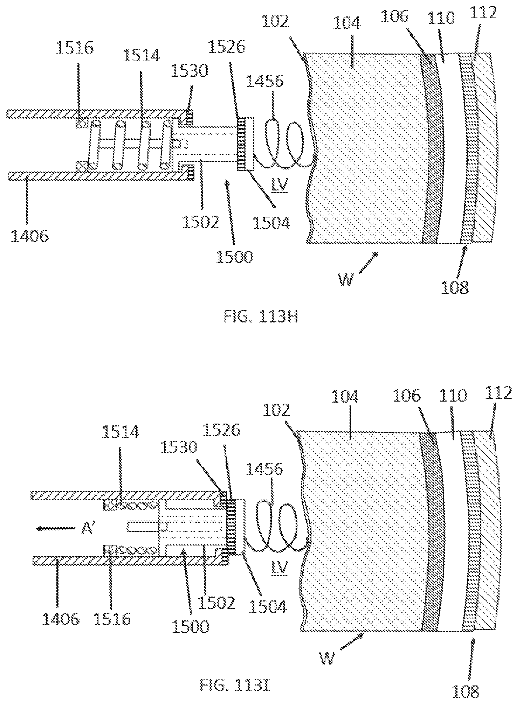

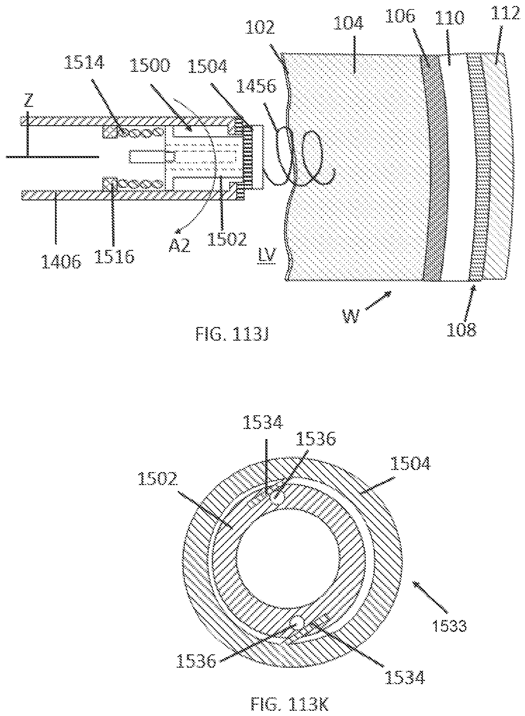

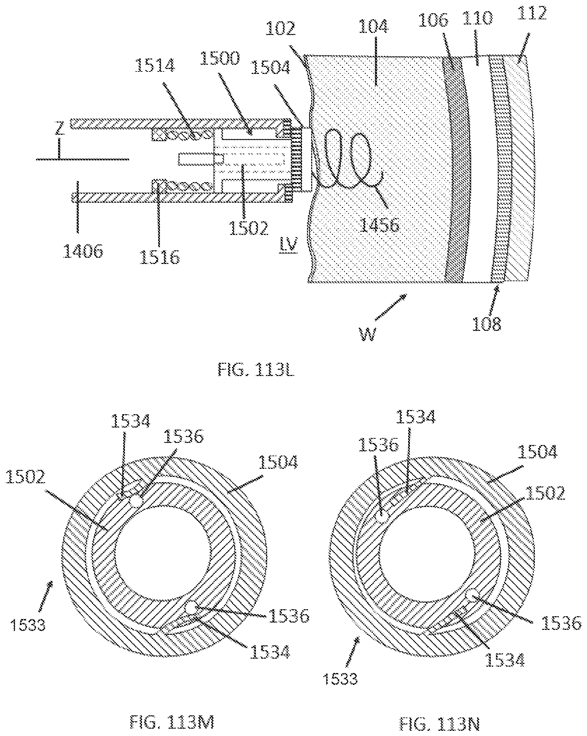

Referring to Figure 113, the delivery catheter 1406 is extended from the distal end 1454 of the steerable catheter 1404. The anchoring device 1456 of the delivery catheter 1406 is attached to the heart wall W, such as for example by rotating the delivery catheter 1406 about axis Z relative to the steerable catheter 1404 to screw the anchoring device 1456 into the heart wall (i.e. through the endocardium 102 and into the myocardium 104). In the illustrated embodiment, the distal end 1458 of the delivery catheter 1406 abuts, or is adjacent, the endocardium 102.

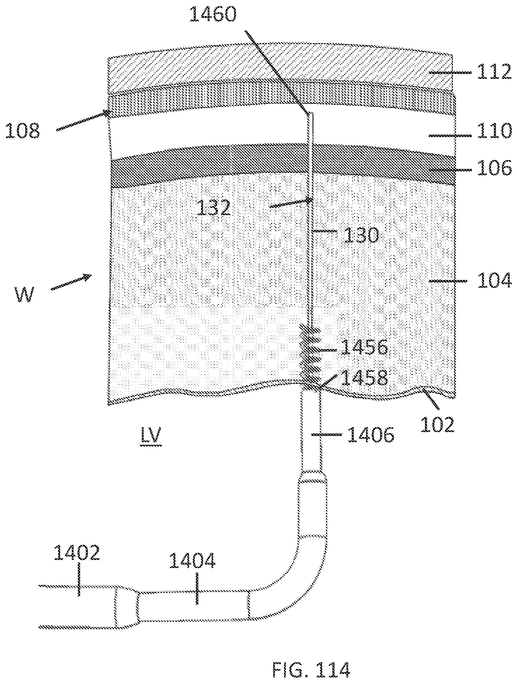

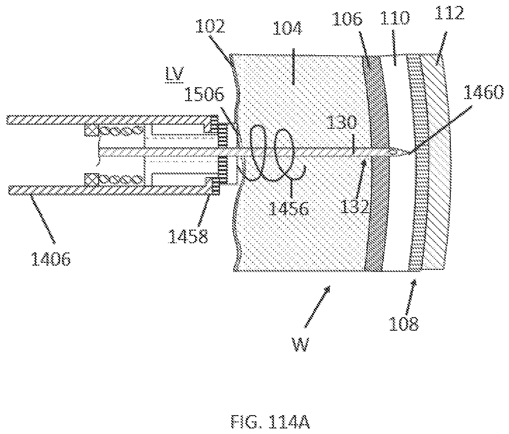

Referring to Figure 114, the piercing device 130 is delivered into an internal chamber (e.g. left ventricle LV) of the heart H via the delivery catheter 1406. The piercing device 130 can be extended from the distal end 1458 of the delivery catheter 1406 such that the distal end 1460 of the piercing device 130 is extended into the heart wall W. In the illustrated embodiment, the distal end 1460 of the piercing device 130 is extended through the endocardium 102, the myocardium 104, the epicardium 106, and into the pericardial cavity 110 to form the passage 132. Since the delivery catheter 1406 is anchored to the heart wall W, the location for insertion of the piercing device 130 can be precisely controlled.

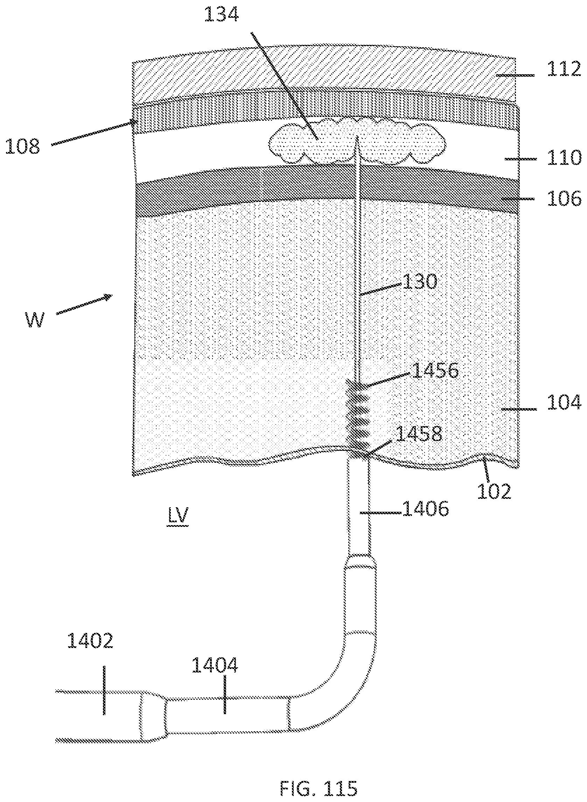

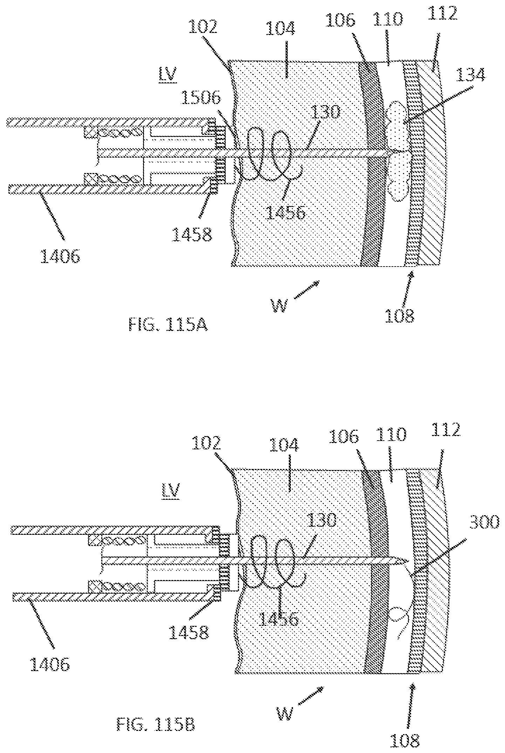

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 115. The dye 134 can be detected by any suitable technique such as X-ray, to verify that the piercing device 130 is properly positioned.

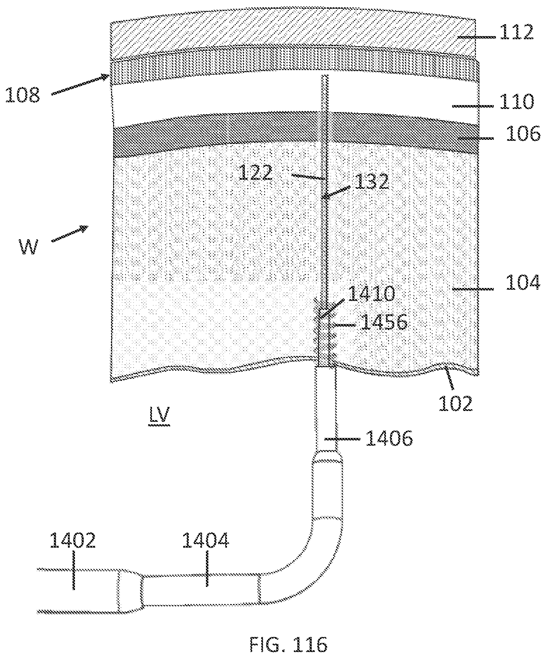

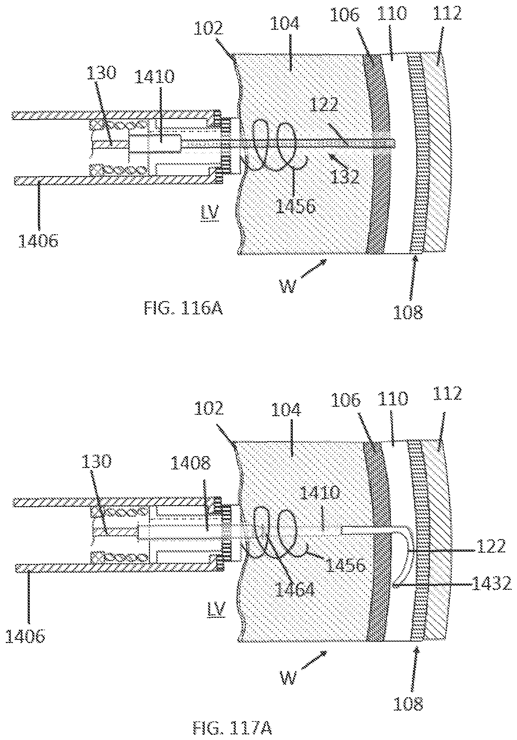

In Figure 116, the anchor 122 and the hemostatic plug 1410 are extended from the delivery catheter 1406 over the piercing device 130 (Figure 115) and through the passage 132. As shown in Figure 116, the anchor 122 remains in the elongated state while sliding along the piercing device 130 into the pericardial cavity 110.

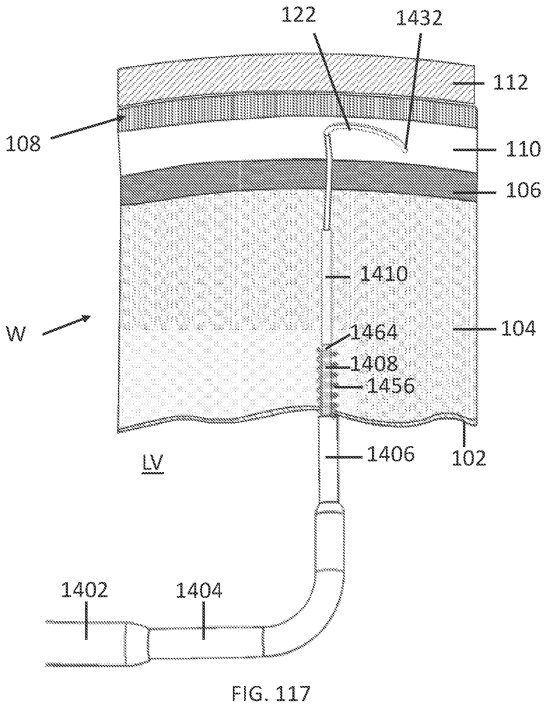

In Figure 117, the anchor 122 is partially deployed beyond the distal end 1460 (Figure 114) of the piercing device 130 (Figure 101). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the hemostatic plug 1410 to push both the hemostatic plug 1410 and the anchor 122 over the piercing device 130 (Figure 101). As the distal end 1432 of the anchor 122 moves beyond the distal end 1460 of the piercing device 130 (Figure 101), the anchor 122 may begin to reshape into its curved deployed state. For example, the anchor 122 may include a shape memory alloy that is shape set to the curved deployed state. Thus, the portion of the anchor 122 that is no longer received on the piercing device 130 may revert to the curved deployed state that that shape memory alloy was set to.

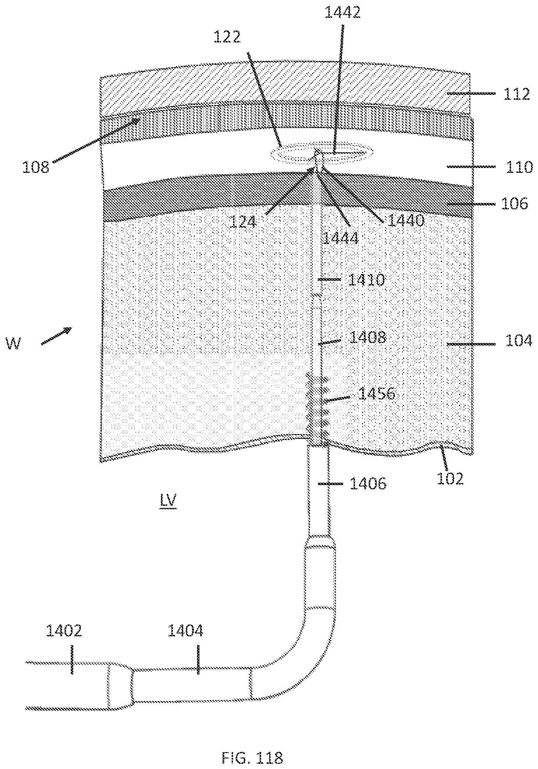

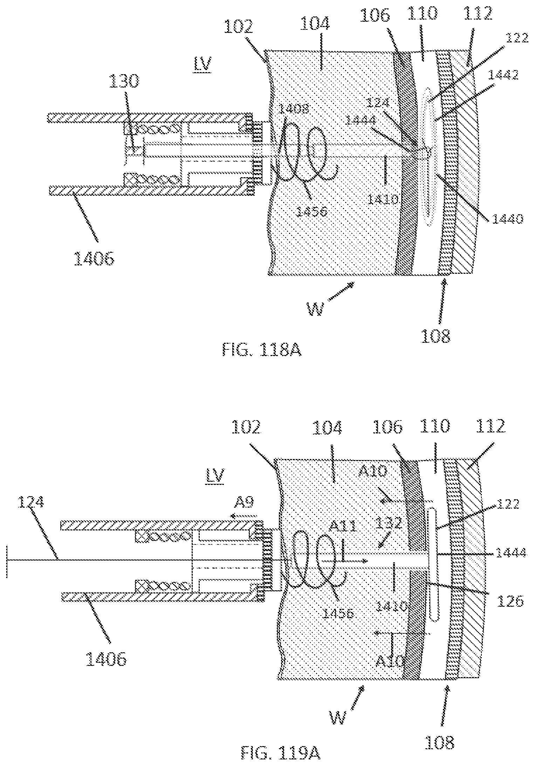

In Figure 118, the anchor 122 is in the deployed state and the hemostatic plug 1410 is positioned within the passage 132 to prevent bleeding from the passage 132. In the illustrated embodiment, the distal end 1444 of the hemostatic plug 1410 is at or near the inner wall of the pericardial cavity 110. To reshape the anchor 122, the line 124 is withdrawn by pulling the line 124 in a direction away from the anchor and into the hemostatic plug 1410. Since the loop 1440 (Figure 97) of the line 124 passes through the loops 1442 on the anchor 122, pulling the line 124 closes the loop 1440, pulls the hemostatic plug 1410 and the anchor 122 together, and pulls the loops 1442 together to help facilitate, along with any shape setting properties of the anchor 122, reshaping the anchor 122 from the elongated state to the curved deployed state shown in Figure 118.

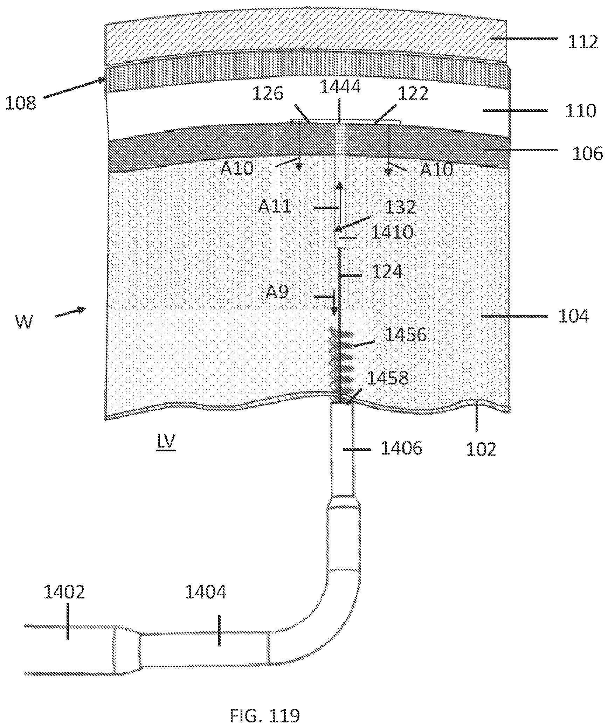

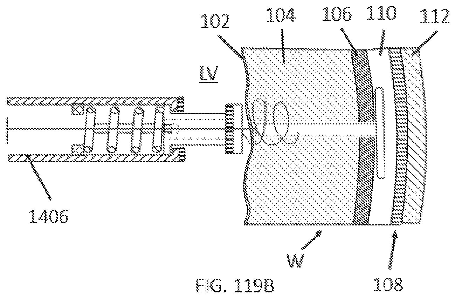

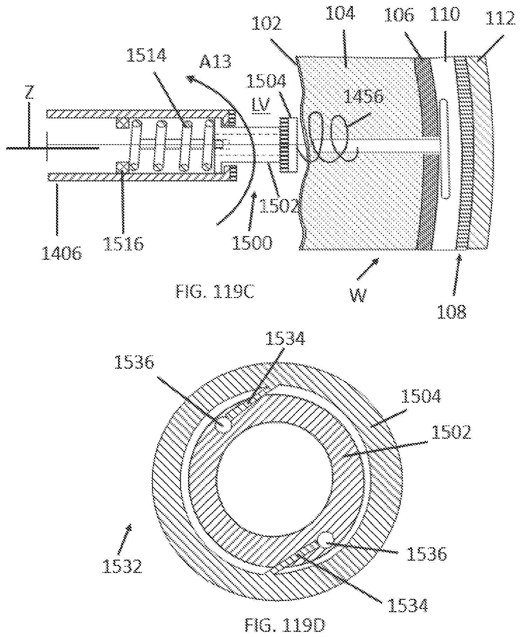

As shown in Figures 119-120, the pusher 1408 and piercing device can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, and the endocardium 102.

To seat the anchor 122, the delivery catheter 1406 may remain attached to the heart wall W and the distal end 1458 can be pushed against the heart wall W. At the same time, the line 124 can be placed in tension by pulling the line 124 in an inward direction toward the heart chamber as shown by the arrow A9. As a result, the anchor 122 will be pulled against the outward facing surface 126 that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130. Thus, once the delivery catheter 1406 is removed, further tensioning of the line 124 can pull the heart wall W inward toward the heart chamber (e.g., the left ventricle LV).

Referring to Figures 112A-112G, 113A-113N, 114A, 115A, 115B, 116A, 117A, 118A, 119A-119D, 120A, system 1400 and method for delivering the device 120 against a surface of a heart can include piston 1500. Piston 1500 can be disposed between and coupled with delivery catheter 1406 and anchoring device 1456.

Referring to Figures 112A-112B, a portion of the delivery catheter 1406, piston 1500, and anchoring device 1456 are illustrated. Piston 1500 includes piston body 1502 coupled with piston head 1504. Anchoring device 1456 can be coupled with distal end 1506 of piston head 1504.

Referring to Figure 112B, delivery catheter 1406 can include an inner lumen 1512 that extends through the distal end 1458 of the delivery catheter 1406. Piston 1500 can comprise inner lumen 1508 extending through piston body 1502 and piston head 1504. Inner lumen 1508 is open at distal end 1506 of piston head 1504 and proximal end 1510 of piston body 1502.

Proximal end 1510 of piston body 1502 is positioned within the inner lumen 1512 of delivery catheter 1406 between a resistance member 1514 and distal end 1458 of delivery catheter 1406. Piston body 1502 can be configured to slide through distal end 1458 of the delivery catheter 1406 and at least partially into inner lumen 1512 of delivery catheter 1406.

Delivery catheter 1406 can include stopper 1516 coupled to inner wall 1518 of delivery catheter 1406. Stopper 1516 couples with and secures the proximal end 1510 of resistance member 1514 in place.

Piston 1500 can be configured to slide in distal end 1458 of the delivery catheter 1406. Resistance member 1514 resists the sliding motion of piston 1500 and biases the piston 1500 toward an extended position. Resistance member 1514 can comprise a variety of shapes and materials. Resistance member 1514 can be a spring, as shown in Figure 112B, a wire formed into a helical shape, or a spring-loaded or force-dampening material.

The piston 1500 and delivery catheter 1406 can have one or more recesses 1522 that slidably engage one or more protrusions 1523. The mating of the recesses 1522 with the protrusions 1523 couples the piston 1500 and delivery catheter 1406, such that the piston 1500 and delivery catheter 1406 can axially slide relative to one another but cannot rotate relative to one another. The resistance member 1514 biases the piston 1500 distally out of the delivery catheter 1406 can include slot 1522. That is, the piston 1500 engages with the delivery catheter 1406 to prevent the rotation of the piston 1500 with respect to the delivery catheter 1406. This engagement can take place in a variety of ways other than the illustrated slot and protrusion arrangement.

Figure 112D illustrates a cross section of a portion of delivery catheter 1406 as shown in Figure 112C. In the example illustrated by Figures 112C and 112D, the recess 1522 is a slot or a plurality of slots in the inner wall 1518 of delivery catheter 1406. Figure 112F illustrates a cross section of proximal end 1510 of piston body 1502, as shown in Figure 112E. In the illustrated example, piston body 1502 includes the projection 1523 extending from outer wall 1524 of piston body 1502. Piston body 1502 can include a plurality of projections 1523. Projection 1523 can comprise a variety of sizes and shapes. Slot 1522 can comprise a shape complementary to projection 1523.

In the example illustrated by Figure 112G, an outer wall 1524 of the proximal end 1510 of piston body 1502 can engages with inner wall 1518 of delivery catheter 1406. The coupling of proximal end 1510 of piston body 1502 with the inner wall 1518 of delivery catheter 1406 allows the piston 1500 to slide into delivery catheter 1406.As delivery catheter 1406 is rotated, the engagement of projection 1523 with slot 1522 rotates piston 1500 with delivery catheter 1406.

With reference back to Figure 112B, piston 1500 is shown in a non-engaged position, characterized by proximal end 1510 of piston body 1502 in contact with the resistance member 1514, inner wall 1518, and distal end 1458 of delivery catheter 1406. A force in a direction A' can be exerted on anchoring device 1456, which in turn exerts a force on piston 1500 and moves piston 1500 proximally relative to the delivery catheter 1406, in the direction A'. The force on anchoring device 1456 can cause piston head 1504 to contact distal end 1458 of delivery catheter 1406. This position shown in Figure 113I and can be referred to as the "activated or engaged position." Force can be applied by pushing anchor 1456 of delivery catheter 1406 against heart wall W or any other material in contact with anchoring device 1456.

The relative position of piston 1500 and delivery catheter 1406 can be detected. A minimum or an optimal force at which anchoring device 1456 is pressed against the material before rotation can be detected based on the relative position of piston 1500 and delivery catheter 1406. Engagement of the piston head 1504 with the distal end 1458 of delivery catheter 1406 (the compressed or activated position) can indicate to a user that anchoring device 1456 is in suitable engagement for use. For example, the engaged or activated position can indicate that anchoring device 1456 is pressed against heart wall W at a suitable pressure for the temporary implanting of the anchoring device 1456 to the heart.

A user can determine if additional force should be applied to anchoring device 1456 by observing the relative position of the piston head 1504 with respect to the distal end 1458 of delivery catheter 1406. With reference to Figures 112B and 112E, to detect the relative position of piston head 1504 with respect to the distal end 1458 of delivery catheter 1406, piston head 1504 and/or catheter 1406 can include a marker 1526, 1530, respectively. The marker 1526 can be located on proximal end 1528 of piston head 1504 as illustrated or any other portion of the piston head. With reference to Figures 112B and 112C, the delivery catheter 1406 marker 1530 can be located on distal end 1458 of delivery catheter 1406.

Referring to Figure 112B, in the non-activated or extended position, marker 1526 is distance D from marker 1530. Piston 1500 can travel in the A' direction upon exertion of a force, for example as anchoring device is pressed against a material, such as a heart wall, such as the endocardium and/or papillary muscle in a ventricle. As piston 1500 travels in the direction A', the distance between marker 1526 and the distal end of delivery catheter 1406 decreases. The distance between marker 1526 and the marker 1530 also decreases. Marker 1526 can abut marker 1530 when the piston 1500 reaches the compressed or activated position. A user can be able to detect the relative position of piston 1500 and delivery catheter 1406 by tracking the position of the marker 1526 and marker 1530 using a visual scope, an electrical sensor that detects contact between markers 1526, 1530, or other suitable means.

The contact, positioning of, and/or pressure applied to anchoring device 1456 and piston 1500 can be determined by a variety of other means. For example, marker 1530 and marker 1526 can be otherwise positioned or replaced. Delivery catheter 1406 or piston 1500 can comprise, for example a force measuring device or pressure sensing device and means for communicating the pressure or force to the user, such as a visual display, indicator light, audible indicator, etc...

Anchoring device 1456 can be rotated into the receiving material, for example heart wall W. As anchoring device 1456 is further rotated into the receiving material, the amount of torque required can increase, until a "torque threshold" is reached. The torque threshold for optimal engagement of the anchoring device 1456 into heart wall W can be pre-determined based on many factors, including the composition of the receiving material, the size shape of the anchoring device, and the distance that the anchoring device must travel into the receiving material. In one exemplary embodiment, the torque threshold is based on a force increase that occurs when the anchor 1456 bottoms out on the tissue. For example, during initial engagement of the anchor 1456 with the tissue, the torque will increase gradually as more of the anchor is engaged in the tissue. But, when the anchor 1456 bottoms out, the torque required to continue rotating the catheter 1406 spikes. In one exemplary embodiment, the "threshold torque" is set between the between the gradually increasing torque and the torque spike that results from bottoming out of the anchor.

With reference to Figure 113A-113B, system 1400 can include an over-torque prevention device 1533 designed to prevent excessive torque of the anchoring device 1456 into the heart wall W. Over-torque can occur when the torque applied to the anchoring device 1456 is greater than the pre-determined torque threshold. Over-torque prevention device 1533 can comprise a clutch mechanism configured to de-couple the delivery catheter 1406 from the piston 1500 and/or anchoring device 1456 when the torque applied to the anchoring device 1456 is greater than the pre-determined torque threshold. The clutch mechanism automatically allows for the rotation of delivery catheter 1406 and piston 1500 separate from the rotation of anchoring device 1456 when the threshold torque is reached, and thus prevents over rotation of anchoring device 1456 into the receiving material. In another exemplary embodiment, the clutch mechanism can be between the delivery catheter 1406 and the piston 1500, instead of between the piston 1500 and the anchor 1456.

The over-torque prevention devise can take a wide variety of different forms. For example, any clutch mechanism can be used. For example, in the example illustrated by Figures 113A-113E, the piston head includes a cap that is rotatably connected to the piston body. The over-torque prevention device 1533 can include one or more arms 1534 extending from inner wall 1540 of the rotatable cap piston head 1504. Arm 1534 can be configured to contact pin 1536 extending distally from distal end 1538 of piston body 1502. Distal end 1538 of piston body 1502 can include one or a plurality of pins 1536.

In the illustrated example, the cap piston head 1504 includes two arms 1534 positioned about 180 degrees apart, and distal end 1538 of piston body 1502 includes two pins 1536 positioned 180 degrees apart. However, any number of arms and pins can be used. The torque threshold value can be altered by various means, including by increasing or decreasing the length of arms 1548 relative to the size of the pins 1550.

Figures 113B-113C correspond to situations where the torque applied to the delivery catheter 1406 and anchor 1456 is less than a pre-determined torque threshold. With reference to Figure 113C, when delivery catheter 1406 is rotated, pins 1536 contact and push arms 1534. Due to this contact, piston head 1504 and anchoring device 1456 rotate at the same rate as delivery catheter 1406 and piston body 1502. Arms 1534 making contact with pins 1536 can result in a resistance experienced by a user applying the torque. The resistance can signify that the torque threshold has not been reached or exceeded by the applied force.

Figures 113D and 113E illustrates situations where the torque applied to the delivery catheter 1406 reaches and exceeds a pre-determined torque threshold. At the position illustrated by Figure 113D, arms 1534 can flex against and begin to slide past pins 1536. Arms 1534 travel past pins 1536, and delivery catheter 1406 and piston body 1502 decouple from piston head 1504 and anchoring device 1456. Delivery catheter 1406 and piston body 1502 rotate with respect to piston head 1504 and anchoring device 1456. The arms 1534 slipping past the pins 1536 prevents excessive torque being applied to the anchoring device 1456, and thereby can prevent anchoring device 1456 being overexerted into the receiving material. Arms 1534 traveling past pins 1536 can cause a sudden decrease in resistance experienced by the user applying the torque. The decrease in resistance can signify that the torque threshold has been reached and that the rotation of delivery catheter 1406 should be slowed, stopped, or reversed.

Arms 1534 and pins 1536 can have numerous shapes, sizes, and other configuration variations to optimize setting and controlling the torque threshold. For example, with reference to Figures 113F-113G, over-torque prevention device 1533 can comprise arms 1534 made of a thin sheet metal material. Arms 1534 can extend along inner wall 1540 of the cap of the piston head 1504. Arms 1534 can couple with flexible spring members 1542, forming a ramp-like surface. The size of the arms 1554 and spring members 1542 can be altered to increase or decrease the desirable pre-determined threshold torque value.

Figure 113F corresponds to situations where the torque applied to the delivery catheter 1406 is less than a pre-determined torque threshold. When delivery catheter 1406 is rotated, pins 1536 contact arms 1534. Due to this contact, piston head 1504 rotates at the same rate as delivery catheter 1406 and piston body 1502.

Figure 113G corresponds to when the torque applied to the delivery catheter 1406 reaches a pre-determined or exceeds the torque threshold. Arms 1534 are can flexed by pins 1536, and piston body 1502 begins to rotate faster relative to piston head 1504. The pins 1536 slip past the arms 1534 to decouple the anchor 1546 from the catheter 1406.

Referring to Figures 113H-113N, 114A, 115A, 115B, 116A, 117A, 118A, 119A-119D, 120A, 121A, and 122A, the deployment of the device 120 for remodeling the shape of a heart wall W and system and method for delivering the device 120 is illustrated. The method illustrated by Figures 113H-113N, 114A, 115A, 115B, 116A, 117A, 118A, 119A-119D, 120A, 121A, and 122A can be adapted to any of the embodiments disclosed herein. For example, the deployment described by Figures 113H-113N, 114A, 115A, 115B, 116A, 117A, 118A, 119A-119D, 120A, 121A, and 122A can be adapted to any of the anchors, hemostasis tubes, delivery devices, and methods described herein.

Referring to Figure 113H, deployment of the device 120 includes delivering delivery catheter 1406 into an internal chamber (e.g. left ventricle LV) of the heart H and adjacent the heart wall W. Delivery catheter 1406 is extended from the distal end 1454 of the steerable catheter (not shown). The desired position on endocardium 102 is selected and anchoring device 1456 is positioned to abut or be adjacent to the endocardium 102. Delivery catheter 1406 is shown in a non-compressed position.

Referring to Figures 113H and 113I, anchoring device 1456 is pressed against endocardium 102 with a force causing piston 1500 to move proximally into delivery catheter 1406. The force at which anchoring device 1456 is pressed against endocardium 102 can be selected to optimize delivery of anchoring device 1456 into endocardium 102 and myocardium 104. Referring to Figure 113I, the movement of piston 1500 in the A' direction relative to delivery catheter 1406 causes the distance between marker 1526 and marker 1530 to decrease.

With reference to Figure 113I, anchoring device 1456 is positioned at a suitable pressure against heart wall W and piston 1500 is shown in the activated position. Marker 1526 abuts marker 1530 and a distal end of the delivery catheter. A user using a scope can be observe the activated position by determining that marker 1526 abuts marker 1530. This can signify to the user that the anchoring device 1456 is ready to be attached to heart wall W.

With reference to Figure 113J-113N, anchoring device 1456 is attached to the heart wall W. In the illustrated embodiment, the anchoring device 1456 can be attached to the heart wall W by rotating the delivery catheter 1406 about illustrated axis Z in the direction of arrow A2 (Figure 113J). As a result, the anchoring device 1456 is screwed into the heart wall W through the endocardium 102 and into the myocardium 104.

With reference to Figures 113A and 113J-113K, anchoring device 1456 is partially rotated into the heart wall W. The torque applied to the delivery catheter 1406 is less than a pre-determined torque threshold. With reference to Figure 113K, when delivery catheter 1406 or piston body 1502 is rotated, arms 1534 contact pins 1536. Due to this contact, piston head 1504 and anchoring device 1456 rotate at the same rate as delivery catheter 1406 and piston body 1502. The resulting gradually increasing torque can signify to a user to continue rotating anchoring device 1456 into heart wall W.

With reference to Figures 113A and 113L-113N, anchoring device 1456 is adequately attached to heart wall W. The torque applied to the delivery catheter 1406 meets and surpasses the pre-determined torque threshold. With reference to Figure 113M, the torque applied to the delivery catheter 1406 reaches a pre-determined torque threshold. Arms 1534 can flex as the pins 1536 travel over the arms.

With reference to Figure 113N, the torque applied to the delivery catheter 1406 surpasses the pre-determined torque threshold. Pins 1536 travel past the arms 1534, causing delivery catheter 1406 and piston body 1502 to rotate with respect to cap of the piston head 1504. The pins 1536 moving past the arms 1534 prevents excessive torque being applied to the anchoring device 1456, and thereby can prevent anchoring device 1456 being over exerted into the heart wall W. Pins 1536 slipping past the arms 1534 can cause a sudden decrease in resistance experienced by the user applying the torque. The decrease in resistance can signify that the threshold torque value has been surpassed and that the rotation of delivery catheter 1406 should be slowed, stopped, or reversed.

Referring to Figures 114A, the piercing device 130 is delivered into an internal chamber (e.g. left ventricle LV) of the heart H via delivery catheter 1406 and piston 1500. The piercing device 130 can be extended from the distal end 1458 of the delivery catheter 1406 and through distal end 1506 of piston head 1504, such that the distal end 1460 of the piercing device 130 is extended into the heart wall W. In the illustrated embodiment, the distal end 1460 of the piercing device 130 is extended through the endocardium 102, the myocardium 104, the epicardium 106, and into the pericardial cavity 110 to form the passage 132. Since the delivery catheter 1406 is anchored to the heart wall W, the location for insertion of the piercing device 130 can be precisely controlled.

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 115A. The dye 134 can be detected by any suitable technique such as X-ray, to verify that the piercing device 130 is properly positioned. Referring to Figure 115B, in another embodiment a wire 300 or other radio-opaque marker can be delivered through the piercing device 130 and into the pericardial cavity 110 to verify that the piercing device 130 is properly positioned, instead of the dye.

In Figure 116A, the anchor 122 and the hemostatic plug 1410 are extended from the delivery catheter 1406 over the piercing device 130 (Figure 114A) and through the passage 132. As shown in Figure 116A, the anchor 122 remains in the elongated state while sliding along the piercing device 130 into the pericardial cavity 110.

In Figure 117A, the anchor 122 is partially deployed beyond the distal end 1460 (Figure 114A) of the piercing device 130. Pusher 1408 is illustrated partially extending from the delivery catheter 1406 and piston 1500 through the anchoring device 1456. As the pusher 1408 extends from the piston 1500, the distal end 1464 of the pusher 1408 engages the hemostatic plug 1410 to push both the hemostatic plug 1410 and the anchor 122 over the piercing device 130. As the distal end 1432 of the anchor 122 moves beyond the distal end 1460 of the piercing device 130, the anchor 122 can begin to reshape into its curved deployed state. For example, the anchor 122 can include a shape memory alloy that is shape set to the curved deployed state. Thus, the portion of the anchor 122 that is no longer received on the piercing device 130 can revert to the curved deployed state that shape memory alloy was set to.

In Figure 118A, the anchor 122 is in the deployed state and the hemostatic plug 1410 is positioned within the passage 132 to prevent bleeding from the passage 132. In the illustrated embodiment, the distal end 1444 of the hemostatic plug 1410 is at or near the inner wall of the pericardial cavity 110. To reshape the anchor 122, the line 124 is withdrawn by pulling the line 124 in a direction away from the anchor and into the hemostatic plug 1410. Since the loop 1440 of the line 124 passes through the loops 1442 on the anchor 122, pulling the line 124 closes the loop 1440, pulls the hemostatic plug 1410 and the anchor 122 together, and pulls the loops 1442 together to help facilitate, along with any shape setting properties of the anchor 122, reshaping the anchor 122 from the elongated state to the curved deployed state shown in Figure 119A.

As shown in Figures 119A-119B, the pusher 1408 and piercing device 130 can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, and the endocardium 102.

Referring to Figure 119A, to seat the anchor 122, line 124 can be placed in tension by pulling the line 124 in an inward direction toward the heart chamber as shown by the arrow A9. As a result, the anchor 122 will be pulled against the outward facing surface 126 that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130.

With reference to Figure 119C, anchoring device 1456 can be detached from the heart wall W. In the illustrated embodiment, the anchoring device 1456 can be detached from the heart wall W by rotating the delivery catheter 1406 about illustrated axis Z in the direction of arrow A13, the direction opposite of direction A2 (Figure 113J). As a result, the anchoring device 1456 can screw out from heart wall W. Piston 1500 can be in the activated or de-activated position when detaching anchoring device 1456 from heart wall W.

With reference to Figures 113A and 119D, anchoring device 1456 is partially rotated out from the heart wall W. When delivery catheter 1406 is rotated about illustrated axis Z in the direction of arrow A13 (Figure 119C), ends of arms 1534 contact pins 1536. Due to this contact, piston head 1504 and anchoring device 1456 rotate at the same rate as delivery catheter 1406 and piston body 1502. When removing anchoring device 1456, pins 1536 will maintain constant contact with arms 1534 at all torques. Arms 1534 will not flex to allow pins 1536 to pass arms 1534 as anchoring device is rotated out from heart wall W, even if the torque applied in the A13 direction reaches or surpasses the torque threshold predetermined in the A2 direction. Torque is therefore not limited in the A13, removal direction as it is in the A2, anchoring direction. This is due to the hard stop between the end of the arms and the pins in the removal direction, instead of the gradual ramp engagement in the am boring direction.

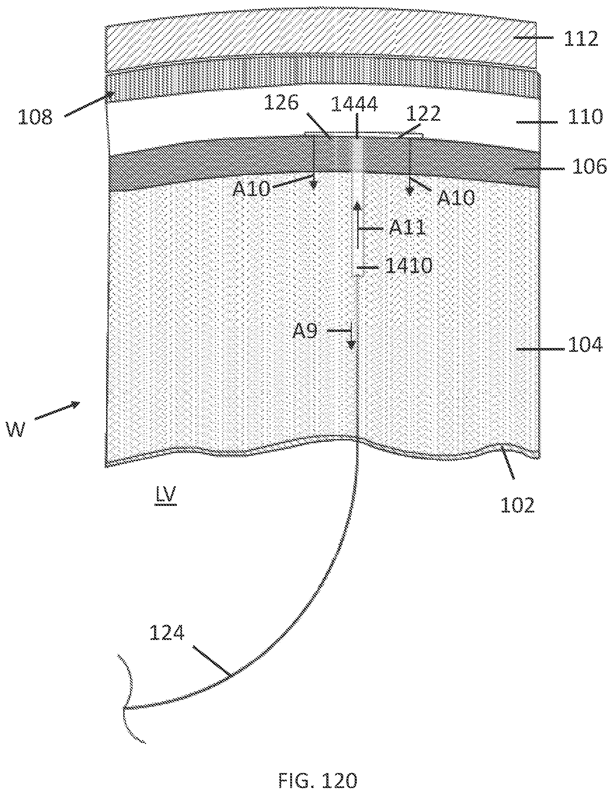

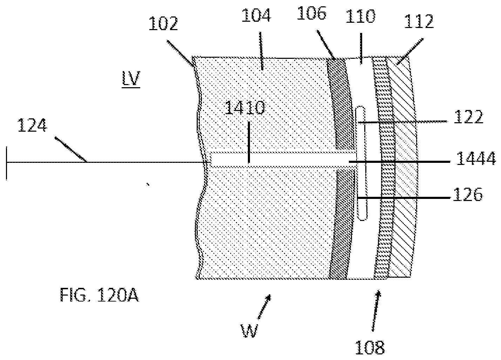

With reference to Figure 120A, once the delivery catheter 1406 is removed, further tensioning of the line 124 can pull the heart wall W inward toward the heart chamber (e.g., the left ventricle LV).

The piston and/or clutch embodiments of Figures 112A-112G, 113A-113N, 114A, 115A, 115B, 116A, 117A, 118A, 119A-119D, 120A can be used in any of the embodiments of the present application. For example, Figures 26A, 27A, and 28A illustrate use of the piston and/or clutch in the embodiment of Figures 26-28 that is described above. In Figures 26A and 27A, device 120 for remodeling the shape of a heart wall W can be delivered through the delivery catheter 136. In particular, the delivery catheter 136 is extended through piston 1500 and through the passage 132 such that the distal end 138 of the delivery catheter 136 is positioned within the pericardial cavity 110. As shown in Figure 27A, with the distal end 138 of the delivery catheter 136 is positioned within the pericardial cavity 110, the device 120 for remodeling the shape of a heart wall W can be delivered through the delivery catheter 136. In particular, the anchor 122 can be extended out of the distal end of the 138 of the delivery catheter 136 and into the pericardial cavity 110 while the attached line 124 extends through the delivery catheter 136 in the passage 132.

With reference to Figures 28A, the delivery catheter 136 and the piercing device 130 can be removed by withdrawing them from the passage 132 and from the heart chamber. The device 120 is left deployed through heart wall W with the anchor 122 engaging an outward facing surface 126 of the heart wall W and the line 124 extending through the epicardium 106, the myocardium 104, and the endocardium 102 and into the heart chamber (e.g., the left ventricle LV). Line 124 can be placed in tension by pulling the line 124 in an inward direction toward the heart chamber as shown by the arrow A12.

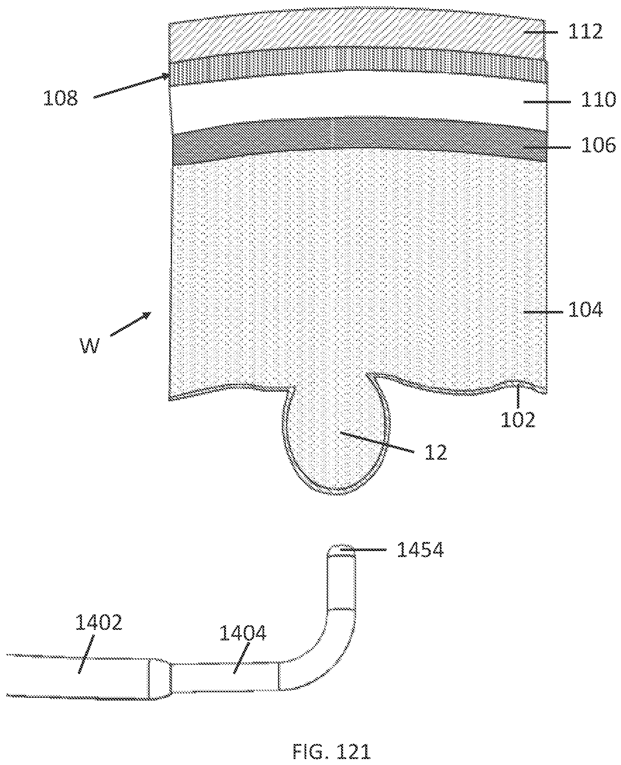

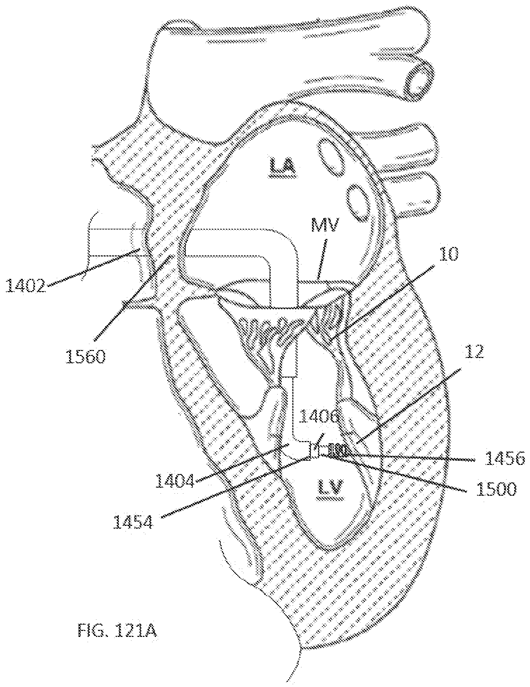

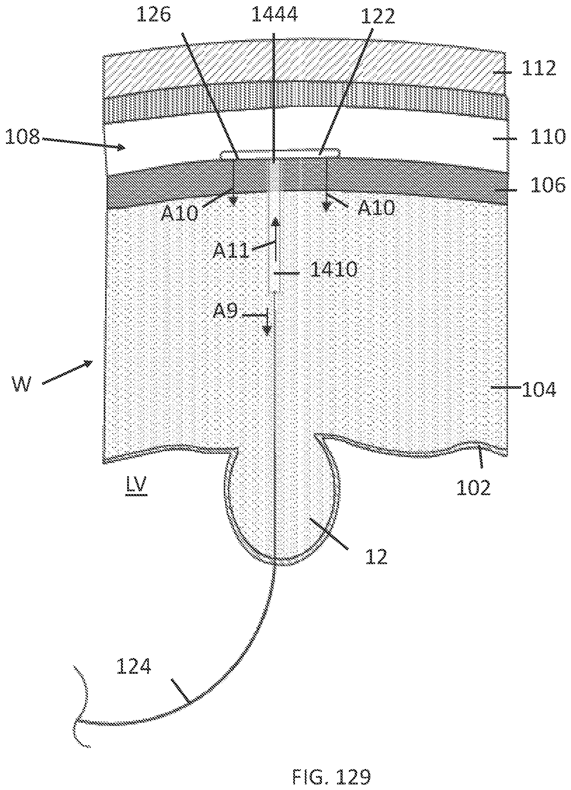

Referring to Figures 121-129, the deployment of the device 120 into the pericardial cavity 110, and through one of the papillary muscles 12, for remodeling the shape of a heart wall W and system and method for delivering the device 120 is illustrated. Referring to Figure 121, deployment of the device 120 includes delivering the guide sheath 1402 and the steerable catheter 1404 into an internal chamber (e.g. left ventricle LV) of the heart H. The steerable catheter 1404 is arranged such that the distal end 1454 of the steerable catheter 1404 is adjacent one of the papillary muscles 12 of the heart H.

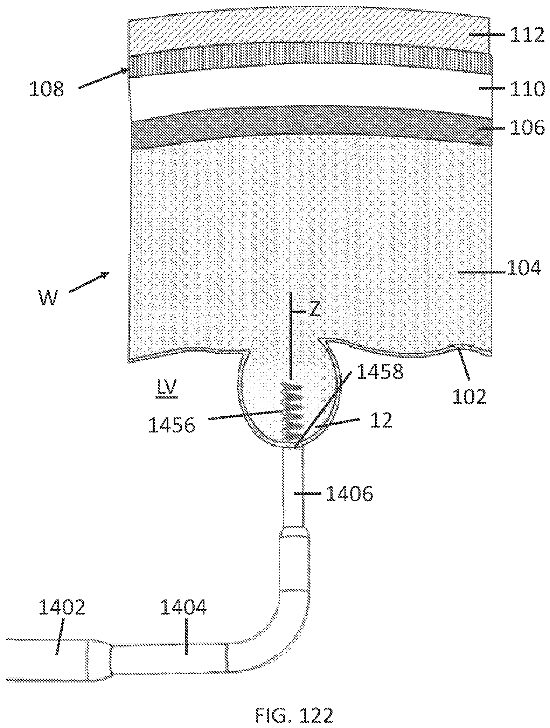

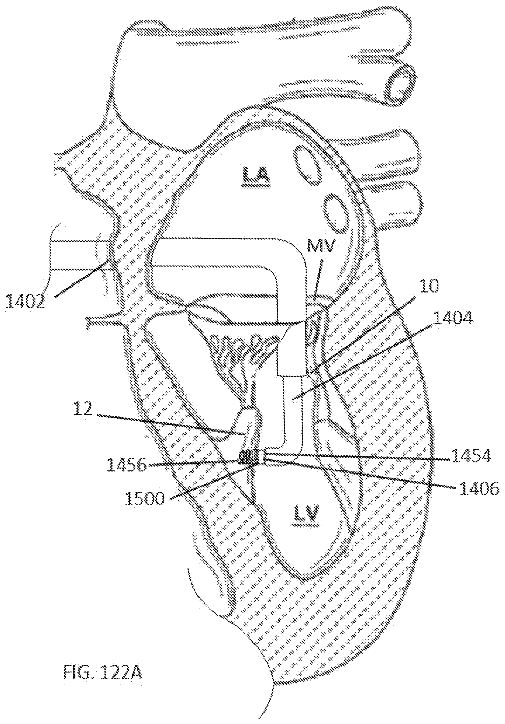

Referring to Figure 122, the delivery catheter 1406 is extended from the distal end 1454 of the steerable catheter 1404. The anchoring device 1456 of the delivery catheter 1406 is attached to the papillary muscle 12, such as for example by rotating the delivery catheter 1406 about axis Z relative to the steerable catheter 1404 to screw the anchoring device 1456 into papillary muscle 12. In the illustrated embodiment, the distal end 1458 of the delivery catheter 1406 abuts, or is adjacent, the papillary muscle 12.

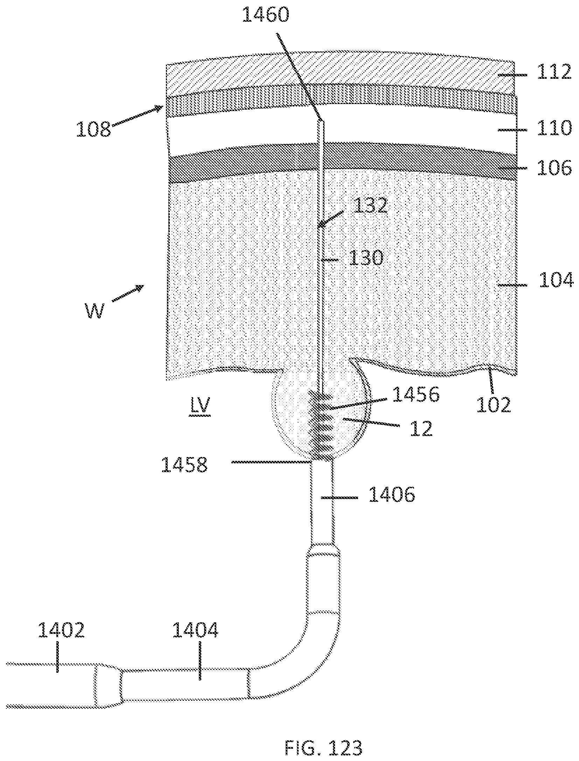

Referring to Figure 123. the piercing device 130 is delivered into an internal chamber (e.g. left ventricle LV) of the heart H via the delivery catheter 1406. The piercing device 130 can be extended from the distal end 1458 of the delivery catheter 1406 such that the distal end 1460 of the piercing device 130 is extended through the papillary muscle 12 and the heart wall W. In the illustrated embodiment, the distal end 1460 of the piercing device 130 is extended through the papillary muscle 12, the endocardium 102, the myocardium 104, the epicardium 106, and into the pericardial cavity 110 to form the passage 132. Since the delivery catheter 1406 is anchored to the papillary muscle 12, the location for insertion of the piercing device 130 can be precisely controlled.

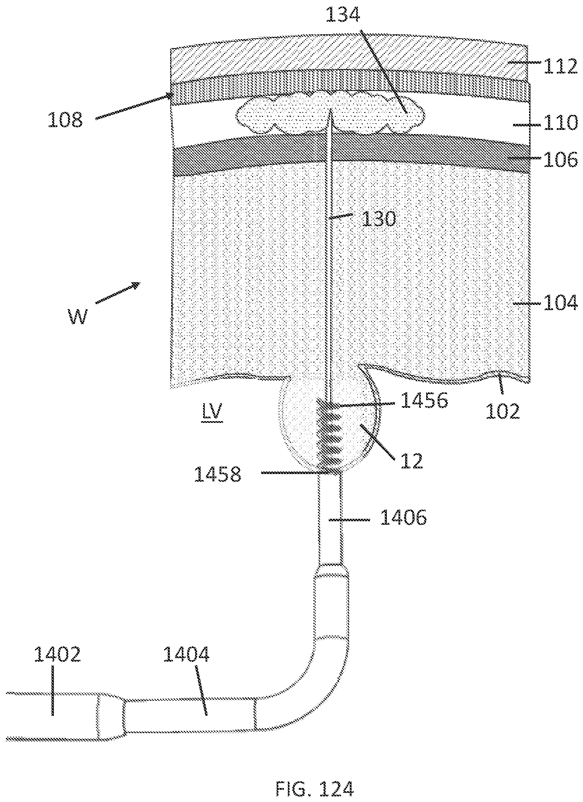

To verify that the piercing device 130 is properly positioned for deploying the anchor 122 into the pericardial cavity 110, a dye 134, or other detectable fluid, can be delivered through the piercing device 130 and injected into the pericardial cavity 110, as shown in Figure 124. The dye 134 can be detected by any suitable technique such as X-ray, to verify that the piercing device 130 is properly positioned.

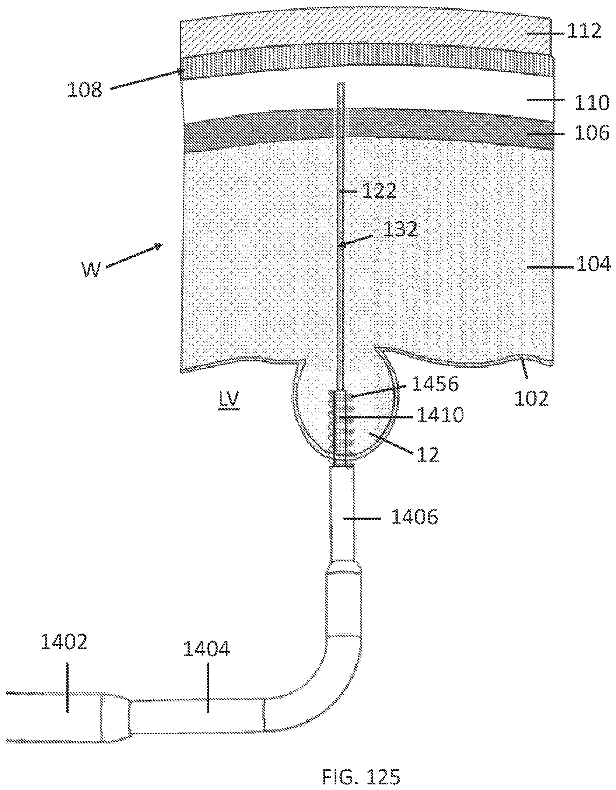

In Figure 125, the anchor 122 and the hemostatic plug 1410 are extended from the delivery catheter 1406 over the piercing device 130 (Figure 115) and through the passage 132. As shown in Figure 116, the anchor 122 remains in the elongated state while sliding along the piercing device 130 into the pericardial cavity 110.

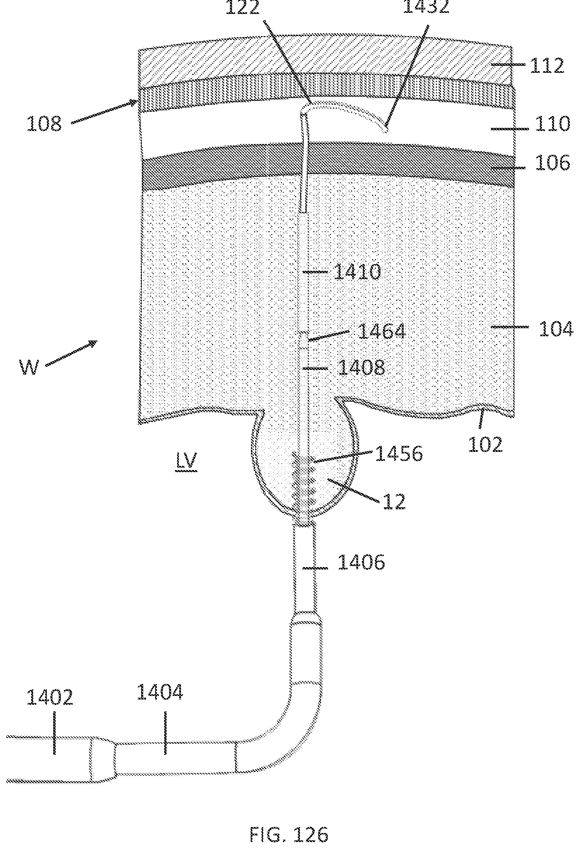

In Figure 126, the anchor 122 is partially deployed beyond the distal end 1460 of the piercing device 130 (Figure 123). The delivery catheter 1406 is illustrated partially extending from the steerable catheter 1404 and the pusher 1408 is illustrated partially extending from the delivery catheter 1406 through the anchoring device 1456. As the pusher 1408 extends from the delivery catheter 1406, the distal end 1464 of the pusher 1408 engages the hemostatic plug 1410 to push both the hemostatic plug 1410 and the anchor 122 over the piercing device 130 (Figure 123). As the distal end 1432 of the anchor 122 moves beyond the distal end 1460 of the piercing device 130 (Figure 123), the anchor 122 may begin to reshape into its curved deployed state. For example, the anchor 122 may include a shape memory alloy that is shape set to the curved deployed state. Thus, the portion of the anchor 122 that is no longer received on the piercing device 130 may revert to the curved deployed state that that shape memory alloy was set to.

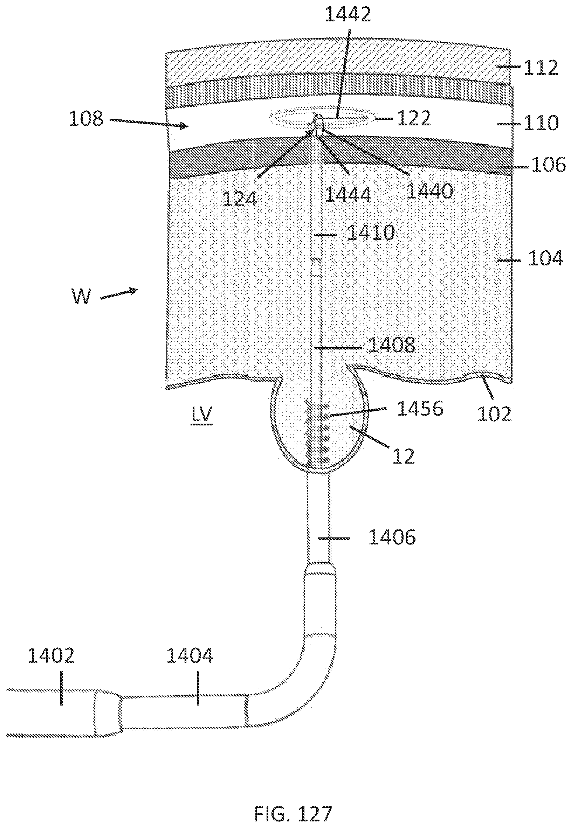

In Figure 127, the anchor 122 is in the deployed state and the hemostatic plug 1410 is positioned within the passage 132 to prevent bleeding from the passage 132. In the illustrated embodiment, the distal end 1444 of the hemostatic plug 1410 is at or near the inner wall of the pericardial cavity 110. To reshape the anchor 122, the line 124 is withdrawn by pulling the line 124 in a direction away from the anchor and into the hemostatic plug 1410. Since the loop 1440 (Figure 97) of the line 124 passes through the loops 1442 on the anchor 122, pulling the line 124 closes the loop 1440, pulls the hemostatic plug toward the anchor, and pulls the loops 1442 together to help facilitate, along with any shape setting properties of the anchor 122, reshaping the anchor 122 from the elongated state to the curved deployed state shown in Figures 127.

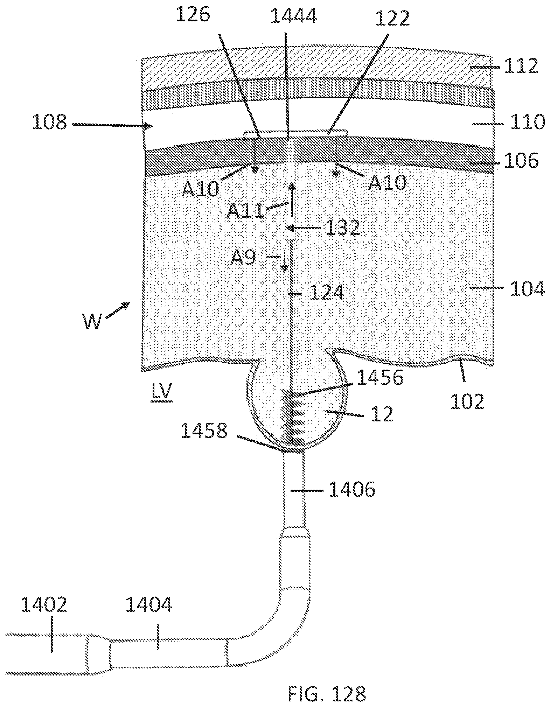

As shown in Figures 128-129, the pusher 1408 and piercing device can be removed by withdrawing them from the passage 132 into the delivery catheter 1406. The anchor 122, hemostatic plug 1410, and line 124 are left deployed in the heart wall W with the anchor 122 within the pericardial cavity 110 and the line 124 extending through the epicardium 106, the myocardium 104, the endocardium 102, and the papillary muscle 12.

To seat the anchor 122, the delivery catheter 1406 may remain attached to the heart wall W and the distal end 1458 can be pushed against the heart wall W. At the same time, the line 124 can be placed in tension by pulling the line 124 in an inward direction toward the heart chamber as shown by the arrow A9. As a result, the anchor 122 will be pulled against the outward facing surface 126, which in the illustrated embodiment is the inner pericardium layer (i.e., the visceral layer of the serous pericardium) that partially defines the pericardial cavity 110, as shown by arrows A10. As the line 124 is being pulled in the direction of A9, the hemostatic plug 1410 is urged in the opposite direction, as shown by arrow A11, such that the distal end 1444 of the hemostatic plug 1410 is positioned at or adjacent the center of the anchor 122. The anchor 122 in its deployed state is too large to fit through the passage 132 formed by the piercing device 130. Thus, once the delivery catheter 1406 is removed, further tensioning of the line 124 can pull the heart wall W inward toward the heart chamber (e.g., the left ventricle LV).

The piston and/or clutch embodiments of Figures 112A-112G, 113A-113N, 114A, 115A, 115B, 116A, 117A, 118A, 119A-119D, 120A can be used to deploy one or more devices 120 into one or more papillary muscles 12, for remodeling the shape of a heart wall W. For example, with reference to Figures 121A and 122A, guide sheath 1402 can puncture and extend through atrial-septal wall 1560 and can optionally be guided through the mitral valve MV. Steerable catheter 1404 can extend from guide sheath 1402. The steerable catheter 1404 is arranged such that the distal end 1454 of the steerable catheter 1404 can be steered to be adjacent one of the papillary muscles 12 of the heart H. Delivery catheter 1406, piston 1500, and anchoring device extend from the distal end 1454 of the steerable catheter 1404. Device 120 can be delivered through the papillary muscle 12 in accordance with Figures 112A-112G, 113A-113N,114A, 115A, 115B, 116A, 117A, 118A, 119A-119D, and 120A.

In certain embodiments, tissue remodeling can be accomplished through use of one or more helical anchors. These anchors can be deployed into targeted regions of heart tissue via a steerable guide rail. An exemplary steerable guide rail 1304 used to deploy a helical anchor 1310 is shown in Figures 130A-D. With reference to Figure 130A, a steerable guide rail 1304 is shown deployed adjacent to tissue 1308. In certain embodiments, guide rail 1304 may engage the tissue 1308 at one or more points. In other embodiments, the steerable guide rail 1304 is positioned against a targeted area of tissue 1308. While the steerable guide rail 1304 is depicted along a substantially flat surface of tissue 1308, it will be appreciated that steerable guide rail 1304 can be positioned against any targeted area of tissue 1308, including along curvatures or non-uniform areas of the tissue.

Steerable guide rail 1304 can be deployed with an associated tether 1306 and stop 1302. As shown, tether 1306 is located within a cavity inside the steerable guide rail 1304. The tether 1306 is held in place by a stop 1302. As shown in Figure 130B, a helical anchor 1310 is positioned along the steerable guide rail 1304 such that when the helical anchor 1310 is rotated, the anchor will engage tissue 1308 along a path established by the steerable guide rail 1304. The engagement of the tissue can be used to remodel the tissue and/or to provide an anchor or connection to the tissue. The helical anchor 1310 can be deployed along the steerable guide rail 1304 to a fully engaged position coils along the entire length of the anchor 1310 are embedded in the tissue, as shown in Figure 130C. It will be appreciated that the stop 1302 is sized and shaped in a manner that will not allow the stop 1302 to be pulled through the end of the helical anchor. In Figure 130D the steerable guide rail 1304 can be removed, while anchor 1310 and the stop 1302 remain fixed in place at the targeted tissue location of tissue 1308.

As shown in Figures 131A-E, after an anchor (e.g. helical anchor 1310) has been deployed into a targeted tissue area, remodeling (or additional remodeling) of the tissue area can be accomplished using the tether 1306. Figure 131A shows an exemplary steerable guide rail 1304 (with associated tether 1306 and stop 1302) deployed adjacent to tissue 1308. In Figure 131B, a helical anchor 1310 can be positioned along the guide rail and then rotated to engage tissue 1308 along a path established by the steerable guide rail 1304. Figure 131C shows the helical anchor 1310 fully deployed in the tissue 1308. In Figure 131D, the steerable guide rail 1304 has been removed and an anchor stop 1312 has been moved along the tether to a proximal end of the helical anchor 1310. Anchor stop 1312 is positioned to contact helical anchor such that when pushing force is applied to the tether stop 1312 and pulling force is applied by the tether 1306 to the stop 1302, the helical anchor is contracted. This contraction of tissue allows for targeted remodeling of tissue 1308. Once a desired level of tissue contraction/remodeling has been accomplished, a locking mechanism 1314 can be affixed to tether 1306 which holds the stop 1312 in place, which maintains the desired compressed position of anchor 1310 in tissue 1308.

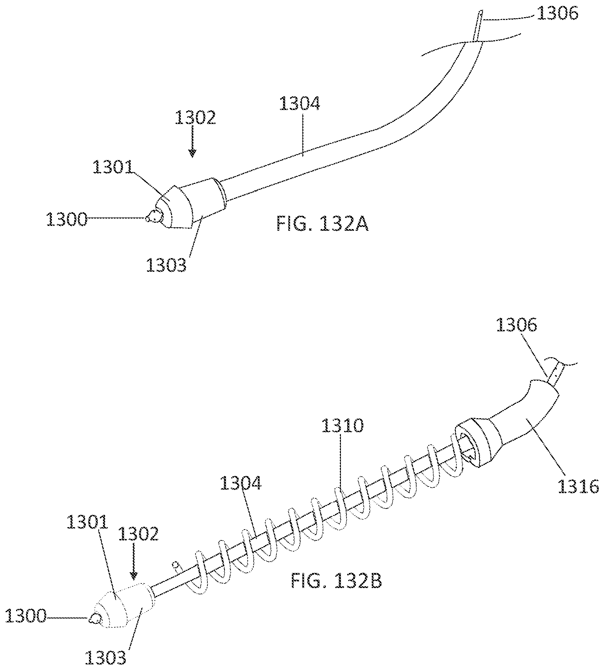

The stop 1302 can take a wide variety of different forms. The stop is illustrated schematically in Figures 130D and 131D. The stop 1302 can be entirely larger than an inner diameter of the coiled anchor 1310, the stop can be sized to frictionally fit inside the inner diameter of the coiled anchor 1310, or the stop have a portion that is larger than inner diameter of the coiled anchor 1310 and a portion that fits inside the coiled anchor. In Figures 132A-B, an exemplary embodiment of stop 1302 is shown. In the illustrated example, stop 1302 is connected to the tether 1306, which can hold the stop against the steerable guide rail 1304 as illustrated by 132A. The stop 1302 is coupled to the end of the tether 1306 by a distal knot 1300. The distal knot 1300 is attached to or part of the tether 1306 and prevents the stop 1302 from moving beyond the distal knot 1300. In some embodiments, distal knot 1300 is formed from tether 1306, for example, via one or more surgical knots as known in the art. In other embodiments, distal knot 1300 can be affixed to the distal end of tether 1306. Additionally, the stop 1302 is sized to prevent the steerable guild rail 1304 from moving past the stop 1302.

In the example illustrated by Figure 132A, the stop 1302 comprises a nose cone 1301 and a stem 1303. The illustrated nose cone 1301 tapers outward from the distal knot 1300 and the stem tapers inward away from the nose cone 1301. In Figure 132B, a helical anchor 1310 is shown being advanced along the steerable guide rail 1304. In certain embodiments, the helical anchor 1310 can be advanced along the steerable guide rail 1304 by a pusher 1316. Pusher 1316 can be manipulated to both advance the helical anchor 1310 along the steerable guide rail 1304 and rotate the helical anchor 1310 about the steerable guide rail 1304. It is appreciated that pusher 1316 can extend outside the body for easy control of the deployment of helical anchor 1310 into a target tissue area.

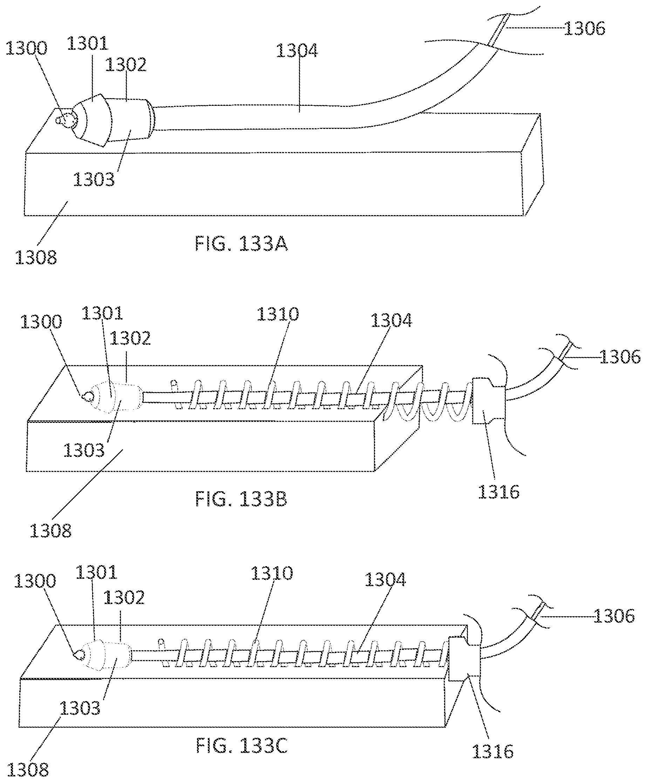

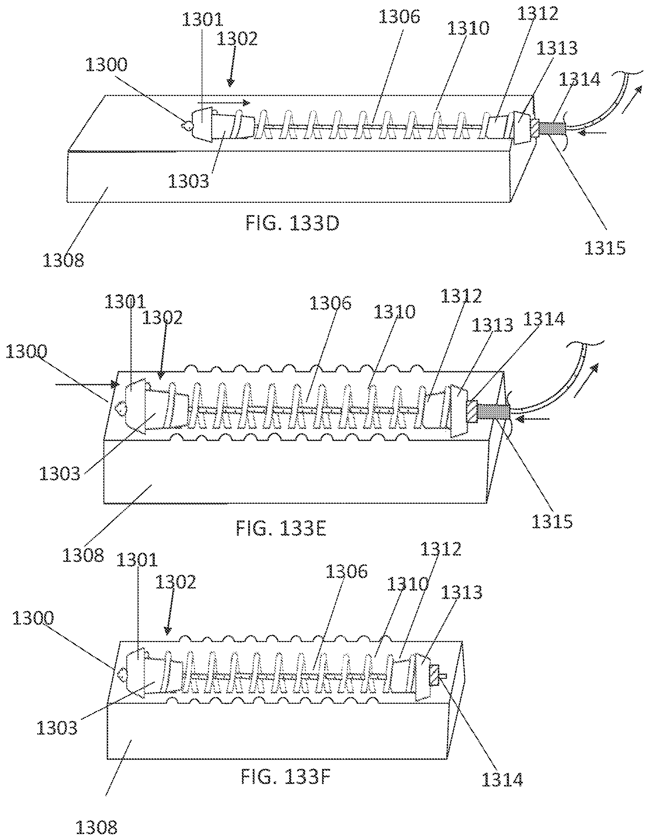

Figures 133A-F show an exemplary deployment of a helical anchor 1310 into tissue 1308 using a steerable guide rail 1304. In Figure 133A, the steerable guide rail 1304 is positioned against a target tissue area 1308. The steerable guide rail 1304 is associated with a stop 1302, which comprises a nose cone 1301 and a stem or strain relief portion 1303. The tether 1306 extends through the steerable guide rail 1304 to the distal knot 1300 also shown. In Figure 133B, the pusher 1316 is used to advance the helical anchor 1310 along the steerable guide rail to an implant position in the target tissue 1308. Once in position, the pusher 1316 rotates the helical anchor 1310 around the guide rail 1304 to implant the helical anchor 1310 into the tissue 1308. In Figure 133C, the pusher 1316 is shown having fully deployed helical anchor 1310 into the tissue 1308. Once the helical anchor 1310 is implanted at the target location, the pusher 1316 and the steerable guide rail 1304 can be retracted. In Figure 133D, pusher 1316 and steerable guide rail 1304 have been retracted. The tether 1306 is pulled to pull the distal stop 1302 against and/or into the distal end of the helical anchor 1310. A proximal anchor stop 1312 is advanced along the tether 1306 against and/or into the proximal end of helical anchor 1310. The proximal stop 1312 can have the same form as the distal stop 1302 or a different form. The proximal stop 1312 can be entirely larger than an inner diameter of the coiled anchor 1310, the stop can be sized to frictionally fit inside the inner diameter of the coiled anchor 1310, or the stop have a portion that is larger than inner diameter of the coiled anchor 1310 and a portion that fits inside the coiled anchor. A locking mechanism 1314 can secure the position of anchor stop 1312 along the tether 1306. As shown in Figure 133E, locking mechanism 1314 can be manipulated by a tool 1315, such as a torque driver or other tool that secures the locking mechanism 1314 in place on the tether 1305. For example, the locking mechanism can have the form of any of the connectors 240 disclosed herein. The pulling of the stop 1302 with the tether and the pushing of the stop 1312 compresses the helical anchor to contract tissue 1308. Once a desired compression of the helical anchor 1310 is achieved, the locking mechanism 1314 can be secured and the tool 1315 removed. As shown in Figure 133F, the locking mechanism 1314 secures the position of the helical anchor 1310 between the stop 1302 and the anchor stop 1312. In certain embodiments, tool 1315 can be used to tighten or loosen the locking mechanism 1314 to further manipulate the tissue engaged with helical anchor 1310 to remodel or reshape the tissue 1308.

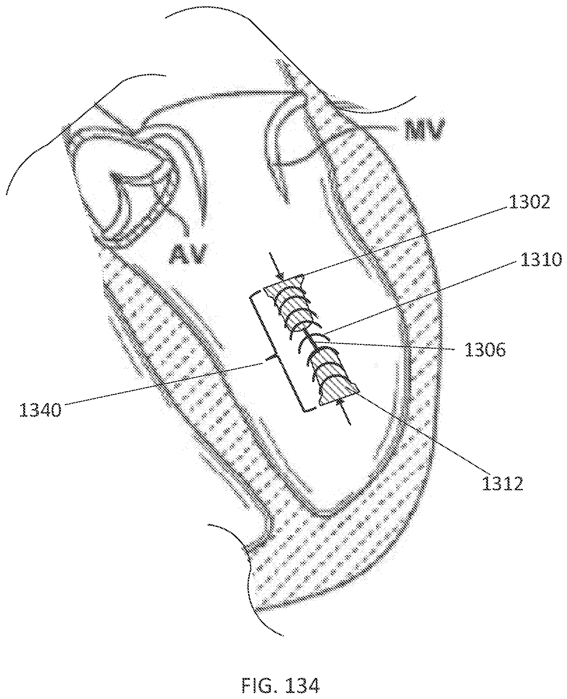

Figure 134 shows a helical anchor 1310 deployed within left ventricular heart wall tissue at a target tissue location 1340 which is along the height of the left ventricle, such as parallel to the direction of flow through the mitral valve MV. As shown, helical anchor 1310 is being contracted by forces as applied by stop 1302 and anchor stop 1312.

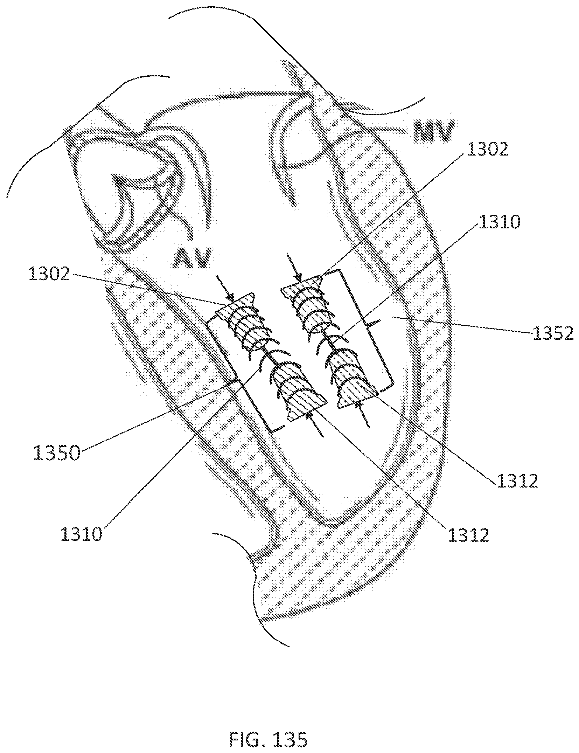

Figure 135 shows a plurality of helical anchor(s) 1310 deployed within heart wall target tissue locations 1350 and 1352 which are along the height of the left ventricle, such as parallel to the direction of flow through the mitral valve MV. As shown, helical anchor(s) 1310 are being contracted by forces as applied by anchor stop(s) 1302 and anchor stop(s) 1312.

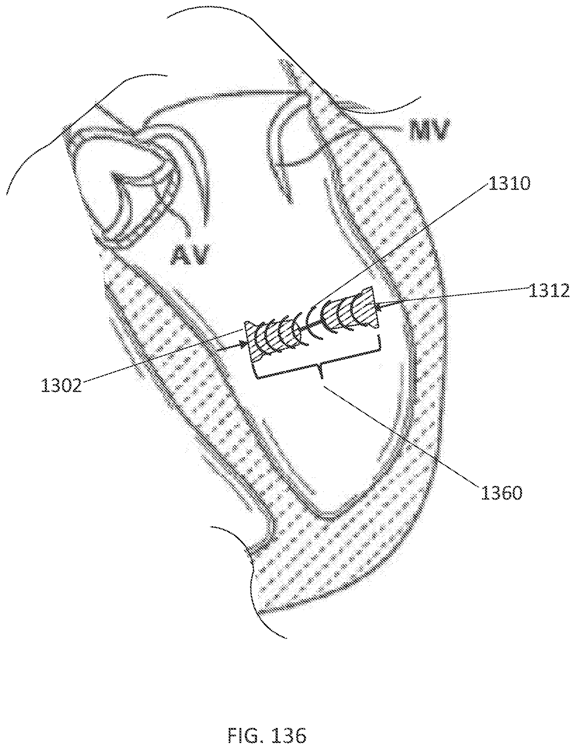

Figure 136 shows a helical anchor 1310 deployed within heart wall tissue at a target tissue location 1360 which is perpendicular to the height of the left ventricle, such as perpendicular to the direction of flow through the mitral valve MV. As shown, helical anchor 1310 is being contracted by forces applied by stop 1302 and anchor stop 1312.

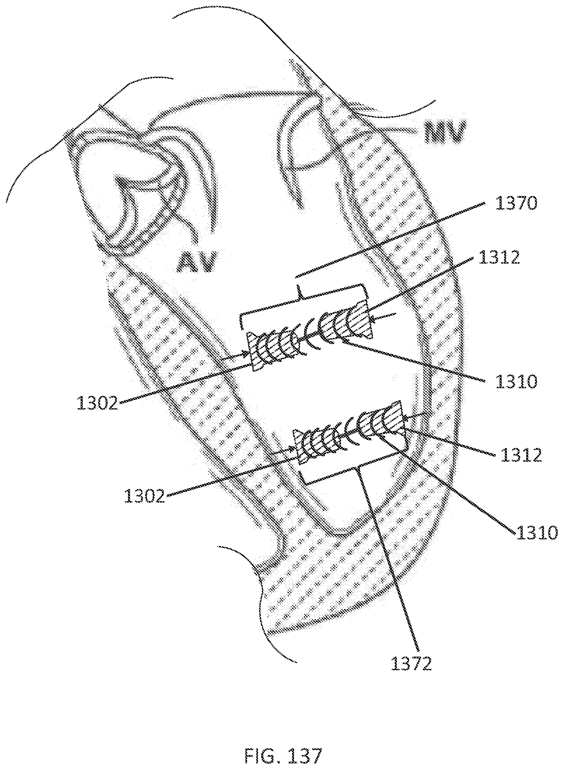

Figure 137 shows a plurality of helical anchor(s) 1310 deployed within heart wall target tissue locations 1370 and 1372 which are perpendicular to the height of left ventricle, such as perpendicular to the direction of flow through the mitral valve MV. As shown, helical anchor(s) 1310 are being contracted by forces applied by stop(s) 1302 and anchor stop(s) 1312.

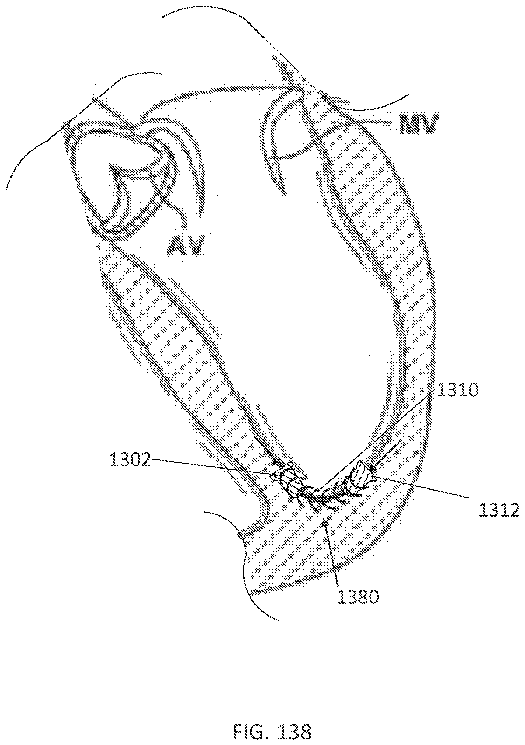

Figure 138 shows a helical anchor 1310 deployed within heart wall tissue at target tissue location 1380 located at the bottom or apex of the left ventricle of the heart. As shown, helical anchor 1310 is being contracted by forces as applied by the anchor stop 1302 and the anchor stop 1312.

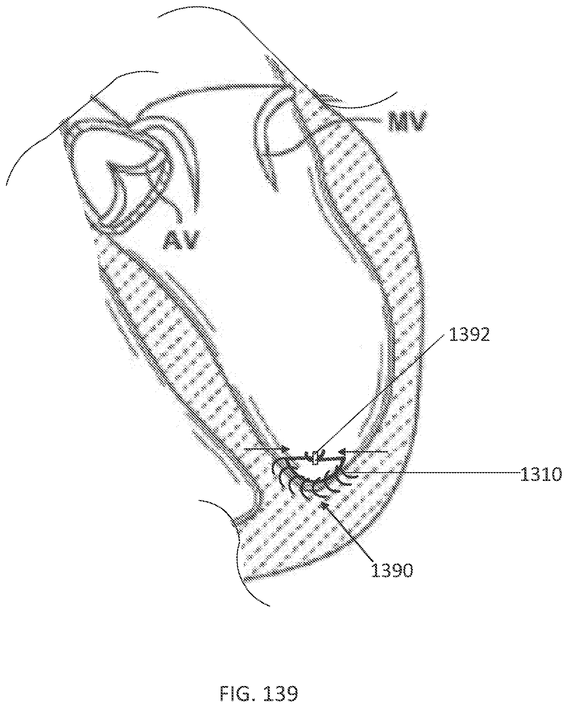

In Figure 139, a helical anchor 1310 is deployed within heart wall tissue at target tissue location 1390 located the bottom or apex of the left ventricle of the heart. As shown, helical anchor 1310 is being contracted by forces as applied by tether 1306. In such an embodiment, tether 1306 can manipulate the helical anchor 1310 to achieve desired contraction/remodeling of the tissue at target tissue location 1390. Once a desired shape or position is achieved, a tether lock 1392 can be used to secure tether 1306 and retain the position of the helical anchor 1310.

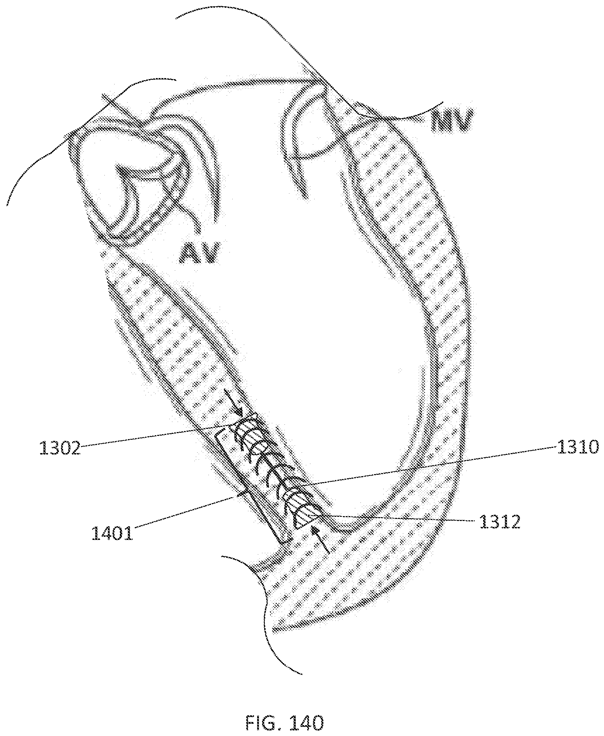

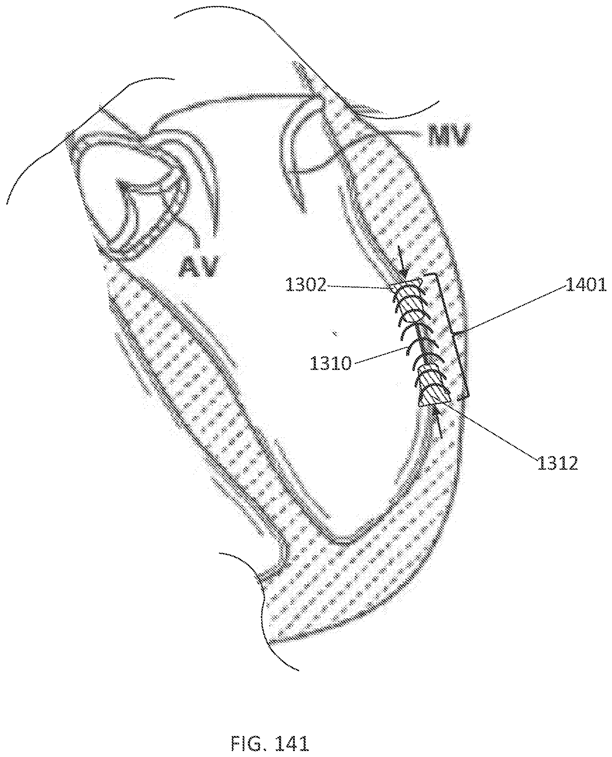

Figure 140 shows a helical anchor 1310 deployed within heart wall tissue at target tissue location 1401 located on the left ventricular side of the ventricular septal wall. As shown, helical anchor 1310 is being contracted by forces as applied by the anchor stop 1302 and the anchor stop 1312. In other exemplary embodiments, as shown in Figure 141, the target tissue location 1401 is located on the right ventricular side of the ventricular septal wall.

It will be appreciated that any of the above exemplary deployments of one or more helical anchor(s) 1310 can be used in combination within one another to reshape multiple target tissue locations within a single heart.

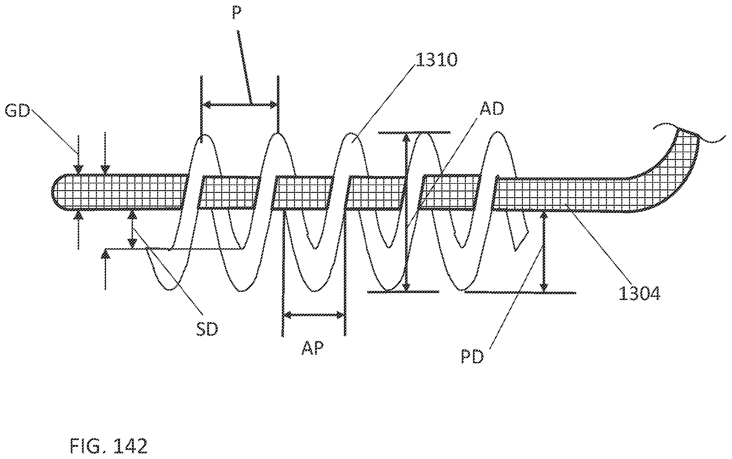

Figure 142 shows a close-up view of a helical anchor 1310 on steerable guide rail 1304. The helical anchor 1310 is shown having an anchor diameter AD. Diameter AD is the diameter of the coil or wire that is used to form helical anchor 1310. Steerable guide rail 1304 is shown having a guide rail diameter GD. The gap between the steerable guide rail 1304 which lies on the surface of the target tissue and the helical anchor 1610 is measured as stitch depth SD. The stitch depth SD can determine how much targeted tissue is engaged by the helical anchor 1310. This is particularly important when targeting specific tissue and/or specific tissue depths. For example, the stitch depth SD can be configured to engage helical anchor 1310 into targeted endocardial tissue of the left ventricle while avoiding or substantially avoiding myocardial tissue. In some exemplary embodiments, stitch depth SD is between 0.02 mm and 2 mm, such as between 0.5 mm and 1.5 mm, such as between 0.75 mm and 1.25 mm, such as 1 mm or less. Helical anchor 1310 can have a pitch P which is measured as the distance between two adjacent coils. It will be appreciated that pitch P can be determined when the coil is at a rest state or may change when helical anchor 1310 is expanded/contracted. Helical anchor 1310 can also have a puncture distance PD measured from the outside of the wire or coil forming helical anchor 1310 and the steerable guide rail 1304.

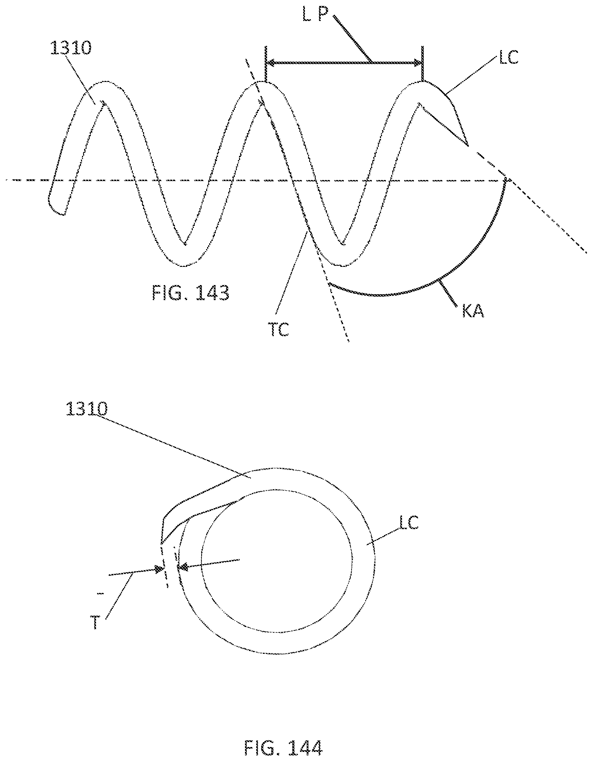

Figure 143 shows an exemplary helical anchor 1310 with a lead coil pitch LP and kick angle KA. The lead coil pitch LP is a measurement of the distance from the apex of the lead coil LC to the apex of the next adjacent trailing coil TC. The geometry of the lead coil (e.g. lead coil pitch LP) can determine the bite angle taken by helical anchor 1310 as it is deployed into tissue. Kick angle KA is measured as the angle between the tip of leading coil LC and the trailing coil TC.

Figure 144 shows a front view of an exemplary helical anchor 1310 with tip bias or kick out T at the tip of the lead coil LC. The tip bias or kick out T is the distance that the tip of the lead coil LC extends outward from the outside diameter of the coiled anchor 1310. This tip bias or kick out causes the tip of the coil to bite into the tissue 1308 when the guide rail 1304 holds the helical anchor against the tissue and the helical anchor is rotated around the guide rail. As previously discussed, the geometry of the lead coil LC can determine the bite angle that the helical anchor 1310 engages with a targeted tissue area.

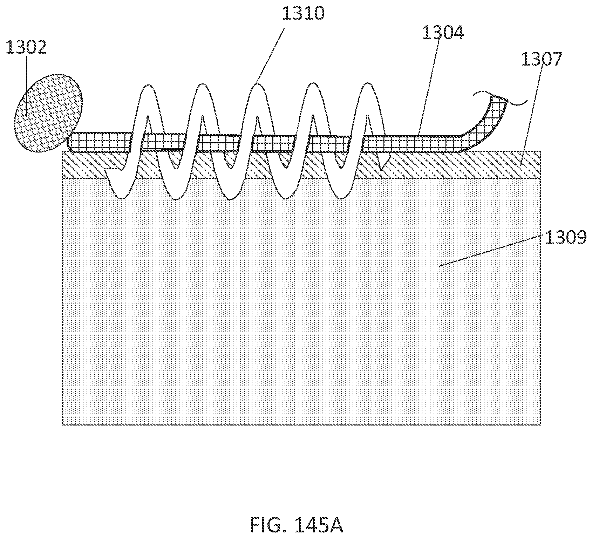

Figures 145A-C show an exemplary targeted deployment of a helical anchor 1310 into a target tissue area 1307, with limited engagement or no engagement with tissue area 1309. In Figure 145A, steerable guide rail 1304 is deployed to rest against the surface of target tissue area 1307. A stop 1302 is shown at the distal end of the steerable guide rail 1304. A helical anchor 1310 is deployed along the steerable guide rail 1304 to engage targeted tissue area 1307, while limiting the depth of the anchor into the tissue to limit engagement with the tissue area 1309. In some exemplary embodiments, targeted tissue area is 1307 is endocardial tissue and tissue area 1309 is myocardial tissue.

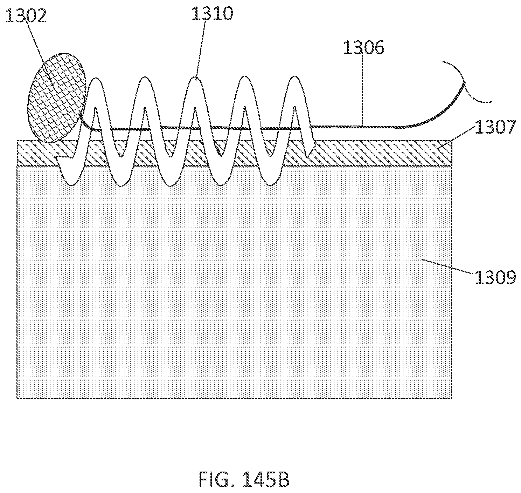

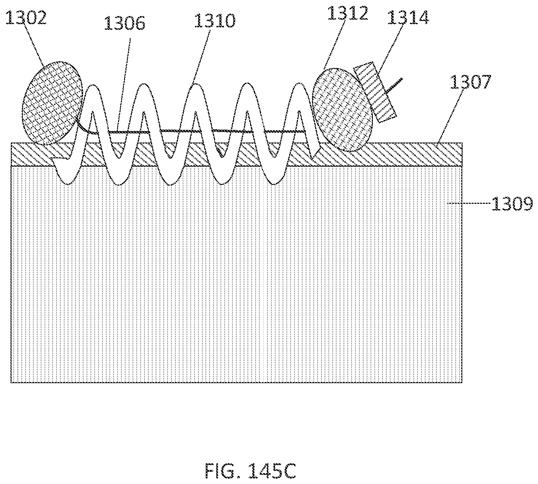

In Figure 145B, the steerable guide rail 1304 has been retracted to expose the tether 1306. An anchor stop 1302 is attached to tether 1306. The tether 1306 is pulled to cause the stop 1302 to engage the coiled anchor 1310. In Figure 145C, an anchor stop 1312 is slidably disposed on the tether 1306. The tether 1306 is pulled to cause the stop 1302 to engage the coiled anchor 1310 while the stop 1312 is pushed against the coiled anchor, to compress the helical anchor 1310. Locking mechanism 1314 is also attached to tether 1306 in order to secure the compressed condition of the helical anchor 1310.

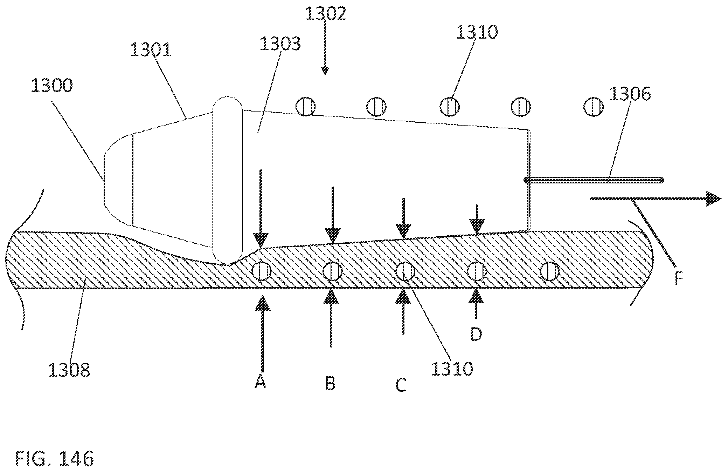

Figure 146 shows an exemplary embodiment of a stop 1302 in contact with a helical anchor 1310 and in contact with tissue 1308, such as endocardial tissue. As shown, stop 1302 comprises a nose cone portion 1301 and a stem portion 1303. As force F is applied to tether 1306, the stem portion 1303 is pulled into the anchor 1310 and compresses the tissue 1308. Due to the optional taper of the stem portion 1303, different amounts of compression of the tissue are provided at coil A, coil B, coil C, and coil D. In the illustrated example, the most tissue compression occurs at coil A and the compression progressively declines at coils B, C, and D due to the taper of the stem portion 1303. and provides load distribution at coils A, B, C, and D. This compression of the tissue 1308 between the stem portion 1303 and the coils A, B, C, and D increases the amount of force F required to be applied to the tether to pull the coiled anchor 1310 out of the tissue. This increase in force F to pull the coiled anchor 1310 out of the tissue is because all of the coils A, B, C, and D that compress tissue share or distribute the force F, instead of the force being focused on the leading coil A alone. When the stem portion is omitted and force is focused on leading coil A alone, the force F can sequentially pull coils A, B, C, D out of the tissue one at a time. The stem portion 1303 can take a wide variety of different forms. For example, the stem portion 1303 can be sixed to compress tissue between any number of coils of the anchor 1310. When included, a proximal stop 1312 having a stem will provide the same increase in pull out force as the distal stop 1302 with a stem.

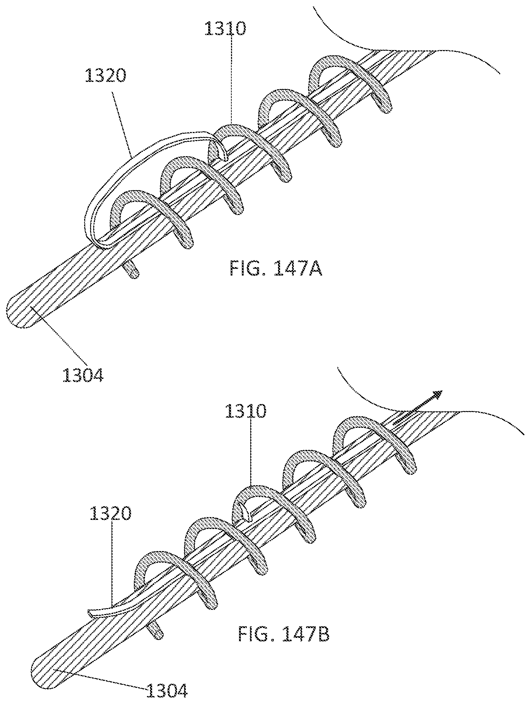

Figures 147A-B show an exemplary optional shield 1320 for use during deployment of a helical anchor 1310 using steerable guide rail 1304. In Figure 147A, the shield 1320 is in its active position which allows for the helical anchor 1310 and steerable guide rail 1304 to be advanced through a patient's anatomy without inadvertent engagement by the lead coil of the anchor with non-target tissue. For example, the shield 1320 can be rotated within the helical anchor 1310 about the steerable guide rail to engage non-target obstacles, instead of the leading end of the helical anchor 1310. For example, the shield 1320 can engage atrial walls, the mitral or tricuspid valve, chordae tendinea, etc., instead of the leading end of the helical anchor. In certain embodiments, target tissue locations may be located in proximity to sensitive non-target tissue or organs. The shield 1320 is operable to push or hold back sensitive non-target tissue so that the helical coil 1310 can be safely deployed.

In Figure 147B, the shield 1320 is shown being retracted. For example, the shield 1320 can be a shape-set wire that can be pulled between the steerable guide 1304 and the helical anchor 1310 to straighten out and remove the shield 1320. In one exemplary embodiment, the shield 1320, the end of the steerable guide 1304, and the end of the helical anchor 1310 are positioned at the target tissue with the shield 1320 in the shielding shape illustrated by Figure 147A. In one use, the helical anchor 1310 can be rotated to implant the helical anchor in the target tissue with the shield in place in the shielding shape. After the helical anchor 1310 is embedded into the tissue, both the guide rail 1304 and the shield 1320 can be pulled through the helical anchor 1310, with the shield straightening out as it is pulled through the helical anchor. In another use, the shield 1320 can be pulled through the helical anchor 1310, with the shield straightening out as it is pulled through the helical anchor, before the helical anchor 1310 is rotated to implant the helical anchor in the target tissue. In other embodiments, shield 1320 is fixed to the steerable guide rail 1304 and can be retracted with same.

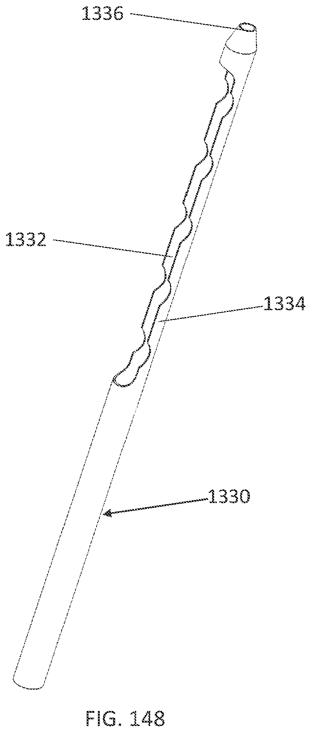

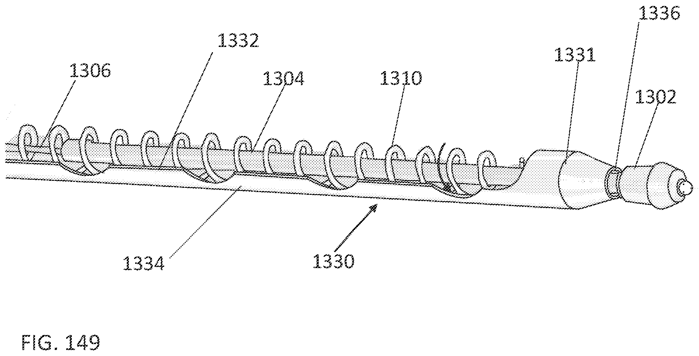

Figure 148 shows an exemplary embodiment of a guard 1330. The guard functions similarly to shield 1320 and is described in more detail with reference to Figures 149-151A-D. The guard allows for the helical anchor 1310 and steerable guide rail 1304 to be advanced through a patient's anatomy without inadvertent engagement by the lead coil of the anchor with non-target tissue. For example, the guard 1330 engages non-target obstacles, instead of the leading end of the helical anchor 1310. For example, the guard 1330 can engage atrial walls, the mitral or tricuspid valve, chordae tendinea, etc., instead of the leading end of the helical anchor.

In Figure 149, the helical anchor 1310 and the steerable guide rail 1304 are disposed inside the guard 1330. The guard 1330 has an open side 1332 where helical anchor is free to engage target tissue and a closed side 1334 which prevents non-target tissue or organs from engagement with helical anchor 1310. A leading end 1331 of the guard is closed on both sides. Before the leading end of the anchor 1310 is engaged with the tissue, the leading end of the anchor 1310 is positioned inside the leading end 1331 to prevent engagement with non-target tissue.

In the example illustrated by Figure 149, the stop 1302 is positioned outside of the leading end 1331 of the guard 1330. The tether 1306 and/or the steerable guide 1304 extend through an opening 1336 in the leading end 1331. The stop 1302 can be retracted through the opening 1336 by pulling on the tether 1306. For example, the opening 1336 can be larger than the stop 1302 and/or the stop can be compressible to allow the stop 1302 to be pulled through the opening 1336. In other exemplary embodiments, the stop 1302 is positioned inside the leading end 1331 of the guard 1330. In these embodiments, the opening 1336 can be omitted.

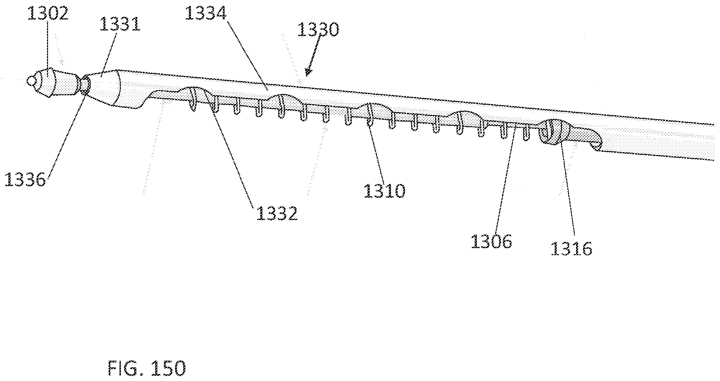

Referring to Figure 150, the open side 1332 forms a "window" which allow the leading coil of helical anchor 1310 to engage targeted tissue. For example, the anchor 1310 can be retracted in the guard 1330 to move the leading end of the helical anchor 1310 out of the leading end 1331 and into the area of the open side 1332. With the opening 1332 facing the target tissue and the steerable guide 1304 pressing the helical anchor 1310 against the tissue, the helical anchor 1310 can be rotated to engage the tissue with the leading end of the anchor and implant the helical anchor into the tissue.

The guard 1330 can extend the length of tether 1306 and/or steerable guide rail 1304 and terminate at the stop 1302. The leading end 1331 of the guard 1330 can be shaped to allow for easier traversal of the guard 1330, anchor, and steerable guide rail 1304 through a patient's cardiovascular system.

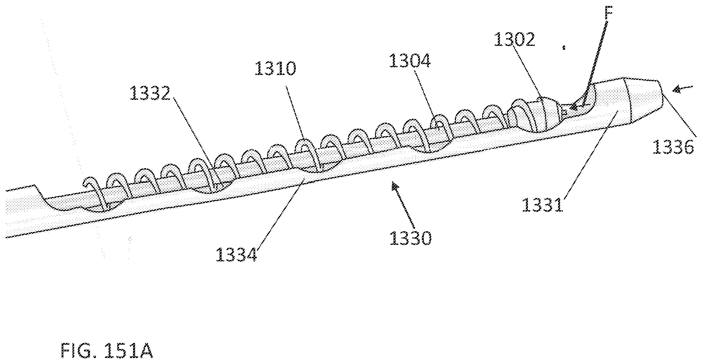

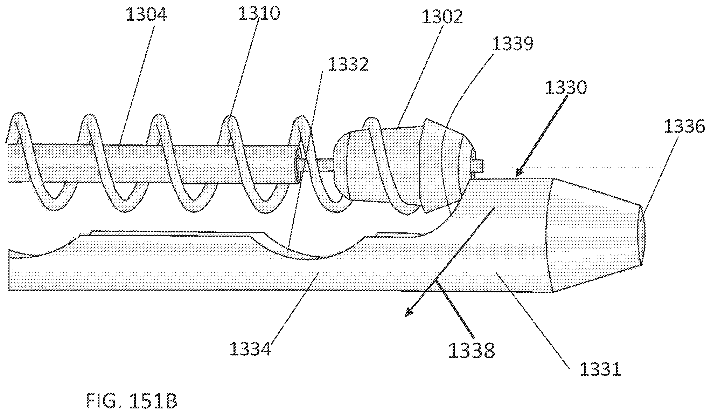

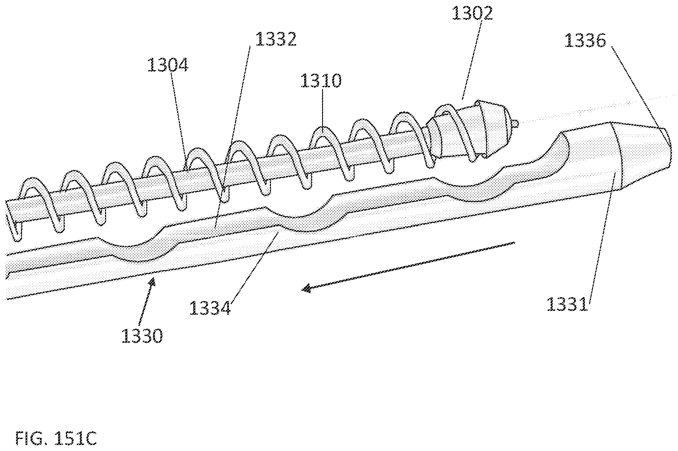

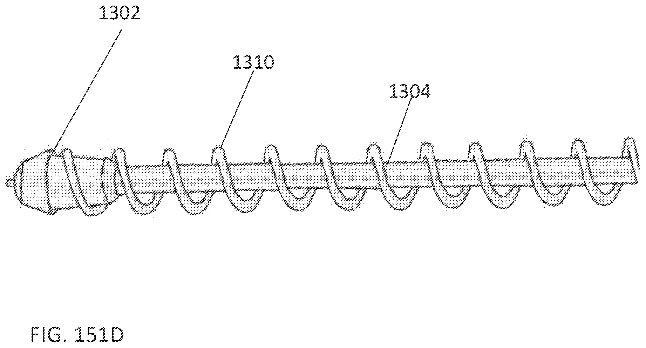

Figures 151A-D show how the exemplary guard 1330 can be removed from an implanted anchor 1310 and retracted from a patient. The steerable rail 1304 can be removed from the anchor and/or patient before, at the same time, or after any of the following guard removal steps. In Figure 151A, force F is applied to the stop 1302 to pull the stop through the opening 1336 and into the distal end of the helical anchor 1310. The guard 1330 is moved such that stop 1302 and helical anchor 1310 are both in the window formed by open side 1332. Referring to Figure 151B, once the stop 1302 and helical anchor 1310 are in the window formed by the open side 1332, the guard 1330 is moved off the stop 1302 and helical anchor 1310 as indicated by arrow 1338. The nosecone 1301 of the stop is optionally tapered and/or the window includes a tapered surface 1339 to allow the guard 1330 to slide off the nosecone 1301 as illustrated by Figure 151B. Referring to Figures 151C and 151D, once the guard 1330 is separated from stop the 1302 and the helical anchor 1310, the guard can be withdrawn from the patient, leaving the implanted stop 1302 and helical anchor 1310.

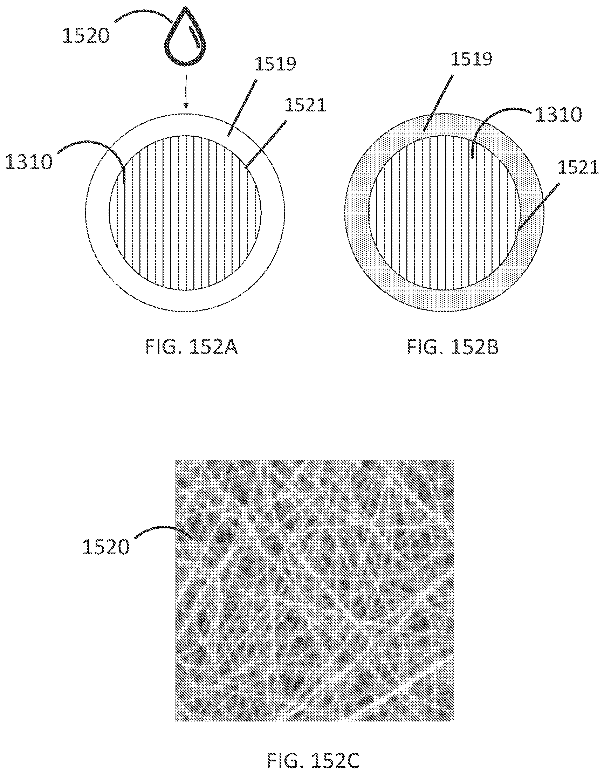

Figure 152B shows an exemplary embodiment of helical anchor 1310 with a covering 1519 and/or a lubricant 1520 on the outside surface 1521 of a wire that forms the helical implant 1310. The covering 1519 and lubricant can take a wide variety of different forms. For example, the covering can be a wrap, a coating, a sleeve, spun-bonded fibers, etc. Any type of covering or coating can be used. The lubricant 1520 can be any lubricant that decreases the friction between the implant 1310 and the tissue 1308 that the anchor 1310 is implanted in. As a result, the amount of torque required to implant the helical anchors can be reduced by the lubricant. In the example illustrated by Figures 152A, a covering 1519 is provided on the outside surface 1521 and then the lubricant 1520 is applied to the covering 1519. This forms the anchor 1310 with a covering 1519 that is saturated with lubricant 1520 illustrated by Figure 152B. In another exemplary embodiment, the material that forms the covering 1519 is already lubricated when applied to the anchor 1310 or the material that forms the covering has lubricating properties, such as a covering material that comprises Teflon. Referring to Figure 152C, in one exemplary embodiment the implant coating 1520 is made of a non-woven material, such as staple non-woven, melt-blown, spunbond/spunlaid, or flashspun. In one exemplary embodiment, the non-woven material is porous and absorbs the lubricant 1520. In one exemplary embodiment, the implant coating 1520 is made from BioSpun polymers which can improve tissue ingrowth at a deployment site. The implant coating 1520 can also cause helical anchor 1310 to have a roughened surface which can reduce metallic reflections and enhance ECHO imagining. In one exemplary embodiment, the roughened surface is provided by a non-woven material. In one exemplary embodiment, the roughened surface is provided by BioSpun polymers. It will be appreciated that coated helical anchor 1310 illustrated by Figure 152B can be used interchangeably with any of the helical anchors 1310 described herein.

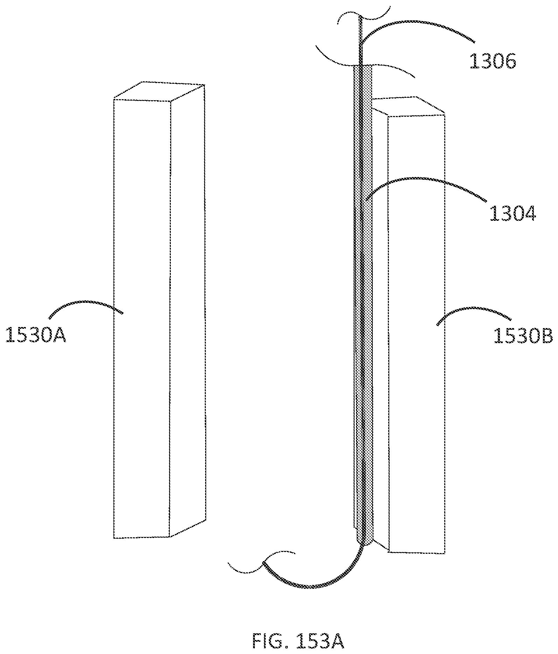

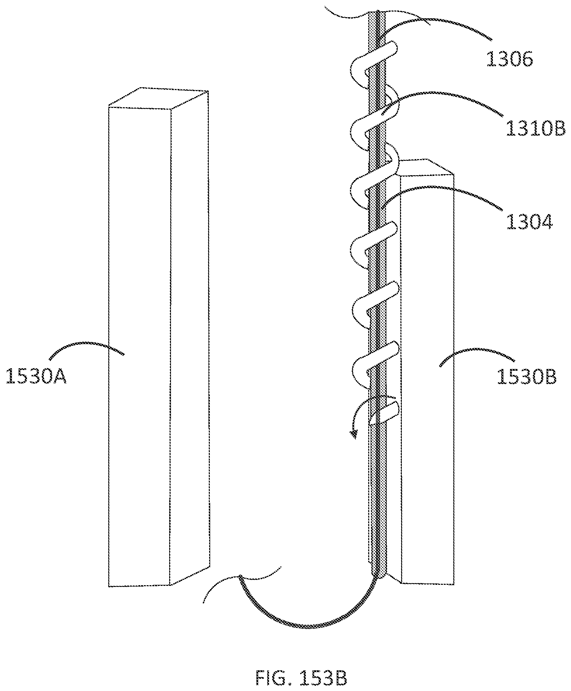

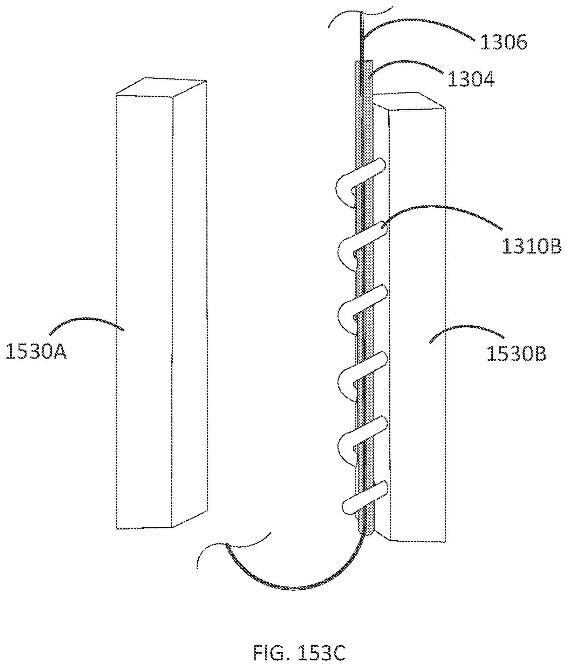

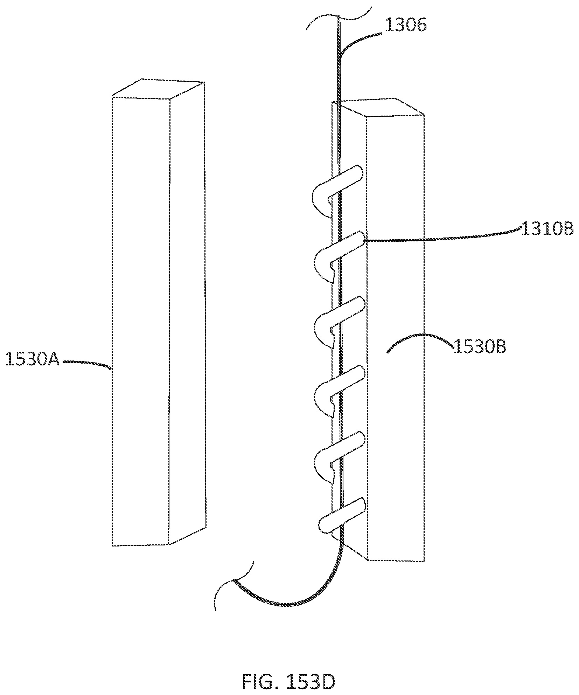

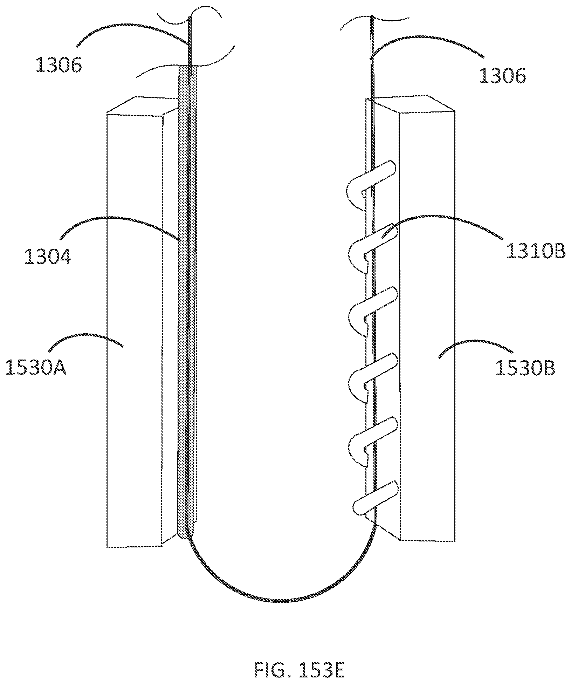

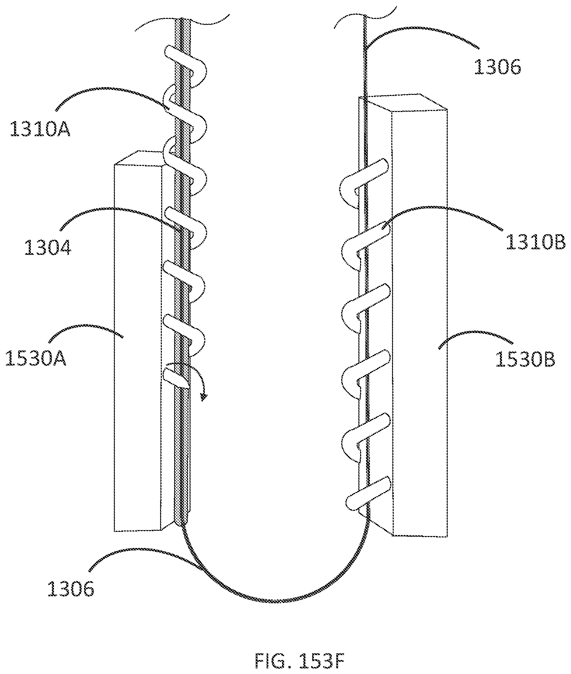

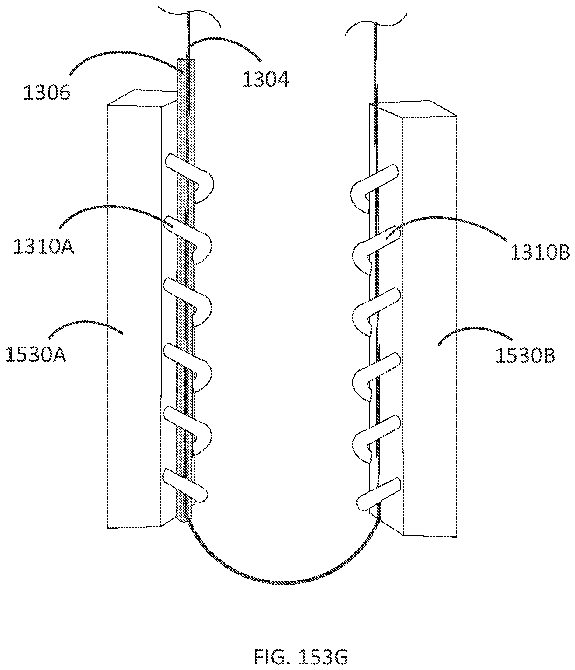

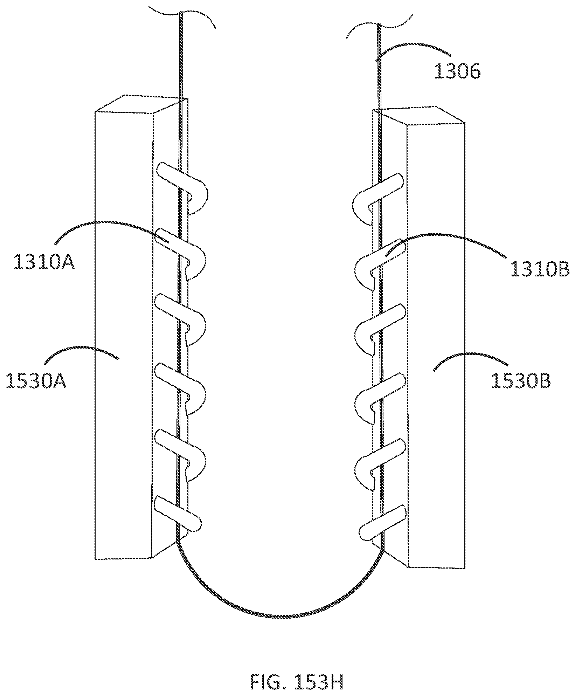

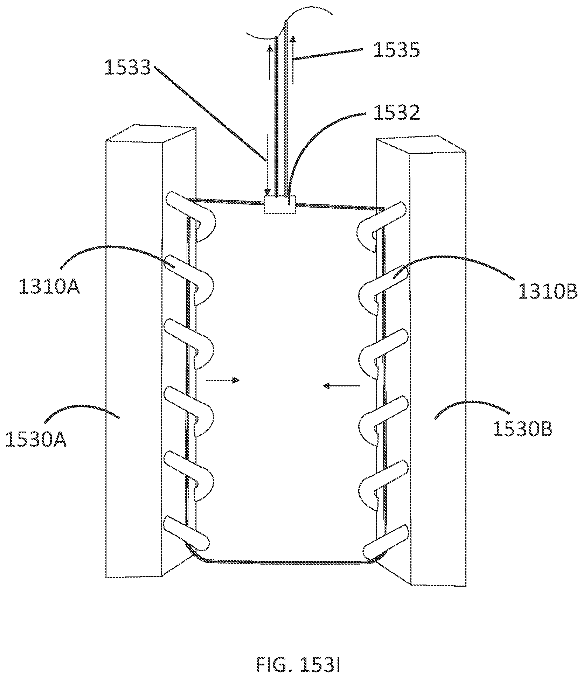

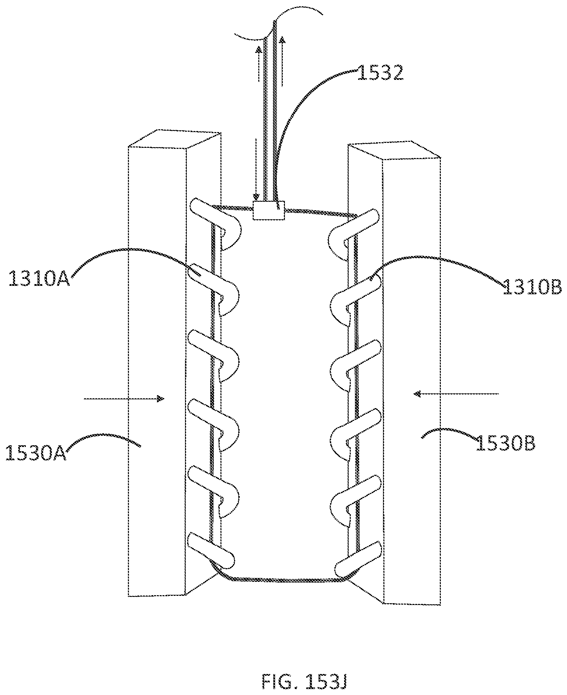

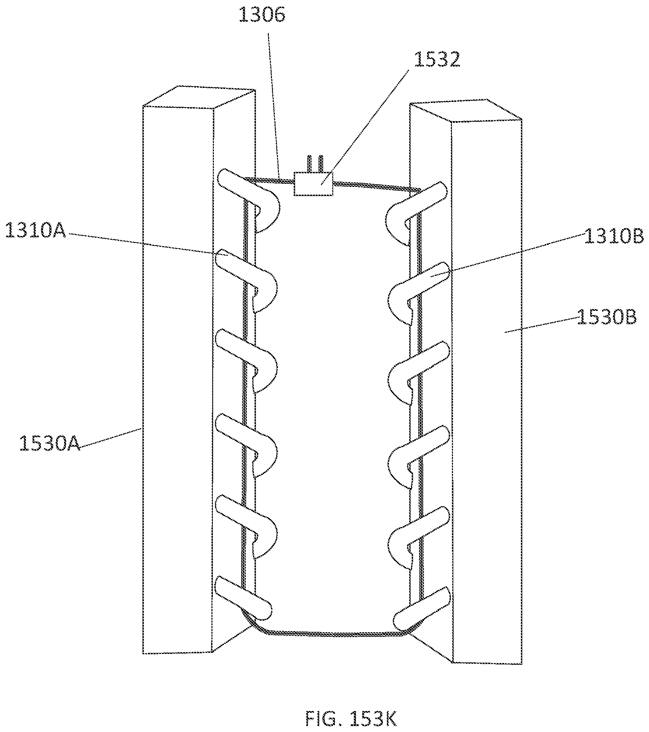

Figures 153A-153K show an exemplary deployment of helical anchors 1310A, 1310B into target tissue areas 1530A and 1530B. The anchors 1310 are then pulled toward one another and secured to remodel the target tissue areas 1530A and/or 1530B. In Figure 153A, a steerable guide rail 1304 is advanced into position against a target tissue area 1530B. In Figure 153B, a helical anchor 1310B is deployed along steerable guide rail 1304 to engage the target tissue area 1530B. In Figure 153C, the helical anchor 1310B is shown fully deployed in the targeted tissue area 1530B. In Figure 153D, the steerable guide rail 1304 has been removed, exposing the tether 1306. In Figure 153E, a steerable guide rail 1304 is advanced into position against target tissue area 1530A. In this exemplary embodiment, the tether 1306 that extends through the anchor 1310B continues through the steerable guide rail 1304 which is against the target tissue location 1530A. In Figure 153F, a second helical anchor 1310A is deployed along the steerable guide rail 1304 into target tissue area 1530A. In Figure 153G, helical anchor 1310A is shown fully deployed at targeted tissue area 1530A. In Figure 153H, the steerable guide rail 1304 has been removed, exposing the tether 1306. In Figure 153I, a tether lock 1532 is advanced along the tether 1306 as indicated by arrow 1537 while the tether 1306 is pulled as indicated by arrow 1535. Referring to Figure 153J, as tether lock 1532 is adjusted, the tether 1306 pulls the helical anchors 1310A and 1310B and the attached tissue areas 1530A and 1530B toward one another. In Figure 153K, the tether lock 1532 is locked into a fixed position, which holds target tissue areas 1530A and B in a remodeled position and the ends of the tether 1306 are cut off. The tether lock 1532 can take a wide variety of different forms. For example, any of the connectors 240 can be used as the tether lock 1532.

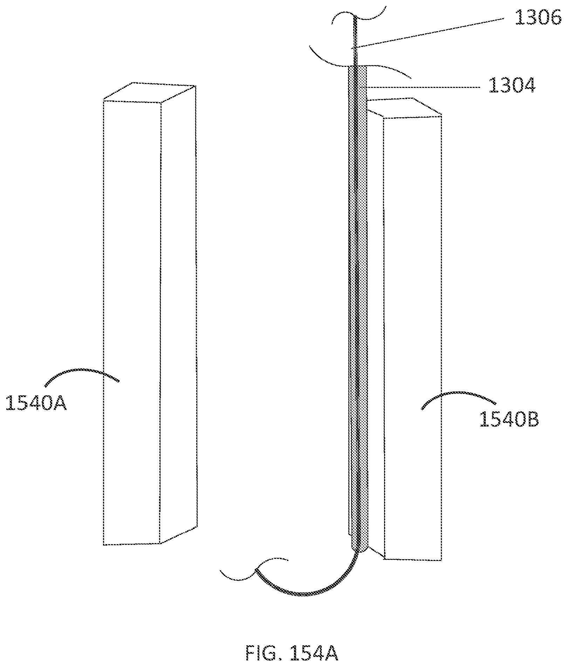

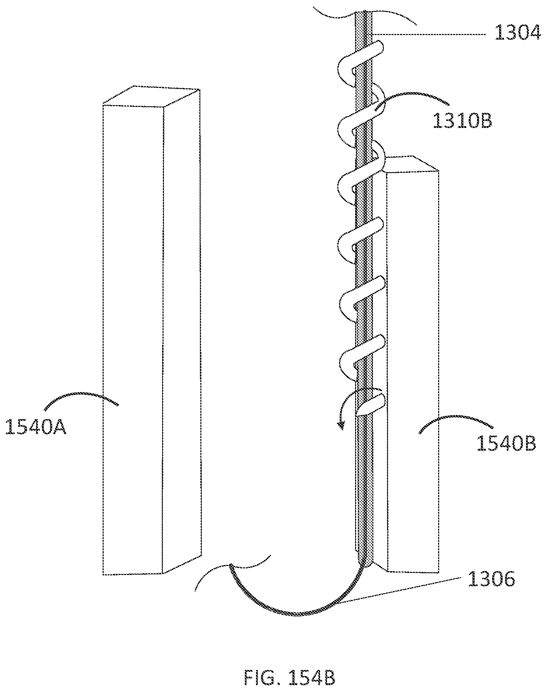

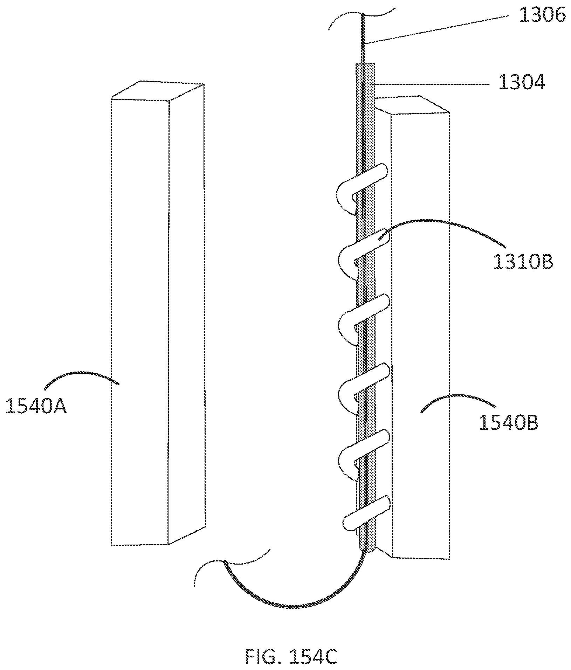

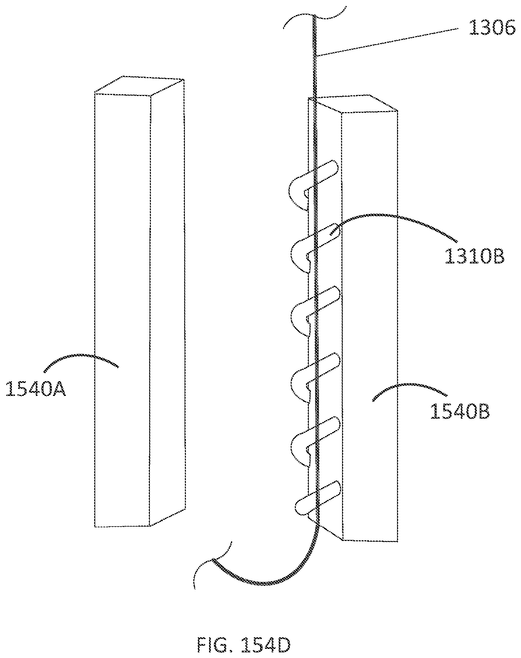

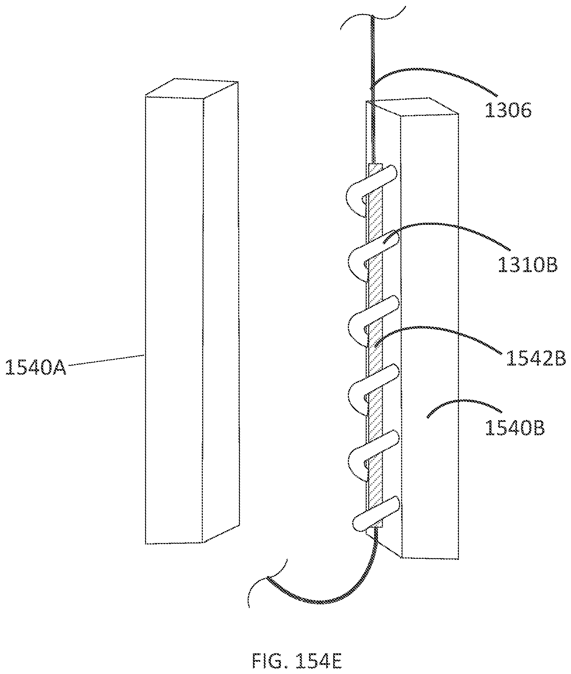

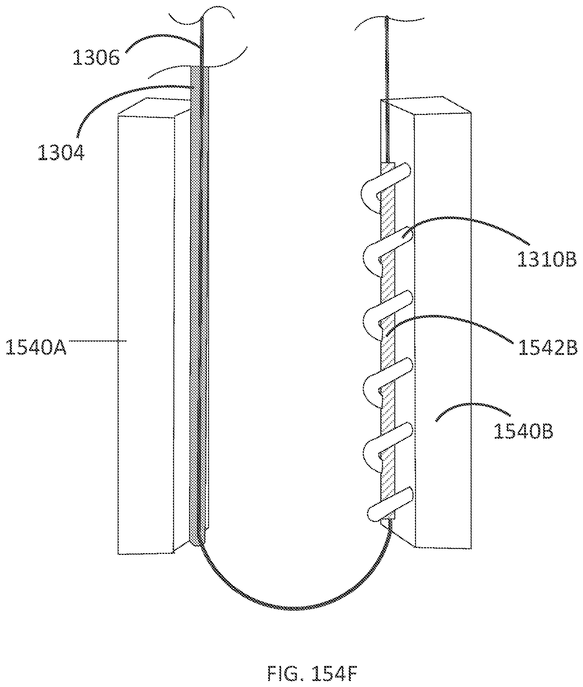

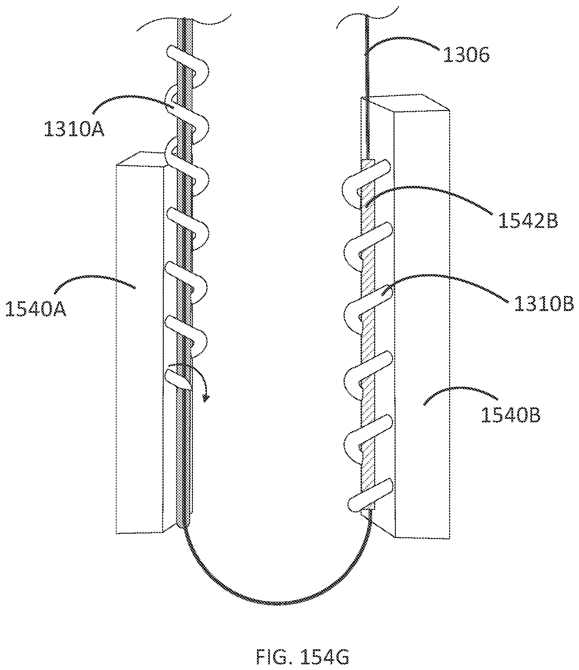

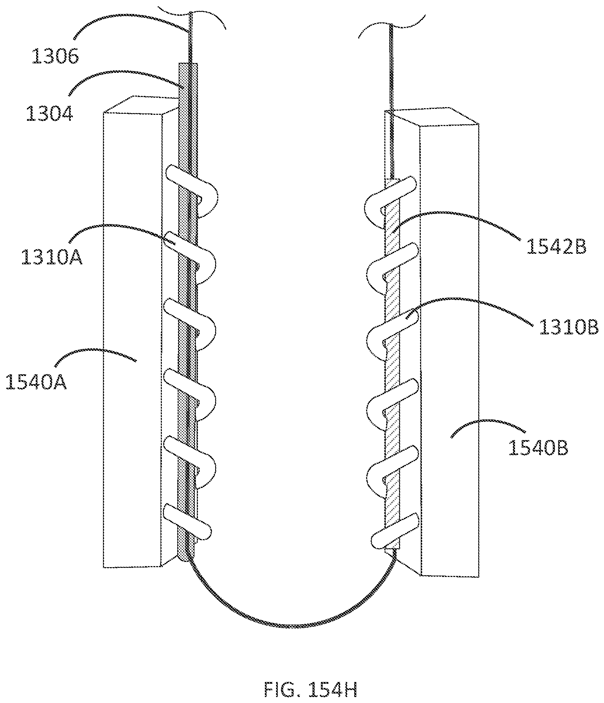

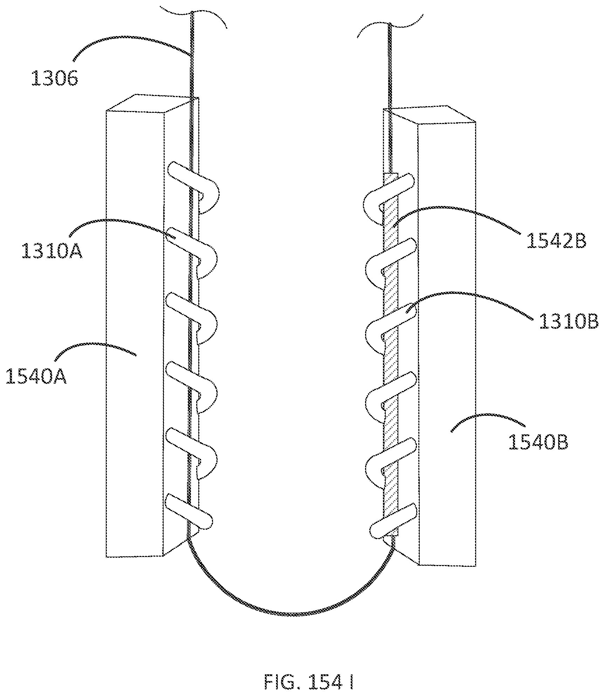

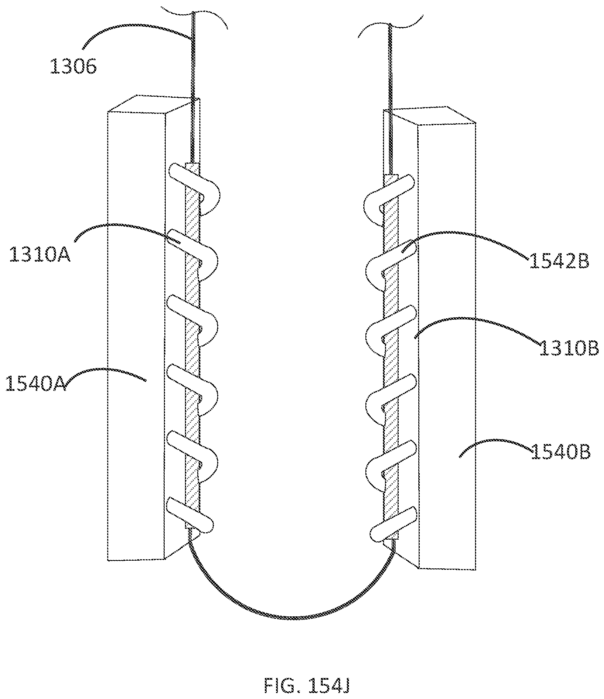

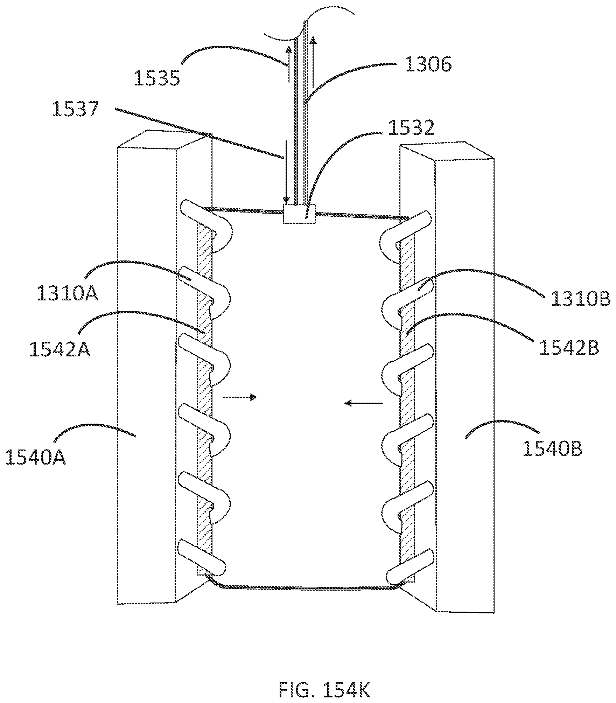

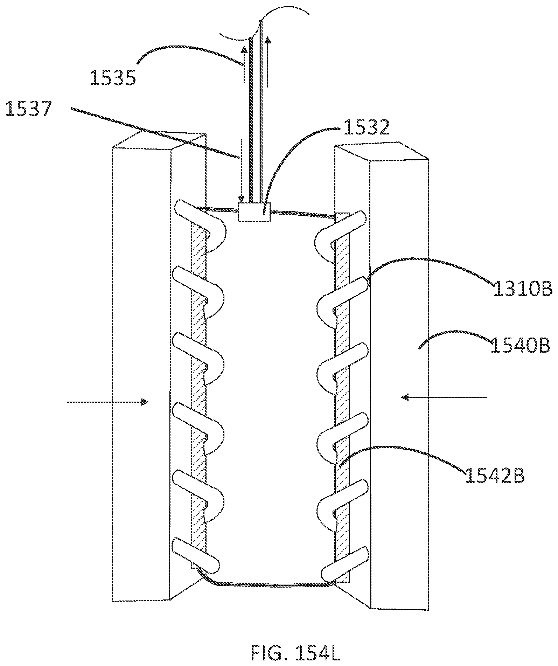

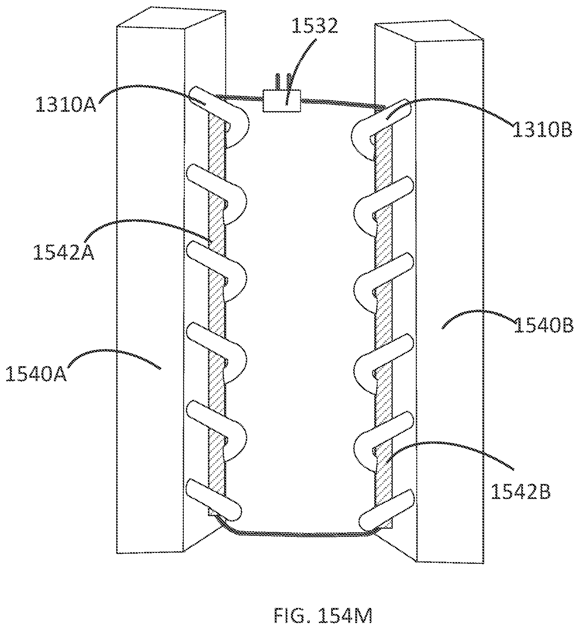

Figures 154A-154M show an exemplary deployment of helical anchors 1310A, 1310B into target tissue area(s) 1540A-B. In Figure 154A, steerable guide rail 1304 is advanced into position against a target tissue area 1540B. In Figure 154B, a helical anchor 1310B is deployed along the steerable guide rail 1304 to engage target tissue area 1540B. In Figure 154C, the helical anchor 1310B is shown fully deployed in the targeted tissue area 1540B. In Figure 154D, the steerable guide rail 1304 has been removed, exposing the tether 1306 in the helical anchor 1310B. In Figure 154E, a stabilizer 1542B is deployed within the space between the target tissue area 1540B and helical anchor 1310B. The stabilizer 1542B can be slidably disposed over the tether 1306. The diameter of stabilizer 1542B can be the same, larger, or smaller than the diameter steerable guide rail 1304. In other embodiments, the stabilizer 1542B is inserted separate from and without the aid of tether 1306. In Figure 154F, a steerable guide rail 1304 is advanced into position against a target tissue area 1540A. The tether 1306 the tether 1306 that extends through the anchor 1310B continues through the steerable guide rail 1304 which is against the target tissue location 1540A. In Figure 154G, a second helical anchor 1310A is deployed along the steerable guide rail 1304 into target tissue area 1540A. In Figure 154H, the helical anchor 1310A is shown fully deployed into the targeted tissue area 1540A. In Figure 154I, the steerable guide rail 1304 has been removed, exposing tether 1306 in the anchor 1310A. In Figure 154J, a second stabilizer 1542A is deployed within the space between the target tissue area 1540A and helical anchor 1310A. The diameter of the stabilizer 1542A can be the same, larger, or smaller than the diameter steerable guide rail 1304. As shown, the stabilizer 1542A the tether 1306 is threaded through the stabilizer 1542A. In other embodiments, stabilizer 1542A is inserted into the anchor 1310A without the aid of tether 1306. In Figure 154K, a tether lock 1532 is advanced along the tether 1306 as indicated by arrow 1537 while the tether 1306 is pulled as indicated by arrow 1535. Referring to Figure 154L, as the tether lock 1532 is advanced, the tether 1306 pulls on the stabilizers 1542A and 1542B, which pull on the anchors 1310A, 1310B, causing target tissue areas 1540A and 1540B to move closer together. In Figure 154M, the tether lock 1532 is locked into a fixed position, which holds target tissue areas 1540A and B in a desired position and the ends of the tether 1306 are cut off.

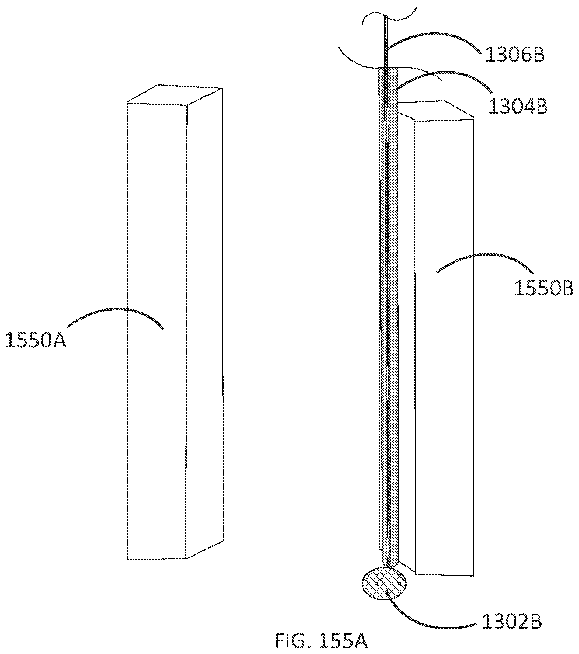

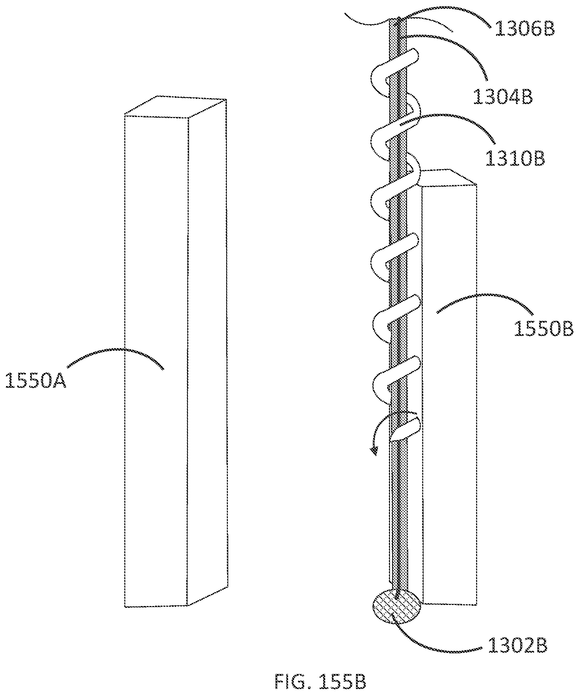

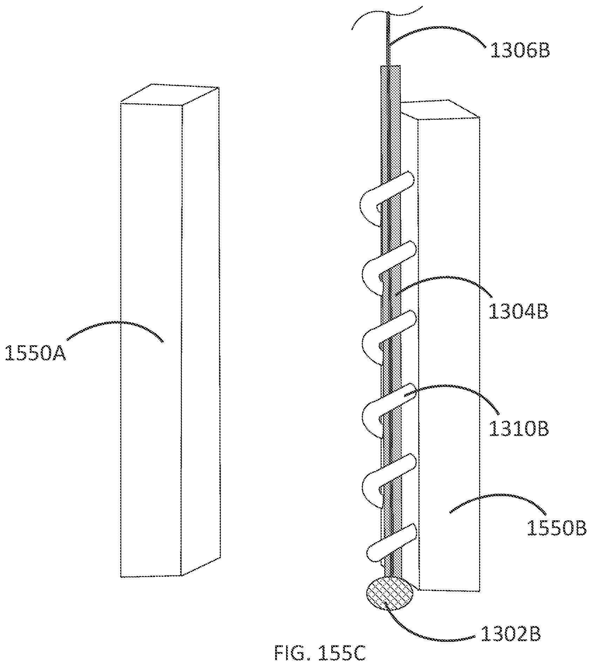

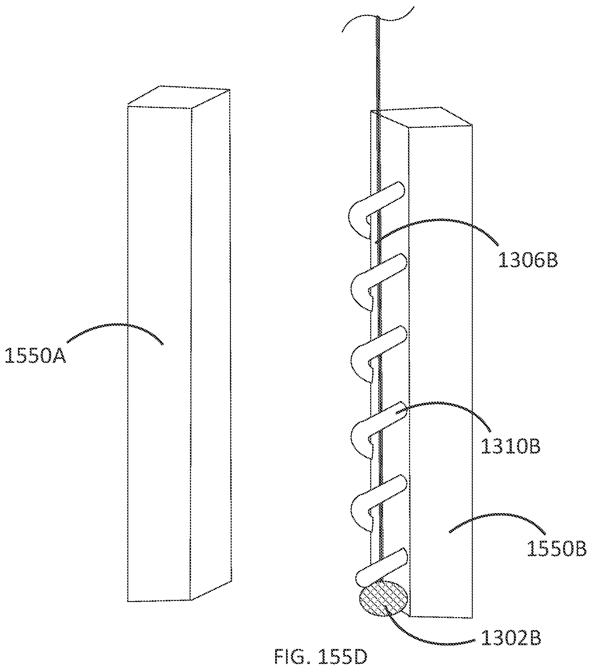

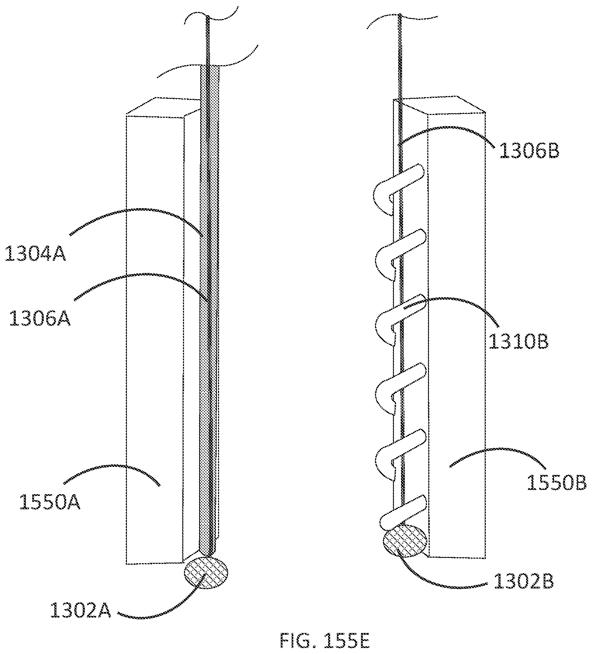

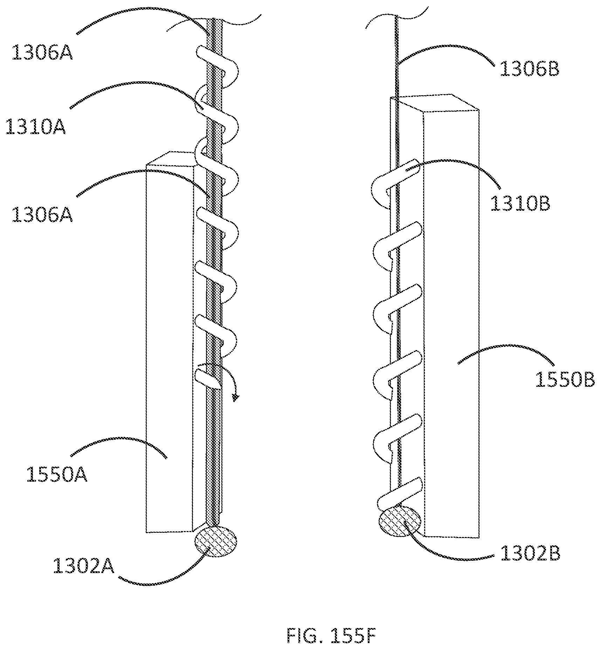

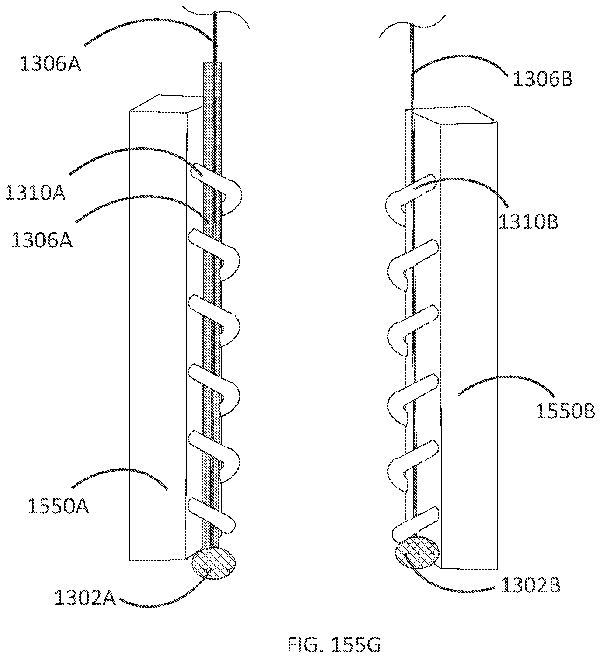

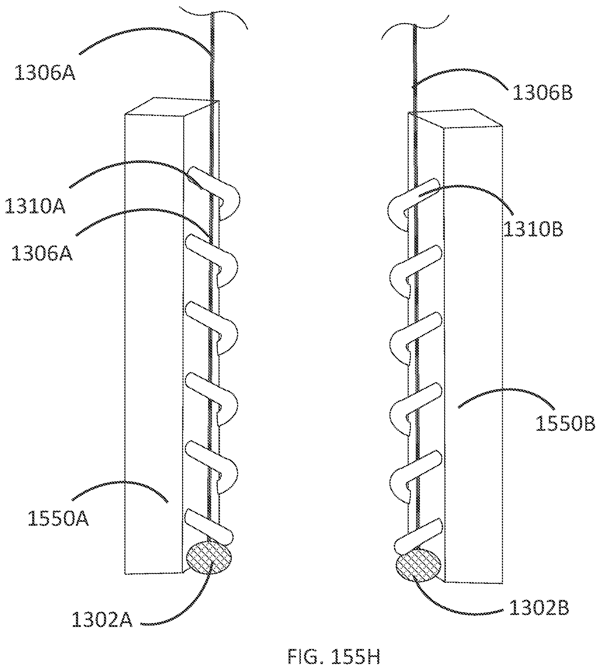

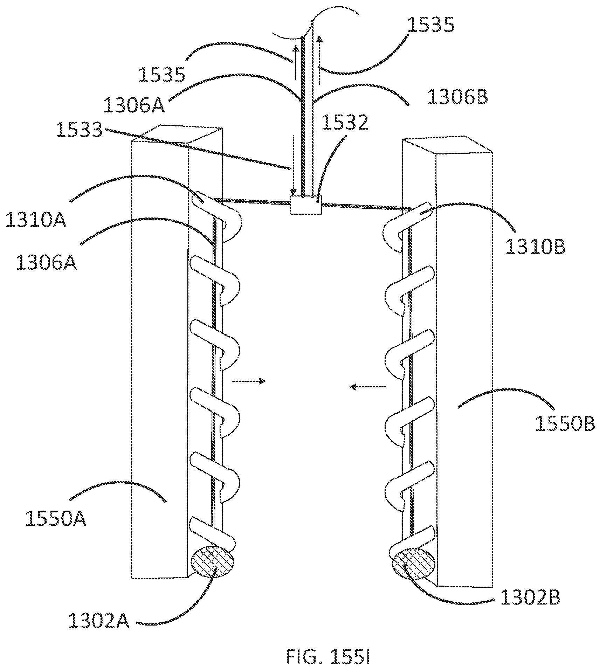

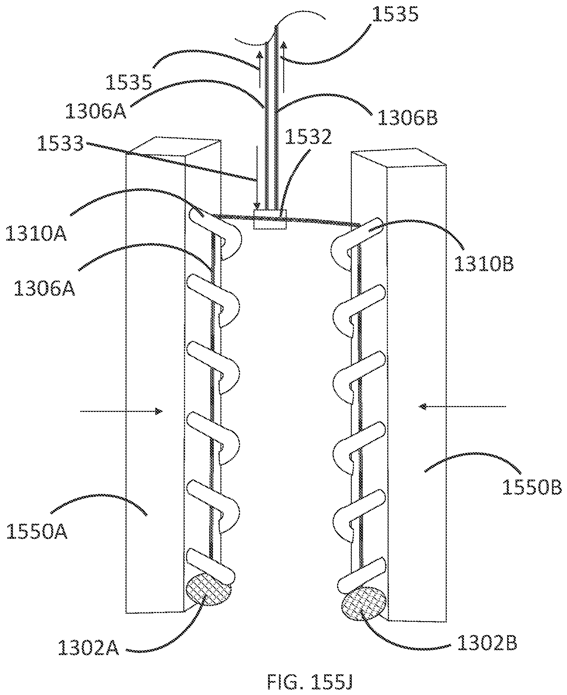

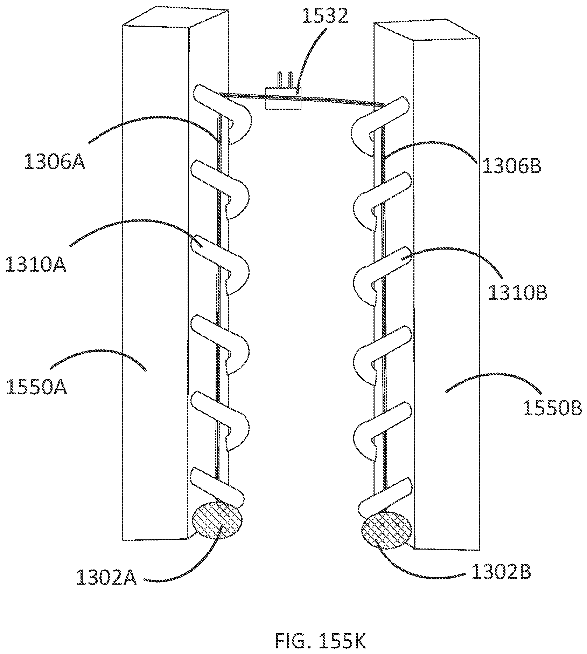

Figures 155A-155K show an exemplary deployment of helical anchors 1310A, 1310B implanted into target tissue areas 1550A and 1550B. In Figure 154A, a steerable guide rail 1304B is advanced into position against a target tissue area 1550B. In Figure 155B, a helical anchor 1310B is deployed along steerable guide rail 1304B to engage the target tissue area 1550B. In Figure 155C, the helical anchor 1310B is shown fully deployed in the targeted tissue area 1550B with stop 1302B connected to the tether 1306B a distal end of the anchor 1310B. In Figure 155D, the steerable guide rail 1304B has been removed, exposing the tether 1306B inside of the anchor 1310B. In Figure 155E, a second steerable guide rail 1304A is advanced into position against a target tissue location 1550A. In Figure 155F, a second helical anchor 1310A is deployed along the steerable guide rail 1304A into target tissue area 1550A. In Figure 155G, the helical anchor 1310A is shown fully deployed in the targeted tissue area 1550A, with the stop 1302A attached to the second tether 1306A. In Figure 155H, the steerable guide rail 1304A has been removed, exposing the tether 1306A. In Figure 155I, a tether lock 1532 is advanced along the tethers 1306A, 1306B as indicated by arrow 1537 while the tethers 1306A, 1306B are pulled as indicated by arrow 1535. Referring to Figure 155J, as tether lock 1532 is adjusted, the tethers 1306A, 1306B pull the helical anchors 1310A and 1310B and the attached tissue areas 1550A and 1550B toward one another. In Figure 155K, the tether lock 1532 is locked into a fixed position, which holds target tissue areas 1550A and 1550B in a desired position.

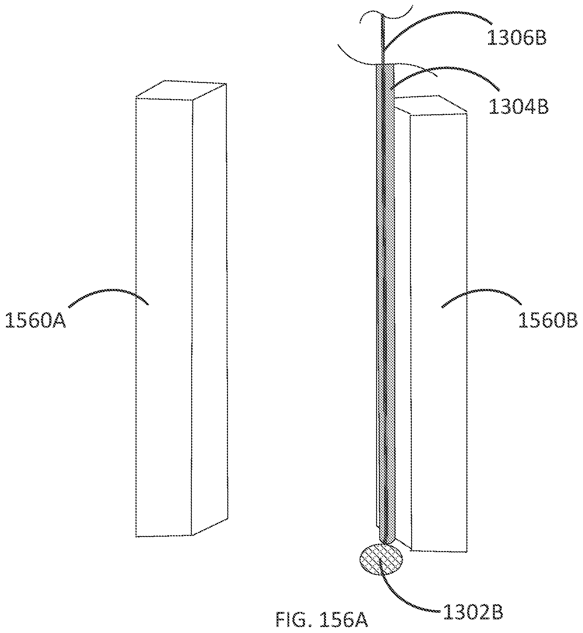

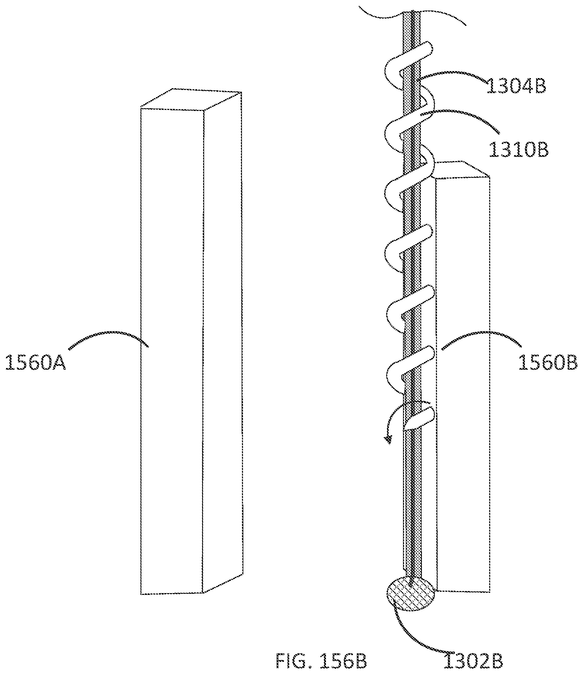

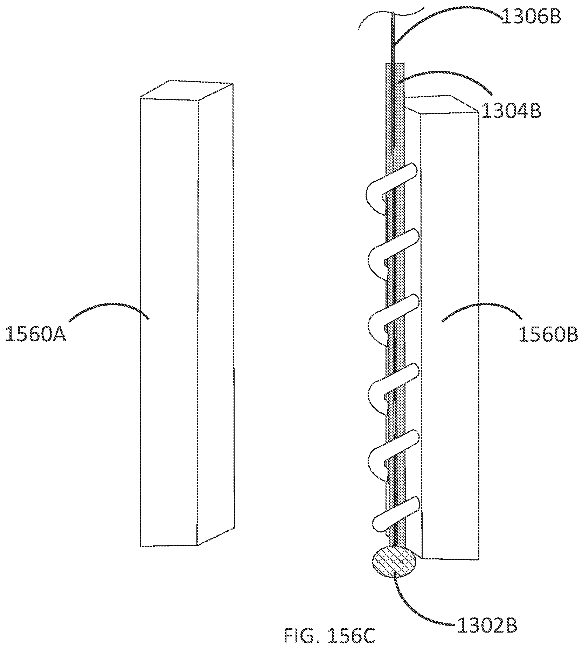

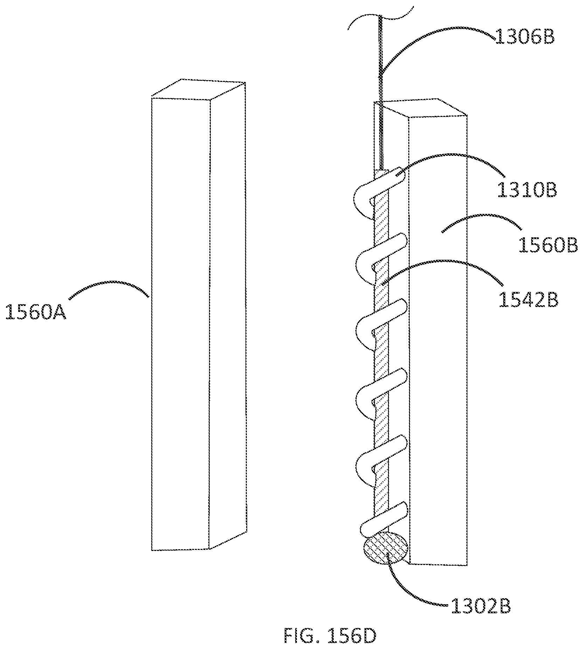

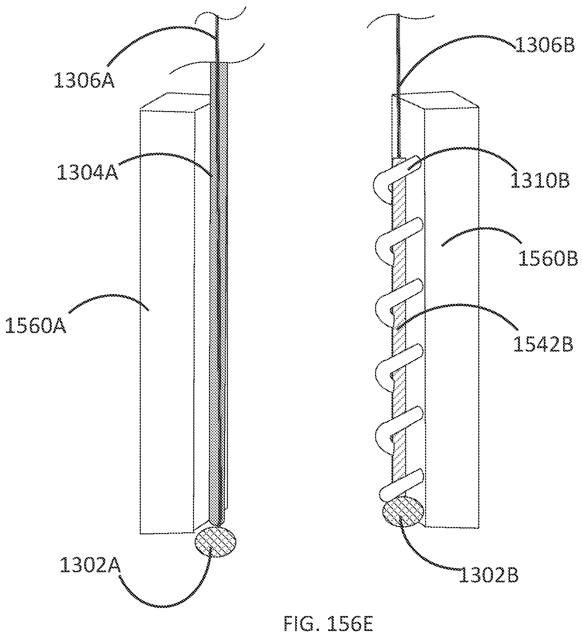

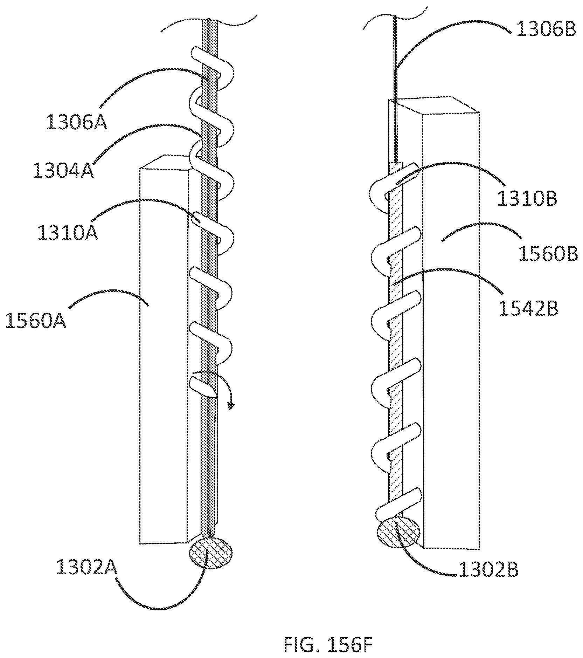

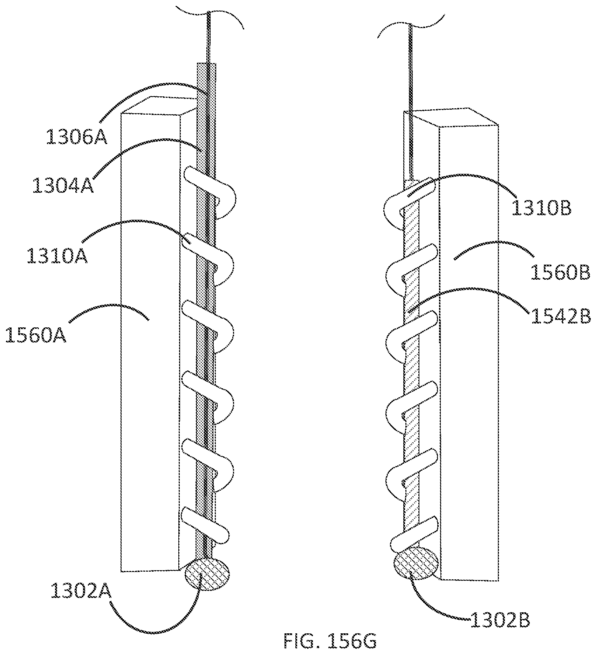

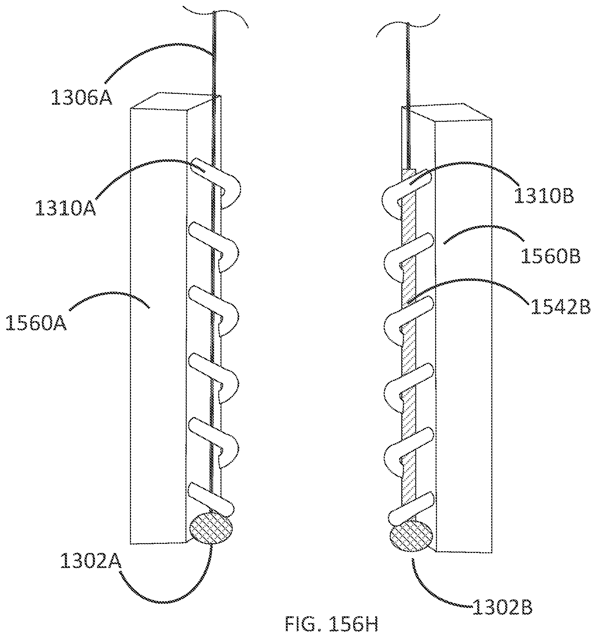

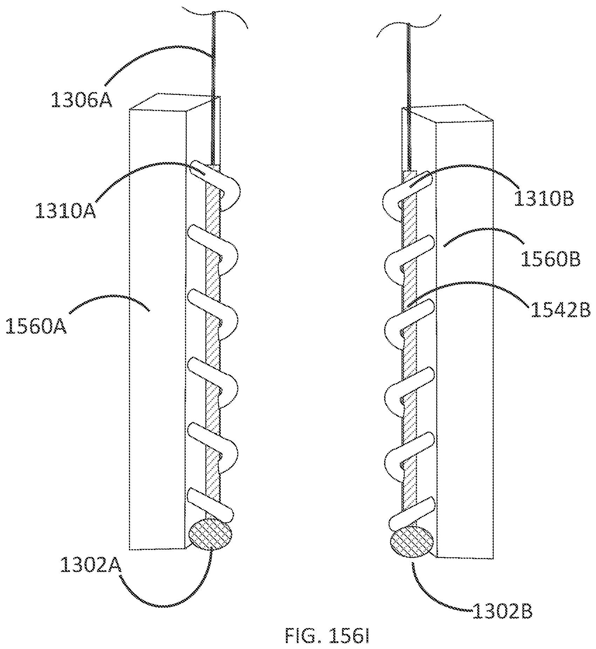

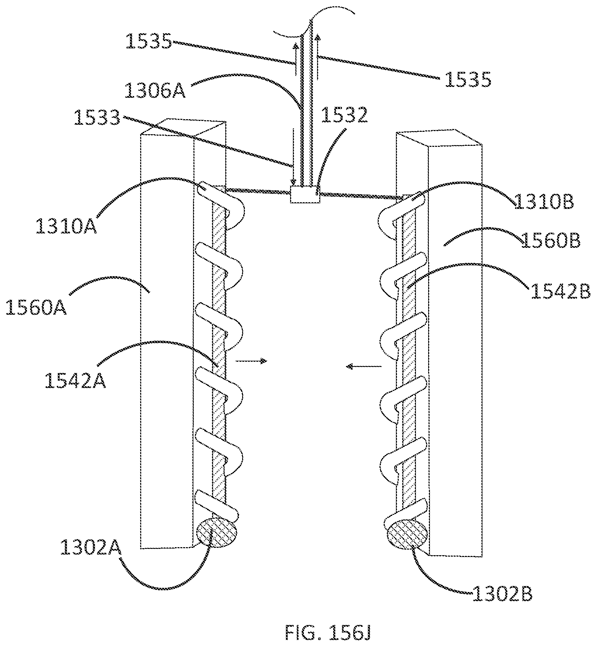

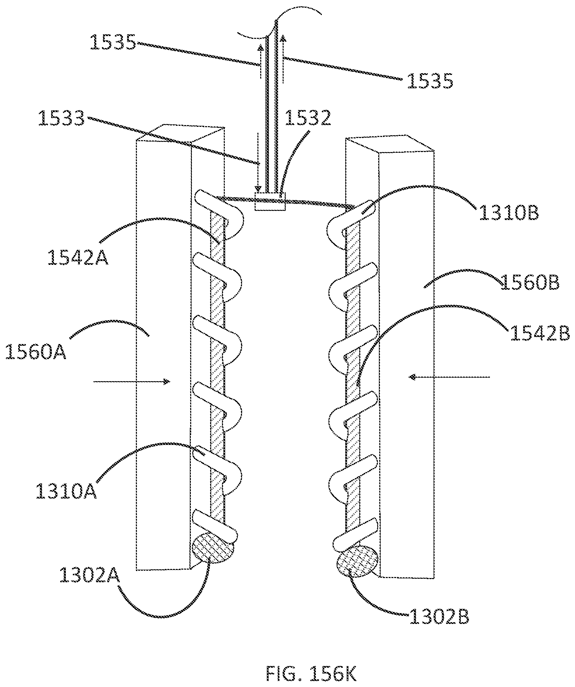

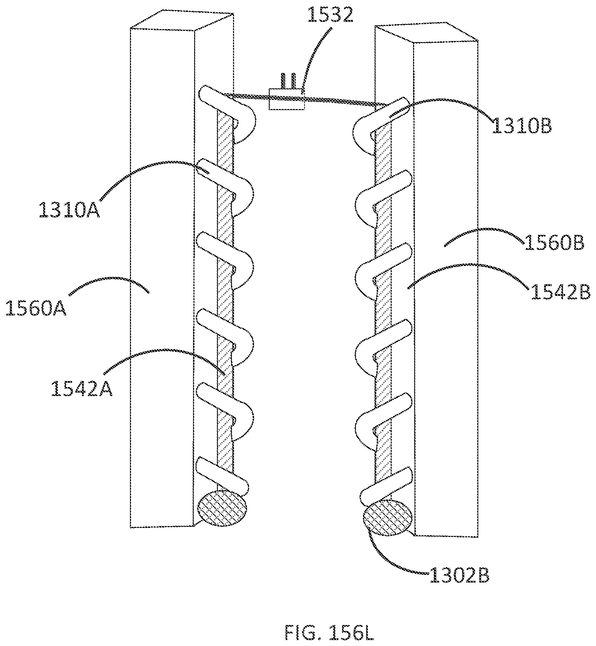

Figures 156A-156L show an exemplary deployment of two helical anchors 1310 deployed into target tissue areas 1560A and 1560B. In Figure 156A, a steerable guide rail 1304B is advanced into position proximate to a target tissue area 1560B. In Figure 156B, the helical anchor 1310B is advanced along steerable guide rail 1304B to engage target tissue area 1560B. In Figure 156C, the helical anchor 1310B is shown fully deployed into the targeted tissue area 1560B with a stop 1302B disposed at an end of the anchor. In figure 156D, the steerable guide rail 1304B has been removed, and replaced with a stabilizer 1542B. The stabilizer 1542B is deployed within the space between the target tissue area 1560B and helical anchor 1310B. As shown, the tether is threaded through the stabilizer 1542B. In other embodiments, the stabilizer 1542B is inserted next to the tether 1306B. In Figure 156E a second steerable guide rail 1304A is deployed proximate to target tissue location 1560A. In Figure 156F, the helical anchor 1310A is deployed along the steerable guide rail 1304A into the target tissue area 1560A. In Figure 156G, the helical anchor 1310A is shown fully deployed into the targeted tissue area 1560A with a stop 1302A disposed at the end of the anchor. In Figure 156H, the steerable guide rail 1304A has been removed, exposing tether 1306A. In Figure 156I, a stabilizer 1542A is deployed within the space between the target tissue area 1560A and helical anchor 1310A. In Figure 156J, a tether lock 1532 is advanced along the tethers 1306A, 1306B as indicated by arrow 1537 while the tethers 1306A, 1306B are pulled as indicated by arrow 1535. Referring to Figure 156J, as tether lock 1532 is adjusted, the tether 1306 pulls the helical anchors 1310A and 1310B and the attached tissue areas 1560A and 1560B toward one another. In Figure 156K, the tether lock 1532 is tightened further pulling on stabilizers 1542A and 1542B causing target tissue areas 1560A and B to move closer together. In Figure 156L, the tether lock 1532 is locked into a fixed position, which holds target tissue areas 1560A and B in a desired position and the ends of the tethers 1306A, 1306B are cut off.

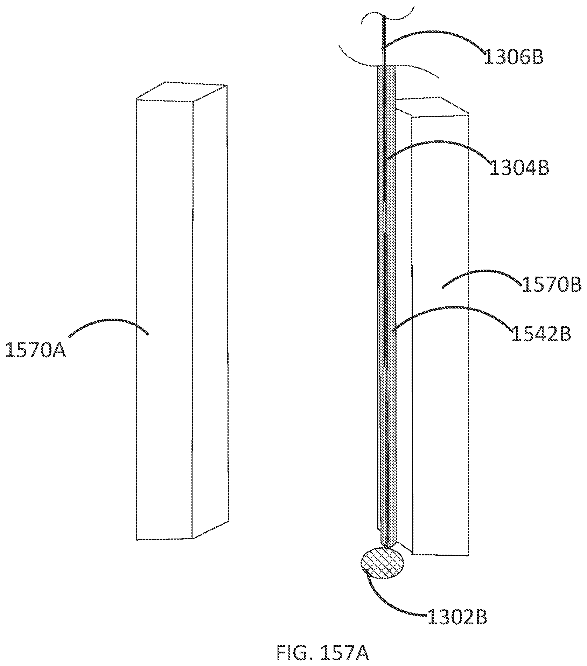

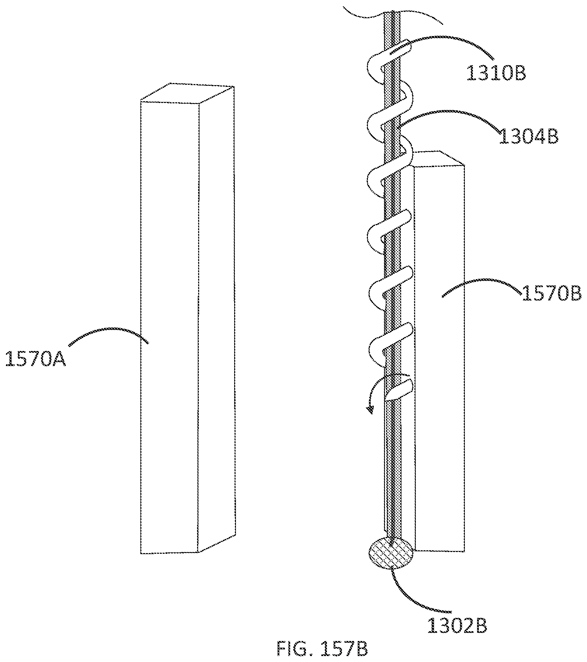

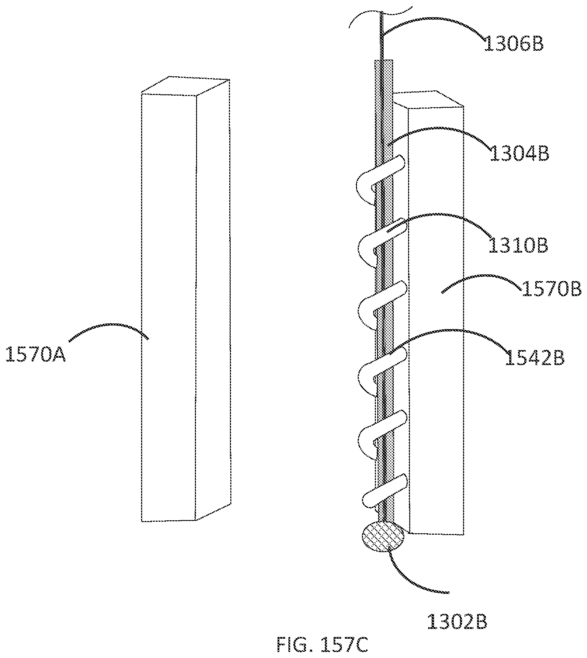

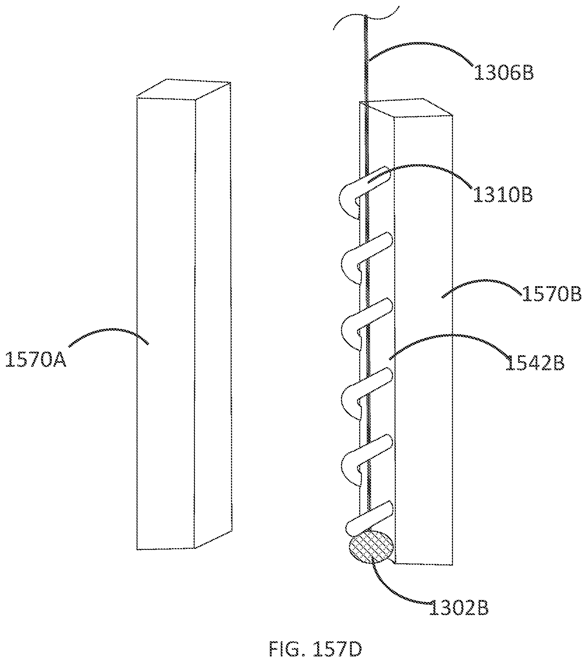

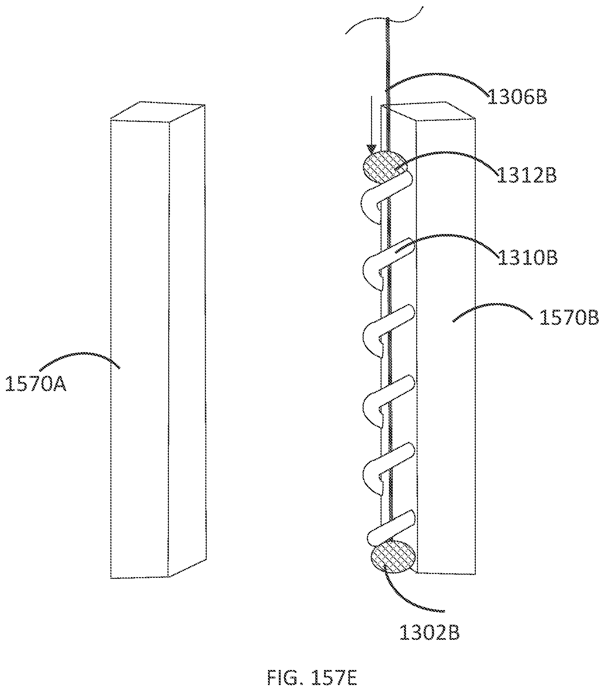

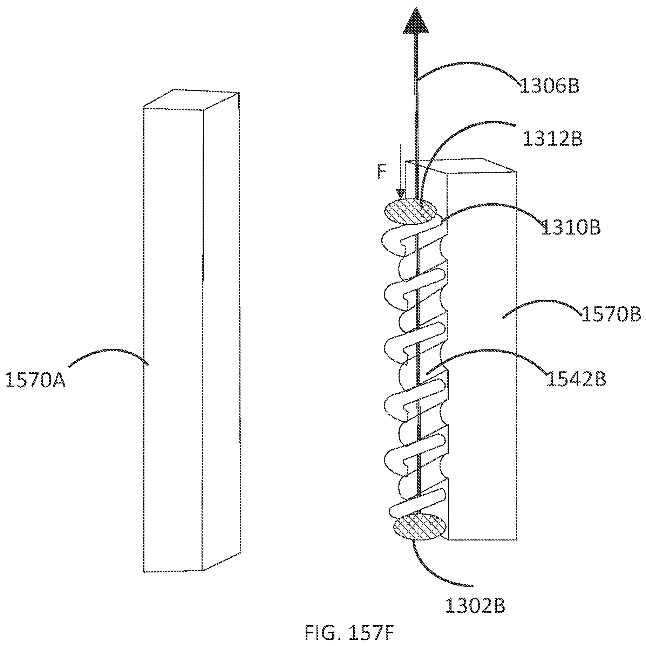

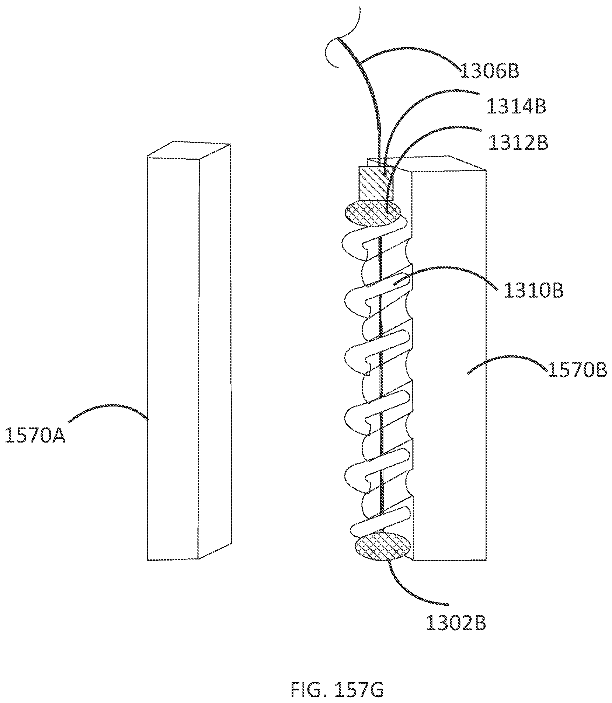

Figures 157A-L show another exemplary deployment of anchors 1310A and 1310B into a plurality of target tissue areas 1570A and 1570B. In Figure 157A, a steerable guide rail 1304B is advanced into position proximate to target tissue area 1570B. In Figure 157B, a helical anchor 1310B is advanced along steerable guide rail 1304B to engage target tissue area 1570B. In Figure 157C, the helical anchor 1310B is shown fully deployed into the targeted tissue area 1570B with the stop 1302B at the end of the guide rail 1304B. In Figure 157D, the steerable guide rail 1304B has been removed, exposing a tether 1306B inside the anchor 1310B. In Figure 157E, an anchor stop 1312B is applied to the proximate end of helical anchor 1310B along the tether 1306B. In Figure 157F, a force F is applied to the anchor stop 1312B while the tether 1306B is pulled to contract the helical anchor 1310B and remodel the target tissue area 1570B. Referring to Figure 157G, once a desired compression of the helical anchor 1310B is achieved, a locking mechanism 1314B is affixed to the tether 1306B. The locking mechanism 1314B holds the stop 1312B in place, which maintains the desired compressed position of anchor 1310B in the tissue 1570B.

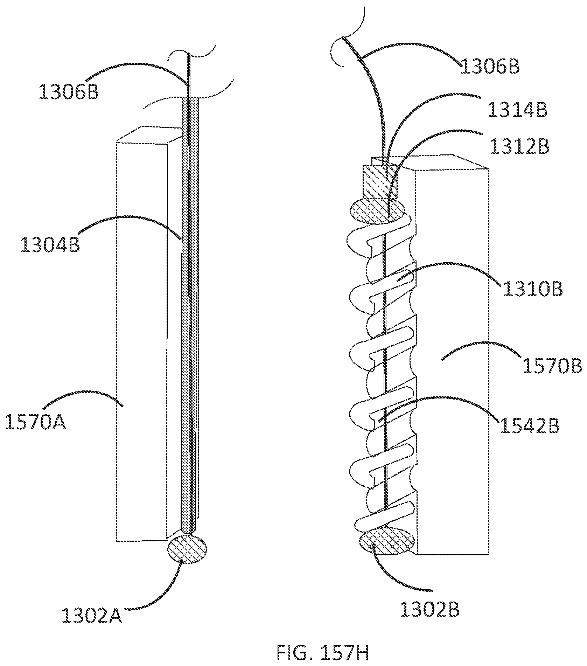

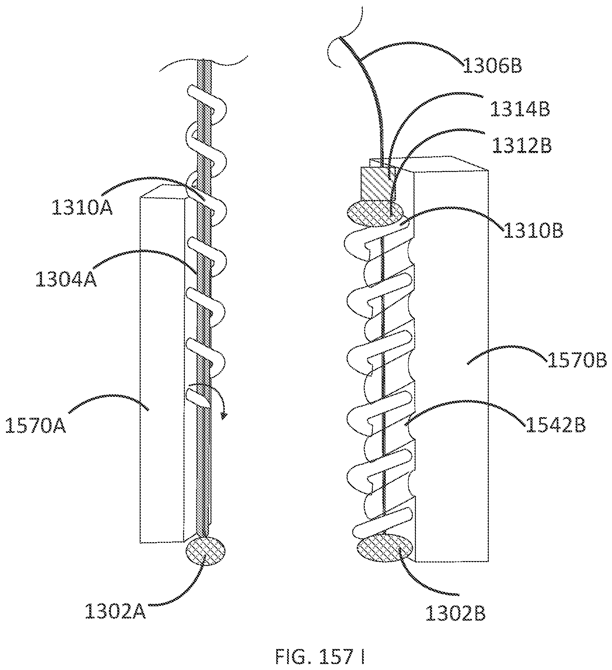

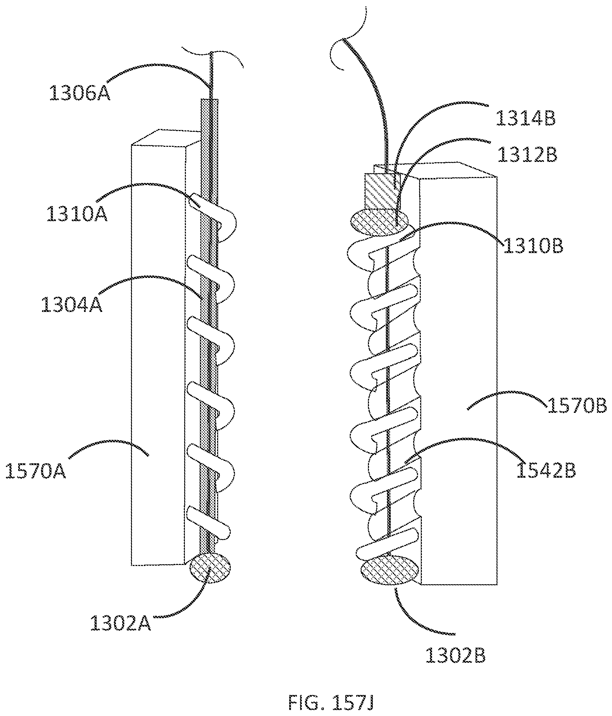

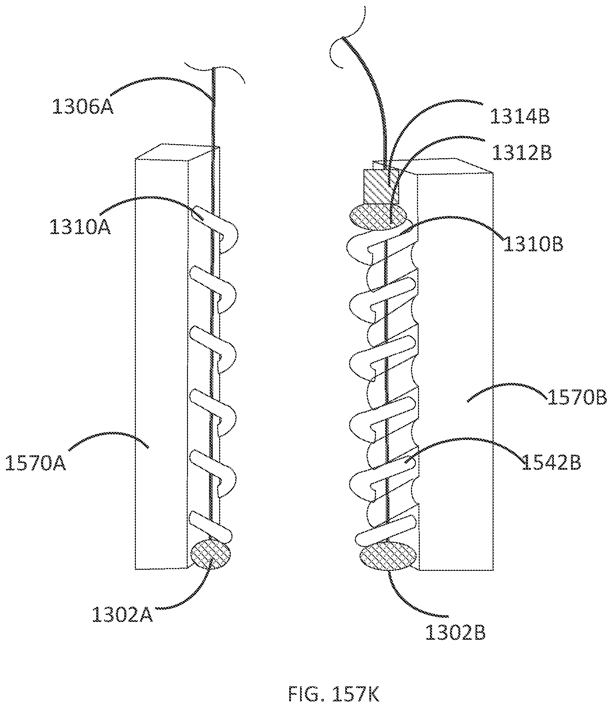

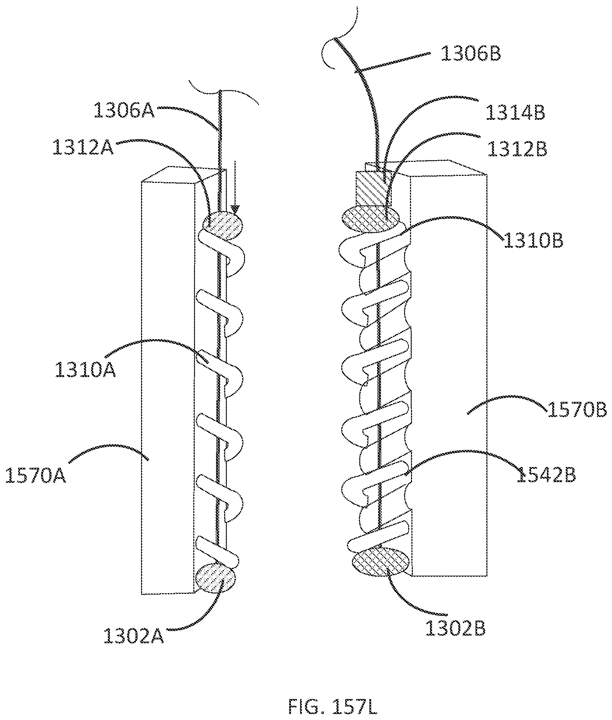

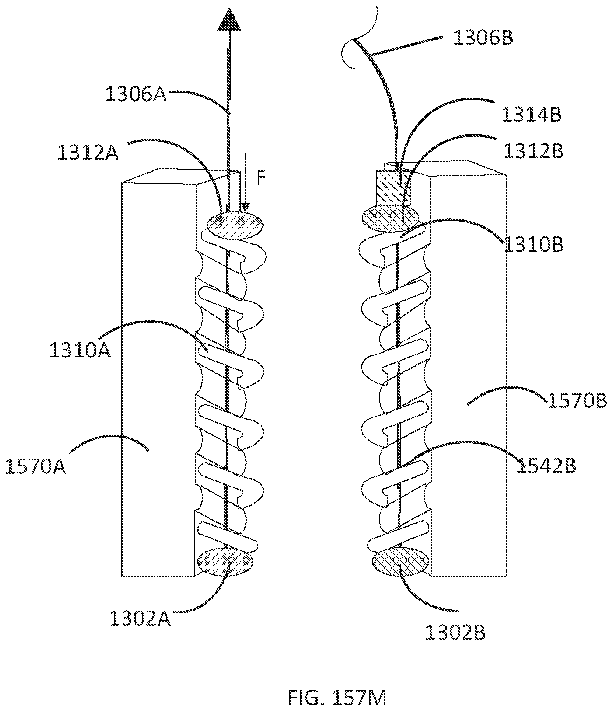

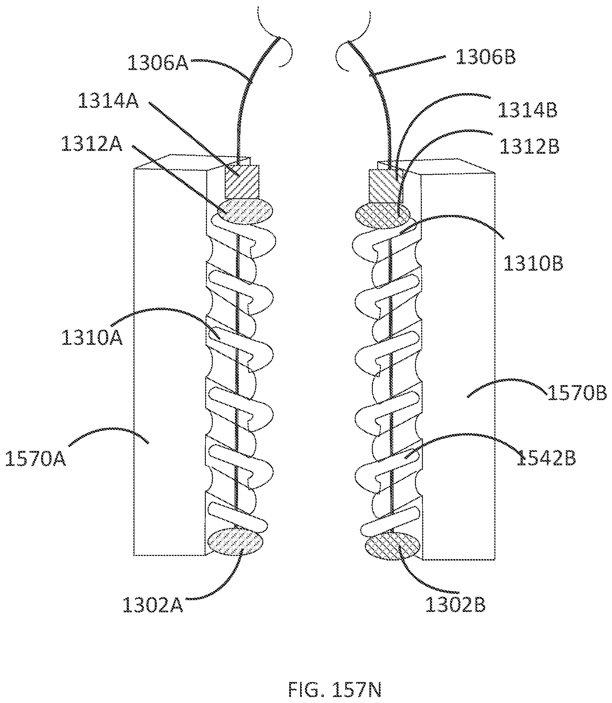

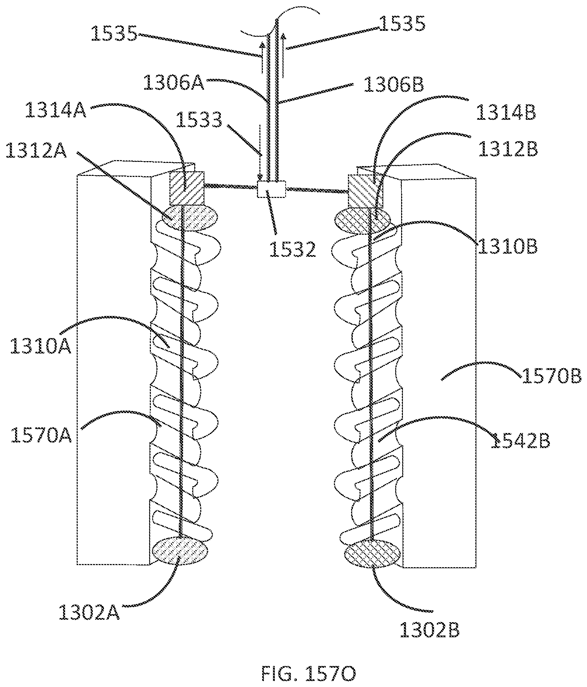

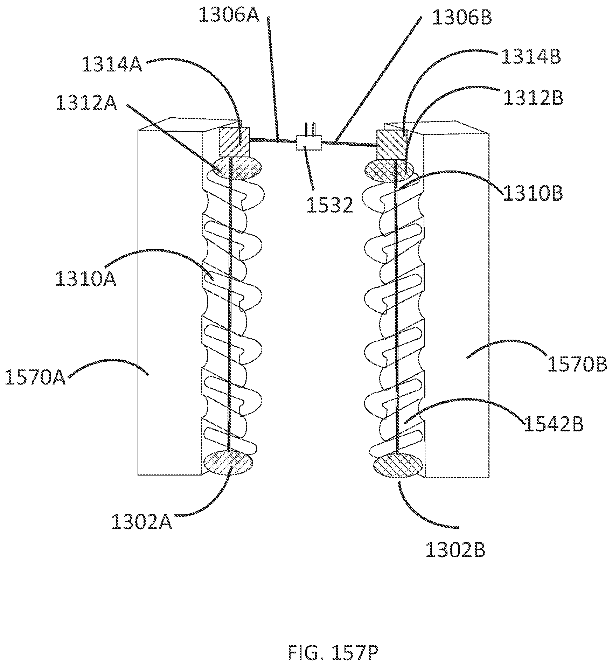

In Figure 157H a second steerable guide rail 1304A is advanced into position adjacent to the target tissue location 1570A. In Figure 157I, a second helical anchor 1310A is deployed along the steerable guide rail 1304A into the target tissue area 1570A. In Figure 157J, the helical anchor 1310A is shown fully deployed at targeted tissue area 1570A with a stop 1302A at the end of the guide rail 1304A. In Figure 157K, the steerable guide rail 1304A has been removed, exposing the tether 1306A inside the helical anchor 1310A. In Figure 157L, an anchor stop 1312A is applied along the tether 1306A to the proximal end of helical anchor 1310A. In Figure 157M, a force F is applied to the anchor stop 1312A while the tether 1306A is pulled to contract the helical anchor 1310A and modify the target tissue area 1570A. Referring to Figure 157N, once a desired compression of the helical anchor 1310A is achieved, a locking mechanism 1314A is affixed to the tether 1306A. The locking mechanism 1314A holds the stop 1312B in place, which maintains the desired compressed position of anchor 1310 in tissue 1308. In Figure 157N, helical anchors 1310A and 1310B are shown fully deployed and locked into a contracted position for reshaping/remodeling tissue at their respective target tissue areas. In Figure 157O, a tether lock 1532 is attached to tethers 1306A and 1306B and is advanced as indicated by arrow 1537 while the tethers 1306A, 1306B are pulled as indicated by arrows 1535. As the tether lock 1532 is adjusted, the tethers 1306A, 1306B pull the helical anchors 1310A and 1310B and the attached tissue areas 1570A and 1570B toward one another. In Figure 157P, the tether lock 1532 is locked into a fixed position, which holds target tissue areas 1570A and 1570B in a remodeled position and the ends of the tether 1306 are cut off.

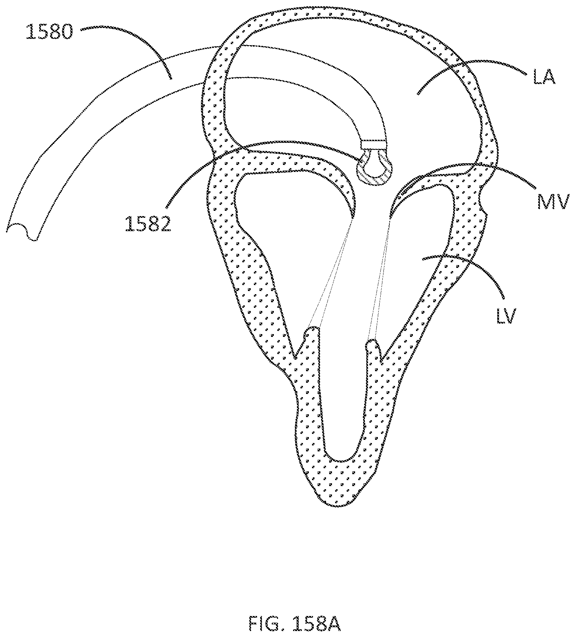

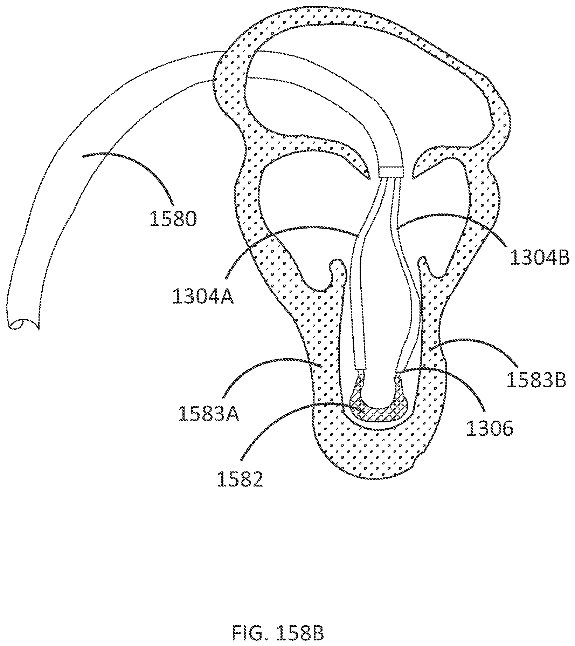

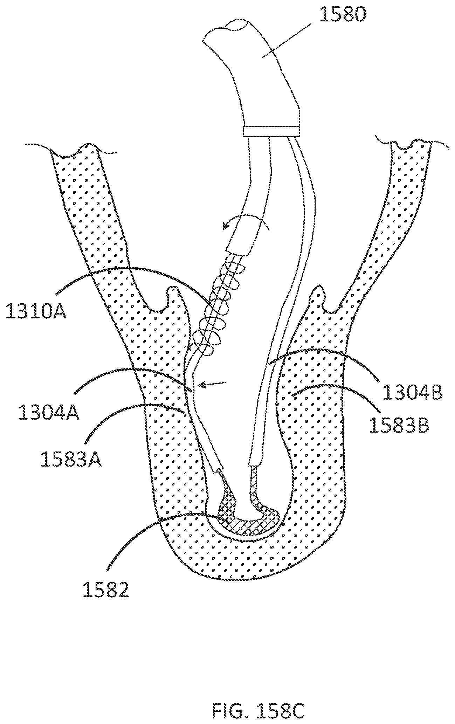

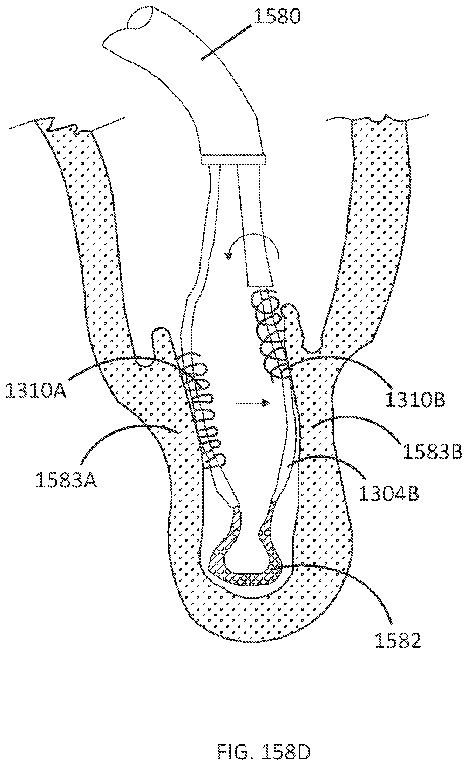

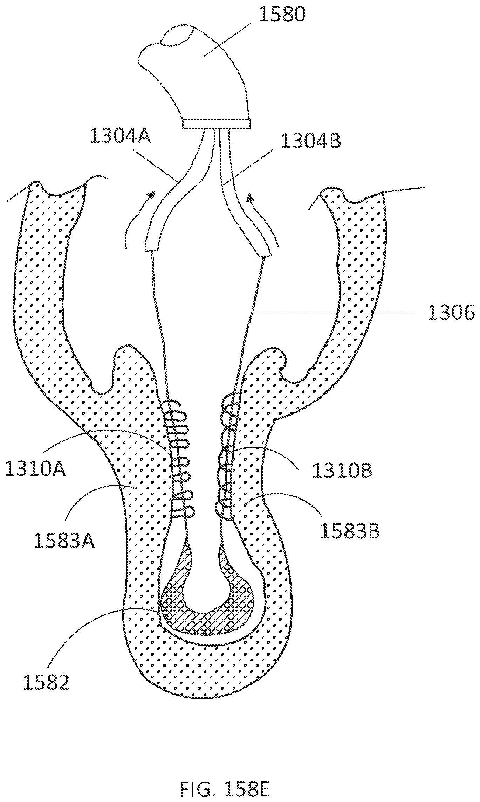

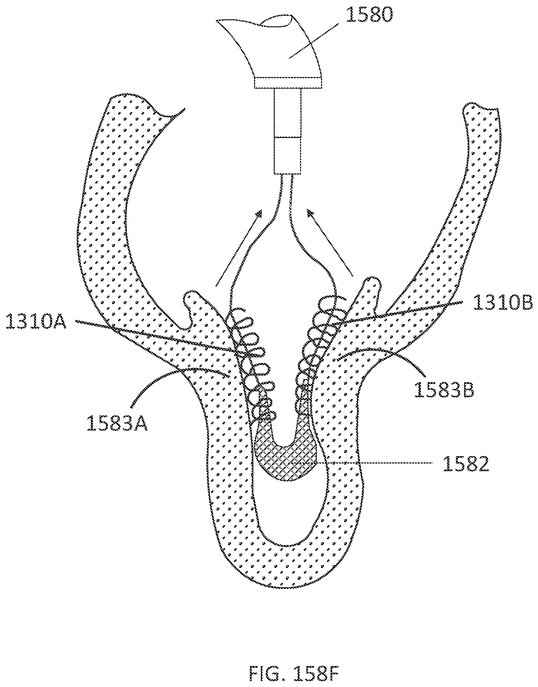

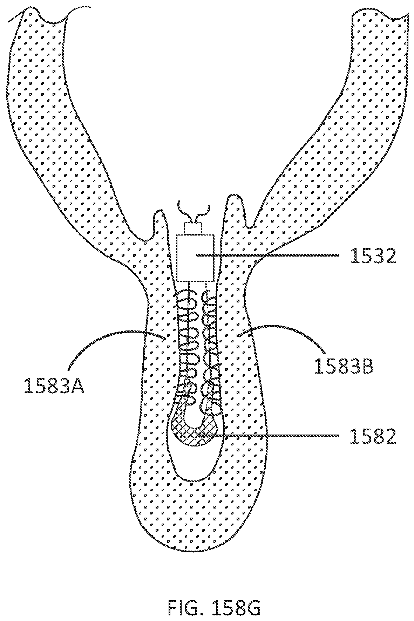

Figures 158A-158G show an exemplary simultaneous deployment of a plurality of guide rails 1304A, 1304B (See Figure 158B) for helical anchors to a plurality of target tissue areas. In Figure 158A, a deployment catheter 1580 guides a horseshoe shaped stabilizer 1582 through the mitral valve MV and into the left ventricle LV. In Figure 158B the horseshoe shaped stabilizer 1582 reaches the apex of the left ventricle LV. In some embodiments, a tether 1306 is disposed within or connected to the horseshoe shaped stabilizer 1582 (See Figure 158E). Referring to Figure 158C, the steerable guide rails 1304A and 1304B are deployed from the deployment catheter 1580 and are positioned against target tissue areas 1583A and 1583B. In Figure 158C the steerable guide rail 1304A is positioned against the target tissue location 1583A and the helical anchor 1310A is deployed along the steerable guide rail 1304A. In Figure 158D the steerable guide rail 1304B is positioned against target tissue location 1583B and the helical anchor 1310B is deployed along the steerable guide rail 1304B. In Figure 158E the steerable guide rails 1304A and 1304B are removed, exposing the tether 1306 that extends through the anchors 1310A, 1310B. In Figure 158F, the tether 1306 is retracted causing the horseshoe shaped stabilizer 1582 to contact and/or extend into helical anchors 1310A and 1310B. The retraction of the tether 1306 also pulls the anchors 1310A and 1310B toward one another to reshape the tissue at the target sites. In some embodiments, the horseshoe stabilizer 1582 contacts the helical anchors 1310A and 1310B and/or the tissue in the gaps between the surface of the target tissue areas and the inner diameters of the helical anchors. Referring to Figure 158G a tether lock 1532 is attached to the ends of the tether 1306 and is advanced while the ends of the tether 1306 are pulled. As the tether lock 1532 is adjusted, the tether pulls the helical anchors 1310A and 1310B and the attached tissue areas 1583A and 1583B toward one another. The tether lock 1532 is locked into a fixed position, which holds target tissue areas 1583AA and 1583B in a remodeled position and the ends of the tether 1306 are cut off.

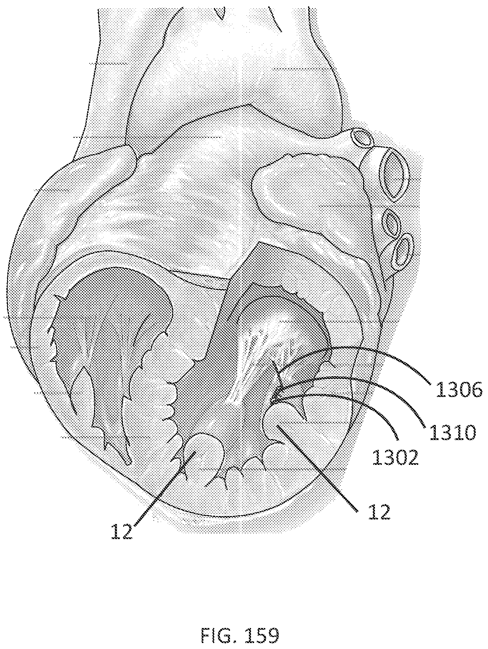

In Figure 159 an anchor 1310 is implanted in one of the papillary muscles 12 in the left ventricle. A first line 1306 extends from the first anchor 1310 and through the mitral valve MV. The anchor 1310 illustrated by Figure 159 can be installed in any manner described herein.

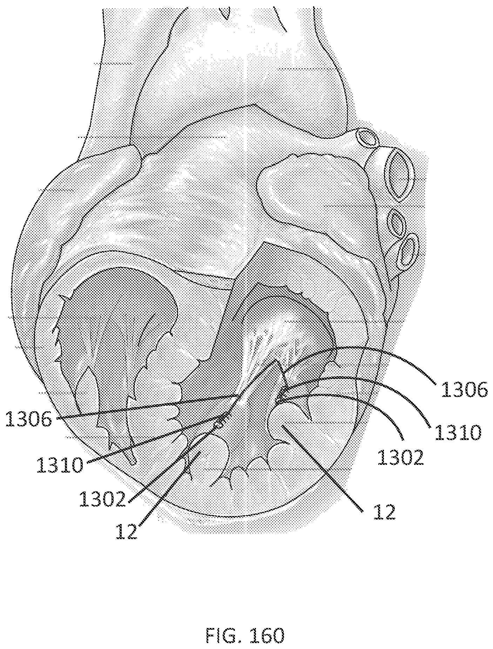

In Figure 160, along with the installed first anchor 1310, a second anchor is illustrated in an implanted position. A second line extends from the second anchor 1310 and through the mitral valve MV. The second device 120 can be installed in any of the manners described herein. In one exemplary embodiment, the second device 220 is installed in the same manner as the first device 120.

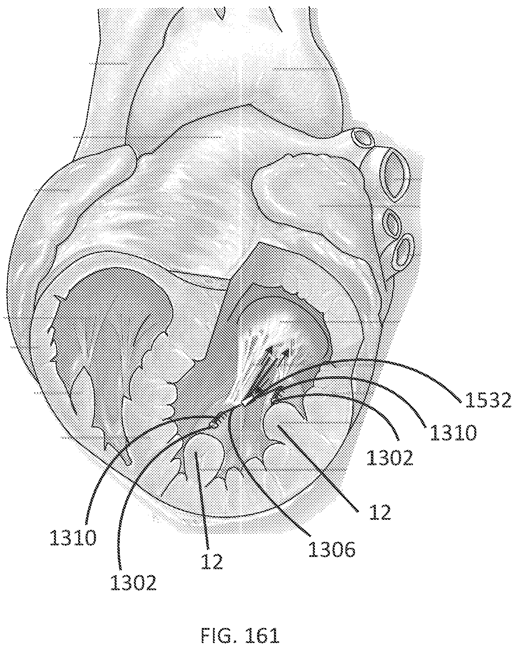

In Figure 161, in one exemplary embodiment the first and second lines 1306 are routed through a connector 1532. The papillary muscles 12 are pulled together or approximated by pushing or holding the position of the connector 1532 and pulling on the lines 1306 as indicated by the arrows. The distance the connector 1532 is pushed and the distances the lines 1306 that extend out of the connector 1532 controls how far the papillary muscles 12 are pulled toward one another, which in turn determines the remodeling of the heart walls.

Still referring to Figure 161, in one exemplary embodiment, the papillary muscles 12 can be pulled toward one another in a manner that improves coaption between the leaflets of the mitral valve. That is, the chordae tendinea are attached to the mitral valve MV leaflets and the papillary muscles. Approximating the papillary muscles toward one another causes the chordae tendinea CT to pull the mitral valve leaflets toward one another and enhance coaption of the mitral valve leaflets. The enhanced or corrected leaflet coaption can reduce or eliminate mitral valve regurgitation.

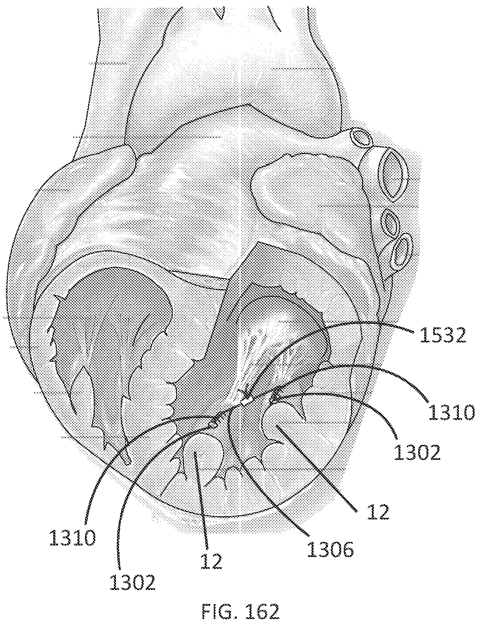

Referring to Figure 162, after the papillary muscles 12 and the heart walls W are pulled to the desired remodeled position by the lines 1306, the connector 1314 secures the positions of the lines in the connector. Once secured, the lines can be trimmed as shown. As such, both anchors 1310 are shown in installed positions. The lines 1306 remain in tension to pull inward to pull the heart wall W inward to remodel the shape of the heart wall W.

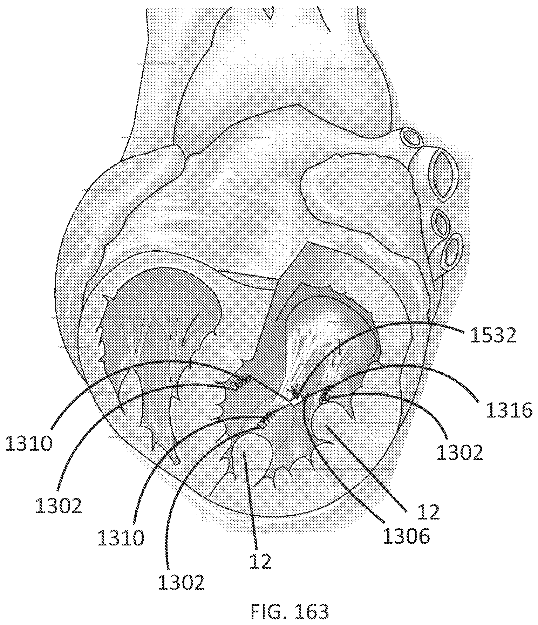

Any number of anchors 1310 and any number of line locking devices 1532 can be used to tailor the heart wall remodeling to each individual patient. In one exemplary embodiment, two or more lines 124 are locked together in each of the locking devices. Figure 163 illustrates one example where three lines are connected together by one locking device. In some embodiments, more than one locking device is used, with at least two lines being connected together by each locking device. In the example illustrated by Figure 163, three anchors 1310 are illustrated in installed positions with a third anchor engaging a heart wall. In the illustrated embodiment of Figure 163, the heart wall associated with the third anchor 1310 is the interventricular septum IS. The lines 1306 can be pulled inward to pull the heart wall W inward to remodel the shape of the heart wall W. To hold the heart wall W in the remodeled position, the lines 1306, while in tension, can be connected within the left ventricle LV, such as for example, by the line locking device 1532 or other suitable means for connecting the lines 1306.

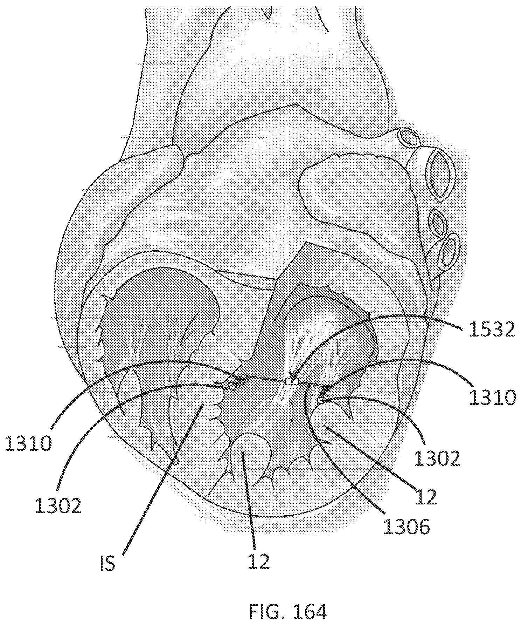

In Figure 164, one anchor 1310 is implanted in a papillary muscle 12 and a second anchor 1310 is installed on the interventricular septum IS. The lines 1306 can be pulled inward to pull the papillary muscle 12 inward and to pull the intraventricular septum IS inward to remodel the shape of the ventricle. To hold the papillary muscle 12 and the intraventricular septum IS in the remodeled positions, the lines 1306, while in tension, can be connected within the left ventricle LV, such as for example, by the line locking device 1532 or other suitable means for connecting the lines 1306.

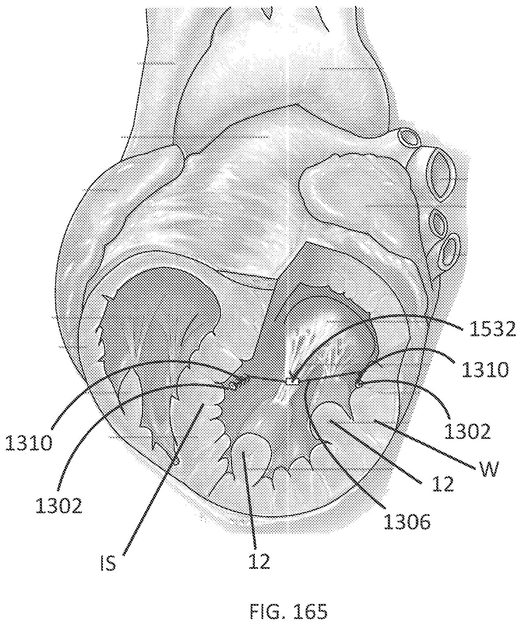

In Figure 165, one anchor 1310 is attached to the heart wall W and a second anchor 1310 is implanted on the interventricular septum IS. The lines 1306, can be pulled inward to pull the heart wall W inward to remodel the shape of the heart wall W and the intraventricular septum IS. To hold the heart wall W and the intraventricular septum IS in the remodeled position, the lines 1306, while in tension, can be connected within the left ventricle LV, such as for example, by the line locking device 1532 or other suitable means for connecting the lines.

The devices can be used in a wide variety of different ways to remodel the heart and/or approximate the papillary muscles. A single locking device 1532 and two or more anchors 1310 can be deployed or more than one line locking device 1532 with two or more anchors 1310 per locking device 1532 can be deployed to remodel the heart of a single patient. For example, any of the configurations disclosed herein can be used in combination on the heart of a single patient.

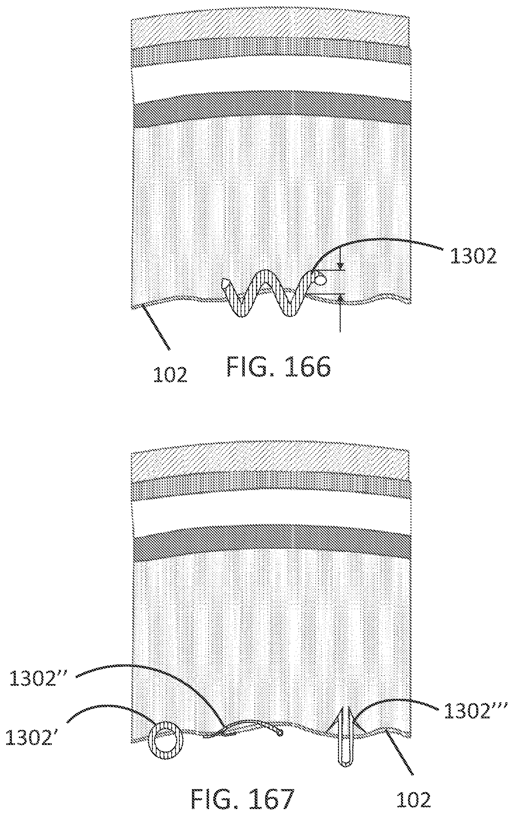

Figure 166 shows an exemplary helical anchor 1310 deployed in endocardial tissue 102. While many embodiments described herein comprise deployment of one or more helical anchors, it is appreciated that, in some embodiments, different types of anchors may be used in substantially similar ways. For example, Figure 167 shows a variety of alternative anchors 1310', 1310", 1310‴ deployed into a target tissue area, for example, endocardial tissue 102. The alternative anchors 1310', 1310", 1310‴ can be configured such that the tissue engagement depth (i.e. the depth of penetration into the tissue) targets endocardial tissue while avoiding or substantially avoiding myocardial tissue. In some exemplary embodiments, stitch depth penetration depth of the alternative anchors is between 0.02 mm and 2 mm, such as between 0.5 mm and 1.5 mm, such as between 0.75 mm and 1.25 mm, such as 1 mm or less. As shown, anchor 1310' is a ring anchor. Anchor 1310" is a hook anchor secured outside of the endocardium tissue 102 while anchor 1310‴ is a hook anchor secured inside the endocardium tissue 102. It is appreciated that anchors 1310', 1310", 1310‴ are merely exemplary, and additional types of anchors may be used in exemplary embodiments as described herein.

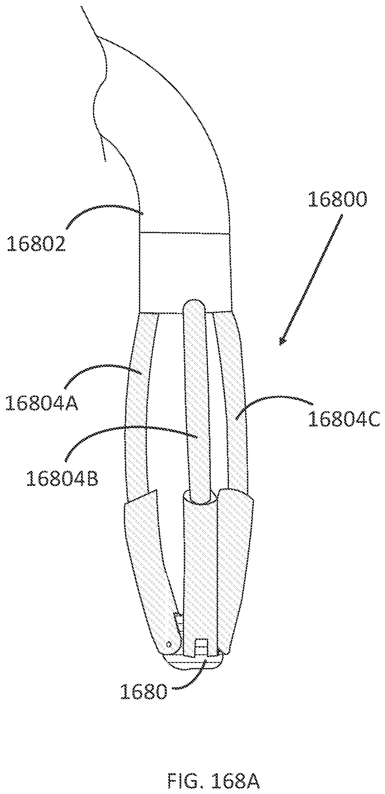

Figures 168A-G show an exemplary embodiment of a device 16800 that includes a plurality of guides/stabilizers 16804. A plurality of helical anchors 1310 can be deployed over the guides/stabilizers 16804. In Figure 168A, the steerable guides 16804A-16804C are connected at an end connector 1680. The end connector 1680 can take a wide variety of different forms. For example, the end connector can comprise a plurality of connectors as illustrated or the guides/stabilizers 16804 can be fixedly connected to the end connector 1680. When the guides/stabilizers 16804 are fixedly connected to the end connector 1680, the guides/stabilizers 16804 can be made from a spring-type material, such that the guides/stabilizers expand outward when released from a delivery catheter 16802. The connector 1680 allows the guides/stabilizers 16804A-16804C to be deployed together to a target tissue location. Further, each guide/stabilizer can be manipulated about the connector 1680 in order to target multiple target tissue locations.

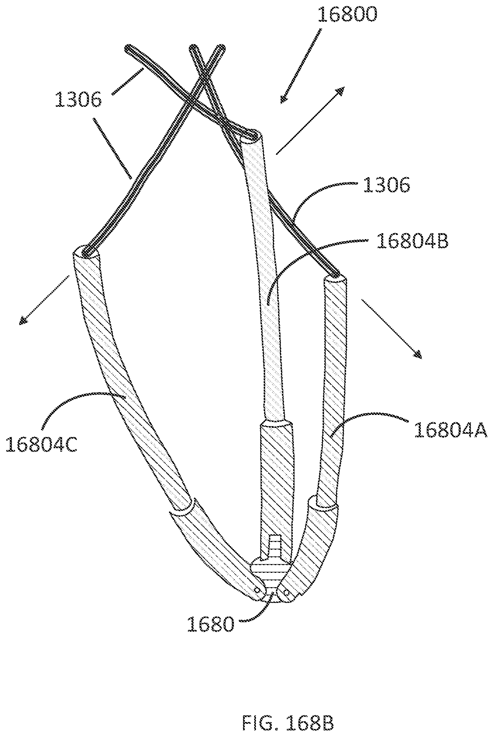

Figure 168B shows the connected steerable guides guides/stabilizers 16804 released from the delivery catheter 16802 to allow the guides/stabilizers 16804 to expand in different directions. As shown, a tether 1306 is attached to each of the guides/stabilizers 16804, such as at an end of each of the guides/stabilizers 16804.

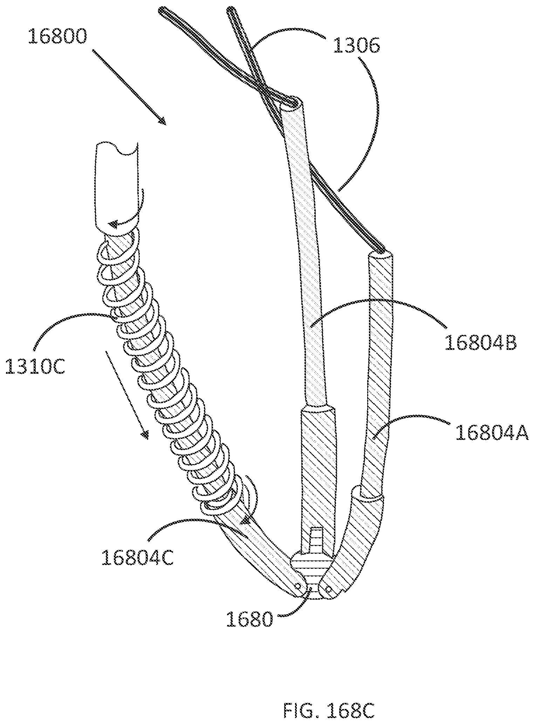

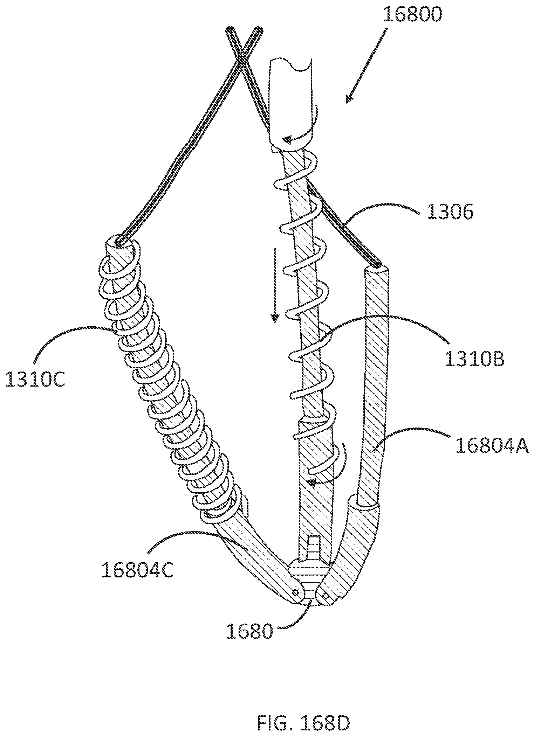

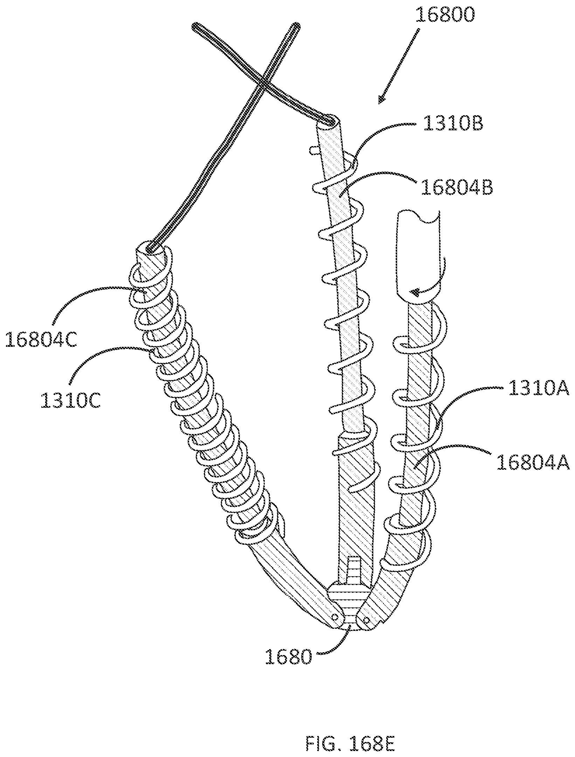

Figure 168C shows an exemplary helical anchor 1310C being deployed along a guide/stabilizer 16804C. Figure 168D shows an exemplary helical anchor 1310B being deployed along a guide/stabilizer 16804B. Figure 168E shows an exemplary helical anchor 1310A being deployed along a guides/stabilizers 16804A. It will be appreciated that while each helical anchor is being deployed along its respective guide/stabilizer, the deployed anchors can also be used to modify tissue locally in the target tissue area which they are implanted into (See e.g., Figure 157N).

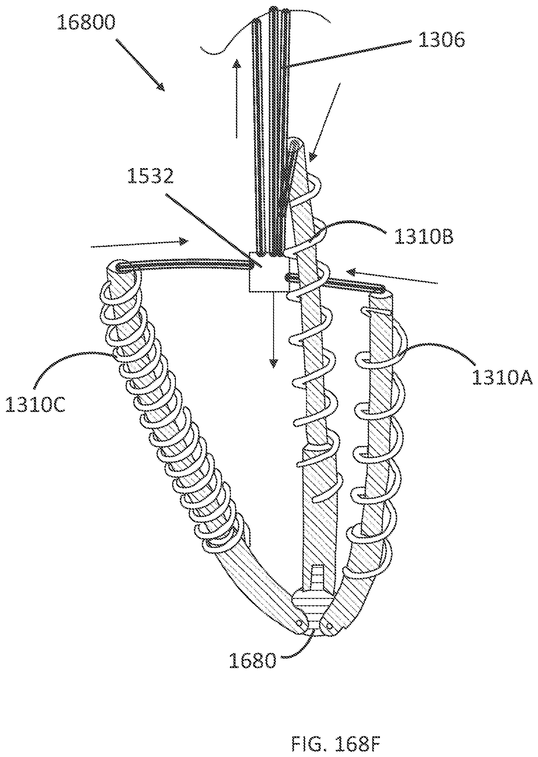

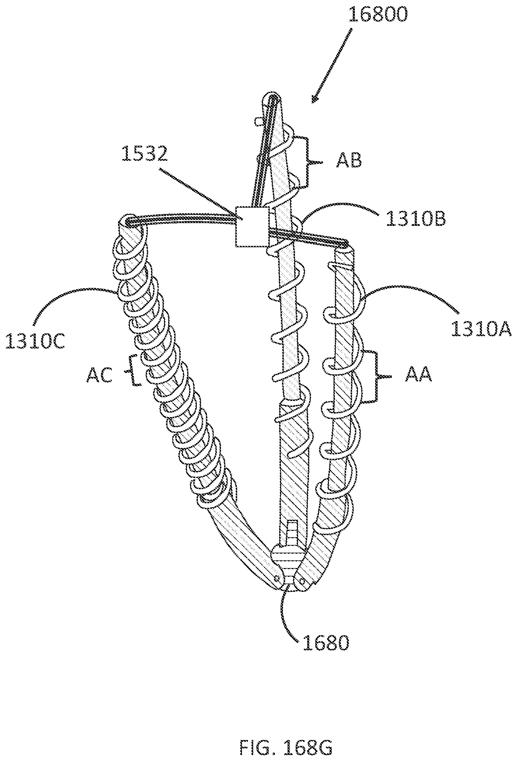

In Figure 168F, the tethers 1306 are pulled while a locking mechanism 1532 is advanced to pull the guides/stabilizers 16804A-16804C and overlying coiled anchors 1310A-1310C toward one another. In Figure 168G, the position of the tethers 1306 in the locking mechanism 1532 the excess tether that extended proximally has been cut off.

It is appreciated that the helical anchors 1310A-1310C can have the same or different bite angles, coil pitches, etc. In the example illustrated by Figure 168G, the helical anchor 1310A has an anchor pitch AA, the helical anchor 1310B has an anchor pitch AB, and the helical anchor 1310C has an anchor pitch AC. In some exemplary embodiments, the anchor pitches AA, AB, AC are the same. In other exemplary embodiments, the anchor pitches AA, AB, AC are different.

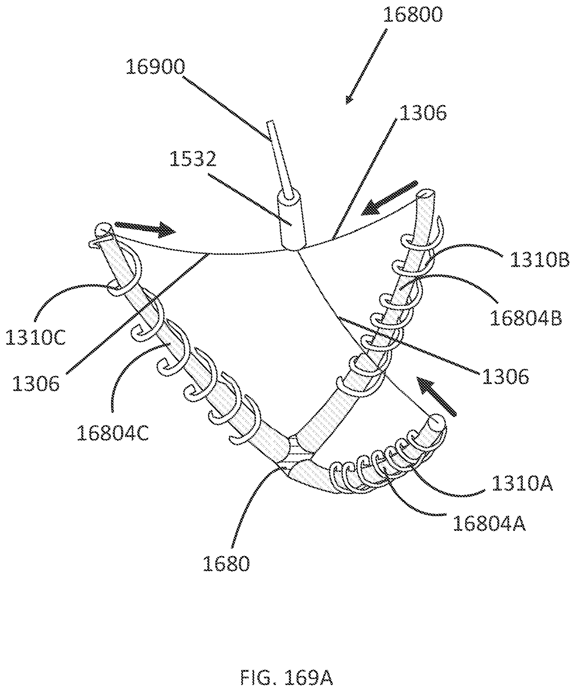

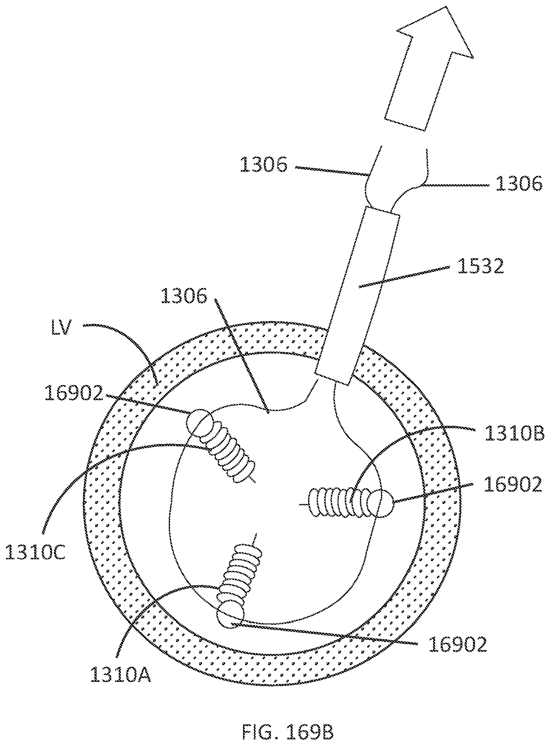

Referring to Figures 168F, 169A, and 169B, the lines 1306 can be pulled independently (See Figure 168F) or pulling a single line 1306 (See Figures 169A and 169B) and can pull the guides/stabilizers 16804A-16804C together. Independent pulling of the lines 1306 allows a different force or tension to be applied to each of the guides/stabilizers 16804A-16804C. Pulling a single line can provide a uniform force or tension to be applied to the guides/stabilizers 16804A-16804C.

In Figure 169A, the lines 1306 that are connected to the guides/stabilizers 16804A-16804C are connected to a single line 16900 that extends through a connector 1532. Pulling on the single line 16900 while pushing on the connector 1532 pulls on all three lines 1306. As a result, all three guides/stabilizers 16804A-16804C are pulled in as indicated by the arrows. In one exemplary embodiment, the force applied by the single line 16900 is uniformly applied to the guides/stabilizers 16804A-16804C. The locking mechanism 1532 can be tightened to the tethers 1306 to hold the position of the helical anchors 1310A-1310C.

Figure 169B is a schematic illustration, looking toward an apex of a ventricle, where three anchors 1310A-1310C are implanted in the ventricle. In this example, a single line 1306 is coupled to all three of the anchors 1310A-1310C. The line 1306 can be coupled to a plurality of anchors in a wide variety of different ways. For example, the line 1306 can be threaded through loops 16902 that are attached to the anchors 1310A-1310C or guides/stabilizers that are disposed in the anchors. The single line 1306 t extends through a connector 1532. Pulling on the single line 1306 on the proximal side of the connector 1532 (i.e. away from the anchors 1310A-1310C) while pushing on the connector 1532 pulls on all three loops 16902. As a result, all three anchors 1310A-1310C are pulled toward one another. In one exemplary embodiment, the force applied by the single line 1306 is uniformly applied to the anchors 1310A-1310C. The locking mechanism 1532 can be tightened to the tether 1306 to hold the position of the helical anchors 1310A-1310C.

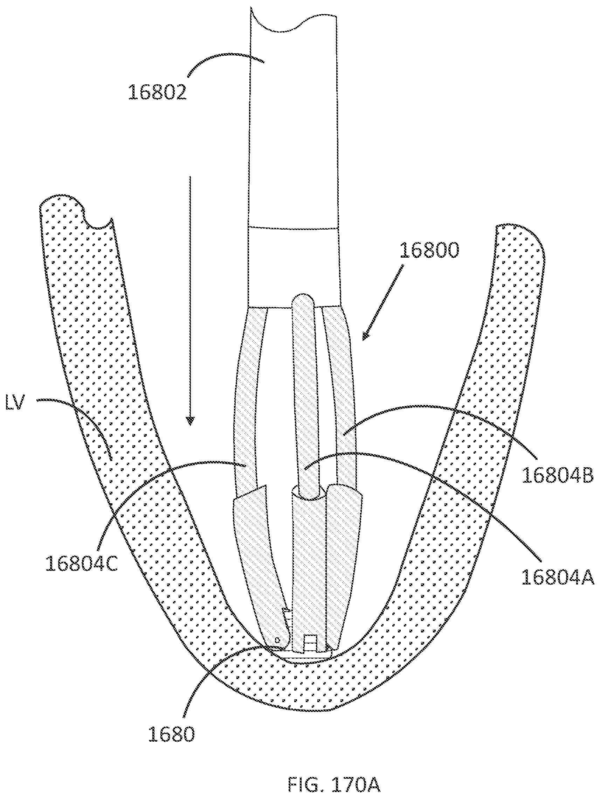

Figures 170A-G show an exemplary deployment of the device 16800 described above with reference to Figure 168A-G into a ventricle, such as the left ventricle LV. In Figure 170A, the delivery catheter 16802 positions the device 16800 in the ventricle. For example, the delivery catheter 16802 can position the device 16800 at the apex of the left ventricle LV as illustrated.

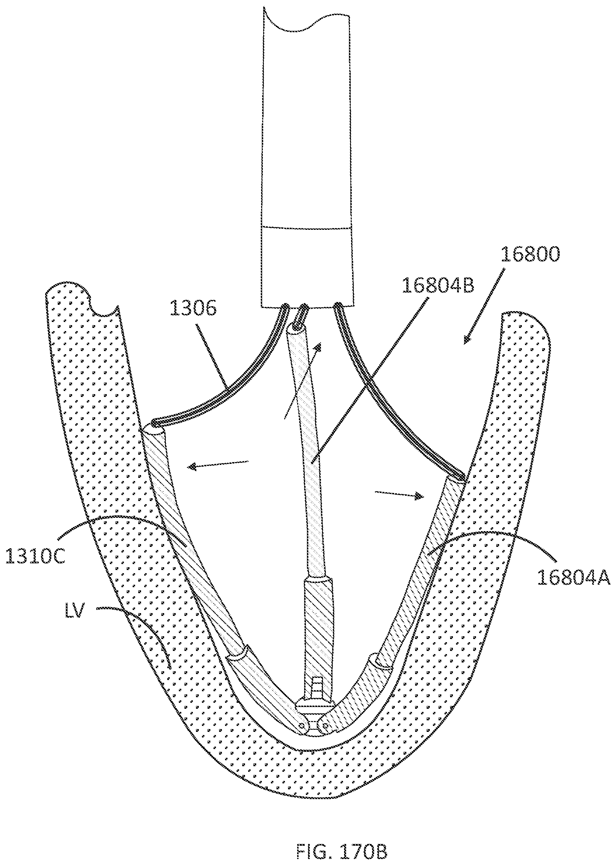

Figure 170B shows the connected steerable guides guides/stabilizers 16804 released from the delivery catheter 16802 to allow the guides/stabilizers 16804 to expand in different directions. In the illustrated example, the guides/stabilizers expand into contact with the walls of the left ventricle. As shown, a tether 1306 is attached to each of the guides/stabilizers 16804, such as at an end of each of the guides/stabilizers 16804.

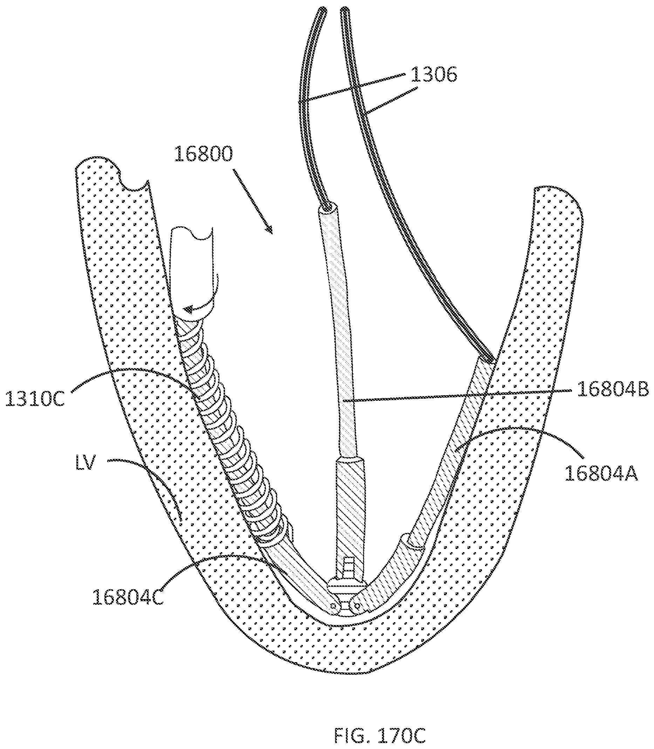

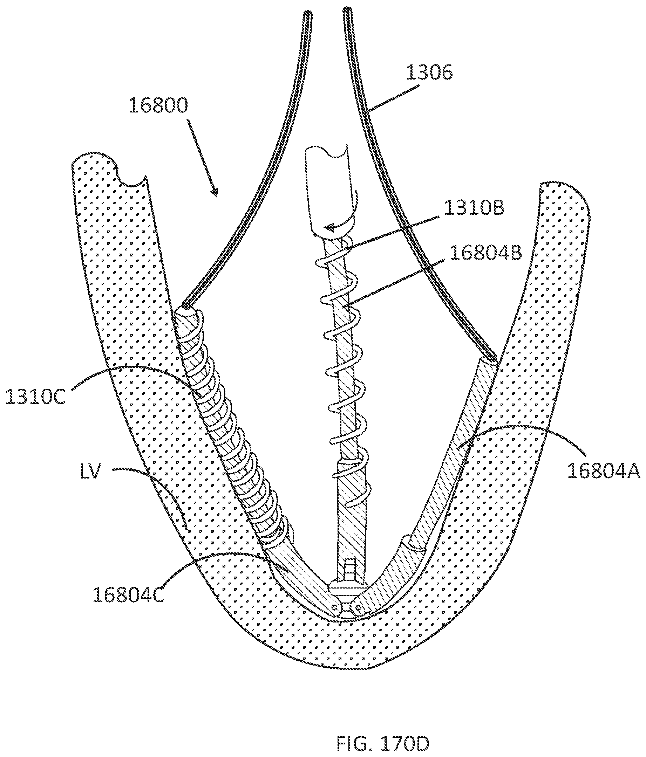

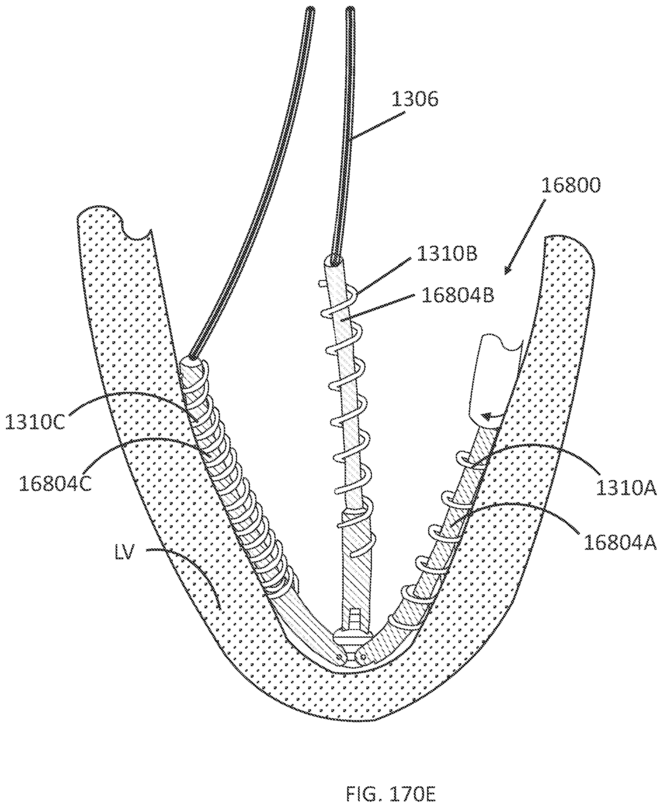

Figure 170C shows an exemplary helical anchor 1310C being deployed along a guide/stabilizer 16804C into a wall of a ventricle. For example, the helical anchor 1310C can be implanted in the intraventricular septum or another wall of the left ventricle LV. Figure 170D shows an exemplary helical anchor 1310B being deployed along a guide/stabilizer 16804B into a wall of a ventricle. For example, the helical anchor 1310B can be implanted in a wall of the left ventricle LV. Figure 170E shows an exemplary helical anchor 1310A being deployed along a guides/stabilizers 16804A into a wall of a ventricle. For example, the helical anchor 1310A can be implanted in a wall of the left ventricle LV. It will be appreciated that while each helical anchor is being deployed along its respective guide/stabilizer, the deployed anchors can also be used to modify tissue locally in the target tissue area which they are implanted into (See e.g. Figure 157N).

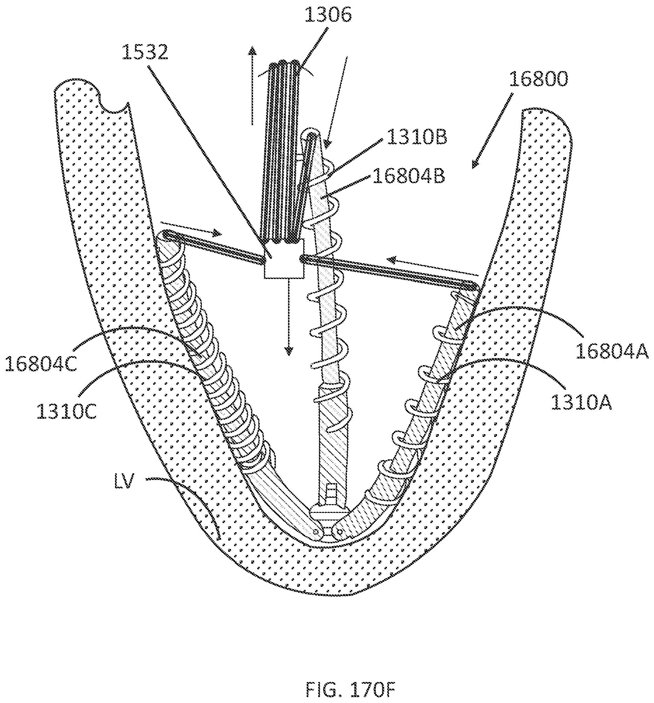

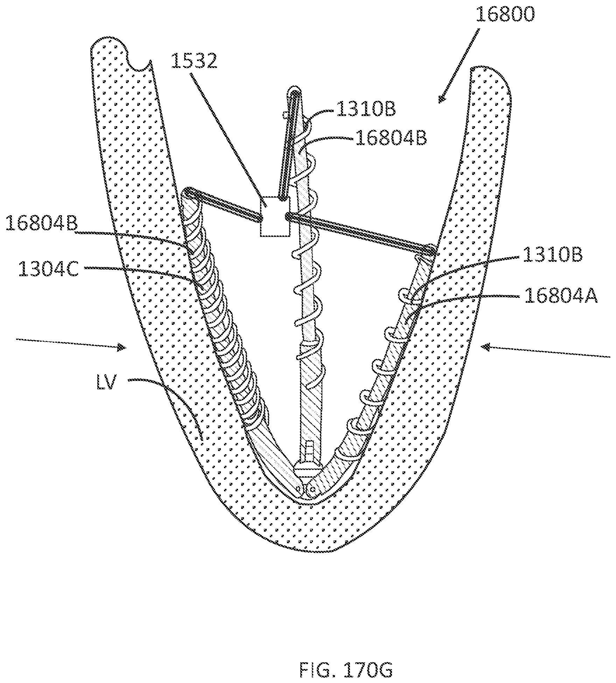

In Figure 170F, the tethers 1306 are pulled while a locking mechanism 1532 is advanced to pull the guides/stabilizers 16804A-16804C and overlying coiled anchors 1310A-1310C toward one another. This pulls the walls of the ventricle inward to remodel the shape of the ventricle. In Figure 170G, the position of the tethers 1306 in the locking mechanism 1532 is fixed and the excess tether that extended proximally has been cut off. This secures the remodeled shape of the ventricle.

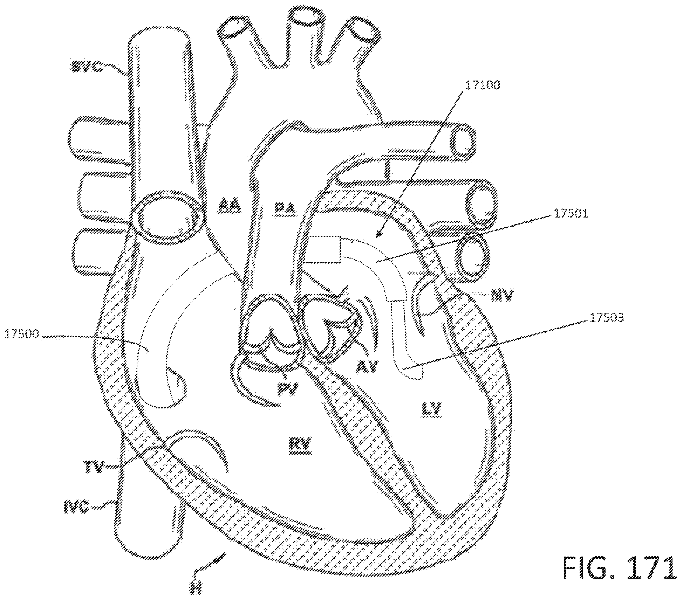

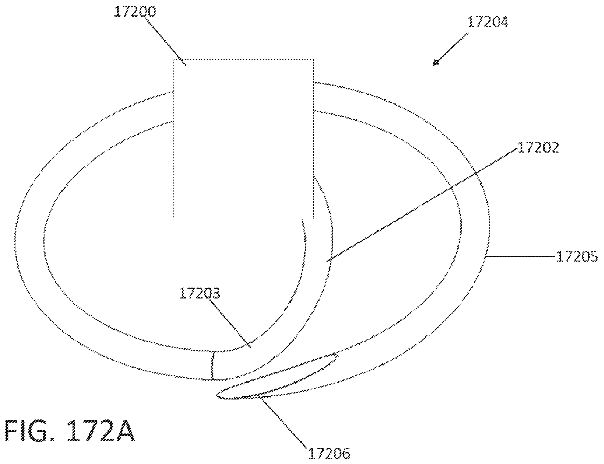

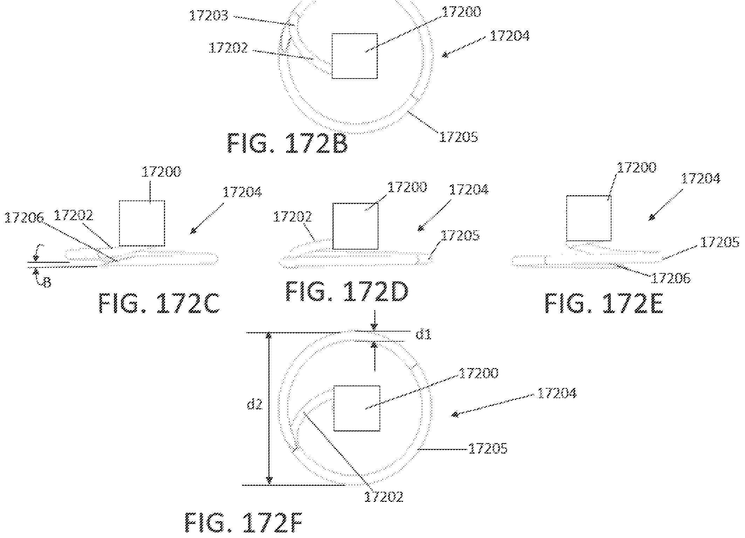

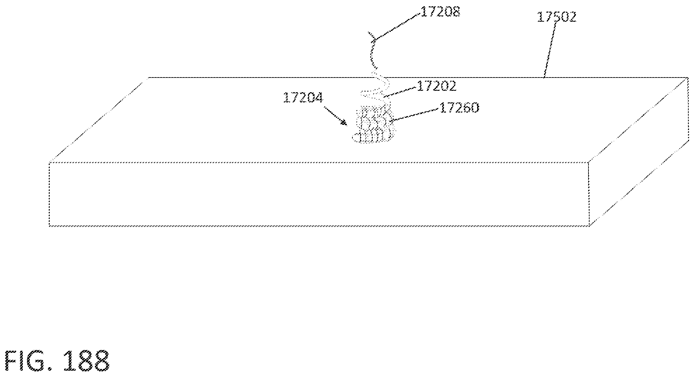

Figures 171-188 show various embodiments relating to a coiled anchor device. Figure 171 shows a cutaway view of the heart with an exemplary deployment system 17100 for implanting a coiled anchor 17204 (See Figure 172A) into the interior wall of the left ventricle LV. The coiled anchor is disposed inside the deployment system 17100 in Figure 171 and is straightened or partially straightened to take the shape of the deployment system.

The deployment system 17100 can take a wide variety of different forms. In the illustrated example, the deployment system includes a guide sheath or catheter 17500, a first steerable catheter 17501, and a second steerable catheter 17503. The coiled anchor is positioned inside and delivered from the second steerable catheter 17503 in the illustrated embodiment. However, the deployment system can include any number of catheters. For example, in one exemplary embodiment the deployment system can have an optional guide sheath, one steerable catheter disposed inside the optional guide sheath, and a steerable implant catheter with an end that the coiled anchor is attached to. As will be described in more detail below, the coiled anchor 17204 is positioned into its deployment position via the second steerable catheter 17503, for example, the heart wall of the left ventricle LV or a papillary muscle of the left ventricle LV.

Figure 172A-172F show an exemplary embodiment of coiled anchor 17204. The coiled anchor 17204 can take a wide variety of different forms. In the illustrated example, the coiled anchor 17204 includes a large, substantially annular or annular, and substantially planar or planar outer ring portion 17205 and a radially inwardly extending portion 17202. The outer ring portion 17205 and the radially inwardly extending portion 17202 can be made from a variety of different materials. For example, the ring portion 17205 and the radially inwardly extending portion 17202 can be formed from metal, such as a titanium, steel, and/or a shape memory alloy, such as nitinol. However, it will be appreciated that in alternative embodiments, the ring portion 17205 and a radially inwardly extending portion 17202 of coiled anchor 17204 may be formed using any number of materials capable of implantation into human tissue.

In the illustrated embodiment, the ring portion 17205 is relatively large compared to the diameter of the wire used to make the ring portion. For example, a diameter of the ring portion 17205 can be many times larger than the diameter of the wire used to make the ring portion 17205. For example, the diameter of the ring portion 17205 can be between 5 and 35 times the diameter of a wire used to make the ring portion, such as 10-30 times the diameter of a wire used to make the ring portion, such as 15-20 times the diameter of a wire used to make the ring portion, such as about 17 times the diameter of a wire used to make the ring portion. In one exemplary embodiment, the diameter d1 of the wire used to make the ring portion is between 0.015 and 0.062 inches, such as between 0.025 and 0.050 inches, such as between 0.030 and 0.040 inches, such as about 0.035 inches. In one exemplary embodiment, a diameter d2 of the ring portion is between 0.250 and 1.0 inches, such as between 0.375 and 0.75 inches, such as between 0.45 and .7 inches, such as about 0.6 inches.

The ring portion 17205 can include any number of turns. For example, the ring portion 17205 can have between ½ and 3 turns, such as between ¾ and 2 turns, such as between 7/8 and 1-1/4 turns, such as between 15/16 and 1-1/8 turns. In the illustrated example, the ring portion 17205 includes about 1 turn.

As shown in Figure 172A, coiled anchor 17204 can be associated with a coupler 17200. The coupler 17200 can take a wide variety of different forms. The coupler can be any structure that facilitates attachment to the coiled anchor 17204, facilitates advancement of the coiled anchor out of the catheter 17501 and/or the catheter 17503, facilitates rotation of the coiled anchor, and facilitates release of the coiled anchor. The coupler 17200 is schematically illustrated in Figures 172A-E. The coupler 17200 can be connected to the coiled anchor 17204 in a wide variety of different ways. In certain embodiments, coupler 17200 is connected to coiled anchor 17204 via frictional force. In other embodiments, coupler 17200 and coiled anchor 17204 may be associated via bonders, glues, binders, adhesives, or the like. Extending from coupler 17200, coiled anchor 17204 may have a radially inwardly extending portion 17202 that includes a transition portion 17203 which has a smaller bend radius than the rest of the coiled anchor 17204. In some embodiments, coiled anchor 17204 is a continuous coil. In certain embodiments, radially inwardly extending portion 17202 is formed from a different material than coiled anchor 17204 and is bonded at a joint. Coiled anchor 17204 terminates at anchor tip 17206. Anchor tip 17206 is sharpened so-as to facilitate implantation of coiled anchor into tissue.

Figure 172C illustrates a bite angle B of the anchor tip. The bite angle B is the angle that the anchor tip 17206 forms with a horizontal plane when the remainder of the coil 17205 is placed on a horizontal plane. In certain embodiments, the "bite" angle of the anchor tip 17206 can be selected to accommodate implantation into different tissue areas. The low profile of coiled anchor 17204 can be used to target tissue remodeling in thin left ventricle walls of the heart. For example, the bite angle B can be selected to engage only endocardial tissue or endocardial tissue and a small depth of tissue past the endocardial tissue. In one exemplary embodiment, the bite angle B is less than 3 degrees, such as less than 2 degrees, such as less than 1 degree, such as less than 0.75 degrees, such as less than 0.050 degrees, such as less than 0.250 degrees, such as less than 0.125 degrees, such as less than 0.05 degrees.

Figure 172C is an overhead view of an exemplary coiled anchor 17204 with coupler 17200 and radially inwardly extending portion 17202. Figure 172C is a side view of coiled anchor 17204. Figure 172D is another side view of coiled anchor 17204. Figure 172E is an opposite side view of coiled anchor 17204. Figure 172F is an underside view of the coiled anchor 17204.

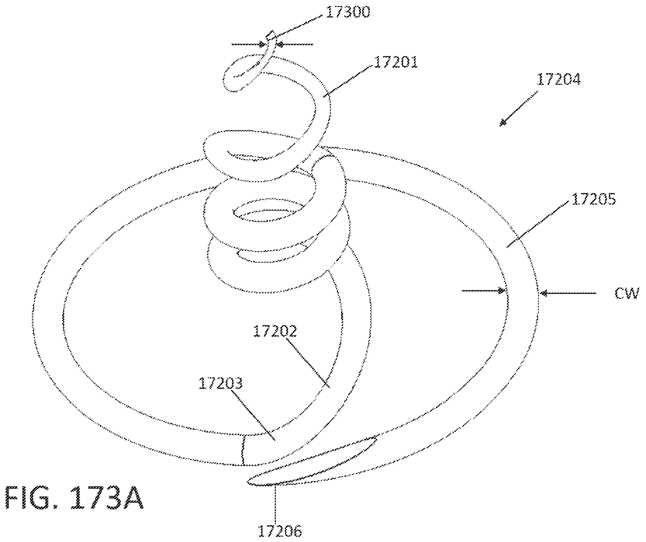

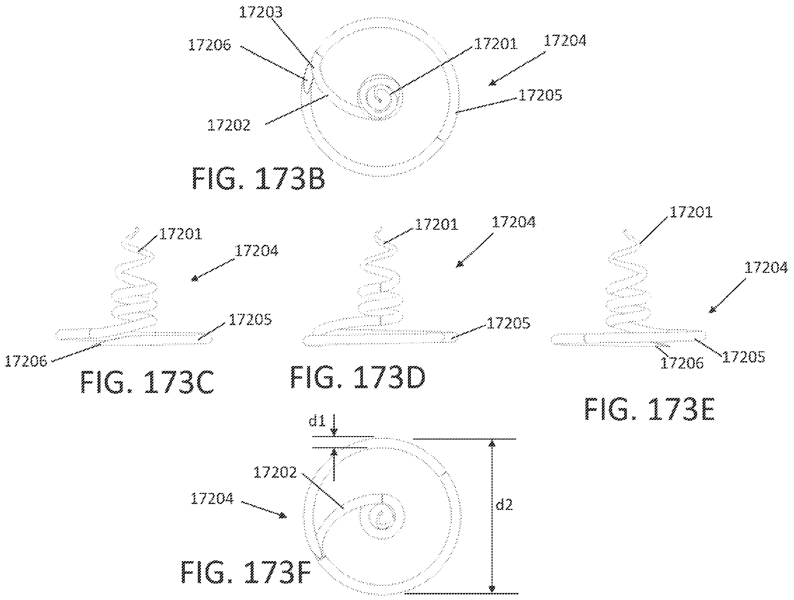

Figures 173A-F show another exemplary embodiment of coiled anchor 17204. In the exemplary embodiment illustrated by Figures 173A-F, the coiled anchor 17204 includes a strain relief. The coiled anchor 17204 can include both a strain relief and a coupler, a strain relief without a coupler, or a coupler without a strain relief. Figure 173A is a view an exemplary coiled anchor 17204 with radially inwardly extending portion 17202 and strain relief 17201. The strain relief 17201 can take a wide variety of different forms. Any spring, shock absorber, or other structure that absorbs, dampens, or otherwise reduces force applied to the strain relief that is transferred to the outer coil 17205 can be used.

In the example illustrated by Figure 173A the strain relief 17201 is a coiled continuation of the transition portion 17202. The additional coil of strain relief 17201 can limit load on tissue by elongation when implanted in tissue. By integrating strain relief 17201 into coiled anchor 17204 the maximum load that coiled anchor 17204 can impart onto the tissue can be reduced.

A coiled strain relief 17201 can take a wide variety of different forms. A coiled strain relief 17201 can be formed with a wire having a constant diameter, can be formed of a coil having a constant diameter, can be formed with a wire that tapers as it extends away from the outer portion 17205, and/or can be a coil that tapers radially inwardly as the coil extends away from the outer portion 17205.

In the example illustrated by Figure 173A, the coiled strain relief 17201 is made from a wire that tapers as the coiled strain relief extends away from the outer ring portion 17205 and the coil that forms the coiled strain relief 17201 tapers radially inwardly as the coil extends away from the outer ring portion 17205. The illustrated coiled strain relief 17201 can be configured to reduce strain applied to the ring 17205 by a predetermined amount. For example, the wire that forms the coiled strain relief can taper from the diameter of the ring portion 17205 (see ranges of the diameter of the ring portion wire above) to between 0.001 and 0.015 inches, such as between 0.003 and 0.012 inches, such as between 0.005 and 0.010 inches, such as about 0.008 inches at reference 17300. The coil that forms the coiled strain relief 17201 can taper from between 0.125 to 0.250 inches to the diameter of the wire at reference 17300, such as from between 0.140 to 0.200 inches to the diameter of the wire at reference 17300, such as from between 0.160 to 0.180 inches to the diameter of the wire at reference 17300.

In some embodiments, radially inwardly extending portion 17202 is tapered toward strain relief 17201 such that the diameter of the coil at radially inwardly extending portion 17202 is larger than at strain relief 17201. In certain embodiments, coiled anchor 17204 may have a coil diameter or width CW of between 0.025-0.040 inches or any of the diameters mentioned above. In certain exemplary embodiments, coiled anchor 17204 has a coil width CW of .035.

Figure 173B is an overhead view of an exemplary coiled anchor 17204 with radially inwardly extending portion 17202 and strain relief 17201. Figure 173C is side view of coiled anchor 17204 with radially inwardly extending portion 17202 and strain relief 17201. Figure 173D is another side view of coiled anchor 17204 with radially inwardly extending portion 17202 and strain relief 17201. Figure 173E is yet another side view of coiled anchor 17204 with radially inwardly extending portion 17202 and strain relief 17201. Figure 173C is an underside view of coiled anchor 17204 with radially inwardly extending portion 17202 and strain relief 17201.

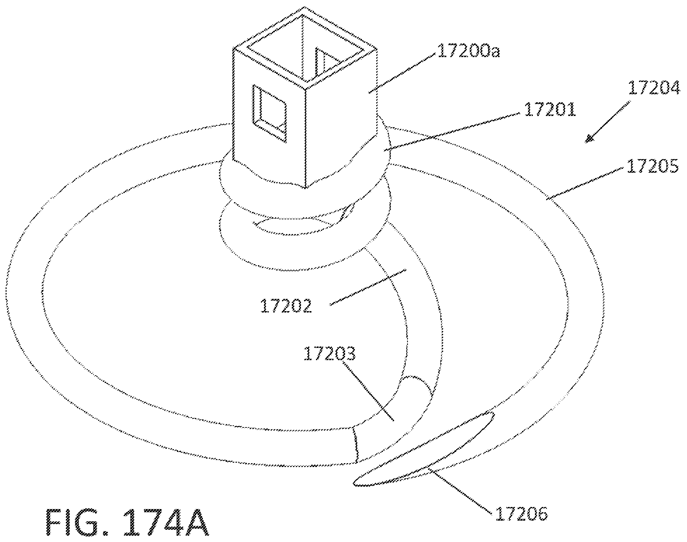

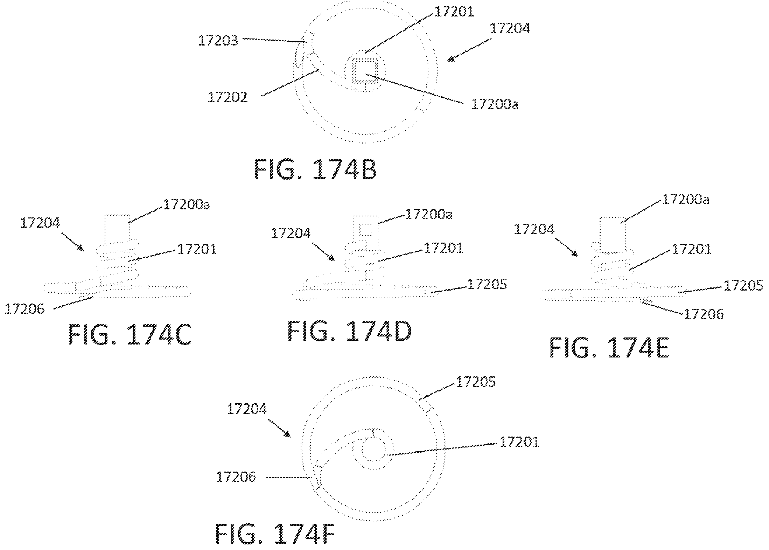

Figures 174A-F illustrate an exemplary embodiment of a coiled anchor 17204 that includes both a strain relief 17201 and a coupler 17200a. Figure 174a shows a coupler 17200a disposed within the strain relief 17201. It will be appreciated that coupler 17200a and strain relief 17201 can be affixed via frictional force or otherwise as described herein. The illustrated coupler 17200a has an opening O which can allow the coupler 17200a to be operably connected and disconnected to a deployment device 17100. In some embodiments, coupler 17200a is hollow such that the deployment device 17100 may be inserted into coupler 17200a to operably connect the deployment device 17100 and coupler 17200a at opening O. During deployment, the connection between deployment device 17100 and coupler 17200a may be used to guide the coiled anchor 17204 into a deployment position, torque the coiled anchor 17204 such that the anchor tip 17206 penetrates tissue, and torque the coiled anchor 17204 until fully deployed into a target tissue area. After deployment, the deployment device 17100 can be detached from coupler 17200a and removed from the body. For example, the coupler can operate in the same or substantially the same manner as the coupling illustrated by Figures 54-74F.

Figure 174B is an overhead view of an exemplary coiled anchor 17204 with strain relief 17201 and coupler 17200a. Figure 174C is side view of coiled anchor 17204 with strain relief 17201 and coupler 17200a. Figure 174D is a side view of coiled anchor 17204 with strain relief 17201 and coupler 17200a showing opening O. Figure 174E is a side view of coiled anchor 17204 with strain relief 17201 and coupler 17200a. Figure 173C is an underside view of coiled anchor 17204 with strain relief 17201 and coupler 17200a (not visible).

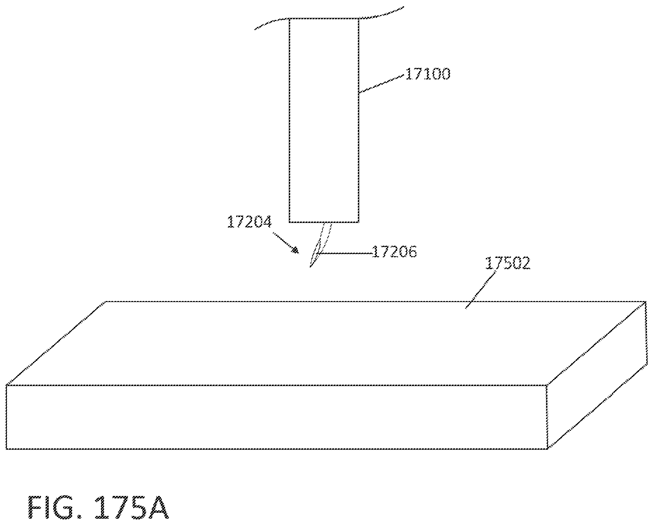

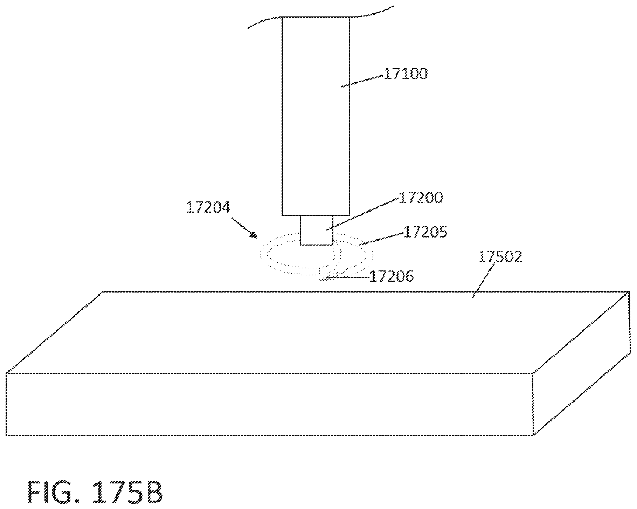

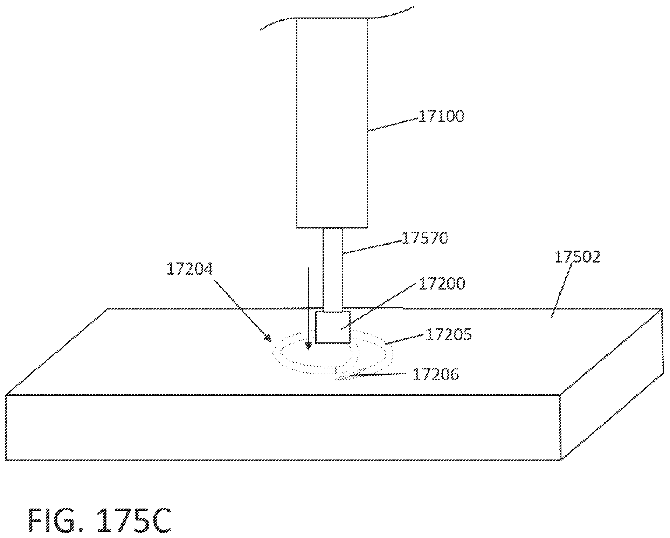

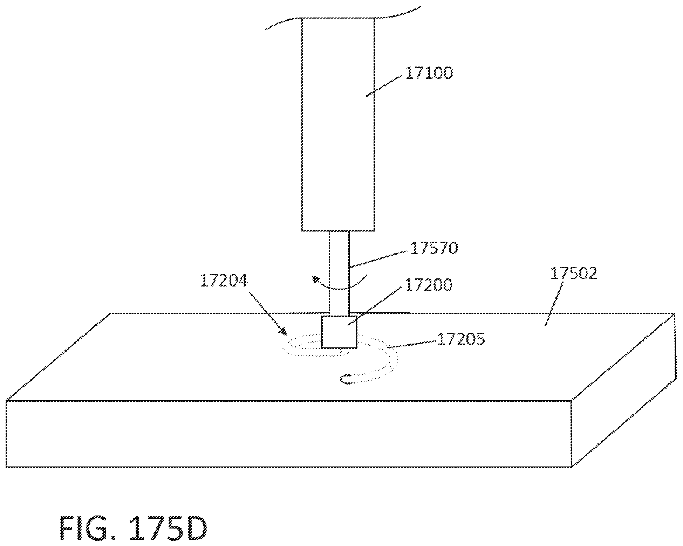

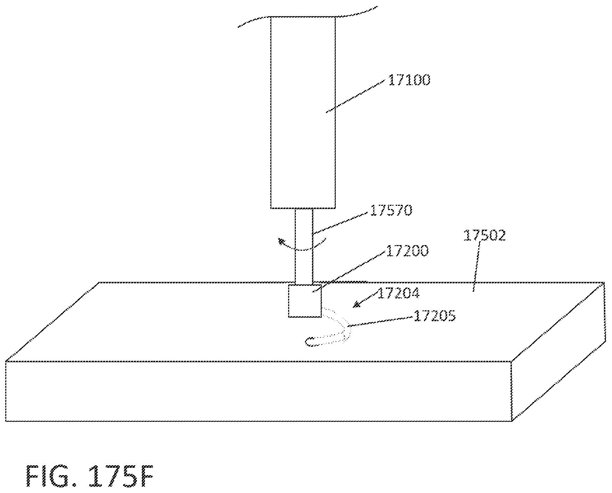

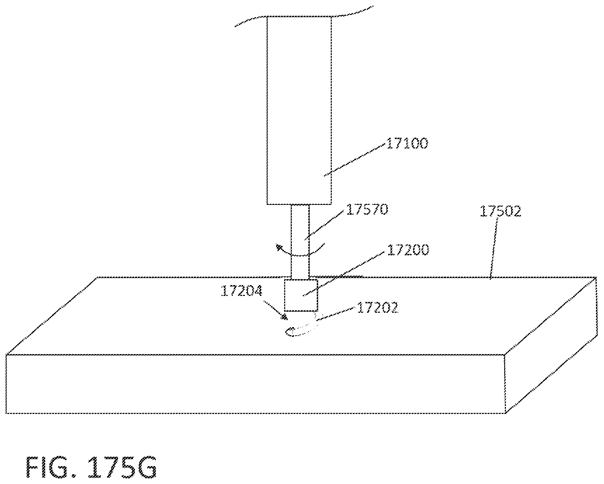

Figure 175A-H illustrate a deployment of an exemplary coiled anchor 17204 into a target tissue area 17502 with a delivery system 17100. The delivery system 17100 can have any number of catheters, as described above, an optional pusher, and/or an optional coupler. The remainder of the coiled anchor 17204 is straightened out or substantially straightened out inside the delivery system 17100. In Figure 175A, a deployment system 17100 is shown in a deployment position, with anchor tip 17206 of coiled anchor 17204 pushed out and exposed at the end of the deployment system 17100. Figure 175B shows coiled anchor 17204 and associated coupler 17200 after being completely pushed out of deployment system (or the deployment system retracted over the anchor). The anchor 17204 moves to its coiled condition due to its shape memory. The deployment system 17100 moves the coiled anchor into deployment position proximate to target tissue area 17502. Figure 175C shows the coiled anchor 17204 with a downward force being applied to position the coiled anchor 17204 at target tissue area 17502 by an optional pusher 17570 of the deployment system 17100, such as a rod, coil, or wire. Figure 175D illustrates deployment of coiled anchor 17204 into target tissue area 17502. As the deployment device 17100 is rotated, anchor tip 17206 penetrates tissue and coiled anchor 17204 advances into the tissue.

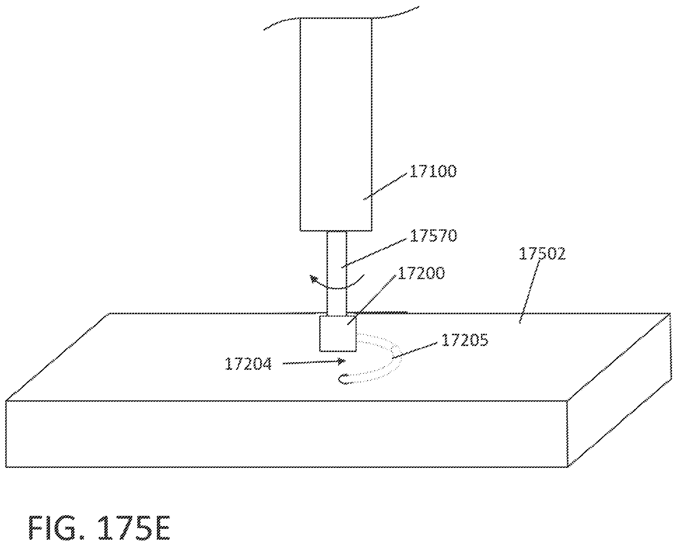

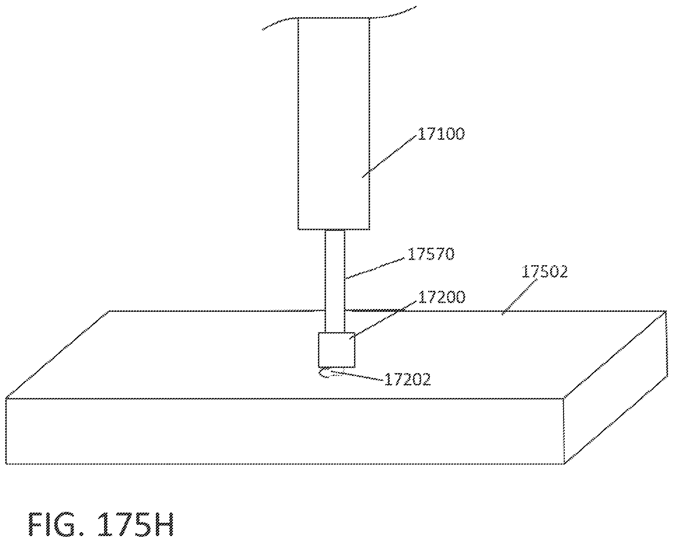

Figures 175E-H show further deployment of coiled anchor 17204 into target tissue area 17502. Each of Figures 175E-H show further rotation of the anchor to further advance the anchor into the tissue. As shown in Figure 175H, the coiled anchor 17204 is fully deployed into target tissue area 17502 such that only a portion of radially inwardly extending portion 17202 and coupler 17200 remain outside of the tissue. Once coiled anchor 17204 is fully deployed in tissue, the deployment device 17100 may be detached from coupler 17200 and removed from the body.

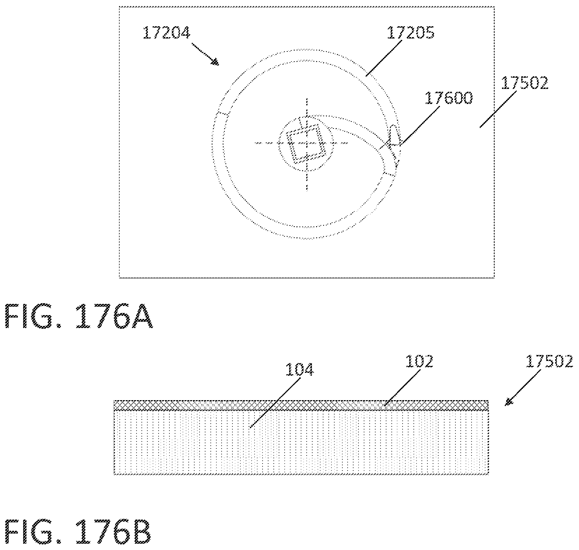

Figures 176A-176H illustrate how the coiled anchor 17204 penetrates the endocardium 102 and embeds in the myocardium 104. In Figure 176A, the coiled anchor 17204 is pushed against the target tissue area 17502 and rotated about the coupler 17200 to puncture the endocardium and create an opening 17600 through the endocardium. The coiled anchor 17204 can slide slightly under the endocardium 102 and engage the myocardium 104.

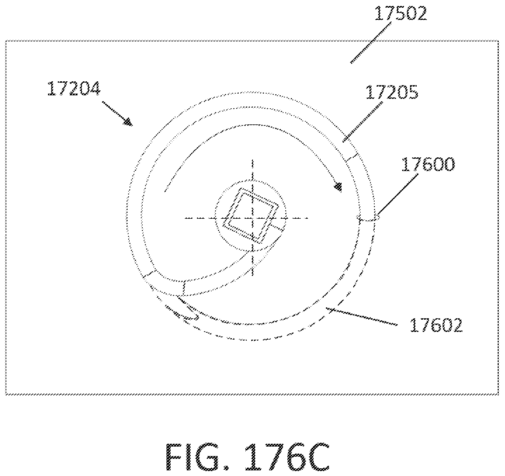

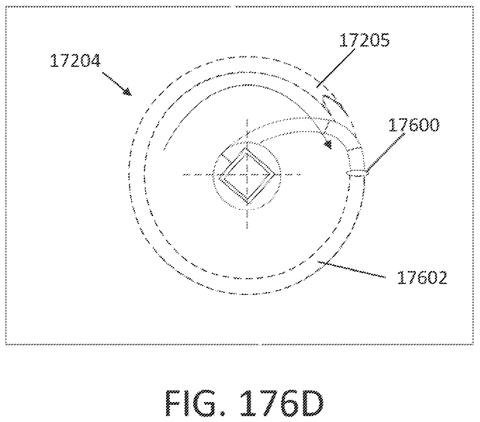

Referring to Figures 176C-176D, rotation of the coiled anchor 17204 about the coupler 17200 is continued. The ring portion 17205 advances through the opening 17600 and creates a path 17602 through the tissue of the myocardium 104. Referring to Figure 176D, the path 17602 and embedded ring portion 17205 is circular or substantially circular. As a result, myocardial tissue is trapped inside the ring portion 17205.

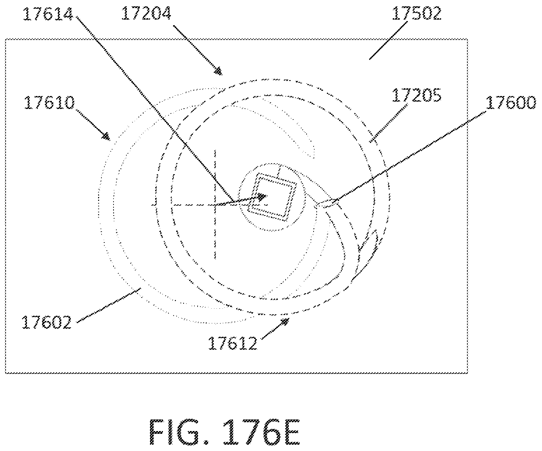

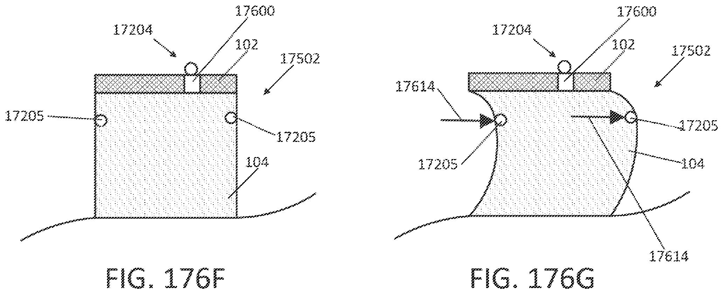

The tissue of the endocardium 102 is significantly stronger than the tissue of the tissue of the myocardium 104. For example, the tissue of the endocardium 102 can be four times as strong as the tissue of the myocardium 104. As a result, the position of the opening 17600 is fixed or is substantially fixed. In Figure 176E, the rotation of the coiled anchor 17204 about the coupler 17200 is continued. This rotation causes the radially inwardly extending portion 17202 to advance into the opening 17600. Since the position of the opening 17600 is essentially fixed, the movement of the radially inwardly extending portion 17202 through the opening translates the coupler 17200 and ring portion 17205 from the position 17610 to the position 17612 as indicated by arrow 17614. This translation pulls the tissue that is trapped in the ring portion 17205 to put the tissue into tension as illustrated by Figures 176F and 176G.

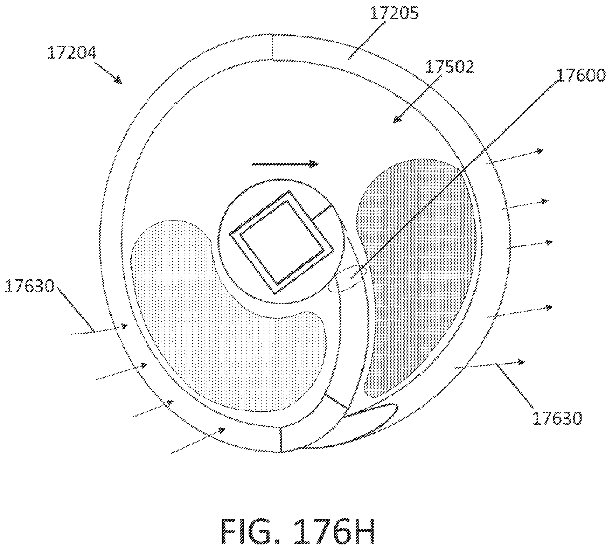

Referring to Figure 176H, the arrows 17630 represent the translation of the ring portion 17205 due to the movement of the radially inwardly extending portion 17202 through the opening 17600. The myocardial tissue portion 17650 between the radially inwardly extending portion 17202 and the ring portion 17205 is placed in tension. The myocardial tissue portion 17652 is compressed between the radially inwardly extending portion 17202 and the ring portion 17205, while the tissue portion 17652 is under tension due to the translation of the ring portion 17205. The translation of the ring portion 17205 illustrated by Figures 176E-176H can cause distortion of the ring portion 17205 and/or lock the ring portion in the tissue (i.e. prevent rotation of the ring portion in the direction opposite to the direction illustrated by Figures 176C-176E). The distortion of the ring portion can take a wide variety of different forms. For example, in Figure 176H a portion of the ring portion 17205 to the right of the radially inwardly extending portion 17202 and a portion of the ring portion 17205 to the left of the radially inwardly extending portion 17202 can both be compressed toward the radially inwardly extending portion 17202, a portion of the ring portion 17205 to the right of the radially inwardly extending portion 17202 and a portion of the ring portion 17205 to the left of the radially inwardly extending portion 17202 can both be stretched away from the radially inwardly extending portion 17202, a portion of the ring portion 17205 to the right of the radially inwardly extending portion 17202 can be compressed toward the radially inwardly extending portion 17202 while a portion of the ring portion 17205 to the left of the radially inwardly extending portion 17202 can be stretched away from the radially inwardly extending portion 17202, a portion of the ring portion 17205 to the left of the radially inwardly extending portion 17202 can be compressed toward the radially inwardly extending portion 17202 while a portion of the ring portion 17205 to the right of the radially inwardly extending portion 17202 can be stretched away from the radially inwardly extending portion 17202, or the ring portion 17205 can be undeformed.

In one exemplary embodiment, by varying the angle of radially inwardly extending portion 17202, the desired locking force can be achieved. In certain exemplary embodiments, the locking force is between 10-20 Newtons.

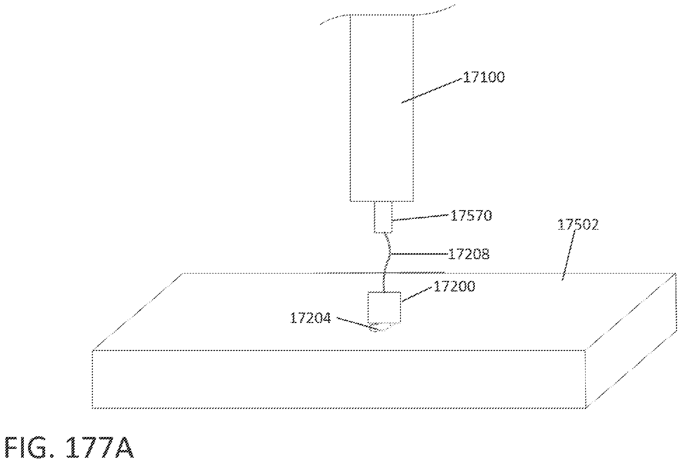

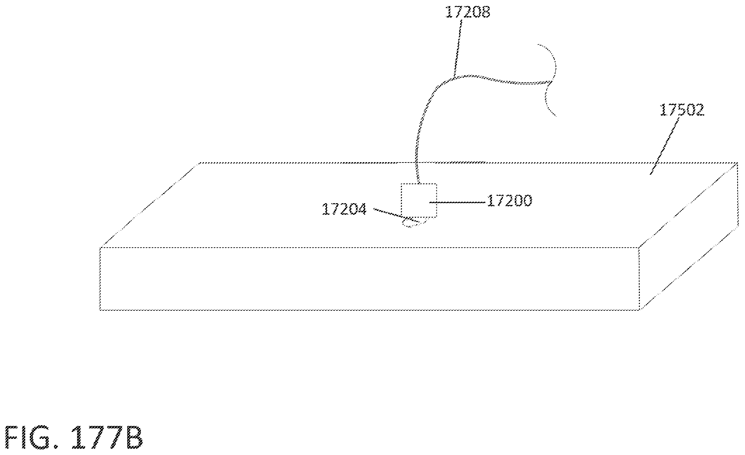

Figure 177A shows a fully deployed coiled anchor 17204 into target tissue area 17502. In some embodiments a tether 17208 may be connected to coiled anchor 17204. In some embodiments, tether 17208 is connected to coiled anchor 17204 via a surgical knot. In some embodiments, tether 17208 is disposed within coiled anchor 17204. In other embodiments, tether 17208 is attached to coupler 17200. Figure 177B shows a fully deployed coiled anchor 17204 with deployment system 17100 and deployment device 17100 removed and tether 17208 exposed.

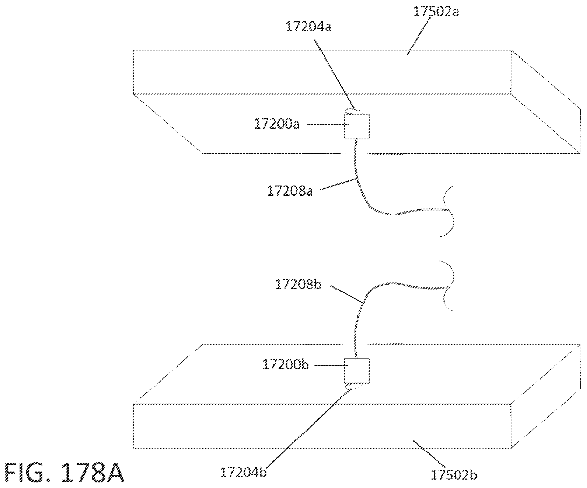

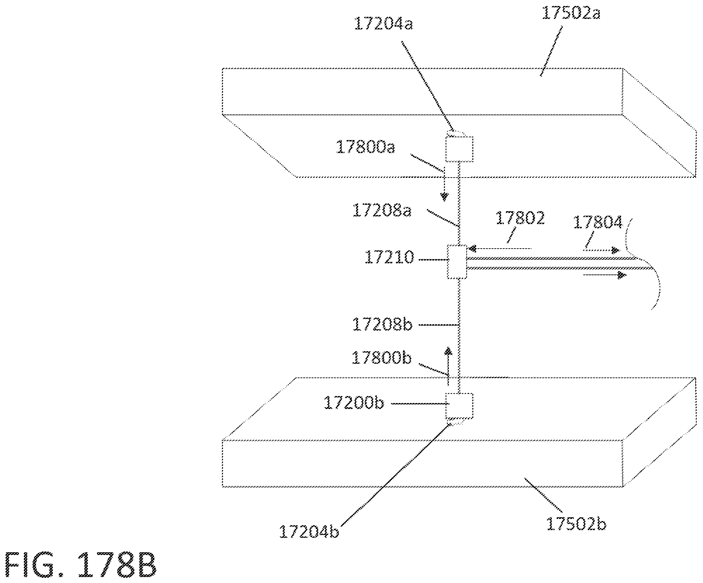

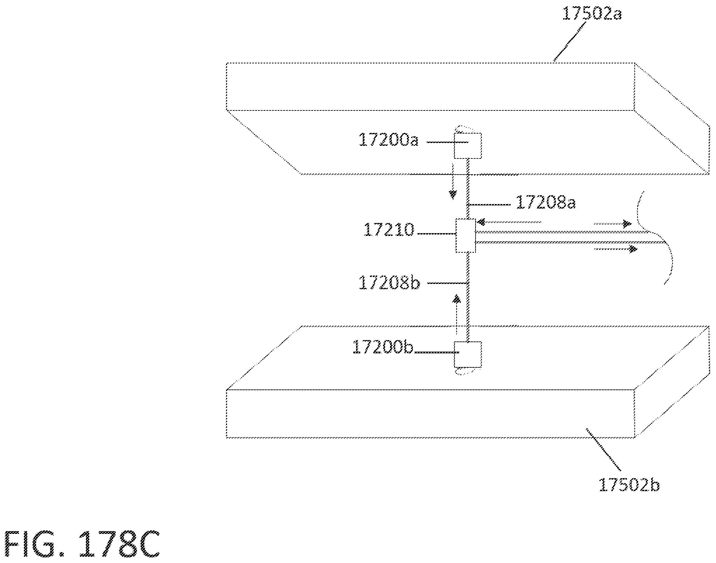

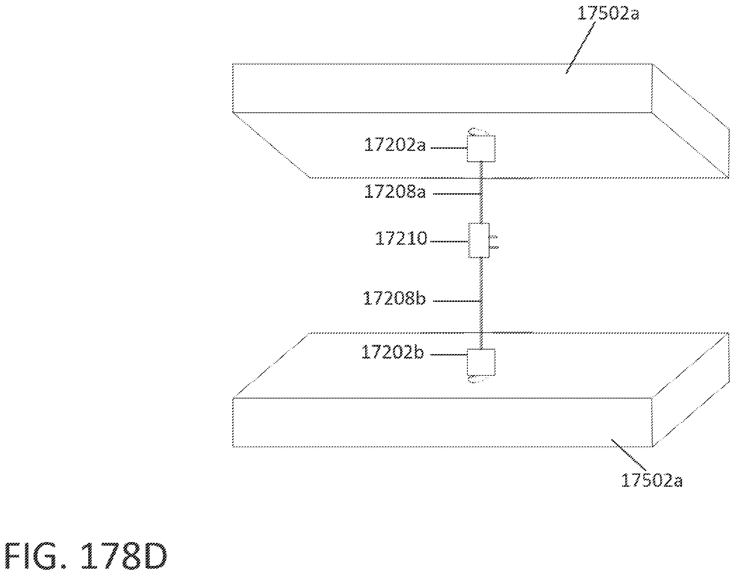

Figures 178A-D illustrate tissue remodeling using coiled anchor 17204. Figure 178A shows and exemplary coiled anchor 17204a deployed in target tissue area 17502a. Connected to coiled anchor 17204a is coupler 17200a and tether 17208a. Proximate to the coiled anchor 17204a deployment location is a second tissue area, target tissue area 17502b, where coiled anchor 17204b and associated coupler 17200b and tether 17208b are deployed. Figure 178B shows tether lock 17210 being advanced along tether 17208a and tether 17208b as indicated by arrow 17802. As tether 17208a and tether 17208b are pulled in the direction of arrow 17804 coiled anchors 17204a and 17204b are pulled toward one another, which manipulates the engaged tissue at target tissue areas 17502a and 17502b. Figure 178C shows tether lock being further advanced along tethers 17208a and 17208b. Once the desired tissue manipulation has been achieved, tether lock 17210 can be locked, which holds target tissue areas 17502a and 17502b in a remodeled position and the ends of tethers 17208a and 17208b are cut off as shown in Figure 178D. Tether lock 17210 can take a variety of forms. For example, any of the connectors 240 can be used as the tether lock 17210.

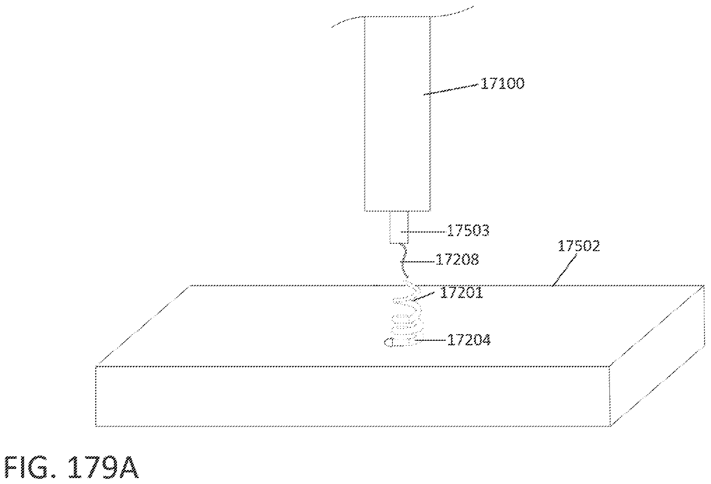

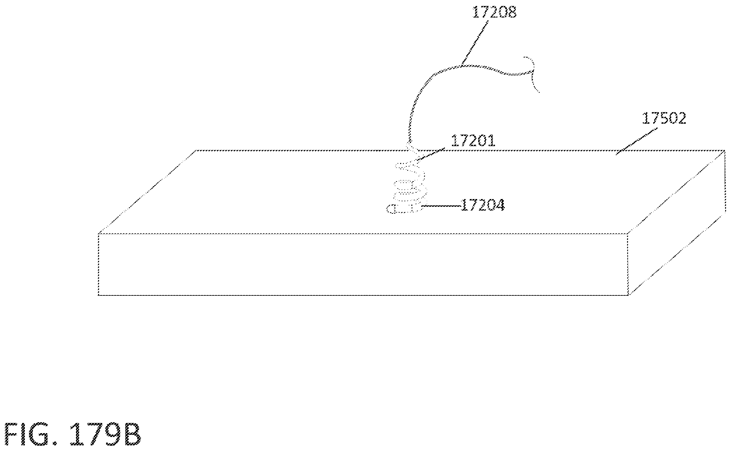

Figure 179A shows an exemplary coiled anchor 17204 with strain relief 17201 deployed into target tissue area 17502. In some embodiments a tether 17208 may be connected to strain relief 17201 of coiled anchor 17204. Figure 179B shows a fully deployed coiled anchor 17204 with strain relief 17201, deployment system 17100, and pusher 17570 removed. This leaves tether 17208 exposed.

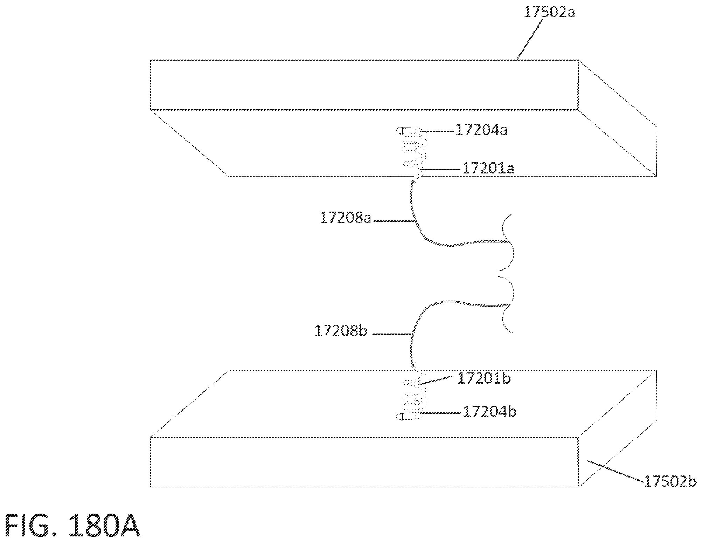

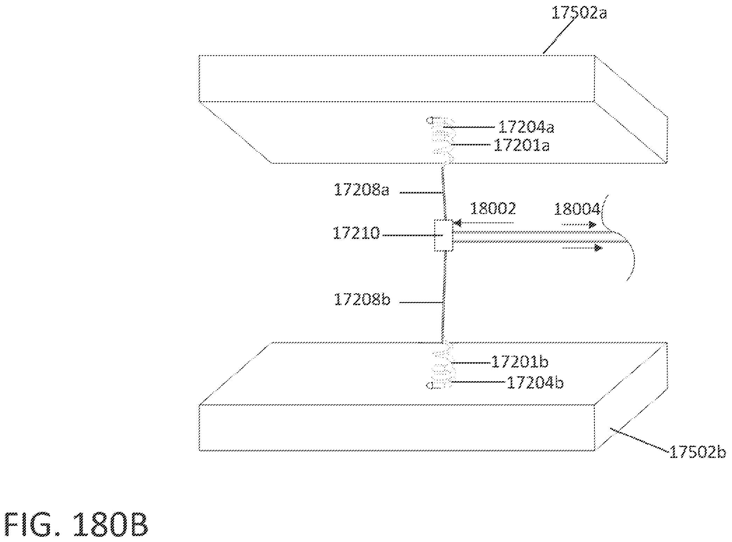

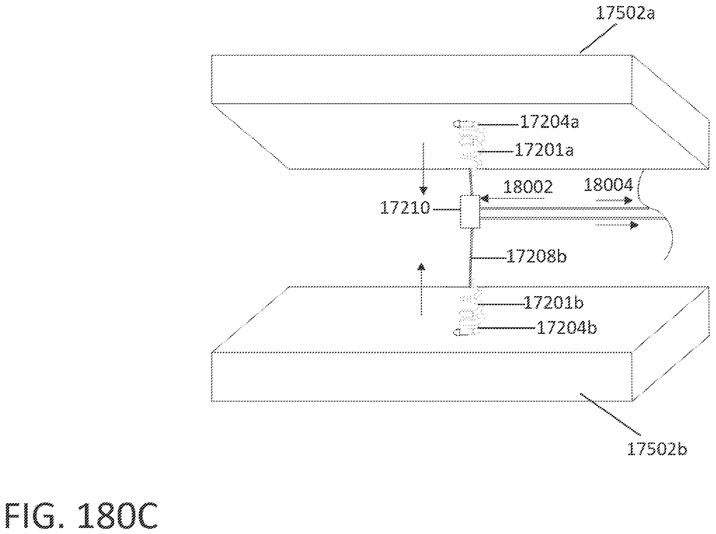

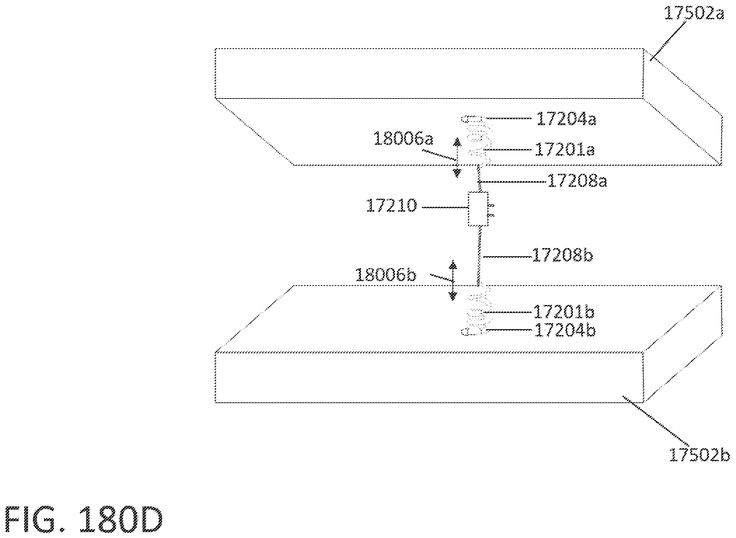

Figures 180A-D illustrate tissue remodeling using coiled anchor 17204 with strain relief 17201. Figure 180A shows and exemplary coiled anchor 17204a with strain relief 17201a deployed in target tissue area 17502a. Tether 17208a is attached to coiled anchor 17204a. Proximate to the coiled anchor 17204a deployment location is a second tissue area, target tissue area 17502b, where coiled anchor 17204b with strain relief 17201b and tether 17208b are deployed. Figure 180B shows tether lock 17210 being advanced along tether 17208a and tether 17208b as indicated by arrow 18002. As tether 17208a and tether 17208b are pulled in the direction of arrow 18004 coiled anchors 17204a and 17204b are pulled toward one another, which manipulates the engaged tissue at target tissue areas 17502a and 17502b. Figure 180C shows tether lock being further advanced along tethers 17208a and 17208b. Once the desired tissue manipulation has been achieved, tether lock 17210 can be locked into place which holds target tissue areas 17502a and 17502b in a remodeled position and the ends of tethers 17208a and 17208b are cut off. Figure 180D shows tether lock 17210 locked into place creating tension in the direction of arrow 18006a at strain relief 17201a and tension in the direction of arrow 18006b at strain relief 17201b. The strain reliefs 17201a, 17201b can limit and/or damp the load on coiled anchors 17204a and 17204b. For example, the strain reliefs 17201a, 17201b can stretch and contract as the tissue areas 17502a, 17502b move toward and away from one another. For example, the tissue areas 17502a, 17502b can be portions of heart walls that move toward and away from one another as the heart beats. As such, the strain reliefs 17201a, 17201b stretch and contract to control the load applied to the heart walls by the tethered anchors.

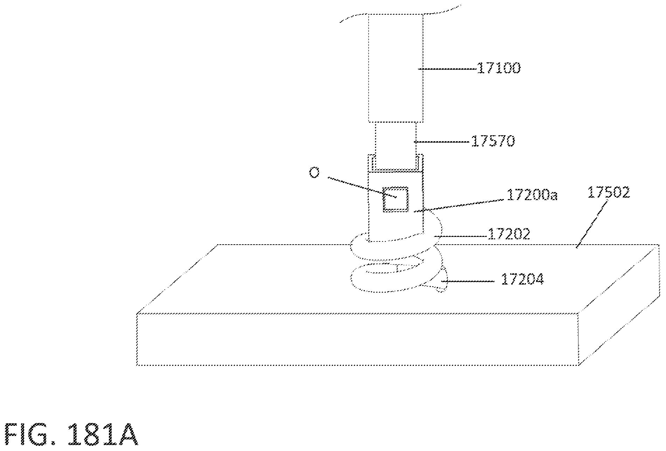

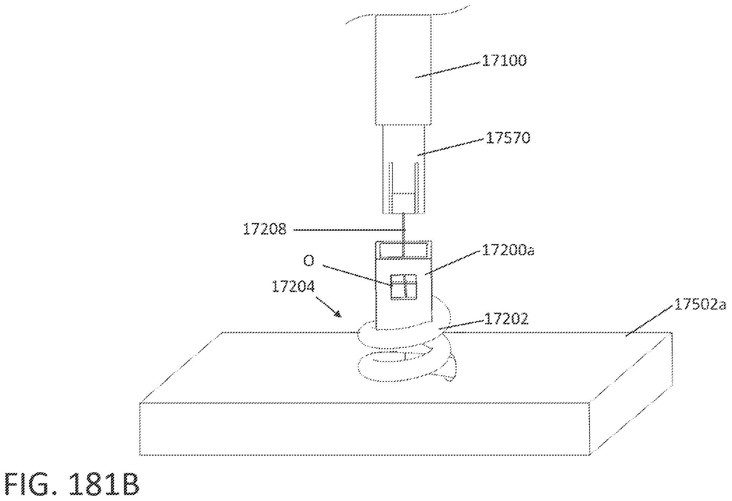

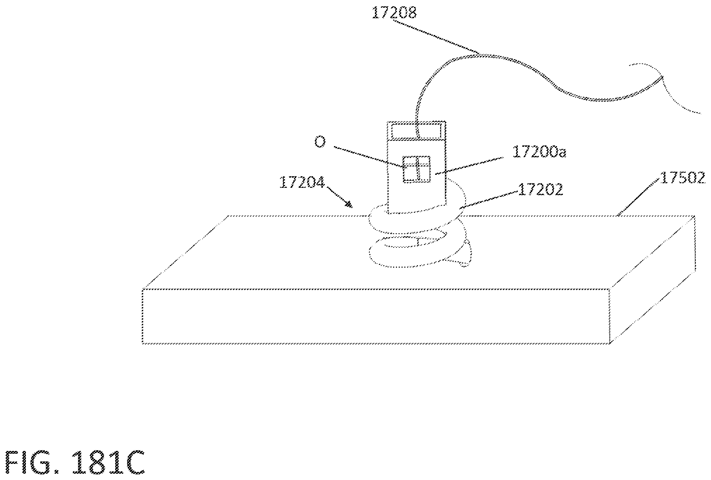

Figure 181A shows a deployment of coiled anchor 17204a and associated coupler 17200a into target tissue area 17502. Coupler 17200a has an opening O which is operably connected and disconnected to a pusher 17570 of deployment device 17100. Figure 181A shows coupler 17200a connected to deployment device 17100. Figure 181B shows coupler 17200b disconnected from deployment device 17100. Figure 181B further depicts tether 17208 disposed within coupler 17200a. Figure 181C shows the deployment device 17100 fully retracted and tether 17208 exposed.

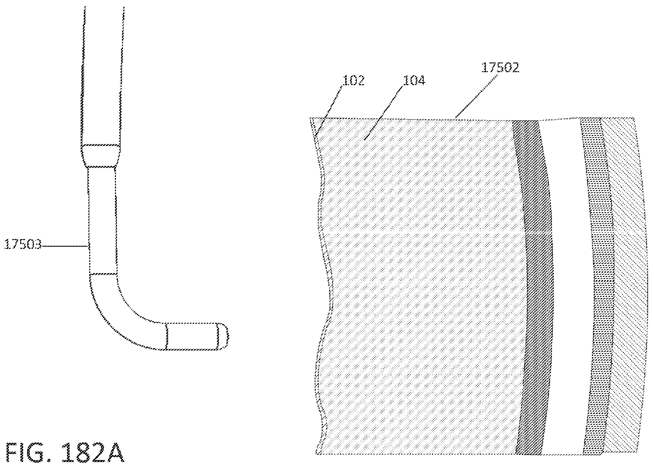

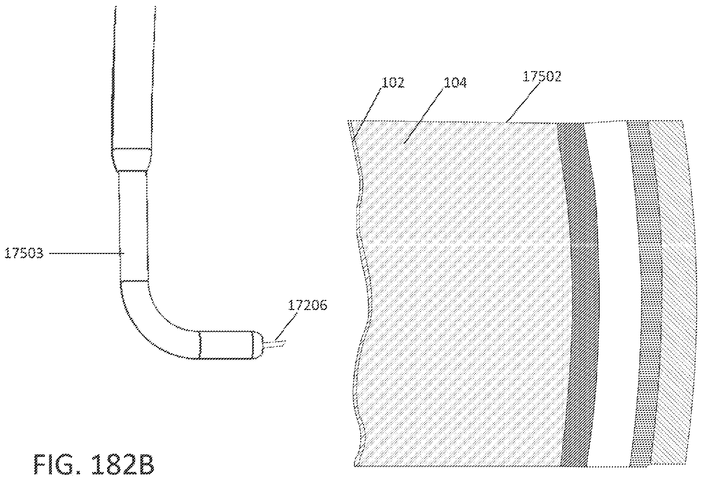

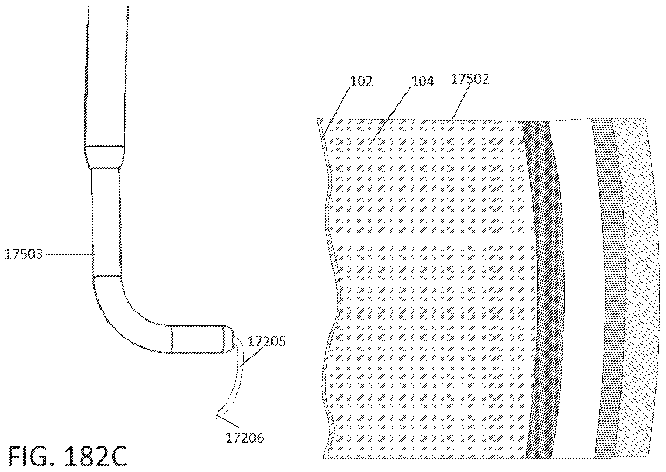

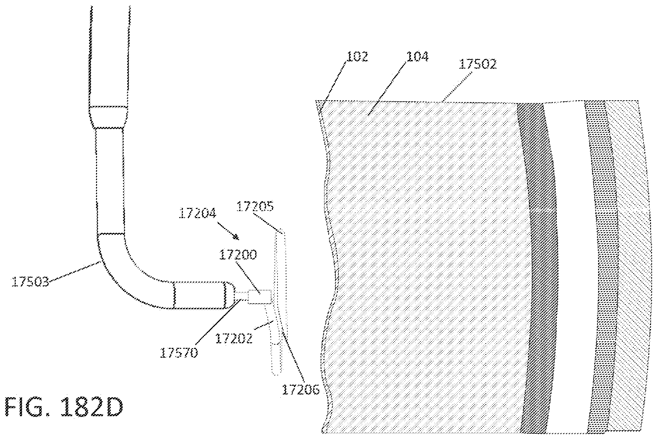

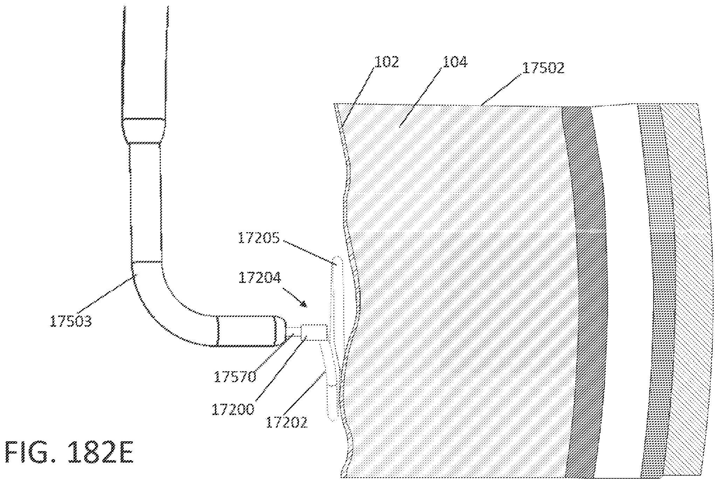

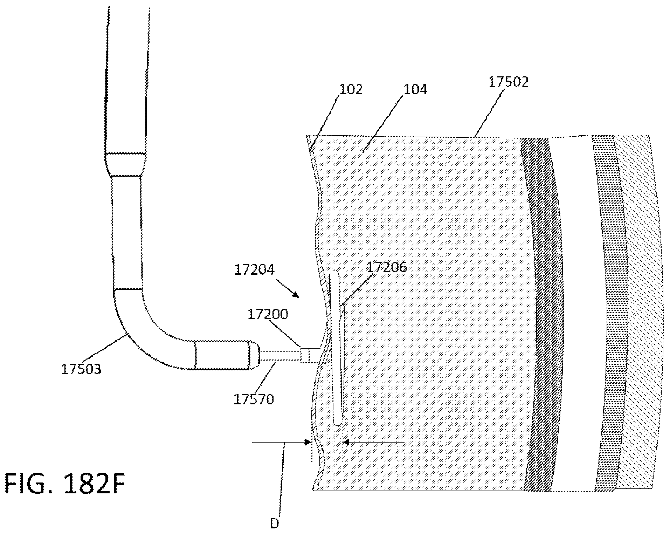

Figures 182A-H illustrate an exemplary deployment of coiled anchor 17204 into target tissue area 17502. Figure 182A shows a catheter 17503 positioned proximate to target tissue area 17502. Figure 182B shows anchor tip 17206 of coiled anchor 17204 protruding from catheter 17503. Figure 182C shows coiled anchor 17204 further protruded from catheter 17503. Figure 182D shows coiled anchor 17204 fully protruded from catheter 17503. Also shown is a pusher 17570 that extends from the catheter 17503 and is connected to coupler 17200. Figure 182E shows coiled anchor 17204 positioned for deployment at target tissue area 17502. Figure 182F shows coiled anchor 17204 deployed within target tissue area 17502 at deployment depth D. The depth D can determine how much endocardial tissue 102 and/or myocardial tissue 104 is engaged by the helical anchor 1310. The depth D is small or shallow to utilize the strength of endocardium. For example, the depth D can be configured to engage helical anchor 1310 into targeted endocardial tissue of the left ventricle while avoiding or substantially avoiding myocardial tissue that is much deeper than the endocardium. In some exemplary embodiments, depth D is between 0.02 mm and 2 mm, such as between 0.5 mm and 1.5 mm, such as between 0.75 mm and 1.25 mm, such as 1 mm or less.

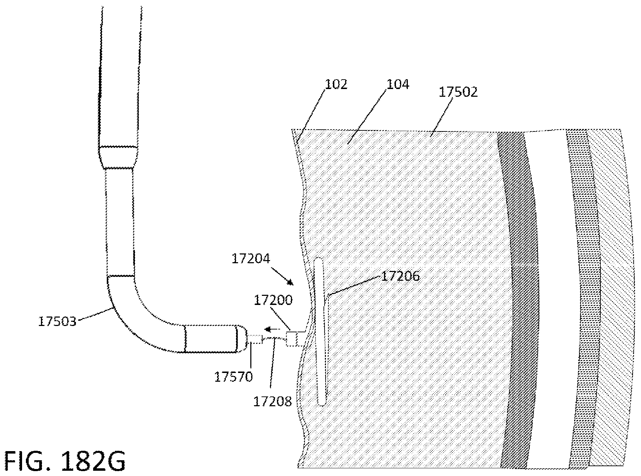

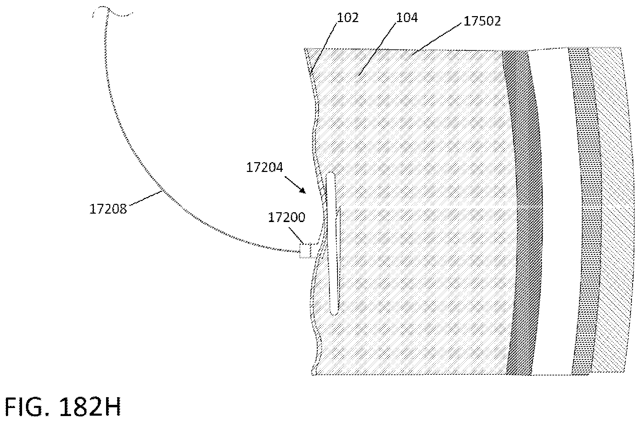

Figure 182G shows the detachment of deployment device 17100 from coupler 17200 and withdrawal of catheter 17503. As the deployment device 17100 is retracted, tether 17208 is exposed. Figure 182H shows coiled anchor 17204 fully deployed with tether 17208 exposed.

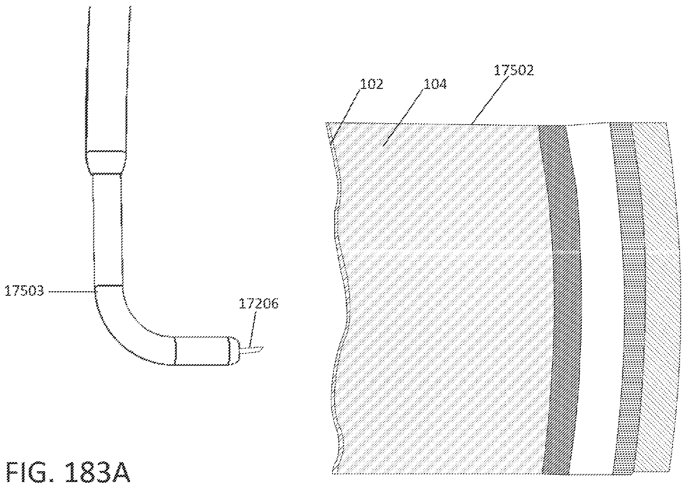

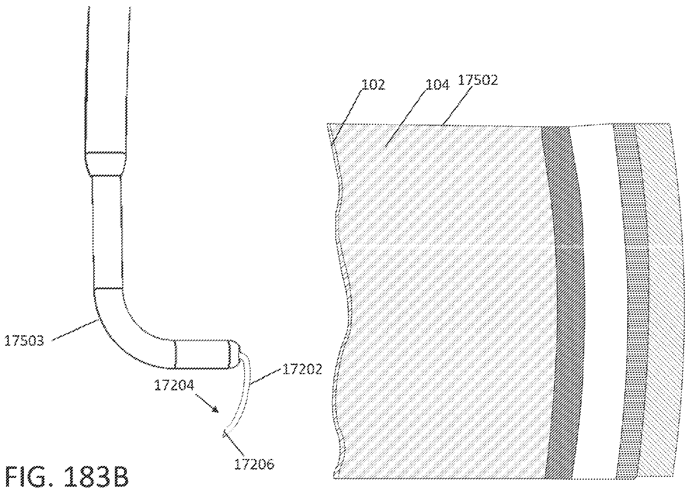

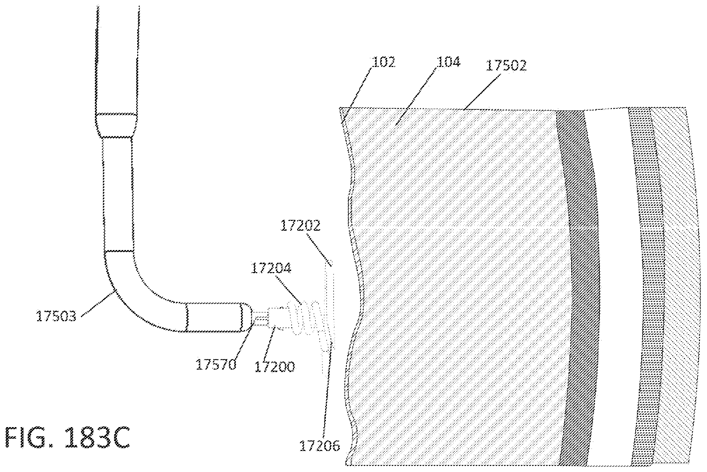

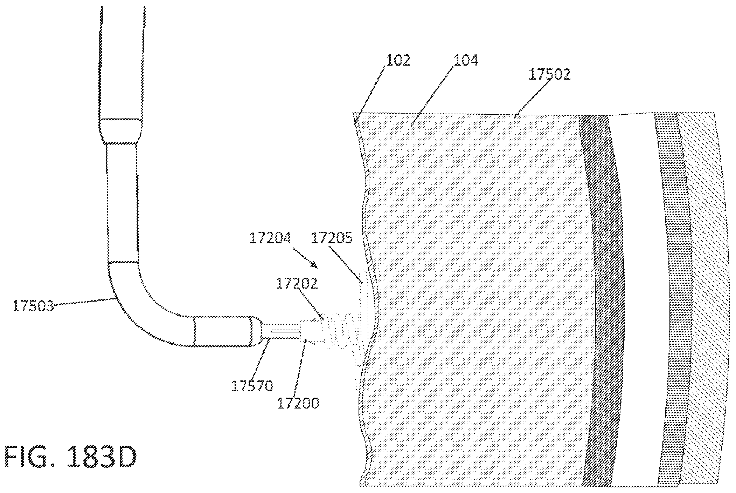

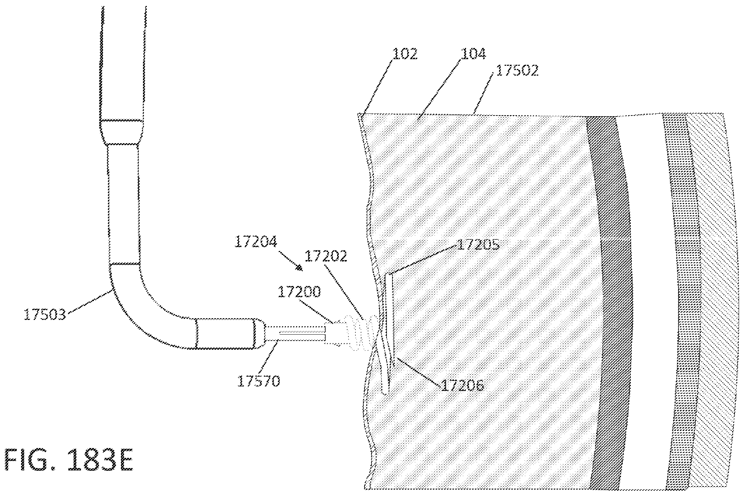

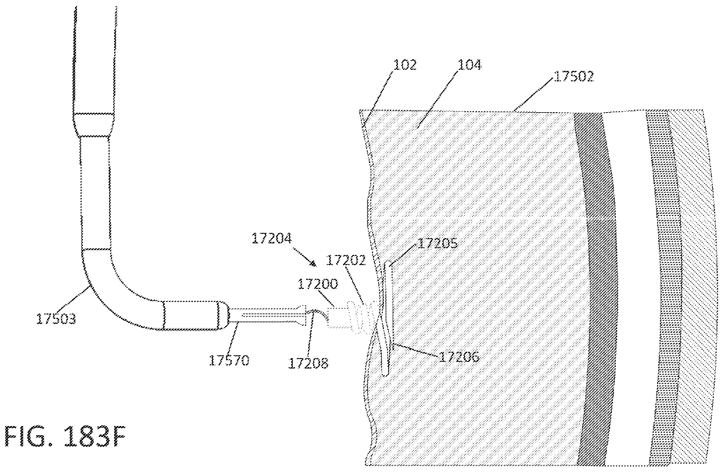

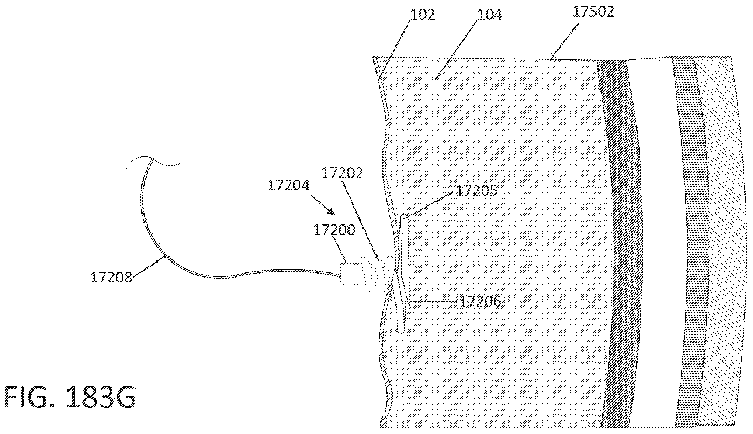

Figures 183A-G illustrate another exemplary deployment of coiled anchor 17204 into target tissue area 17502. Figure 183A shows a catheter 17503 positioned proximate to target tissue area 17502. Figure 183B shows anchor tip 17206 of coiled anchor 17204 protruding from catheter 17503. Figure 183C shows coiled anchor 17204 fully protruded from catheter 17503. Also shown is deployment device pusher 17570 that includes a coupler (See coupler of Figures 58-69 that can be used) which is connected to coupler 17200. Figure 183D shows coiled anchor 17204 positioned for deployment at target tissue area 17502. Figure 183E shows coiled anchor 17204 deployed within target tissue area 17502. Figure 183F shows the detachment of deployment device 17100 from coupler 17200 and withdrawal of catheter 17503. As the deployment device 17100 is retracted, tether 17208 is exposed. Figure 183G shows coiled anchor 17204 fully deployed with tether 17208 exposed.

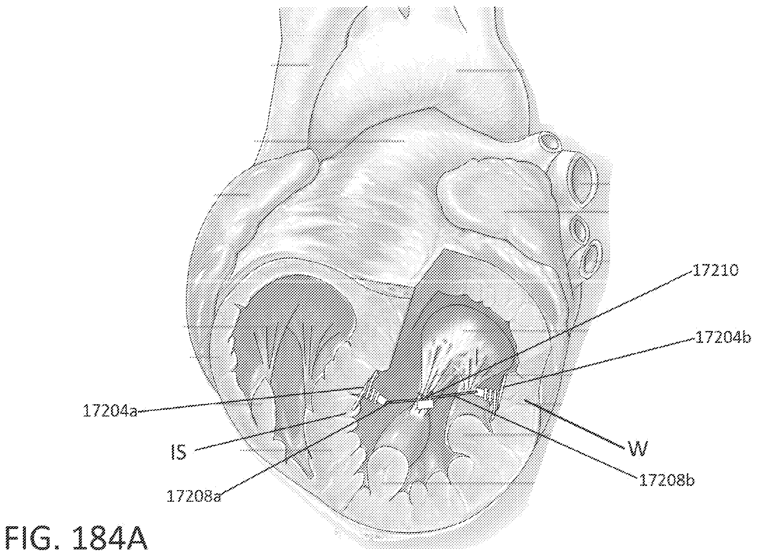

In Figure 184A, coiled anchor 17204b is attached to the heart wall W and a coiled anchor 17204a is implanted on the interventricular septum IS. The tethers 17208a and 17208b can be pulled inward to pull the heart wall W inward to remodel the shape of the heart wall W and the intraventricular septum IS. To hold the heart wall W and the intraventricular septum IS in the remodeled position, the tethers 17208a and 17208b, while in tension, can be connected within the left ventricle LV, such as for example, by the line tether lock 17210 or other suitable means for connecting the tethers. The strain reliefs can limit and/or damp the load on coiled anchors 17204a and 17204b. For example, the strain reliefs can stretch and contract as the heart walls move toward and away from one another as the heart beats. As such, the strain reliefs stretch and contract to control the load applied to the heart walls by the tethered anchors.

The coiled anchors 17204a and 17204b can be used in a wide variety of different ways to remodel the heart and/or approximate the papillary muscles. A tether lock 17210 and two or more coiled anchors can be deployed or more than one tether lock 17210 with two or more anchors per tether lock can be deployed to remodel the heart of a single patient. For example, any of the configurations disclosed herein can be used in combination on the heart of a single patient.

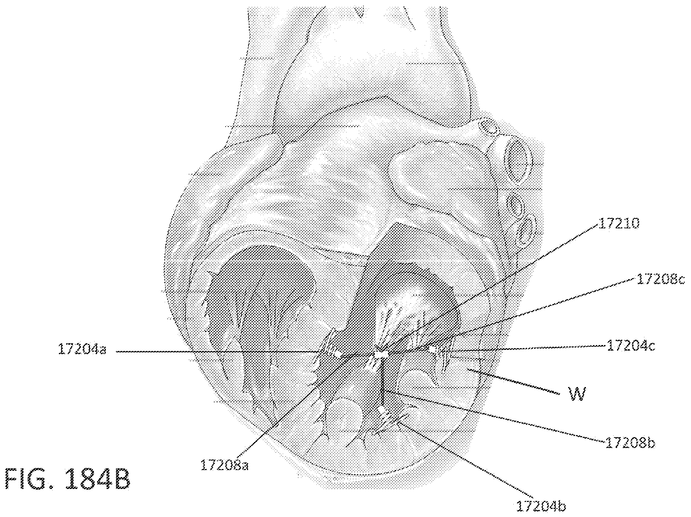

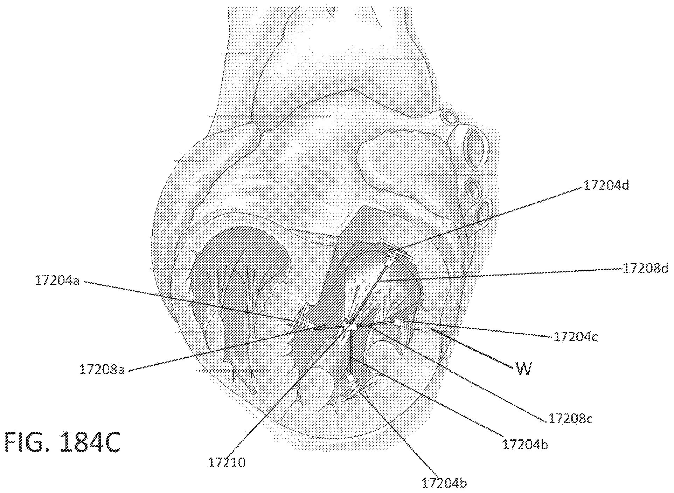

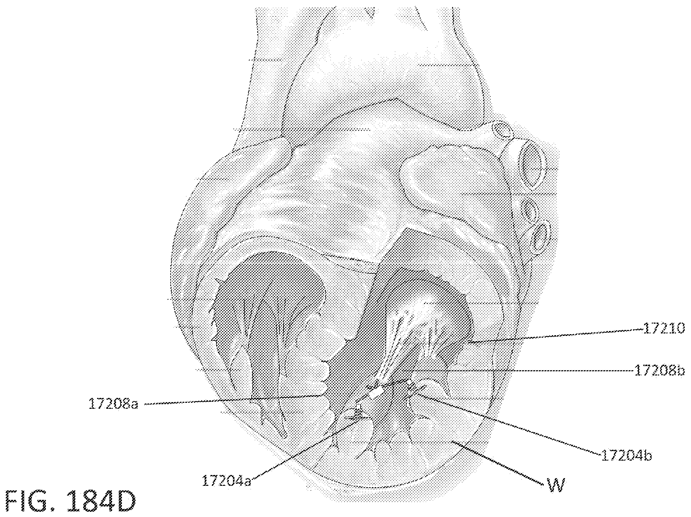

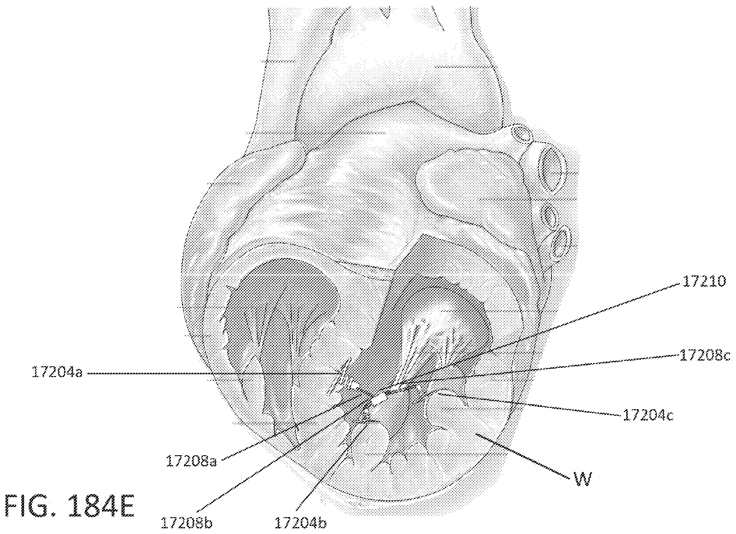

Figure 184B shows remodeling of the heart wall W and intraventricular septum IS using coiled anchors 17204b and 17204c deployed in heart wall W and coiled anchor 17204a deployed in intraventricular septum IS. Figure 184C shows remodeling of the heart wall W and intraventricular septum IS using coiled anchors 17204b-d deployed in heart wall W and coiled anchor 17204a deployed in intraventricular septum IS. Figure 184D shows remodeling of papillary muscles using coiled anchors 17204a and 17204b. Figure 184E shows remodeling of papillary muscles and intraventricular septum IS using coiled anchors 17204a and 17204b deployed within the papillary muscles and coiled anchor 17204a deployed in the intraventricular septum IS.

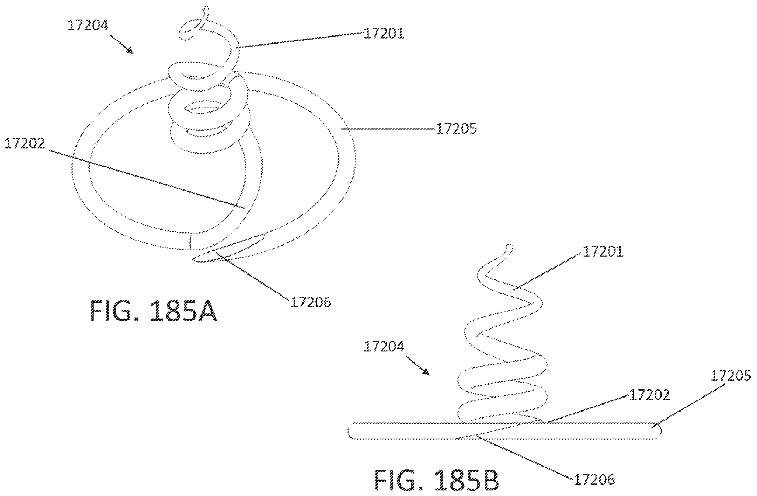

Figure 185A shows an exemplary coiled anchor 17204 with strain relief 17201. Figure 185B is a side view of the coiled anchor 17204 of Figure 185A. It is appreciated that in some embodiments, the outer coil or ring 17205 of coiled anchor 17204 is in a single plane. In one exemplary embodiment, radially inwardly portion 17202 or a majority of the radially inwardly extending portion is also in the same, single plane as the outer ring 17205. For example, the portion of the radially inwardly extending portion 17202 that extends from the ring 17205 is in the same plane as the ring 17205 and the radially inwardly extending portion 17202 can begin to incline or curve out of the plane at the strain relief 17201. In such embodiments, there is not incline in the transition between the outer coil of coiled anchor 17204 and radially inwardly extending portion 17202. This can prevent the coiled anchor 17204 from unwinding from its deployed position in tissue.

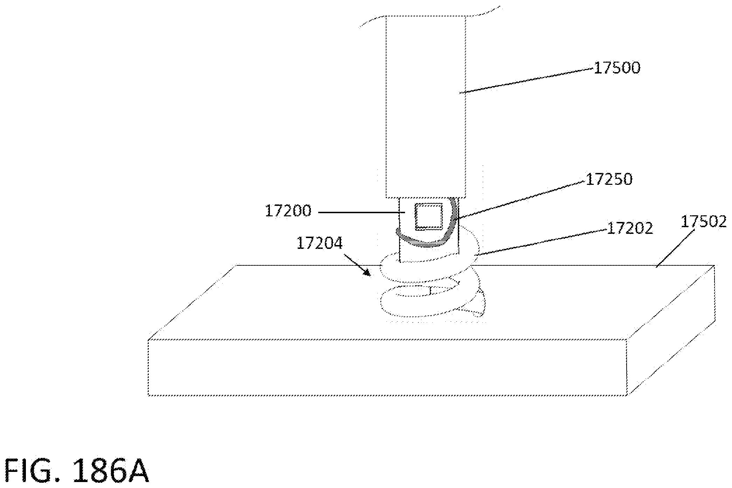

Figure 186A shows an exemplary coiled anchor 17204 deployed in target tissue area 17502. To prevent unwinding of the anchor, certain embodiments may include a locking device 17250. The locking device 17250 can take a wide variety of different forms. In one exemplary embodiment, the locking device 17520 is configured to frictionally engage a surface of the tissue 17502 to rotation of the anchor 17204 in a disengaging direction.

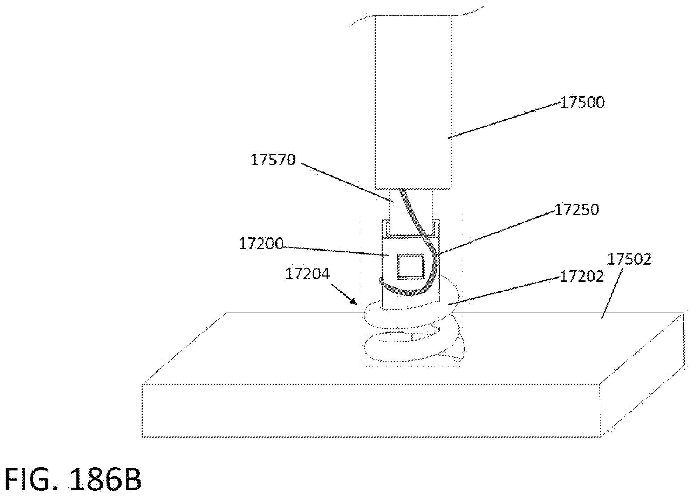

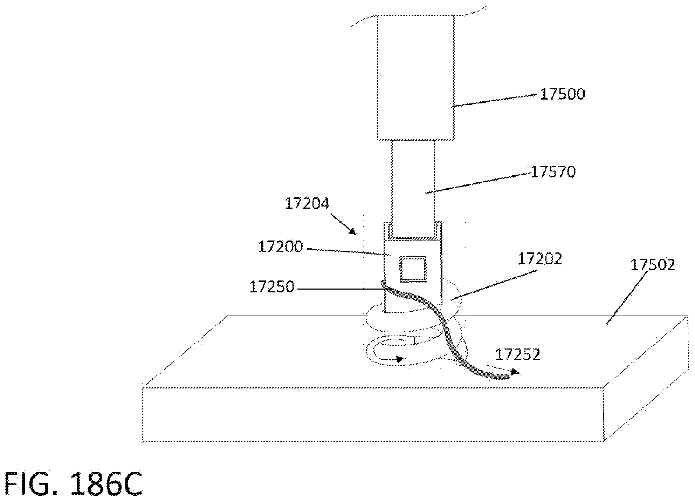

In the illustrated embodiment, the locking device 17250 has a "kickstand" configuration that extends from the coupler 17200 to the surface of the tissue 17502. The locking device 17250 is a shaped wire with an end portion oriented in the reverse direction of coiled anchor 17204. In certain embodiments, locking device 17250 is a smaller than the coiled anchor 17204. Figure 186B shows locking device 17250 being deployed from catheter 17503. Figure 186C shows locking device 17250 making contact with target tissue area 17502. Locking device 17250 is shape set to assume the position illustrated by Figure 186C and apply a frictional force in the direction of arrow 17252. This frictional force locks the anchor 17204 in the tissue 17502.

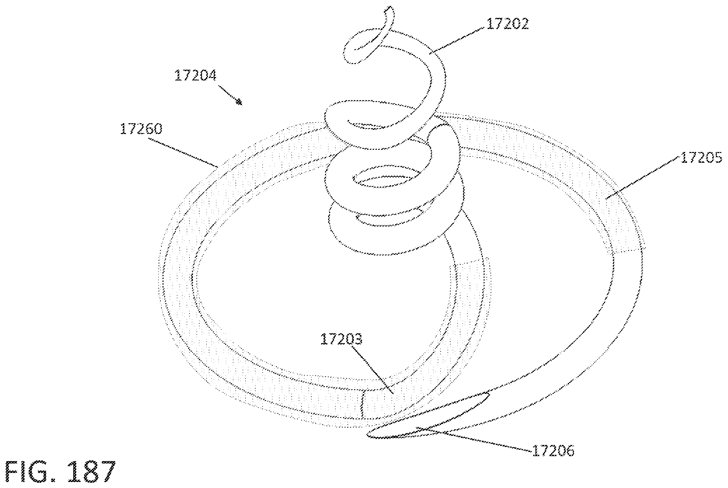

Figure 187A shows exemplary coiled anchor 17204 with a cloth 17260. The cloth can take a wide variety of different forms. The cloth can be textured to enhance friction. The cloth 17260 can be a sleeve disposed over the ring portion 17205 as illustrated or can be otherwise attached to the ring 17205.

As coiled anchor 17204 is deployed into tissue the cloth 17260 bunches up around radially inwardly extending portion 17202 and/or strain relief 17201 as shown in Figure 17. The bunched-up cloth 17260 pinches against the tissue. This bunching up and/or pinching of the cloth 17260 locks the coiled anchor in place or increases the torque required to unwind the anchor.

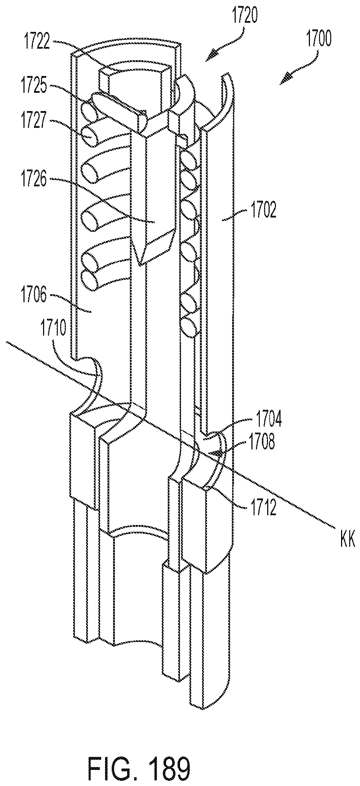

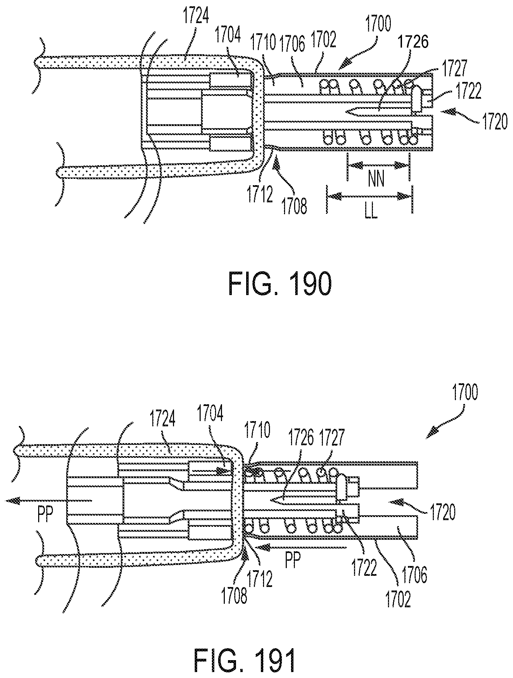

Referring to Figures 189-194, an exemplary embodiment of a trimming tool is provided. The trimming tool 1700 can be used to trim a line 1724 (Figure 190). With reference to Figure 190, the line 1724 can take a wide variety of different forms. Examples of lines include, but are not limited to, sutures, wires, cables, chords, bendable rods, any combination thereof, etc. The line 1724 can be similar in all material aspects to the lines 124 and 224 (Figure 71A). The line 1724 can be any element or combination of elements that is configured to extend from the anchor 122, through the heart wall W, and into the internal chamber (Figures 9-17).

Figure 189 illustrates a perspective cross sectional view of the trimming tool 1700. The trimming tool 1700 can take a wide variety of different forms. In an exemplary embodiment, the trimming tool 1700 includes an outer sleeve 1702 and an inner housing 1720. The outer sleeve 1702 includes a backstop 1704 disposed along the inner wall 1706 of the outer sleeve 1702. The backstop 1704 can be manufactured as a part of the outer sleeve 1702 or as a separate component. The outer sleeve 1702 includes a channel 1708. The channel 1708 can be disposed between a first end 1710 of the outer sleeve 1702 and a second end 1712 of the outer sleeve 1702. As shown in Figure 189, the first end 1710 and the second end 1712 are arranged along the plane KK and are opposite from each other about the outer sleeve 1702. The channel 1708, as illustrated in Figure 189, is circular, but the channel 1708 can be triangular, rectangular, or any other desirable shape.

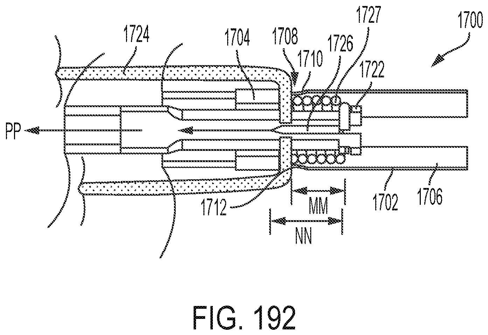

The inner housing 1720 includes an inner shaft 1722, a compressible member 1727 and a cutter 1726. The compressible member 1727 can take a wide variety of different forms and include a spring, sponge, or other compressible or elastic material. The compressible member 1727 can be coupled to the inner shaft 1722 and/or the cutter 1726. As shown in Figure 189, the compressible member 1727 is a spring and is disposed between the inner shaft 1722 and the inner wall 1706 of the outer sleeve 1702. In an exemplary embodiment, the compressible member 1727 is coupled to the inner shaft 1722 at a distal end 1725 of the compressible member 1727. The compressible member 1727 has an expanded length LL (see Figure 190) and a compressed length MM (see Figure 192).

The cutter 1726 is coupled with the inner shaft 1722 and can take a wide variety of different forms. The cutter 1726 can include a blade, guillotine, knife, or other member capable of severing the line 1724. With reference to Figure 190, the cutter 1726 has a length NN that is between the expanded length LL of the compressible member 1727 and the compressed length MM of the compressible member 1727.

With reference to Figures 190-194, one or more lines 1724 can be threaded or otherwise placed through the channel 1708 of the trimming tool 1700. Specifically, the line 1724 can be inserted such that it enters the first end 1710 of the outer sleeve 1702 and exits the second end 1712 of the outer sleeve 1702. The line 1724 can be positioned such that it rests along or against the backstop 1704 of the outer sleeve 1702.

To trim the line 1724, the inner housing 1720 is advanced in a first direction PP (shown in Figure 191) relative to the outer sleeve 1702. To prevent the movement of the line 1724 immediately prior to or during the trimming of the line, the compressible member 1727 engages the line 1724 and presses the line 1724 against the backstop 1704. Stated another way, the compression of the compressible member 1727 on the line 1724 and the backstop 1704 holds the line 1724 in place and preserves the tension of the line 1724 prior to and during the subsequent trimming of the line 1724. This can result in a reduction in the amount of force that the cutter 1726 must put on the line 1724, resulting in a swifter and cleaner cut of the line 1724 as well as an elongated life of the cutter 1726.

As shown in Figure 192, the compressible member 1727 further compresses against the line 1724 and the backstop 1704. The compressible member 1727 can compress to a length from the expanded length LL up to, and including, the compressed length MM. The cutter 1726 advances in the first direction PP and through the line 1724, thereby trimming the line 1724. The inner shaft 1722 advances in the direction PP until the compressible member 1727 is fully compressed.

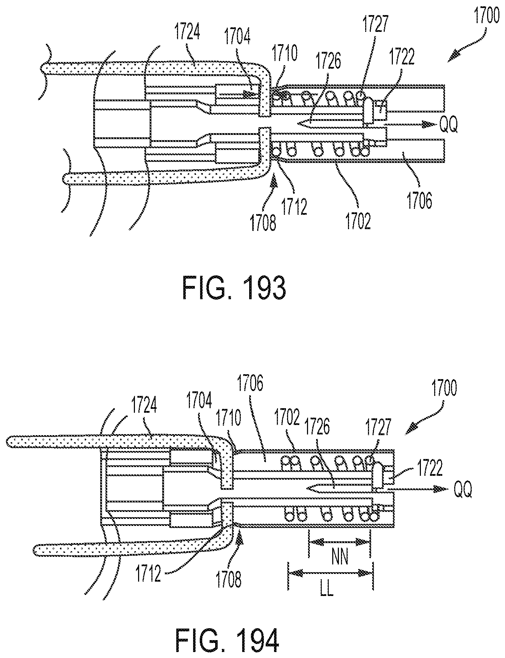

Referring to Figures 193-194, after the line 1724 is trimmed, the inner housing 1720 can travel in a second direction QQ, which is opposite of the first direction PP. The compressible member 1727 disengages the backstop 1704 and the line 1724 and expands to its expanded length LL.

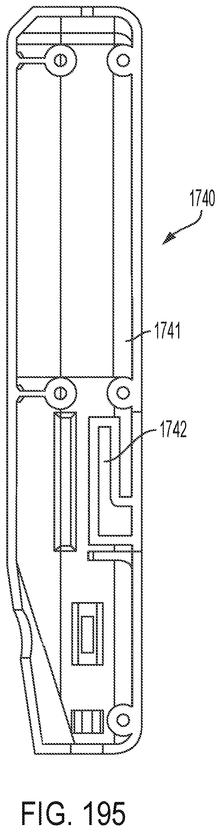

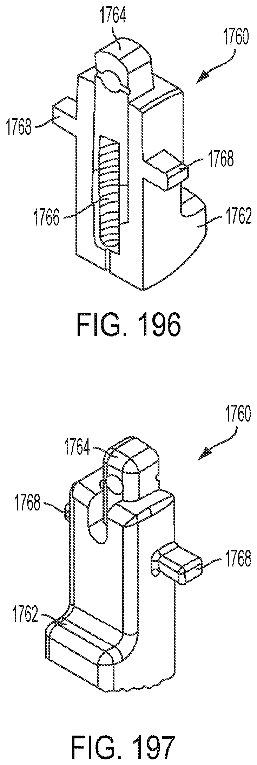

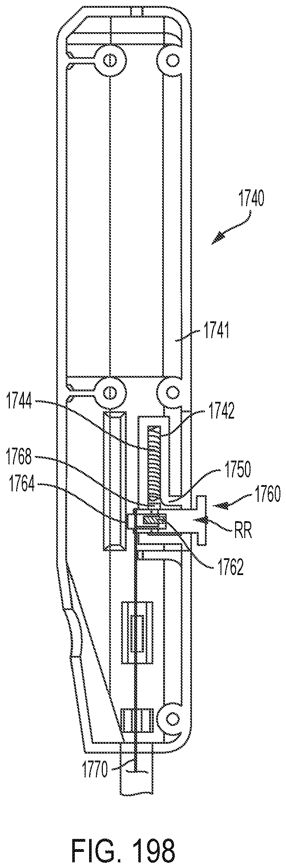

The trimming tool 1700 can be used in a variety of settings and can be activated by a variety of handles, triggers, and/or actuators. Referring to Figure 195, one such handle 1740 for the trimming tool 1700 is illustrated. The handle 1740 includes an outer frame 141 and a cavity 1742. In various embodiments, the cavity 1742 can house a spring 1744 (Figure 198). The outer frame 141 is coupled to a trigger 1760 (Figures 196-197).

As shown in Figures 196-197, the trigger 1760 includes an outer housing 1762 and an inner member 1764. The inner member 1764 can be compressed against a spring 1766, which biases against the inner member 1764. The outer housing 1762 also includes one or more protrusions 1768 extending from the outer housing 1762. As shown in Figures 196-197, two protrusions 1768 extend in opposite directions from the outer housing 1762.

Referring to Figures 198-199, handle 1740 is illustrated. The handle 1740 and trigger 1760 can utilize a multi-step motion to prevent accidental activation of the trimming tool 1700. In a non-activated state, the protrusions 1768 of the trigger 1760 are not in line with the cavity 1742, but rather abut inner wall 1750 of handle 140. This prevents the trigger 1760 from prematurely moving through the cavity 1742.

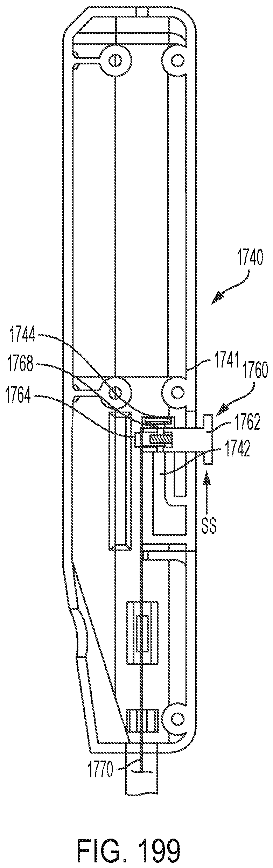

To activate the trimming tool 1700, the trigger 1760 is pushed in direction RR. When the trigger 1760 is pushed in direction RR, the inner member 1764 is pushed against the spring 1766 (Figure 196) to allow the trigger 1760 to be depressed in the first direction RR. The trigger 1760 is sufficiently depressed when the protrusions 1768 become in line with the cavity 1742. The spring 1744 is expanded against the trigger 1760 and/or the protrusions 1768 to prevent accidental or premature motion of the trigger 1760 in the cavity 1742. As illustrated in Figure 199, to deploy the trimming tool 1700 to trim the line 1724, the trigger 1760 is then pressed in a second direction SS through the cavity 1742 and against the spring 1744. The trigger 1760 activates the trimming tool 1700 via an actuator 1770. After the line 1724 is trimmed, the trigger 1760 can be released against the spring 1744 and travels back to its resting state.

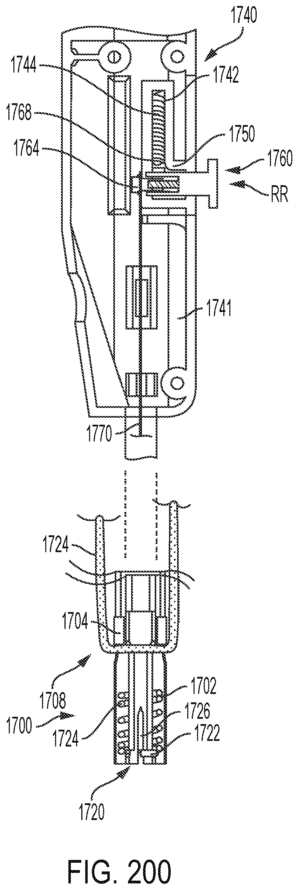

With reference to Figures 200-202, the trimming tool 1700, handle 1740, and trigger 1760 are shown in use. Referring to Figure 200, the trigger 1760 is pushed in the direction RR such that the inner member 1764 is pushed against the spring 1766 (see Figure 196). This exposes the protrusions 1768 to the cavity 1742. To prevent further unnecessary motion, the spring 1744 in the cavity 1742 biases against the protrusions 1768 until a second force in the direction SS is applied.

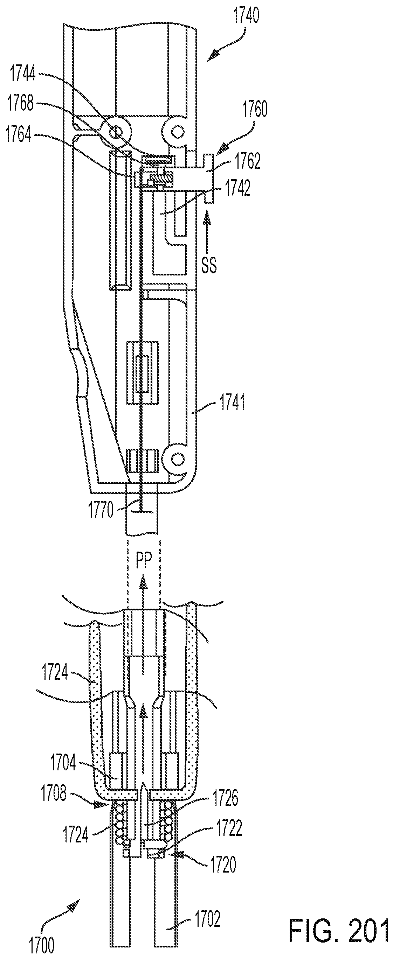

Referring to Figure 201, the trigger 1760 is pressed in a second direction SS through the cavity 1742 and against the spring 1744. The trigger 1760 activates the motion of the inner housing 1720, which is advanced in the direction PP. The compressible member 1727 engages the line 1724 and presses the line 1724 against the backstop 1704. The cutter 1726 also advances in the first direction PP. The cutter 1726 advances through the line 1724, thereby trimming the line 1724. The inner shaft 1722, cutter 1726, and compressible member 1727 advance in the direction PP until the compressible member 1727 is fully compressed.

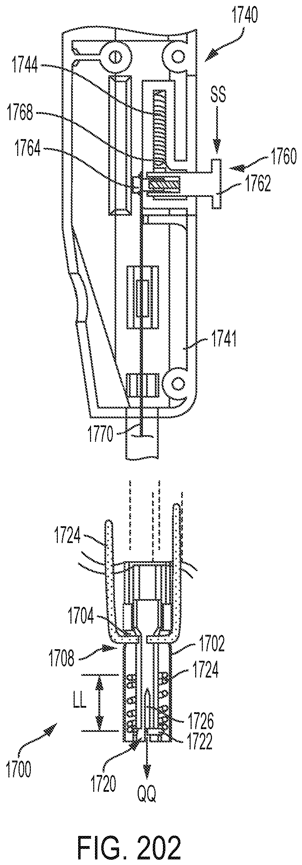

Referring to Figure 202, the trigger 1760 is released from the spring 1744 such that it travels back to its resting state. The inner housing 1720 of the trimming tool 1700 is released and travels in the second direction QQ. The compressible member 1727 disengages the backstop 1704 and the line 1724 and expands to its expanded length LL. The trimming tool 1700 can then be repositioned to trim a subsequent line 1724.

## Claims

1. A trimming tool (700; 1700) for trimming a line (124, 224; 1724) within a patient, comprising:
an actuator (1770) for manipulation by a user;
a moveable inner shaft (703; 1722) coupled to the actuator (1770);
a compressible member (1727) coupled to the moveable inner shaft (703; 1722);
a cutter (704; 1726) connected to the moveable inner shaft (703; 1722);
wherein the compressible member (1727) comprises an expanded length and a compressed length;
wherein the cutter (704; 1726) has a length between the expanded length and the compressed length of the compressible member (1727);
an outer sleeve (701; 1702) coupled to the inner shaft (703; 1722), the outer sleeve (701; 1702) comprising a backstop (1704) disposed along an inner wall (1706) of the outer sleeve (701; 1702), and a channel (1708) disposed between a first end (1710) and a second end (1712) of the outer sleeve (701; 1702); and
wherein the trimming tool (700; 1700) is configured to trim the line (124, 224; 1724) received inside of the channel (1708) of the outer sleeve (701; 1702).

2. The trimming tool (700; 1700) of claim 1, wherein the channel (1708) is circular in cross section.

3. The trimming tool (700; 1700) of any one of claims 1-2, wherein the compressible member (1727) comprises at least one of a spring (1744), sponge, or other elastic material.

4. The trimming tool (700; 1700) of any one of claims 1-3, wherein the compressible member (1727) and the cutter are coupled to the inner shaft (703; 1722).

5. The trimming tool (700; 1700) of any one of claims 1-4, wherein the compressible member (1727) is disposed between the inner shaft (703; 1722) and an inner wall (1706) of the outer sleeve (701; 1702).

6. The trimming tool (700; 1700) of any one of claims 1-5, wherein the compressible member (1727) is coupled to the inner shaft (703; 1722) at a distal end of the compressible member (1727).

7. The trimming tool (700; 1700) of any one of claims 1-6, wherein the cutter (704; 1726) comprises a blade, guillotine, or knife.

8. The trimming tool (700; 1700) of any one of claims 1-7, wherein the compressible member (1727) engages the line (124, 224; 1724) and presses the line (124, 224; 1724) against the backstop (1704) prior to the cutter (704; 1726) advancing through the line (124, 224; 1724).

9. The trimming tool (700; 1700) of claim 8, wherein when the compressible member (1727) presses the line (124, 224; 1724) against the backstop (1704), the compressible member (1727) shortens to the compressed length.

10. The trimming tool of claim 9, wherein when the compressible member (1727) shortens to the compressed length, the cutter (704; 1726) advances through the line (124, 224; 1724).

11. An assembly for trimming a line (124, 224; 1724) within a patient, comprising:
the trimming tool (700; 1700) of any one of claims 1-10;
a handle (1740) coupled to the trimming tool (700; 1700), the handle (1740) comprising:
an outer frame (1741),
a cavity (1742) extending partially into the outer frame (1741), wherein the cavity (1742) houses a first spring, and
a trigger (1760) coupled with the outer frame (1741) and moveable within the cavity (1742), wherein the trigger (1760) comprises an outer housing (1762), an inner member (1764), and a second spring coupled with the outer housing (1762) and the inner member (1764).

12. The assembly of claim 11, wherein the outer housing (1762) comprises one or more protrusions (1768) extending from the outer housing (1762).

13. The assembly of claim 11 or 12, wherein the trigger (1760) is moveable in a first direction and a second direction.

14. The assembly of any one claims 11 to 13, wherein the trigger (1760) must be moved in the first direction prior to being moved in the second direction or moving the trigger (1760) in the second direction initiates the movement of the trimming tool (700; 1700).

15. The assembly of any one of claims 11 to 14, wherein moving the trigger (1760) in the second direction comprises moving the trigger (1760) in the cavity (1742).

## Patentansprüche

1. Trimmwerkzeug (700; 1700) zum Trimmen einer Leine (124, 224; 1724) innerhalb eines Patienten, umfassend:
einen Aktuator (1770) für die Manipulation durch einen Benutzer;
einen beweglichen inneren Schaft (703; 1722), der mit dem Aktuator (1770) gekoppelt ist;
ein komprimierbares Element (1727), das mit dem beweglichen inneren Schaft (703; 1722) gekoppelt ist;
eine Schneidevorrichtung (704; 1726), die mit dem beweglichen inneren Schaft (703; 1722) verbunden ist;
wobei das komprimierbare Element (1727) eine expandierte Länge und eine komprimierte Länge aufweist;
wobei die Schneidevorrichtung (704; 1726) eine Länge zwischen der expandierten Länge und der komprimierten Länge des komprimierbaren Elements (1727) aufweist;
eine äußere Hülle (701; 1702), die mit dem inneren Schaft (703; 1722) gekoppelt ist, wobei die äußere Hülle (701; 1702) einen Anschlag (1704) umfasst, der entlang einer inneren Wand (1706) der äußeren Hülle (701; 1702) angeordnet ist, und einen Kanal (1708), der zwischen einem ersten Ende (1710) und einem zweiten Ende (1712) der äußeren Hülle (701; 1702) angeordnet ist; und
wobei das Trimmwerkzeug (700; 1700) dazu konfiguriert ist, die innerhalb des Kanals (1708) der äußeren Hülle (701; 1702) aufgenommene Leine (124, 224; 1724) zu trimmen.

2. Trimmwerkzeug (700; 1700) nach Anspruch 1, wobei der Kanal (1708) im Querschnitt kreisförmig ist.

3. Trimmwerkzeug (700; 1700) nach einem der Ansprüche 1 bis 2, wobei das komprimierbare Element (1727) mindestens eines umfasst von einer Feder (1744), einem Schwamm oder einem anderen elastischen Material.

4. Trimmwerkzeug (700; 1700) nach einem der Ansprüche 1 bis 3, wobei das komprimierbare Element (1727) und die Schneidevorrichtung mit dem inneren Schaft (703; 1722) gekoppelt sind.

5. Trimmwerkzeug (700; 1700) nach einem der Ansprüche 1 bis 4, wobei das komprimierbare Element (1727) zwischen dem inneren Schaft (703; 1722) und einer Innenwand (1706) der äußeren Hülle (701; 1702) angeordnet ist.

6. Trimmwerkzeug (700; 1700) nach einem der Ansprüche 1 bis 5, wobei das komprimierbare Element (1727) mit dem inneren Schaft (703; 1722) an einem distalen Ende des komprimierbaren Elements (1727) gekoppelt ist.

7. Trimmwerkzeug (700; 1700) nach einem der Ansprüche 1 bis 6, wobei die Schneidevorrichtung (704; 1726) eine Klinge, eine Guillotine oder ein Messer umfasst.

8. Trimmwerkzeug (700; 1700) nach einem der Ansprüche 1 bis 7, wobei das komprimierbare Element (1727) in Eingriff mit der Leine (124, 224; 1724) kommt und die Leine (124, 224; 1724) gegen den Anschlag (1704) drückt, bevor die Schneidevorrichtung (704; 1726) durch die Leine (124, 224; 1724) vorgeschoben wird.

9. Trimmwerkzeug (700; 1700) nach Anspruch 8, wobei dann, wenn das komprimierbare Element (1727) die Leine (124, 224; 1724) gegen den Anschlag (1704) drückt, das komprimierbare Element (1727) sich auf die komprimierte Länge verkürzt.

10. Trimmwerkzeug nach Anspruch 9, wobei dann, wenn sich das komprimierbare Element (1727) auf die komprimierte Länge verkürzt, die Schneidevorrichtung (704; 1726) durch die Linie (124, 224; 1724) vorgeschoben wird.

11. Anordnung zum Trimmen einer Leine (124, 224; 1724) in einem Patienten, umfassend:
das Trimmwerkzeug (700; 1700) nach einem der Ansprüche 1 bis 10;
einen Griff (1740), der mit dem Trimmwerkzeug (700; 1700) gekoppelt ist, wobei der Griff (1740) umfasst:
einen äußeren Rahmen (1741),
einen Hohlraum (1742), der sich teilweise in den äußeren Rahmen (1741) erstreckt, wobei der Hohlraum (1742) eine erste Feder aufnimmt, und
einen Auslöser (1760), der mit dem äußeren Rahmen (1741) gekoppelt ist und innerhalb des Hohlraums (1742) beweglich ist, wobei der Auslöser (1760) ein äußeres Gehäuse (1762), ein inneres Element (1764) und eine zweite Feder umfasst, die mit dem äußeren Gehäuse (1762) und dem inneren Element (1764) gekoppelt ist.

12. Anordnung nach Anspruch 11, wobei das äußere Gehäuse (1762) einen oder mehrere Vorsprünge (1768) umfasst, die sich von dem äußeren Gehäuse (1762) aus erstrecken.

13. Anordnung nach Anspruch 11 oder 12, wobei der Auslöser (1760) in einer ersten Richtung und in einer zweiten Richtung beweglich ist.

14. Anordnung nach einem der Ansprüche 11 bis 13, wobei der Auslöser (1760) in die erste Richtung bewegt werden muss, bevor er in die zweite Richtung bewegt wird, oder das Bewegen des Auslösers (1760) in die zweite Richtung leitet die Bewegung des Trimmwerkzeugs (700; 1700) ein.

15. Anordnung nach einem der Ansprüche 11 bis 14, wobei das Bewegen des Auslösers (1760) in die zweite Richtung das Bewegen des Auslösers (1760) in den Hohlraum (1742) umfasst.

## Revendications

1. Outil de coupe (700 ; 1700) pour la coupe d'une ligne (124, 224 ; 1724) à l'intérieur d'un patient, comprenant :
un actionneur (1770) destiné à être manipulé par un utilisateur ;
une tige interne (703 ; 1722) mobile accouplée à l'actionneur (1770) ;
un élément compressible (1727) accouplé à la tige interne (703 ; 1722) mobile ;
un dispositif de coupe (704 ; 1726) relié à la tige interne (703 ; 1722) mobile ;
l'élément compressible (1727) comprenant une longueur étendue et une longueur comprimée ;
le dispositif de coupe (704 ; 1726) présentant une longueur entre la longueur étendue et la longueur comprimée de l'élément compressible (1727) ;
un manchon externe (701 ; 1702) accouplé à la tige interne (703 ; 1722), le manchon externe (701 ; 1702) comprenant une butée arrière (1704) disposée le long d'une paroi interne (1706) du manchon externe (701 ; 1702) et un canal (1708) disposé entre une première extrémité (1710) et une seconde extrémité (1712) du manchon externe (701 ; 1702) ; et
l'outil de coupe (700 ; 1700) étant conçu pour couper la ligne (124, 224 ; 1724) logée à l'intérieur du canal (1708) du manchon externe (701 ; 1702).

2. Outil de coupe (700 ; 1700) selon la revendication 1, dans lequel le canal (1708) est circulaire en coupe transversale.

3. Outil de coupe (700 ; 1700) selon l'une quelconque des revendications 1 à 2, dans lequel l'élément compressible (1727) comprend au moins un élément parmi un ressort (1744), une éponge ou un autre matériau élastique.

4. Outil de coupe (700 ; 1700) selon l'une quelconque des revendications 1 à 3, dans lequel l'élément compressible (1727) et le dispositif de coupe sont accouplés à la tige interne (703 ; 1722).

5. Outil de coupe (700 ; 1700) selon l'une quelconque des revendications 1 à 4, dans lequel l'élément compressible (1727) est disposé entre la tige interne (703 ; 1722) et une paroi interne (1706) du manchon externe (701 ; 1702).

6. Outil de coupe (700 ; 1700) selon l'une quelconque des revendications 1 à 5, dans lequel l'élément compressible (1727) est accouplé à la tige interne (703 ; 1722) au niveau d'une extrémité distale de l'élément compressible (1727).

7. Outil de coupe (700 ; 1700) selon l'une quelconque des revendications 1 à 6, dans lequel le dispositif de coupe (704 ; 1726) comprend une lame, une guillotine ou un couteau.

8. Outil de coupe (700 ; 1700) selon l'une quelconque des revendications 1 à 7, dans lequel l'élément compressible (1727) vient en prise avec la ligne (124, 224 ; 1724) et presse la ligne (124, 224 ; 1724) contre la butée arrière (1704) avant que le dispositif de coupe (704 ; 1726) n'avance à travers la ligne (124, 224 ; 1724).

9. Outil de coupe (700 ; 1700) selon la revendication 8, dans lequel, lorsque l'élément compressible (1727) presse la ligne (124, 224 ; 1724) contre la butée arrière (1704), l'élément compressible (1727) se raccourcit à la longueur comprimée.

10. Outil de coupe selon la revendication 9, dans lequel, lorsque l'élément compressible (1727) se raccourcit à la longueur comprimée, le dispositif de coupe (704 ; 1726) avance à travers la ligne (124, 224 ; 1724).

11. Ensemble de coupe d'une ligne (124, 224 ; 1724) à l'intérieur d'un patient, comprenant :
l'outil de coupe (700 ; 1700) selon l'une quelconque des revendications 1 à 10 ;
une poignée (1740) accouplée à l'outil de coupe (700 ; 1700), la poignée (1740) comprenant :
un cadre externe (1741),
une cavité (1742) s'étendant partiellement dans le cadre externe (1741), la cavité (1742) logeant un premier ressort et
un déclencheur (1760) accouplé au cadre externe (1741) et mobile à l'intérieur de la cavité (1742), le déclencheur (1760) comprenant un boîtier externe (1762), un élément interne (1764) et un second ressort accouplé au boîtier externe (1762) et à l'élément interne (1764).

12. Ensemble selon la revendication 11, dans lequel le boîtier externe (1762) comprend une ou plusieurs saillies (1768) s'étendant à partir du boîtier externe (1762).

13. Ensemble selon la revendication 11 ou 12, dans lequel le déclencheur (1760) est mobile dans un premier sens et un second sens.

14. Ensemble selon l'une quelconque des revendications 11 à 13, dans lequel le déclencheur (1760) doit être déplacé dans le premier sens avant d'être déplacé dans le second sens ou le déplacement du déclencheur (1760) dans le second sens initie le déplacement de l'outil de coupe (700 ; 1700).

15. Ensemble selon l'une quelconque des revendications 11 à 14, dans lequel le déplacement du déclencheur (1760) dans le second sens comprend le déplacement du déclencheur (1760) dans la cavité (1742).
